(19) Europäisches Patentamt
European Patent Office
Office européen des brevets

(11) EP 4 461 735 A1

(12) **EUROPEAN PATENT APPLICATION**
published in accordance with Art. 153(4) EPC

(43) Date of publication:
**13.11.2024 Bulletin 2024/46**

(21) Application number: **23737031.7**

(22) Date of filing: **04.01.2023**

(51) International Patent Classification (IPC):
***C07D 519/00*** *(2006.01)* ***C07D 487/04*** *(2006.01)*
***A61K 31/519*** *(2006.01)* ***A61K 47/55*** *(2017.01)*
***A61P 29/00*** *(2006.01)* ***A61P 35/00*** *(2006.01)*
***A61P 37/00*** *(2006.01)*

(52) Cooperative Patent Classification (CPC):
**A61K 31/519; A61K 47/55; A61P 29/00; A61P 35/00; A61P 37/00; C07D 487/04; C07D 519/00**

(86) International application number:
**PCT/CN2023/070367**

(87) International publication number:
**WO 2023/131167 (13.07.2023 Gazette 2023/28)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **04.01.2022 CN 202210000510**
**27.01.2022 CN 202210078765**
**14.04.2022 CN 202210389191**
**13.05.2022 CN 202210501720**
**12.08.2022 CN 202210962203**
**07.12.2022 CN 202211567270**

(71) Applicant: **Xizang Haisco Pharmaceutical Co., Ltd.**
**Lhoka, Tibet 856099 (CN)**

(72) Inventors:
- **ZHANG, Chen**
  **Shannan, Tibet 856000 (CN)**
- **LIAO, Yuting**
  **Shannan, Tibet 856000 (CN)**
- **ZHAO, Chenfei**
  **Shannan, Tibet 856000 (CN)**
- **YU, Yan**
  **Shannan, Tibet 856000 (CN)**
- **TANG, Pingming**
  **Shannan, Tibet 856000 (CN)**
- **MA, Junjie**
  **Shannan, Tibet 856000 (CN)**
- **CHEN, Xiaogang**
  **Shannan, Tibet 856000 (CN)**
- **YUAN, Shuai**
  **Shannan, Tibet 856000 (CN)**
- **CHENG, Xinfan**
  **Shannan, Tibet 856000 (CN)**
- **YE, Fei**
  **Shannan, Tibet 856000 (CN)**
- **LI, Yao**
  **Shannan, Tibet 856000 (CN)**
- **NI, Jia**
  **Shannan, Tibet 856000 (CN)**
- **YAN, Pangke**
  **Shannan, Tibet 856000 (CN)**

(74) Representative: **IP TRUST SERVICES**
**Europole - Le Grenat**
**3, avenue Doyen Louis Weil**
**38000 Grenoble (FR)**

# (54) COMPOUND FOR INHIBITING AND DEGRADING IRAK4, AND PHARMACEUTICAL COMPOSITION AND PHARMACEUTICAL APPLICATION THEREOF

(57) The present invention relates to a compound represented by general formula (I) or a stereoisomer, deuterated compound, solvate, prodrug, metabolite, pharmaceutically acceptable salt or eutectic crystal thereof, and an intermediate thereof, and use thereof in IRAK4-related diseases such as an autoimmune disease, an inflammatory disease or cancer. B-L-K (I)



Processed by Luminess, 75001 PARIS (FR)

## Description

### Technical Field

The present invention relates to a compound represented by general formula (I) or a stereoisomer, deuterated compound, solvate, prodrug, metabolite, pharmaceutically acceptable salt or eutectic crystal thereof, and an intermediate thereof and a preparation method therefor, and use thereof in IRAK4-related diseases such as cancer or an autoimmune system disease.

### Background Art

Kinases catalyze the phosphorylation of proteins, lipids, sugars, nucleosides, and other cellular metabolites and play key roles in various aspects of physiology in eukaryotic cells. In particular, protein kinases and lipid kinases are involved in controlling the activated signal events in cells for growth, differentiation and survival in response to extracellular mediators or stimuli such as growth factors, cytokines or chemokines. In general, protein kinases are divided into two classes, one that preferentially phosphorylates tyrosine residues and the other that preferentially phosphorylates serine and/or threonine residues.

Interleukin-1 receptor kinase 4 (IRAK4) is a serine/threonine-specific protein kinase that belongs to the tyrosine-like kinase (TLK) family and is a key factor in the innate immune response involving interleukin-1, interleukin-18, and interleukin-33 receptors and Toll-like receptors. After extracellular signal molecules bind to interleukin receptors or Toll-like receptors, they are recruited to form a MyD88:IRAK4:IRAKI/2 multiprotein complex, leading to the phosphorylation of IRAK1/2 and mediating a series of downstream signalings, thereby activating the p38, JNK and NF-kB signaling pathways, and ultimately leading to the expression of pro-inflammatory cytokines. Clinical pathological studies have shown that individuals with IRAK4 mutations are protected against chronic lung disease and inflammatory bowel disease. IRAK4 deficiency itself is not lethal; individuals survive to adulthood and their risk of infection decreases with age. Therefore, IRAK4 has become an important therapeutic target and has attracted widespread research and development interest.

PROTAC (proteolysis targeting chimera) molecules are a class of bifunctional compounds that can simultaneously bind targeting proteins and E3 ubiquitin ligases. Such compounds can be recognized by proteasomes of cells, causing the degradation of targeting proteins, and can effectively reduce the content of targeting proteins in the cells. By introducing a ligand capable of binding to various targeting proteins into the PROTAC molecules, it is possible to apply the PROTAC technology to the treatment of various diseases, and this technology has attracted extensive attention in recent years.

Therefore, it is necessary to develop a novel IRAK4 inhibitor and a PROTAC drug of E3 ubiquitin ligase for the treatment of IRAK4-related diseases.

### Summary of the Invention

An objective of the present invention is to provide a compound with a novel structure, good efficacy, high bioavailability and higher safety that can inhibit or degrade IRAK4, for use in the treatment of IRAK4-related diseases such as an autoimmune disease, an inflammatory disease or cancer.

The present invention provides a compound or a stereoisomer, deuterated compound, solvate, prodrug, metabolite, pharmaceutically acceptable salt or eutectic crystal thereof, wherein the compound is a compound represented by general formula (I),

B-L-K (I);

In certain embodiments, B is selected from

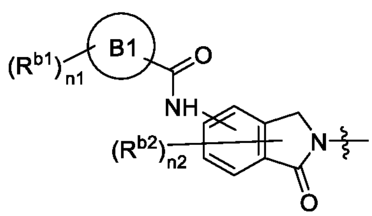

or

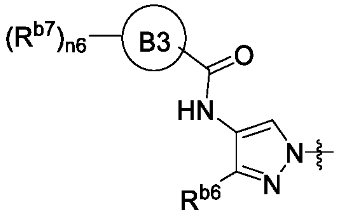

;

in certain embodiments, B1 and B3 are each independently selected from $C_{6-10}$ aryl, 5- to 10-membered heteroaryl or 4- to 10-membered heterocyclyl, wherein the heteroaryl or heterocyclyl contains 1 to 4 heteroatoms selected from O, S or N;

in certain embodiments, B1 and B3 are each independently selected from pyrazolyl, oxazolyl, dioxazolyl, oxadiazolyl, triazolyl, imidazolyl, tetrazolyl, pyrrolyl, thienyl, thiazolyl, thiadiazolyl, pyridyl, phenyl, pyrazinyl, pyrimidyl, pyridazinyl, thienopyrazinyl, benzimidazolyl, pyridotriazolyl, pyrimidopyrazolyl, imidazopyridazinyl, pyridopyrazolyl, pyrrolopyridazinyl or

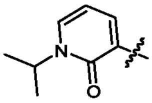

;

in certain embodiments, B1 and B3 are selected from

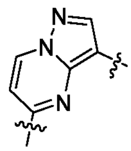

,

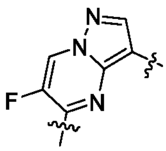

or

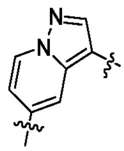

;

in certain embodiments, $R^{b1}$ and $R^{b7}$ are each independently selected from H, F, Cl, Br, I, =O, OH, $NH_2$, CN, $CF_3$, C(=O)OH, $CHF_2$, $C_{1-4}$ alkyl, $C_{1-4}$ alkoxy, $C_{3-6}$ cycloalkyl, $-(CH_2)_n-R^{b21}$, $-OR^{b21}$, $-N(R^{b21})_2$, $C_{6-10}$ aryl, 5- to 10-membered heteroaryl or 4- to 10-membered heterocyclyl, wherein the alkyl, alkoxy, cycloalkyl, heterocyclyl, aryl or heteroaryl is optionally further substituted with 0 to 4 substituents selected from H, F, Cl, Br, I, OH, =O, $-N(R^{b21})_2$, CN, $CF_3$, C(=O)OH, $C_{1-4}$ alkyl, $C_{1-4}$ alkoxy, $C_{3-6}$ cycloalkyl, 5- to 10-membered heteroaryl, 4- to 10-membered heterocyclyl or $R^{b7a}$, and the heteroaryl or heterocyclyl contains 1 to 4 heteroatoms selected from O, S, or N;

in certain embodiments, $R^{b1}$ and $R^{b7}$ are each independently selected from H, F, Cl, Br, I, =O, OH, $NH_2$, CN, $CF_3$, $CHF_2$, $CH_2F$, methyl, ethyl, methoxy, ethoxy, phenyl, pyrrolyl, pyridyl, morpholinyl,

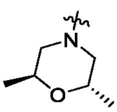, 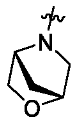, 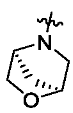, 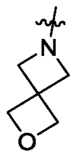, 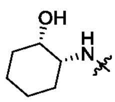, 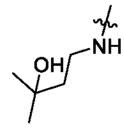, 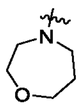, 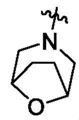, 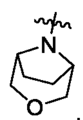, 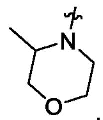, 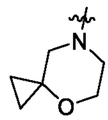,

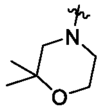, 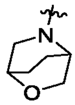, 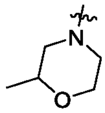, 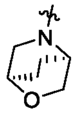, 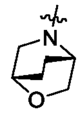, 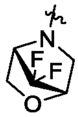, 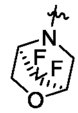 or 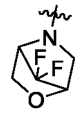,

wherein the methyl, ethyl, methoxy, ethoxy, phenyl, pyrrolyl, pyridyl or morpholinyl is optionally further substituted with 0 to 4 substituents selected from H, F, Cl, Br, I, OH, CN, $CF_3$, $NH_2$, $NHC_{1-4}$ alkyl, $N(C_{1-4}$ alkyl$)_2$, $NHCH_2C_{3-6}$ cycloalkyl, $C_{1-4}$ alkyl, $C_{1-4}$ alkoxy, $C_{3-6}$ cycloalkyl or $R^{b7a}$;

in certain embodiments, $R^{b1}$ and $R^{b7}$ are each independently selected from azetidinyl, azacyclopentyl, piperidyl, piperazinyl, morpholinyl, 2-oxa-5-azabicyclo[2.2.1]heptanyl, wherein the $R^{b1}$ and $R^{b7}$ are optionally substituted with 1 to 4 substituents selected from F, Cl, Br, I, OH, =O, CN, $CF_3$, $NH_2$, $NHC_{1-4}$ alkyl, $N(C_{1-4}$ alkyl$)_2$, $NHCH_2C_{3-6}$ cycloalkyl, $C_{1-4}$ alkyl, $C_{1-4}$ alkoxy, $C_{3-6}$ cycloalkyl or $R^{b7a}$, and the heterocyclyl contains 1 to 4 heteroatoms selected from O, S, or N;

in certain embodiments, $R^{b1}$ and $R^{b7}$ are each independently selected from azetidinyl, azacyclopentyl, piperidyl, piperazinyl, morpholinyl or 2-oxa-5-azabicyclo[2.2.1]heptanyl, wherein the $R^{b1}$ and $R^{b7}$ are optionally substituted with 1 to 4 substituents selected from F, Cl, Br, I, OH, =O, CN, $CF_3$, $NH_2$, $NHC_{1-4}$ alkyl, $N(C_{1-4}$ alkyl$)_2$, $NHCH_2C_{3-6}$ cycloalkyl, halogen-substituted $C_{1-4}$ alkyl, cyano-substituted $C_{1-4}$ alkyl, $-C_{1-4}$ alkylene -OH, $C_{1-4}$ alkyl, $C_{1-4}$ alkoxy, $-CH_2-O-C_{1-4}$ alkyl, $-CH_2-C_{3-6}$ cycloalkyl, $-O-C_{3-6}$ cycloalkyl, $-NH-C_{3-6}$ cycloalkyl, $C_{3-6}$ cycloalkyl, $-CH_2$-4- to 7-membered heterocycloalkyl, -O-4- to 7-membered heterocycloalkyl, -NH-4- to 7-membered heterocycloalkyl, or 4- to 7-membered heterocycloalkyl, and the heterocyclyl contains 1 to 4 heteroatoms selected from O, S, or N;

in certain embodiments, $R^{b1}$ and $R^{b7}$ are each independently selected from azetidinyl, azacyclopentyl, phenyl, pyrrolyl, pyridyl, morpholinyl,

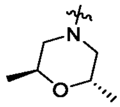, 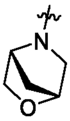, 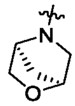, 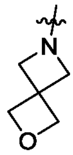, 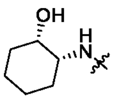,

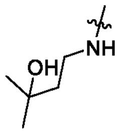, 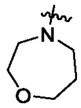, 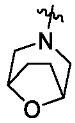, 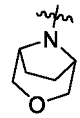, 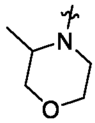, 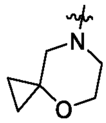, 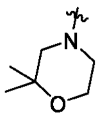, 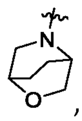, 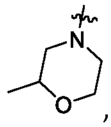, 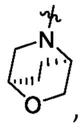, 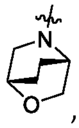,

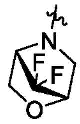, 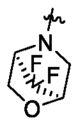, or 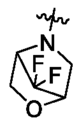;

in certain embodiments, $R^{b1}$ and $R^{b7}$ are each independently selected from

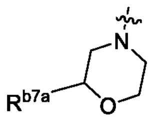,

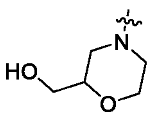, 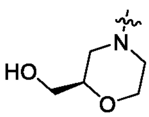, 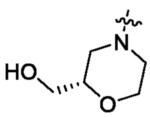, 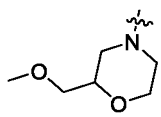, 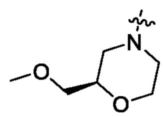, 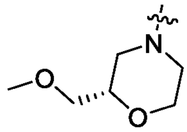 or 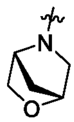;

in certain embodiments, $R^{b1}$ and $R^{b7}$ are optionally substituted with 1 to 4 substituents selected from F, Cl, Br, I, OH, =O, CN or $R^{b7a}$,

in certain embodiments, $R^{b7a}$ is selected from $C_{1-4}$ alkyl, $-C_{3-6}$ cycloalkyl, 4- to 10-membered heterocyclyl, $-C_{1-4}$ alkylene $-C_{3-6}$ cycloalkyl, $-C_{1-4}$ alkylene 4- to 10-membered heterocyclyl, $-O-C_{3-6}$ cycloalkyl, -O-4- to 10-membered heterocyclyl, $-NH-C_{3-6}$ cycloalkyl, -NH-4- to 10-membered heterocyclyl, $-N(C_{1-4}$ alkyl$)-C_{3-6}$ cycloalkyl or $-N(C_{1-4}$ alkyl)-4- to 10-membered heterocyclyl, wherein the $R^{b7a}$ is optionally substituted with 1 to 4 substituents selected from H, F, Cl, Br, I, OH, =O, $-N(R^{b21})_2$, CN, $CF_3$, C(=O)OH, $C_{1-4}$ alkyl, $C_{1-4}$ alkoxy, $C_{3-6}$ cycloalkyl or 4- to 10-membered heterocyclyl, and the heterocyclyl contains 1 to 4 heteroatoms selected from O, S, or N;

in certain embodiments, $R^{b7a}$ is selected from methyl, ethyl, propyl, isopropyl, cyclopropyl, cyclobutyl, cyclopentyl, cyclohexyl, azetidinyl, azacyclopentyl, azacyclohexyl, piperazinyl, morpholinyl, oxacyclobutyl, oxacyclopentyl, oxacyclohexyl, $-CH_2$-cyclopropyl, $-CH_2$-cyclobutyl, - $CH_2$-cyclopentyl, $-CH_2$-cyclohexyl, $-CH_2$-azetidinyl, $-CH_2$-azacyclopentyl, $-CH_2$-azacyclohexyl, $-CH_2$-piperazinyl, $-CH_2$-morpholinyl, $-CH_2$-oxacyclobutyl, $-CH_2$-oxacyclopentyl, $-CH_2$-oxacyclohexyl, -O-cyclopropyl, -O-cyclobutyl, -O-cyclopentyl, -O-cyclohexyl, -O-azetidinyl, -O-azacyclopentyl, -O-azacyclohexyl, -O-piperazinyl, -O-morpholinyl, -O-oxacyclobutyl, -O-oxacyclopentyl, -O-oxacyclohexyl, -NH-cyclopropyl, -NH-cyclobutyl, -NH-cyclopentyl, -NH-cyclohexyl, -NH-azetidinyl, -NH-azacyclopentyl, -NH-azacyclohexyl, -NH-piperazinyl, -NH-morpholinyl, -NH-oxacyclobutyl, - NH-oxacyclopentyl, or -NH-oxacyclohexyl, wherein the $R^{b7a}$ is optionally substituted with 1 to 4 substituents selected from F, Cl, Br, I, OH, =O, CN, $CF_3$, C(=O)OH, $C_{1-4}$

alkyl, $C_{1-4}$ alkoxy, $C_{3-6}$ cycloalkyl, or 4- to 10-membered heterocyclyl, and the heterocyclyl contains 1 to 4 heteroatoms selected from O, S, or N;

in certain embodiments, $R^{b7a}$ is selected from $CF_3$, $CHF_2$, $CH_2F$, $CH_2CN$, $CH_2OH$, $CH_2OCH_3$, methyl, ethyl, $-CH_2$-cyclopropyl, $-CH_2$-azetidinyl, $-CH_2$-oxacyclobutyl, -O-cyclopropyl, -O-azetidinyl, -O-oxacyclobutyl, -NH-cyclopropyl, -NH-azetidinyl, -NH-oxacyclobutyl,

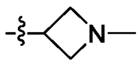,

or

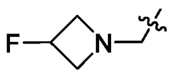;

in certain embodiments, $R^{b2}$ and $R^{b6}$ are each independently selected from H, F, Cl, Br, I, =O, OH, $-C(=O)N(R^{b21})_2$, $-N(R^{b21})_2$, CN, $CF_3$, C(=O)OH, $CHF_2$, $C_{1-4}$ alkyl, $C_{1-4}$ alkoxy, $C_{3-6}$ cycloalkyl, $-(CH_2)_n-R^{b21}$, $-OR^{b21}$, $C_{6-10}$ aryl, 5- to 10-membered heteroaryl or 4- to 10-membered heterocyclyl, wherein the alkyl, alkoxy, cycloalkyl, aryl, heteroaryl or heterocyclyl is optionally further substituted with 0 to 4 substituents selected from H, F, Cl, Br, I, OH, =O, $NH_2$, CN, $CF_3$, C(=O)OH, $C_{1-4}$ alkyl, $C_{1-4}$ alkoxy, $C_{3-6}$ cycloalkyl, 5- to 10-membered heteroaryl or 4- to 10-membered heterocyclyl, and the heteroaryl or heterocyclyl contains 1 to 4 heteroatoms selected from O, S, or N;

in certain embodiments, $R^{b2}$ and $R^{b6}$ are each independently selected from H, F, Cl, Br, I, =O, $CF_3$, $CHF_2$, OH, $NH_2$, NH(methyl), NH(ethyl), NH(propyl), NH(isopropyl), $N(methyl)_2$, $N(ethyl)_2$, CN, methyl, ethyl, methoxy, ethoxy, propoxy, isopropyloxy, morpholinyl, piperazinyl, pyrrolidyl, piperidyl or oxazolidinyl, wherein the methyl, ethyl, methoxy, ethoxy, propoxy, isopropyloxy, morpholinyl, piperazinyl, pyrrolidyl, piperidyl or oxazolidinyl is optionally further substituted with 0 to 4 substituents selected from H, F, Cl, Br, I, OH, CN, $CF_3$, $C_{1-4}$ alkyl, $C_{1-4}$ alkoxy or $C_{3-6}$ cycloalkyl;

in certain embodiments, each $R^{b21}$ is independently selected from H, $C_{1-6}$ alkyl, $C_{1-4}$ alkoxy, $C_{3-6}$ cycloalkyl, $C_{6-10}$ aryl, 5- to 10-membered heteroaryl or 4- to 10-membered heterocyclyl, wherein the alkyl, alkoxy, cycloalkyl, aryl, heteroaryl or heterocyclyl is optionally further substituted with 0 to 4 substituents selected from H, F, Cl, Br, I, OH, =O, $NH_2$, CN, $CF_3$, C(=O)OH, $C_{1-4}$ alkyl, $C_{3-6}$ cycloalkyl or $C_{1-4}$ alkoxy, and the heteroaryl or heterocyclyl contains 1 to 4 heteroatoms selected from O, S, or N;

in certain embodiments, B is selected from

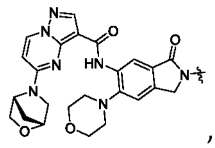, 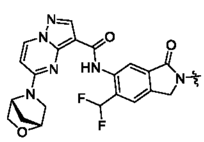, 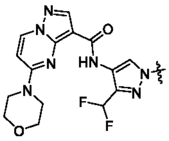,

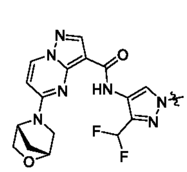, 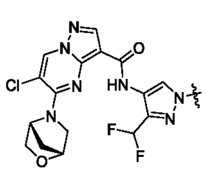, 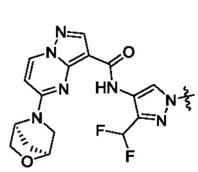, 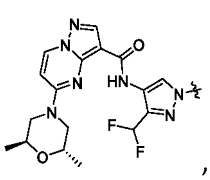, 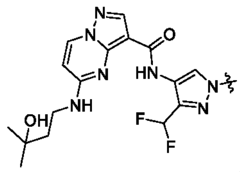,

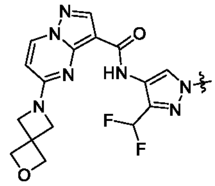, 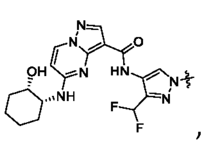, 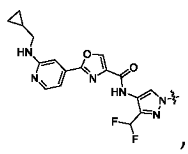, 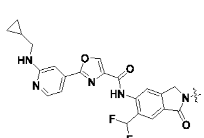, 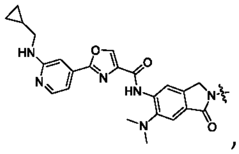,

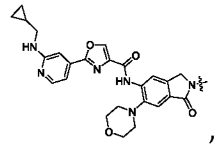, 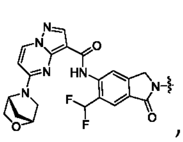, 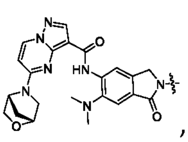, 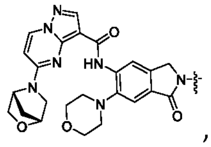, 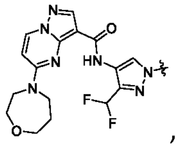,

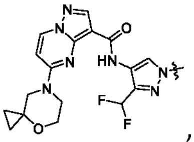, 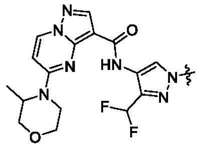, 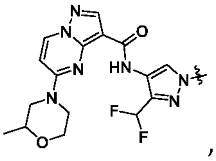, 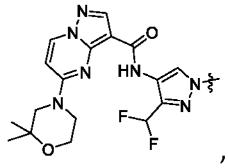, 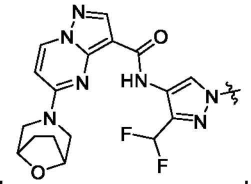,

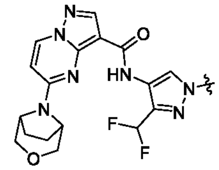, 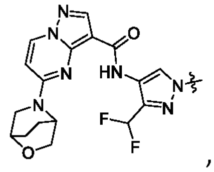, 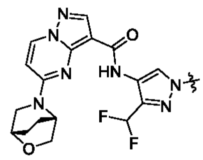, 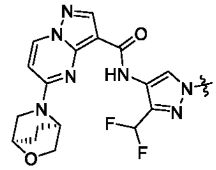, 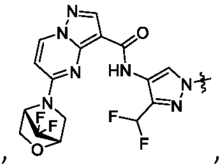,

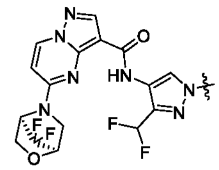, 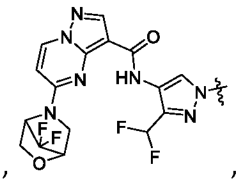, 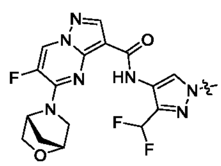, 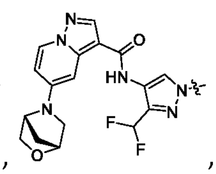, 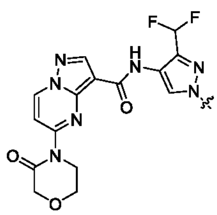,

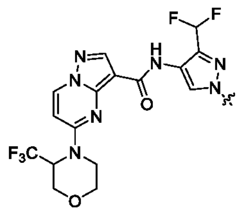, 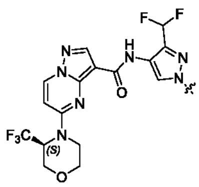, 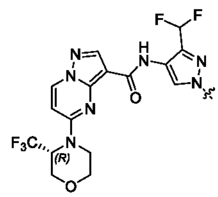, 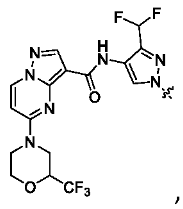, 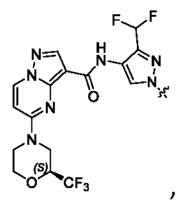,

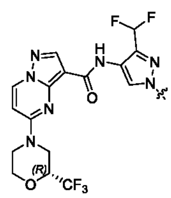, 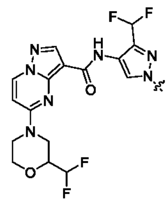, 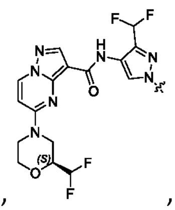, 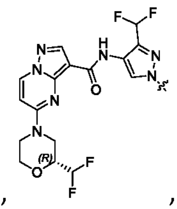, 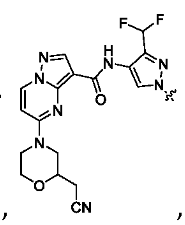, 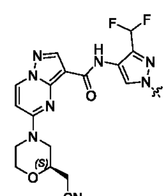,

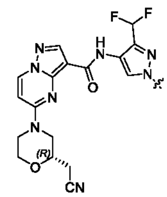, 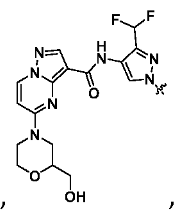, 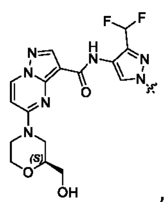, 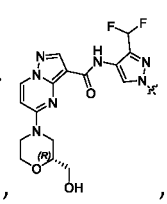, 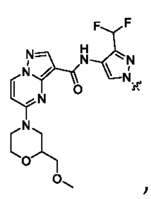, 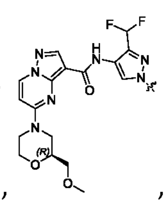, 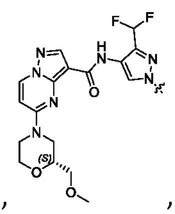,

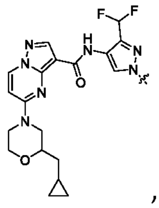, 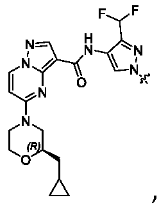, 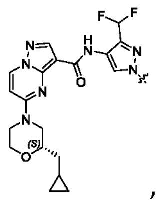, 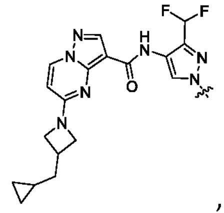, 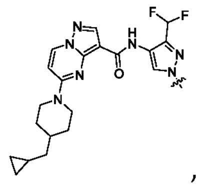, 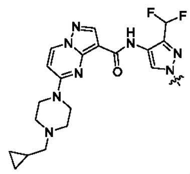,

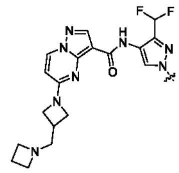, 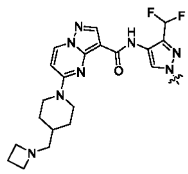, 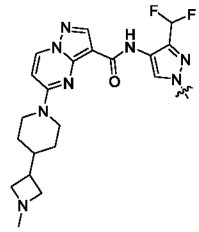, 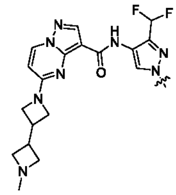, 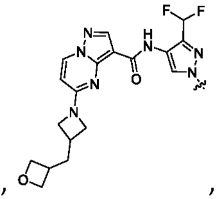,

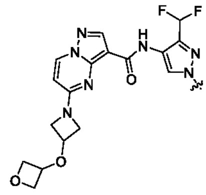, 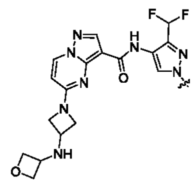, 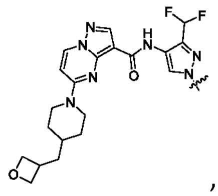, 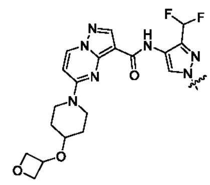, 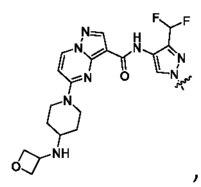,

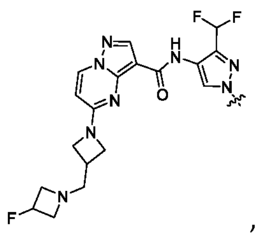, or 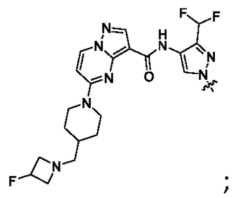;

in certain embodiments, L is selected from -Ak1-Cy1-Ak2-Cy2-Ak3-Cy3-Ak4-Cy4-Ak5-;

in certain embodiments, L is selected from a bond, -Cy1-, -Cy1-Ak2-, -Cy1-Ak2-Ak3-, -Cy1-Ak2-Ak3-Ak4-Ak5-, -Cy1-Cy2-, -Cy1-Ak2-Cy2-, -Cy1-Cy2-Ak3-, -Cy1-Cy2-Ak3-Cy4-, -Cy1-Ak2-Cy2-Ak3-, - Cy1-Ak2-Cy2-Ak3-Ak4-, -Cy1-Ak2-Cy2-Cy3-Ak4-, -Cy1-Cy2-Ak3-Ak4-, -Cy1-Cy2-Ak3-Ak4-Ak5-, - Cy1-Ak2-Cy2-Ak3-Ak4-Ak5-, -Cy1-Ak2-Ak3-Cy3-Ak4-, -Cy1-Ak2-Ak3-Cy3-Ak4-Ak5-, -Cy1-Ak2-Ak3-Ak4-Cy4-Ak5-, -Cy1-Cy2-Cy3-, -Cy1-Ak2-Cy2-Cy3-, -Cy1-Cy2-Cy3-Ak4-, -Cy1-Ak2-Cy2-Cy3-Ak4-, - Cy1-Ak2-Cy2-Ak3-Cy3-, -Cyl-Ak2-Cy2-Cy3-Ak4-Ak5, -Cy1-Ak2-Cy2-Ak3-Cy3-Ak4-Ak5-, -Cy1-Cy2-Cy3-Cy4-, -Cy1-Cy2-Ak3-Cy3-Cy4-, -Cy1-Cy2-Cy3-Ak4-Cy4-, -Ak1-Cy1-Ak2-Cy2-, -Ak1-Cy1-Ak2-Cy2-Ak3-, -Ak1-Ak2-Cy2-Ak3-, -Ak1-Ak2-Cy2-, -Ak1-Ak2-Cy2-Cy3-Ak4-, -Ak1-Ak2-Ak3-Cy3-Ak4-, -Ak1-Cy1-Ak2-, -Ak1-Cy1-Cy2-Ak3-Ak4-, -Ak1-Cy1-Cy2-Ak3-, -Ak1-Cy1-Ak2-Ak3-Ak4-, -Ak1-Cy1-, - Ak1-Cy1-Ak2-Ak3-, -Ak1-Ak2-Cy2-Ak3-Ak4-, -Ak1-Cy1-Ak2-Cy2-Ak3-Ak4-, -Cy1-Ak2-Ak3-Cy3-Cy4-Ak5-, -Ak1-Cy1-Ak2-Ak3-Ak4-Ak5-, -Cy1-Ak1-Ak2-Ak3-, -Ak1-Cy1-Cy2-, -Ak1-Ak2-Ak3-Ak4- or - Cy1-Ak2-Cy2-Ak3-Cy3-Ak4-;

in certain embodiments, Ak1, Ak2, Ak3, Ak4 and Ak5 are each independently selected from - $(CH_2)_q$-, O, -$(CH_2)_q NR^L$-, NR‘C=O, C=ONR$^L$, C=O, -R$^L$C=CR$^L$-, C≡C or a bond;

in certain embodiments, Ak1, Ak2, Ak3, Ak4 and Ak5 are each independently selected from O, C≡C, $CH_2$, $CH_2CH_2$, $CH_2CH_2CH_2$, $CH_2CH_2CH_2CH_2$, $CH_2N(CH_3)$, $CH_2CH_2N(CH_3)$, $N(CH_3)$, NH, C(=O), $C(=O)N(CH_3)$, $N(CH_3)C(=O)$, C(=O)NH or NHC(=O);

in certain embodiments, $R^L$ is selected from H or $C_{1-6}$ alkyl;

in certain embodiments, $R^L$ is selected from H, methyl or ethyl;

in certain embodiments, Cy1, Cy2, Cy3 and Cy4 are each independently selected from a bond, 4-to 7-membered mono-heterocyclic ring, 4- to 10-membered fused-heterocyclic ring, 5- to 12-membered spiro-heterocyclic ring, 7- to 10-membered bridged-heterocyclic ring, 3- to 7-membered monocycloalkyl, 4- to 10-membered fused cycloalkyl, 5-to 12-membered spiro cycloalkyl, 7- to 10-membered bridged cycloalkyl, 5- to 10-membered heteroaryl or 6- to 10-membered aryl, wherein the aryl, heteroaryl, cycloalkyl, mono-heterocyclic ring, fused-heterocyclic ring, spiro-heterocyclic ring or bridged-heterocyclic ring is optionally further substituted with 0 to 4 substituents selected from

H, F, Cl, Br, I, OH, C(=O)OH, CN, $NH_2$, =O, $C_{1-4}$ alkyl, halogen-substituted $C_{1-4}$ alkyl, hydroxyl-substituted $C_{1-4}$ alkyl or $C_{1-4}$ alkoxy, and the heteroaryl, mono-heterocyclic ring, fused-heterocyclic ring, spiro-heterocyclic ring or bridged-heterocyclic ring contains 1 to 4 heteroatoms selected from O, S, or N;

in certain embodiments, Cy1, Cy2, Cy3 and Cy4 are each independently selected from a bond, 4-to 7-membered nitrogen-containing mono-heterocyclic ring, 4- to 10-membered nitrogen-containing fused-heterocyclic ring, 5- to 12-membered nitrogen-containing spiro-heterocyclic ring, 7- to 10-membered nitrogen-containing bridged-heterocyclic ring, 3- to 7-membered monocycloalkyl, 4- to 10-membered fused cycloalkyl, 5- to 12-membered spiro cycloalkyl, 7- to 10-membered bridged cycloalkyl, 5- to 10-membered heteroaryl or 6- to 10-membered aryl, wherein the mono-heterocyclic ring, fused-heterocyclic ring, bridged-heterocyclic ring, spiro-heterocyclic ring, cycloalkyl, aryl or heteroaryl is optionally further substituted with 0 to 4 substituents selected from H, F, Cl, Br, I, OH, C(=O)OH, CN, $NH_2$, =O, $C_{1-4}$ alkyl, halogen-substituted $C_{1-4}$ alkyl, hydroxyl-substituted $C_{1-4}$ alkyl or $C_{1-4}$ alkoxy, and the mono-heterocyclic ring, fused-heterocyclic ring, bridged-heterocyclic ring, spiro-heterocyclic ring or heteroaryl contains 1 to 4 heteroatoms selected from O, S, or N;

in certain embodiments, Cy1, Cy2, Cy3 and Cy4 are each independently selected from a bond or one of the following substituted or unsubstituted groups: cyclopropyl, cyclobutyl, cyclopentyl, cyclohexyl, azetidinyl, azacyclopentyl, piperidyl, morpholinyl, piperazinyl, phenyl, cyclopropyl-fused-cyclopropyl, cyclopropyl-fused-cyclobutyl, cyclopropyl-fused-cyclopentyl, cyclopropyl-fused-cyclohexyl, cyclobutyl-fused-cyclobutyl, cyclobutyl-fused-cyclopentyl, cyclobutyl-fused-cyclohexyl, cyclopentyl-fused-cyclopentyl, cyclopentyl-fused-cyclohexyl, cyclohexyl-fused-cyclohexyl, cyclopropyl-spiro-cyclopropyl, cyclopropyl-spiro-cyclobutyl, cyclopropyl-spiro-cyclopentyl, cyclopropyl-spiro-cyclohexyl, cyclobutyl-spiro-cyclobutyl, cyclobutyl-spiro-cyclopentyl, cyclobutyl-spiro-cyclohexyl, cyclopentyl-spiro-cyclopentyl, cyclopentyl-spiro-cyclohexyl, cyclohexyl-spiro-cyclohexyl, cyclopropyl-fused-azetidinyl, cyclopropyl-fused-azacyclopentyl, cyclopropyl-fused-azacyclohexyl, cyclobutyl-fused-azetidinyl, cyclobutyl-fused-azacyclopentyl, cyclobutyl-fused-azacyclohexyl, cyclopentyl-fused-azetidinyl, cyclopentyl-fused-azacyclopentyl, cyclopentyl-fused-azacyclohexyl, cyclohexyl-fused-azetidinyl, cyclohexyl-fused-azacyclopentyl, cyclohexyl-fused-azacyclohexyl, azetidinyl-fused-azetidinyl, azetidinyl-fused-azacyclopentyl, azetidinyl-fused-azacyclohexyl, azacyclopentyl-fused-azacyclopentyl, azacyclopentyl-fused-azacyclohexyl, azacyclohexyl-fused-azacyclohexyl, cyclobutyl-spiro-azetidinyl, cyclobutyl-spiro-azacyclopentyl, cyclobutyl-spiro-azacyclohexyl, cyclopentyl-spiro-azetidinyl, cyclopentyl-spiro-azacyclopentyl, cyclopentyl-spiro-azacyclohexyl, cyclohexyl-spiro-azetidinyl, cyclohexyl-spiro-azacyclopentyl, cyclohexyl-spiro-azacyclohexyl, azetidinyl-spiro-azetidinyl, azetidinyl-spiro-azacyclopentyl, azetidinyl-spiro-azacyclohexyl, azacyclopentyl-spiro-azacyclopentyl, azacyclopentyl-spiro-azacyclohexyl, azacyclohexyl-spiro-azacyclohexyl,

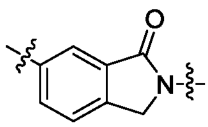, 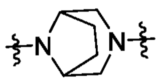, 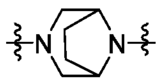, 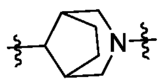, 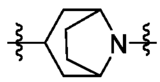, 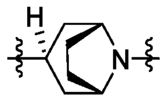,

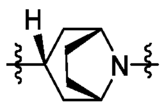, 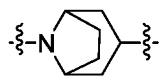, 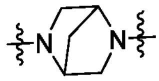, 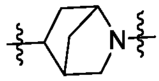, 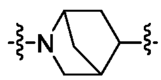, 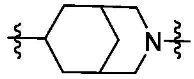,

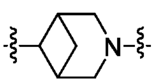, 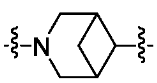, 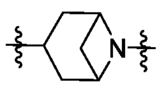, 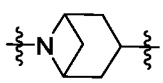, 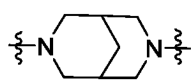, 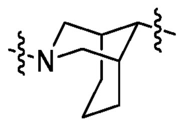,

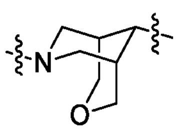 or 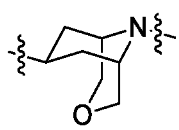, 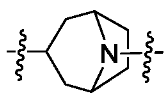, which, when substituted, is optionally further substituted with 0 to 4 substituents selected from H, F, Cl, Br, I, OH, $NH_2$, C(=O)OH, CN, =O, $C_{1-4}$ alkyl, halogen-substituted $C_{1-4}$ alkyl, hydroxyl-substituted $C_{1-4}$ alkyl or $C_{1-4}$ alkoxy;

in certain embodiments, Cy1, Cy2, Cy3 and Cy4 are each independently selected from a bond or one of the following substituted or unsubstituted groups:

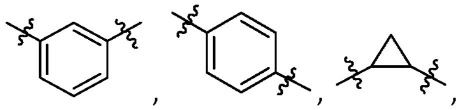

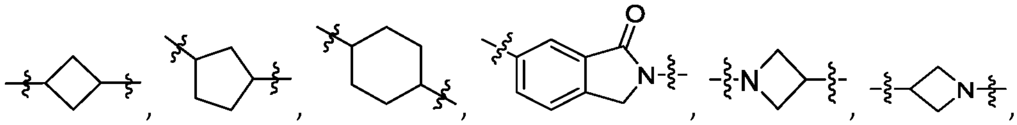

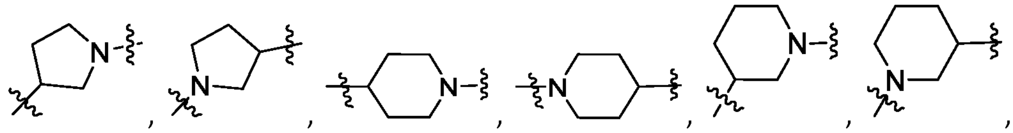

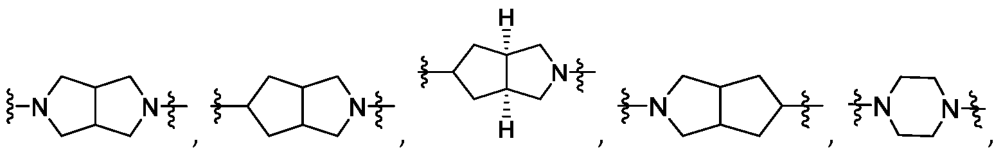

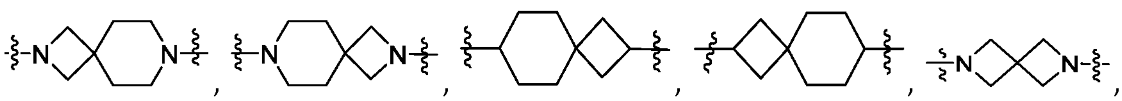

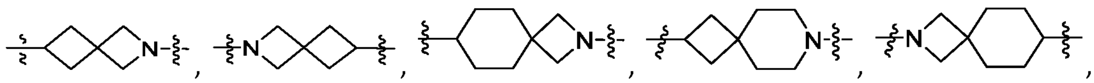

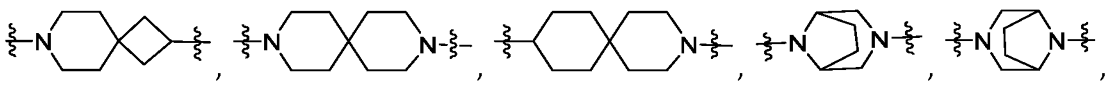

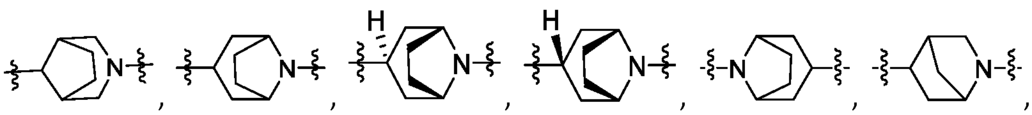

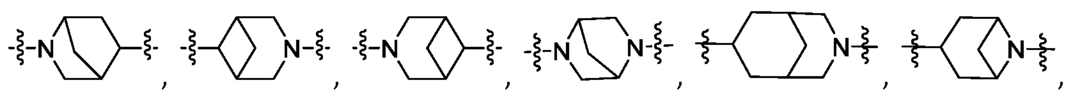

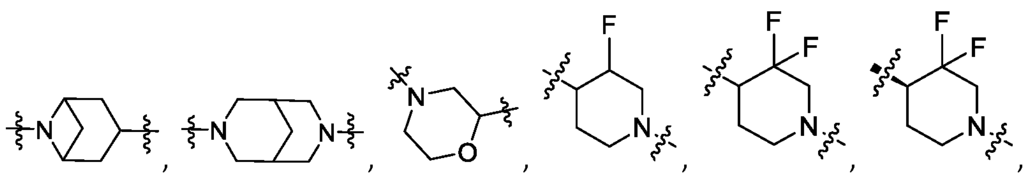

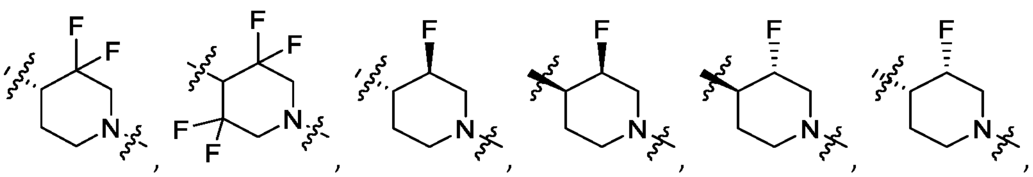

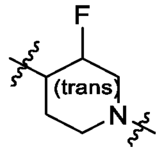 or 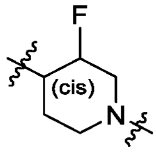,

which, when substituted, is optionally further substituted with 0 to 4 substituents selected from H, F, $CF_3$, methyl, =O, hydroxymethyl, C(=O)OH, CN or $NH_2$;
in certain embodiments, L is selected from

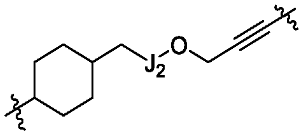, 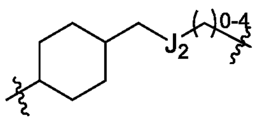,

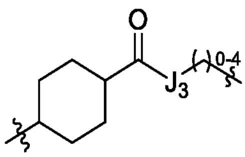, 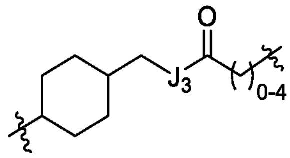, 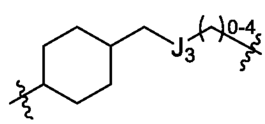,

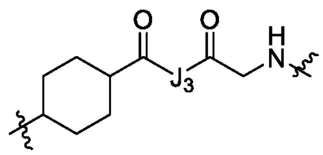, 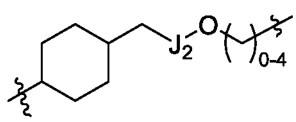, 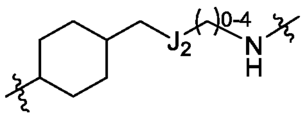,

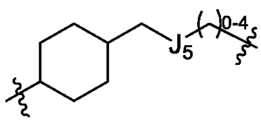, 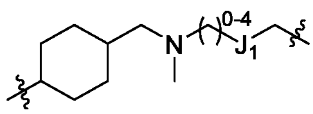, 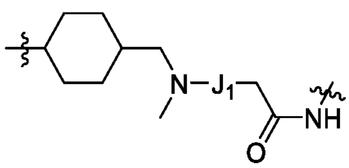,

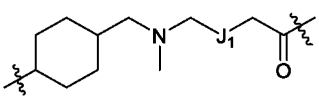, 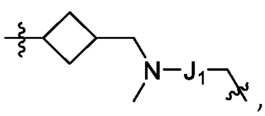, 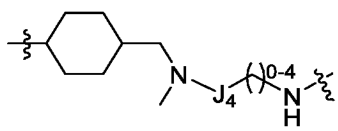,

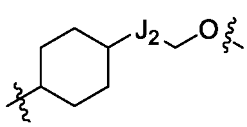, 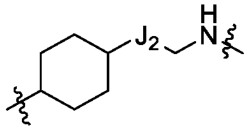, 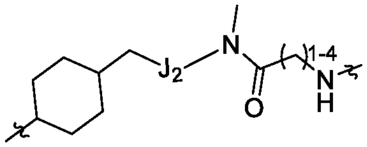,

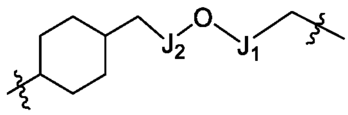, 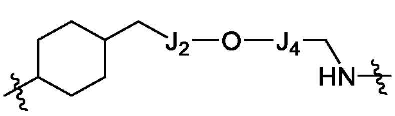, 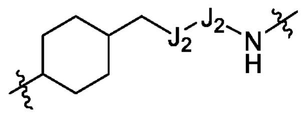,

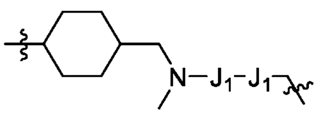, 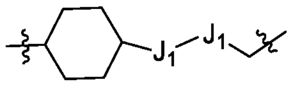, 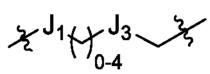, 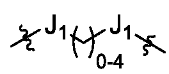,

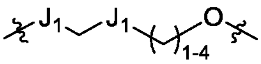, 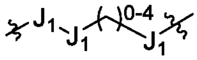, 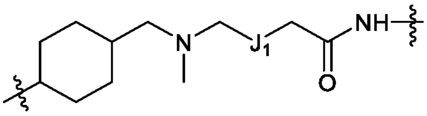,

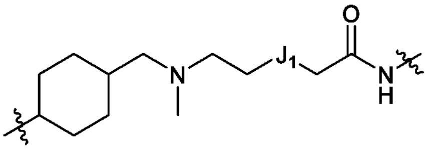, 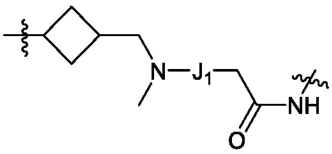, 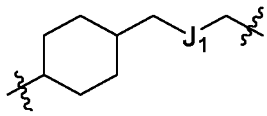,

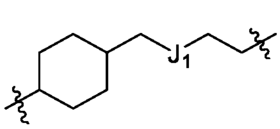, 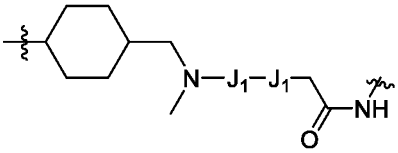, 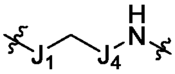, 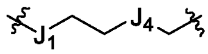,

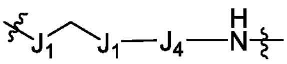, 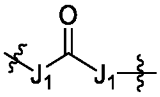, 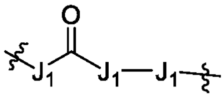, 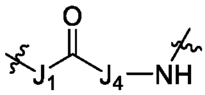,

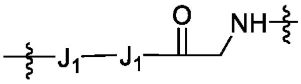, 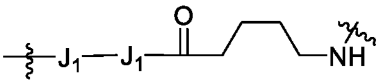, 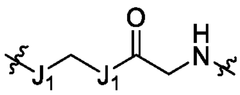,

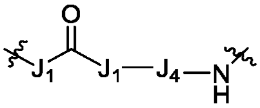, 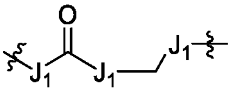, 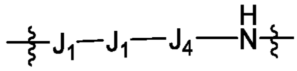, 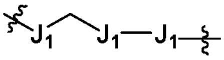,

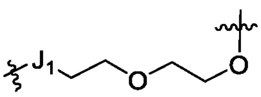, 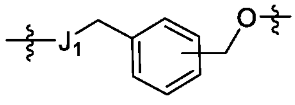, 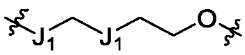, 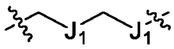,

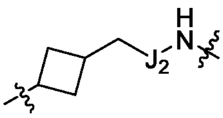, 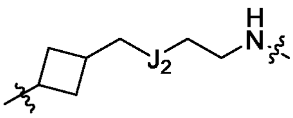, , 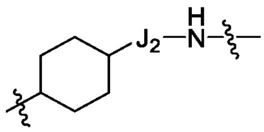, 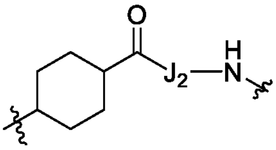,

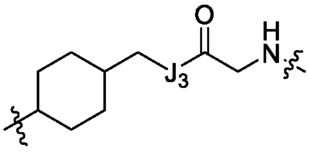, 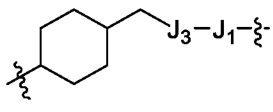, 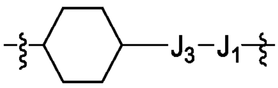, 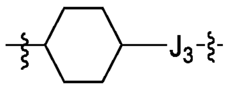,

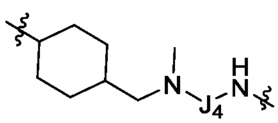, 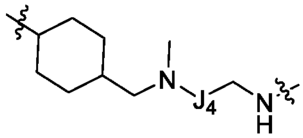, 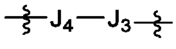, 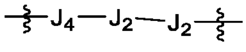,

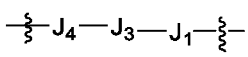 or 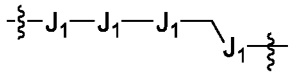;

in certain embodiments, each $J_1$ is independently selected from

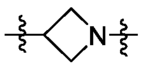, 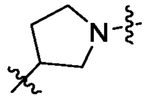,

or

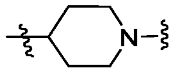;

in certain embodiments, each $J_2$ is independently selected from

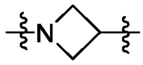, 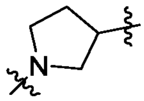,

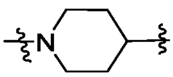, 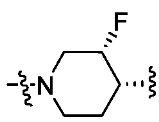, 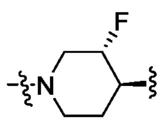, 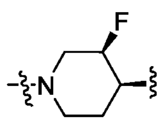, 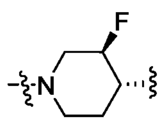, 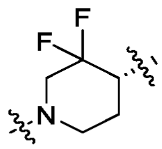,

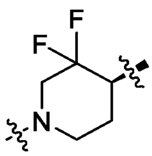, 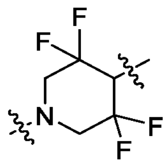, 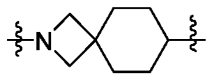, or 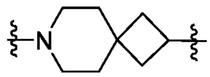;

in certain embodiments, each $J_3$ is independently selected from

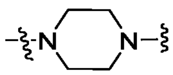,

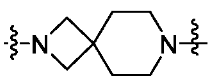, 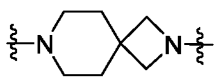, 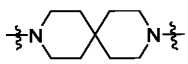, 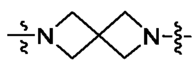 or 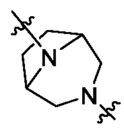;

in certain embodiments, each $J_4$ is independently selected from

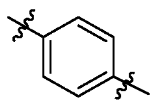, 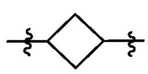,

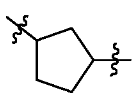, 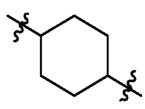, 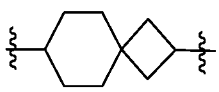 or 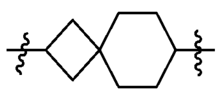;

in certain embodiments, each $J_5$ is independently selected from

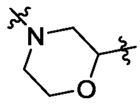

;

in certain embodiments, L is selected from

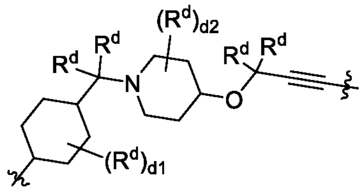

,

$R^d$ is selected from H or D, and at least one of $R^d$ is selected from D, d1 is selected from 0, 1, 2, 3, 4, 5, 6, 7, 8, 9 or 10, and d2 is selected from 0, 1, 2, 3, 4, 5, 6, 7, 8 or 9;

in certain embodiments, L is selected from a group shown in Table L-1, wherein the left side of the group is linked to B;

Table L-1 L group

| | | | |
|---|---|---|---|
| 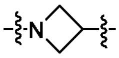 | 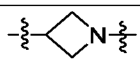 | 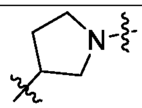 | 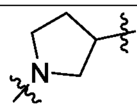 |
| 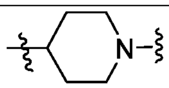 | 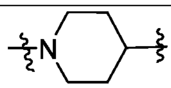 | 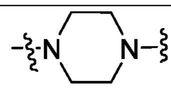 | 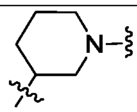 |
| 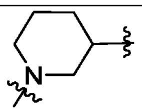 | 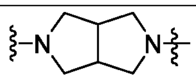 | 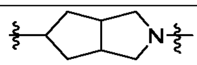 | 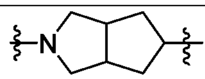 |
| 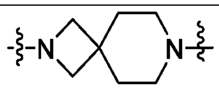 | 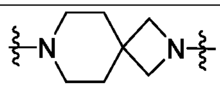 | 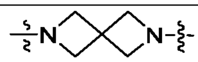 | 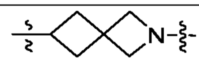 |
| 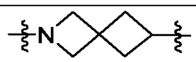 | 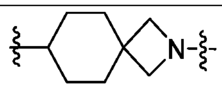 | 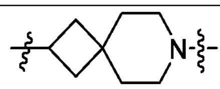 | 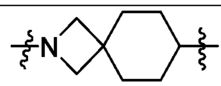 |

| | | | |
|---|---|---|---|
| 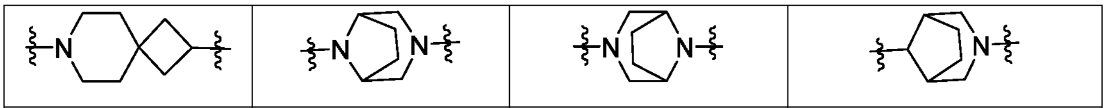 | | | |
| 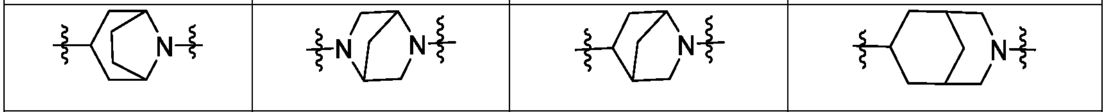 | | | |
| 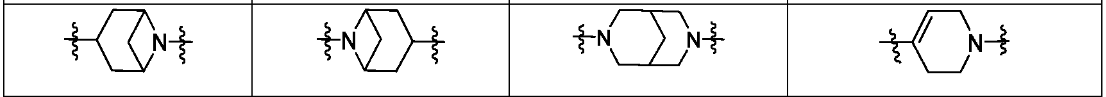 | | | |
| 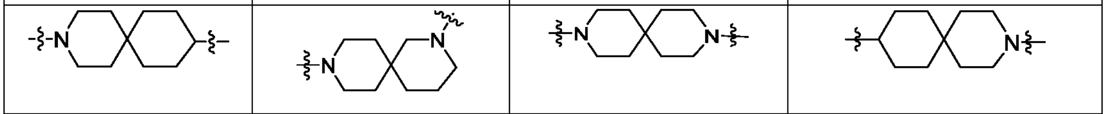 | | | |
| 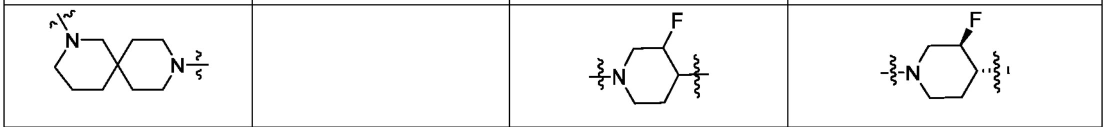 | | | |
| 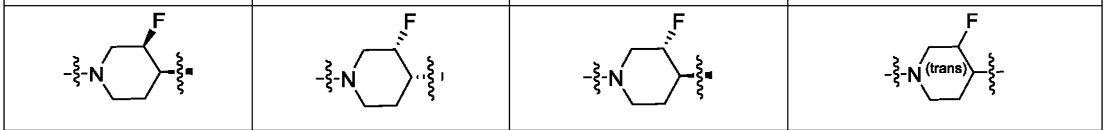 | | | |
| 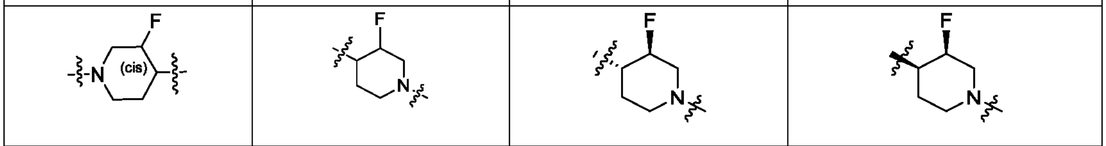 | | | |
| 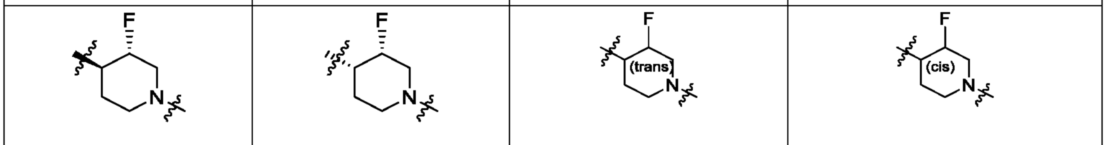 | | | |
| 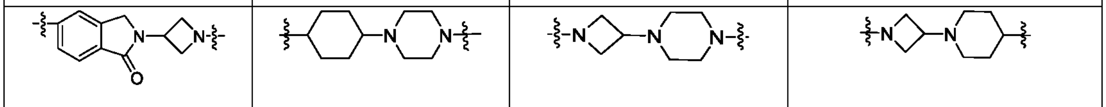 | | | |
| 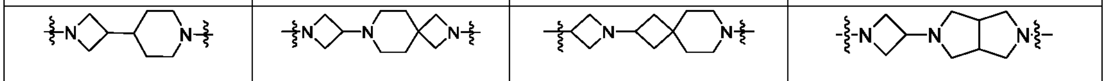 | | | |
| 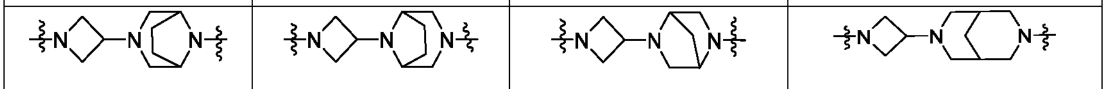 | | | |
| 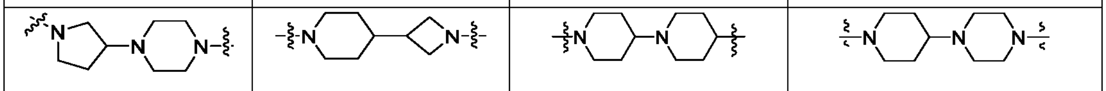 | | | |
| 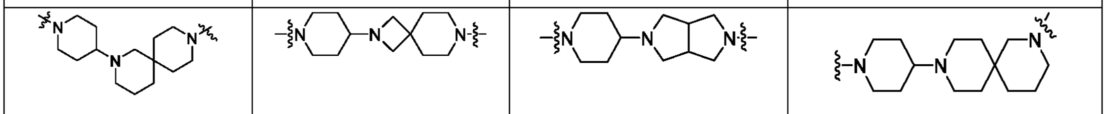 | | | |
| 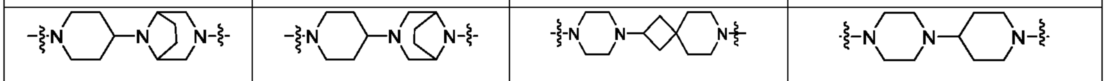 | | | |
| 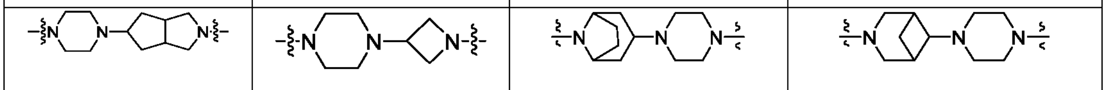 | | | |
| 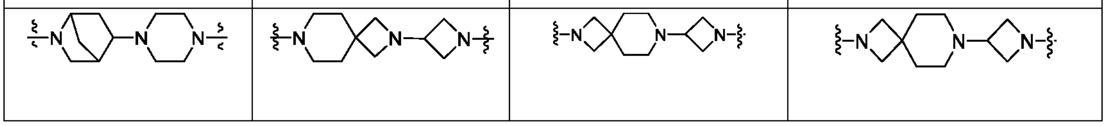 | | | |

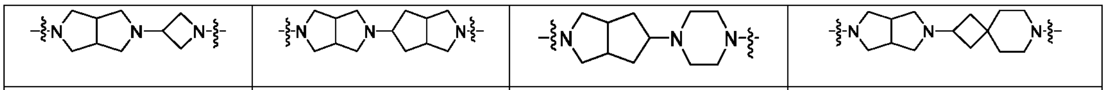
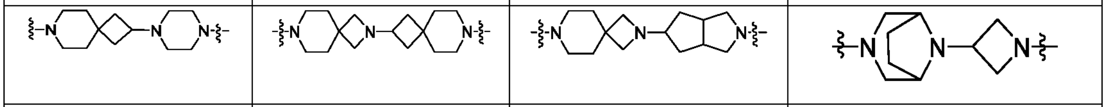
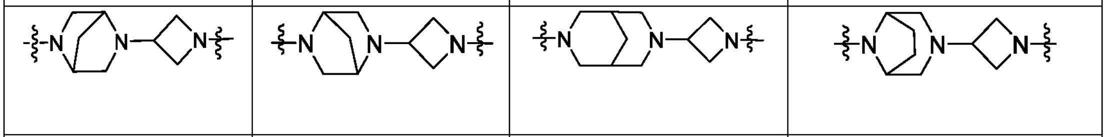
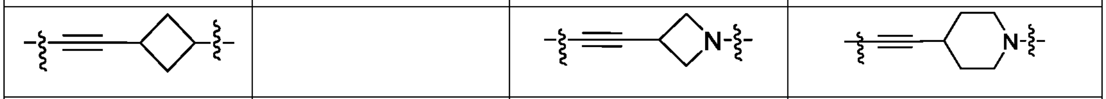
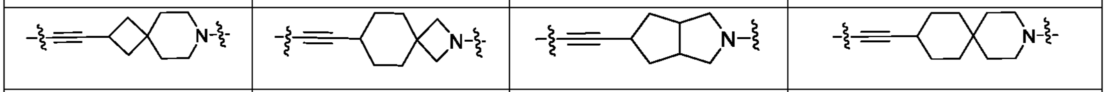
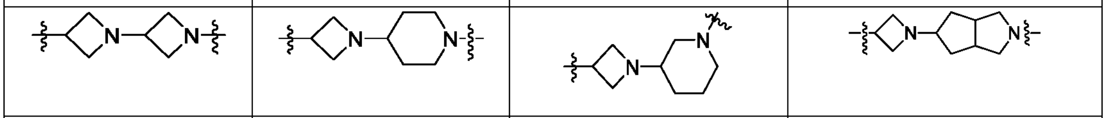
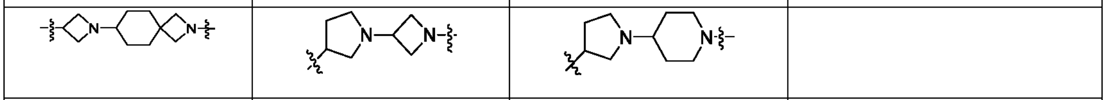
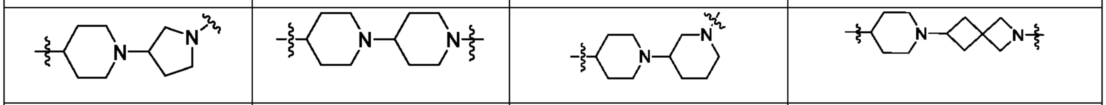
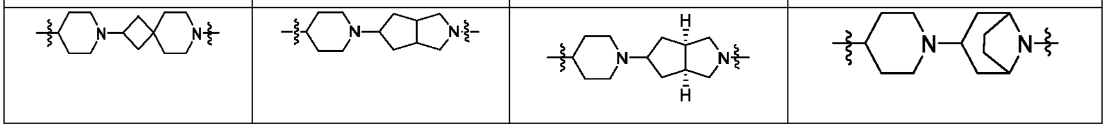
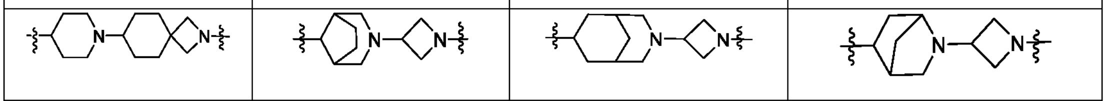
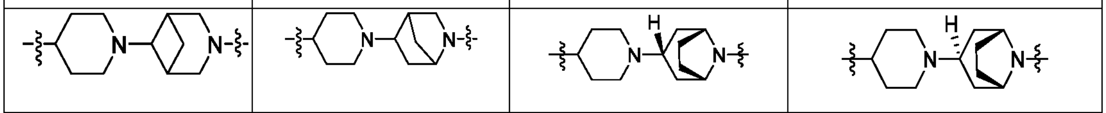
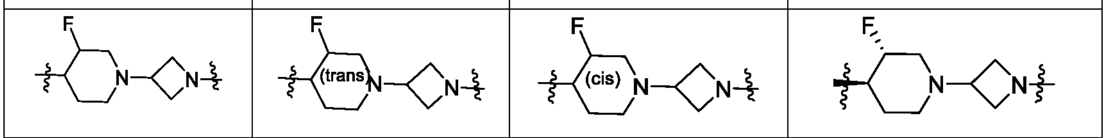
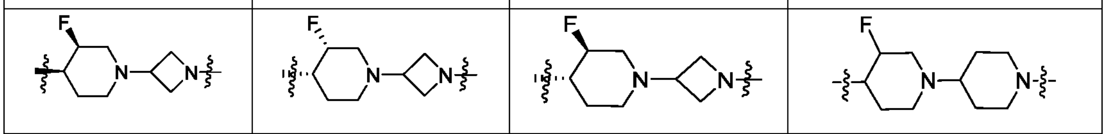
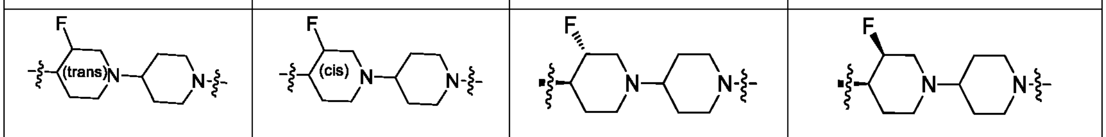
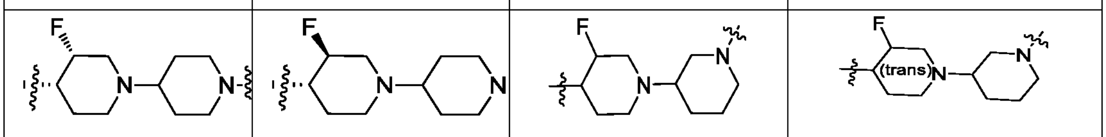
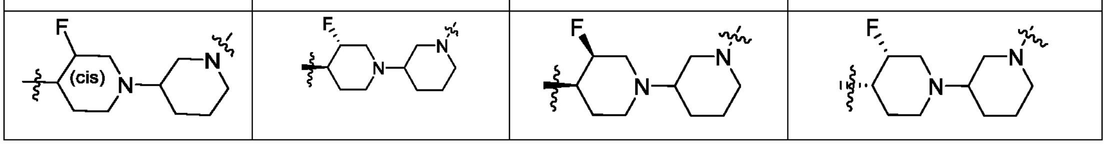

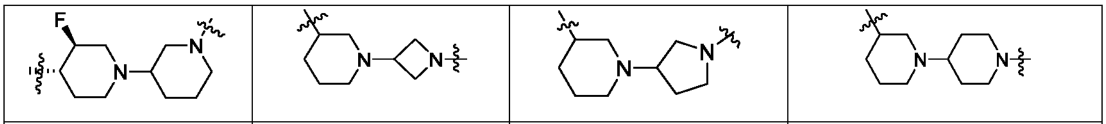
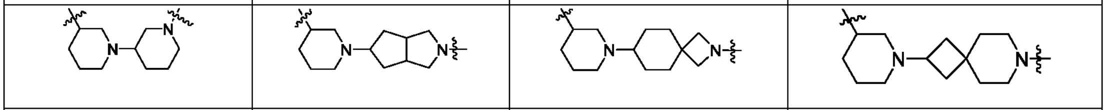
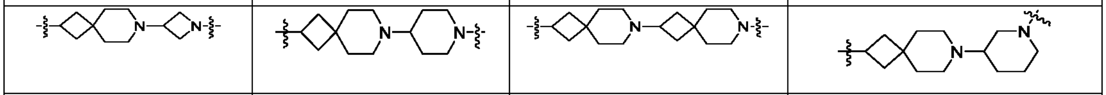
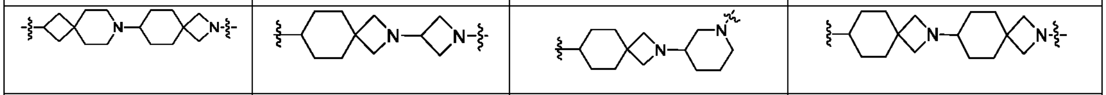
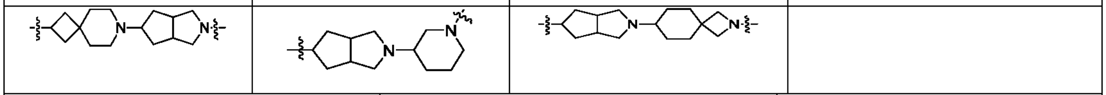
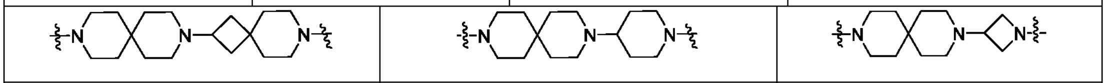
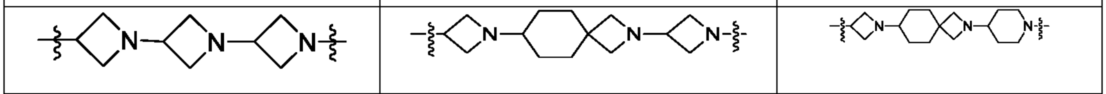
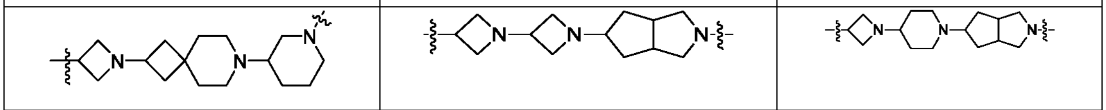
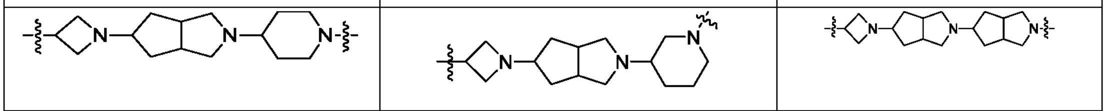
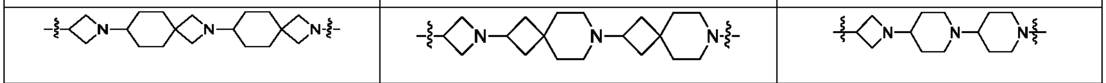
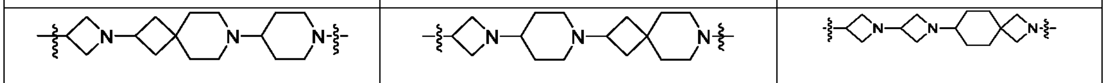
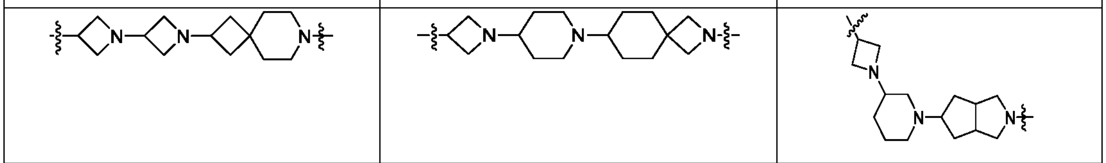
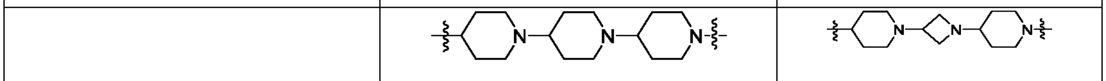
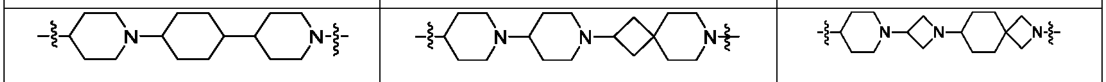
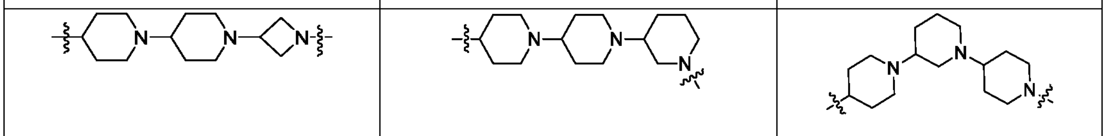
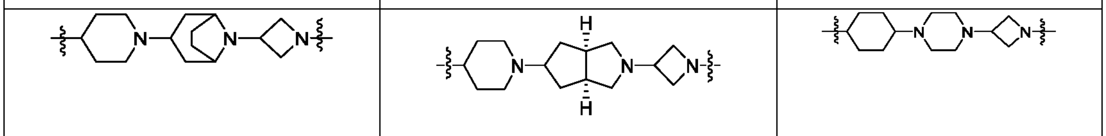
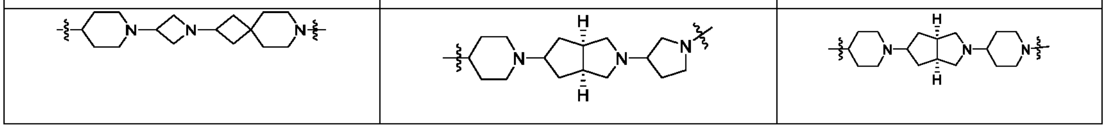

| | | |
|---|---|---|
| 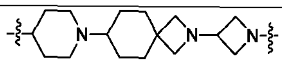 | 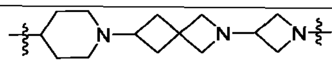 | 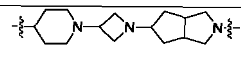 |
| 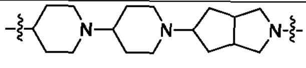 | 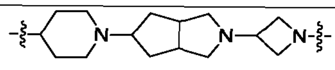 | 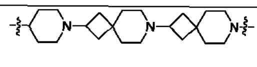 |
| 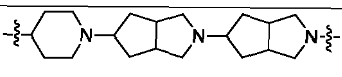 | 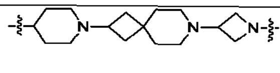 | 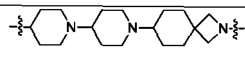 |
| 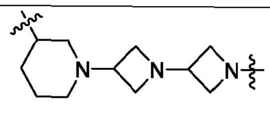 | 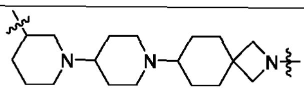 | 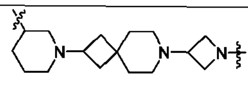 |
| 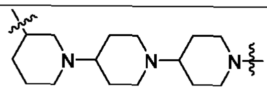 | 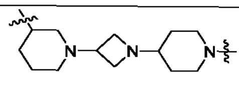 | 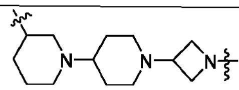 |
| 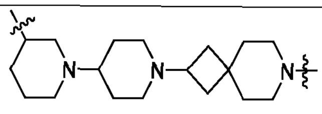 | 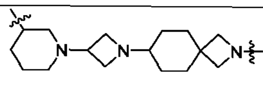 | 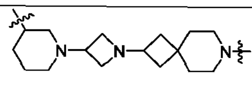 |
| 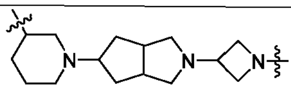 | 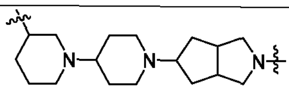 | 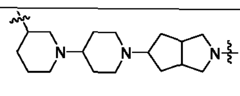 |
| 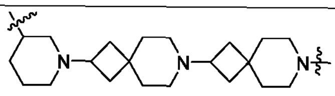 | 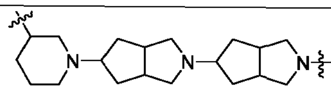 | 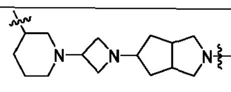 |
| 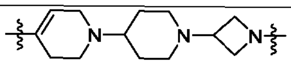 | 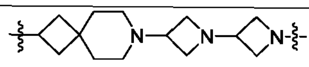 | 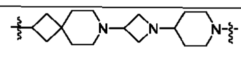 |
| 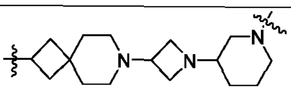 | 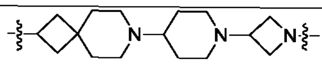 | |
| 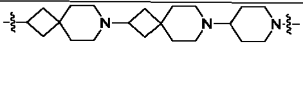 | 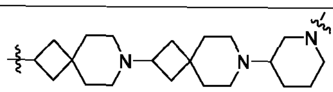 | 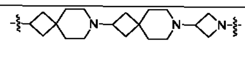 |
| 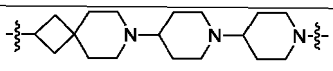 | 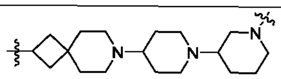 | 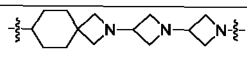 |
| 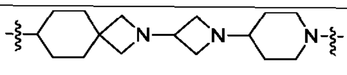 | 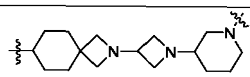 | 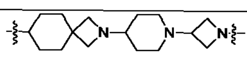 |
| 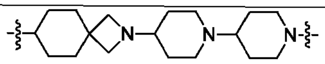 | 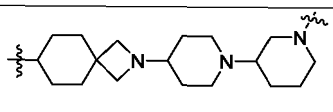 | |
| | | |
| | | |
| | | |

| | | |
|---|---|---|
| 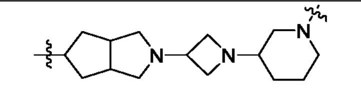 | 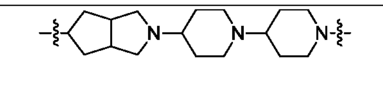 | 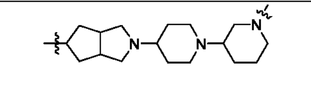 |
| 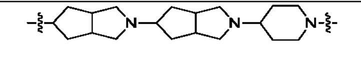 | 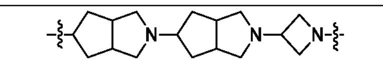 | 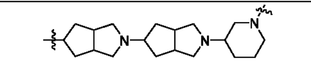 |
| 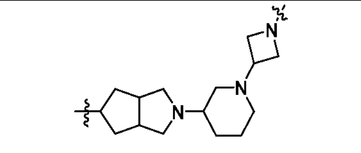 | 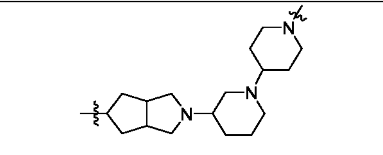 | 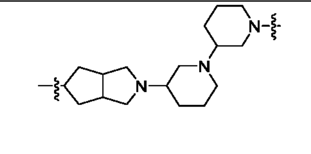 |
| 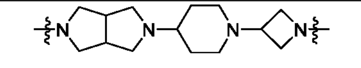 | 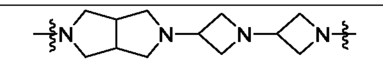 | 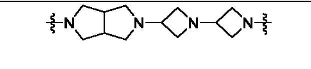 |
| 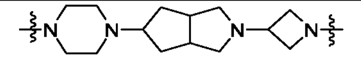 | 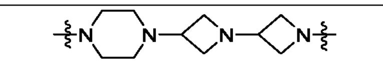 | 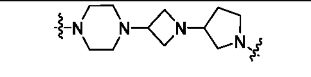 |
| 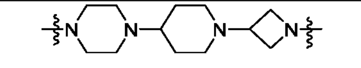 | 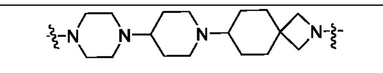 | 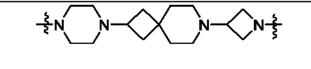 |
| 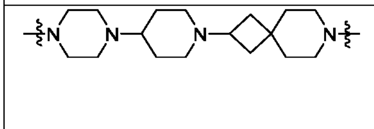 | 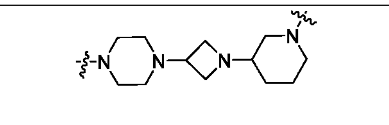 | 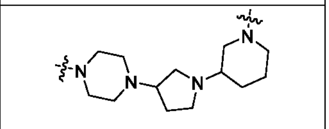 |
| 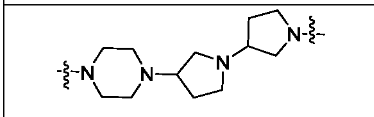 | 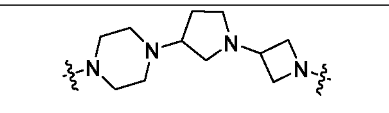 | 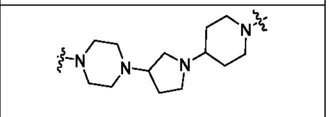 |
| 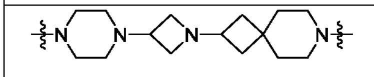 | 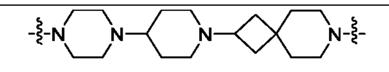 | 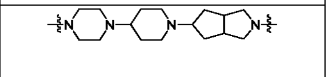 |
| 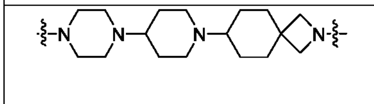 | 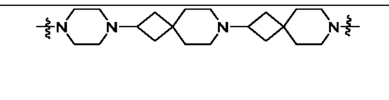 | 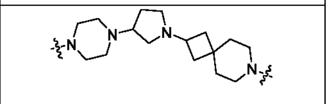 |
| 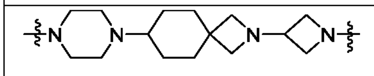 | 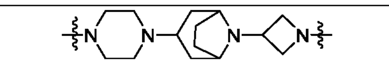 | 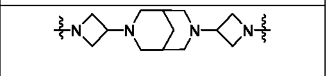 |
| 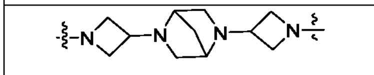 | 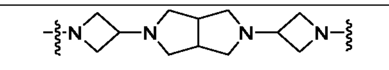 | 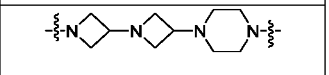 |
| 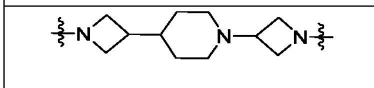 | 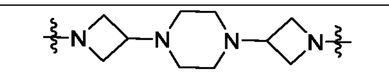 | 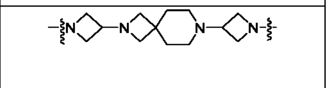 |
| 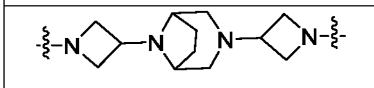 | | |
| | | |
| | | |
| | | |
| | | |

| | | |
|---|---|---|
| 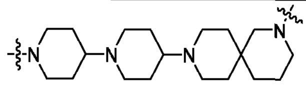 | 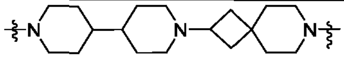 | 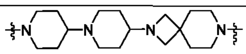 |
| 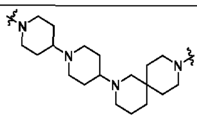 | 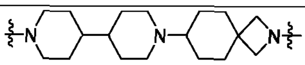 | 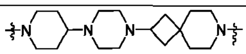 |
| 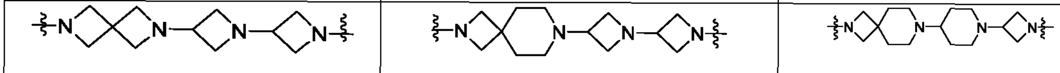 | | |
| 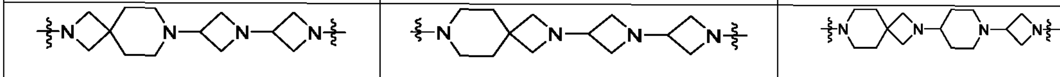 | | |
| 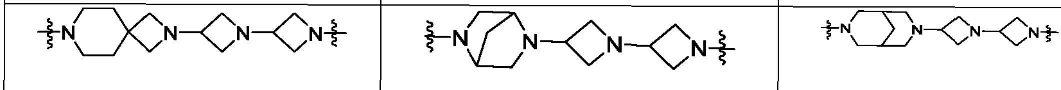 | | |
| 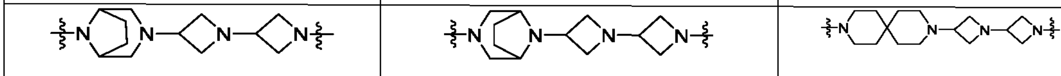 | | |
| 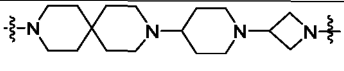 | 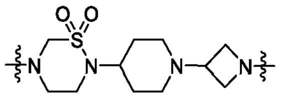 | 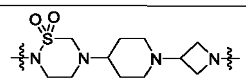 |
| 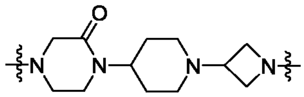 | 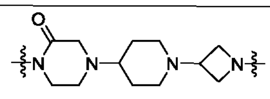 | |
| 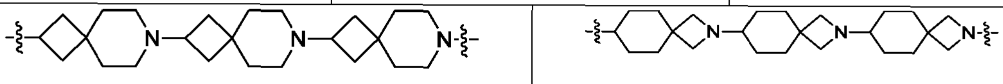 | | |
| 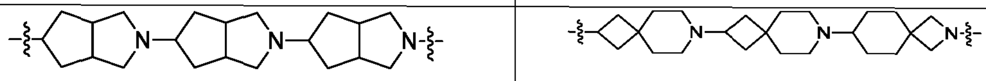 | | |
| 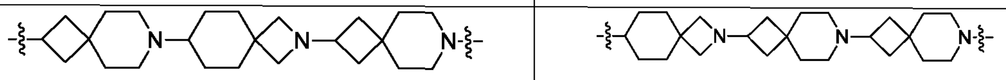 | | |
| 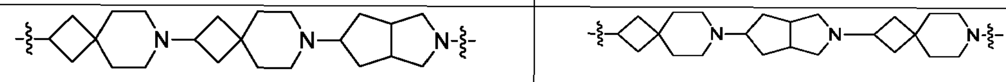 | | |

| | | | |
|---|---|---|---|
| 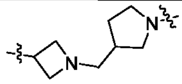 | 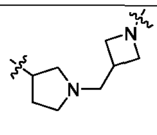 | 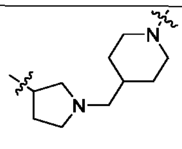 | 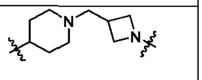 |
| 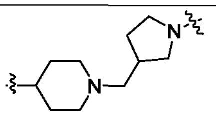 | 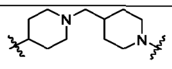 | 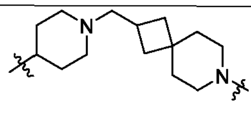 | 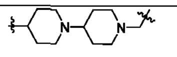 |
| 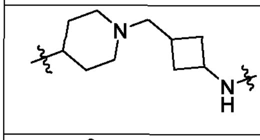 | 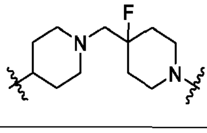 | 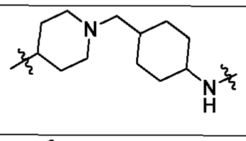 | 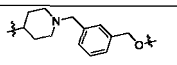 |
| 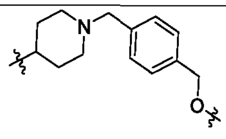 | 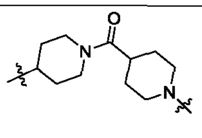 | 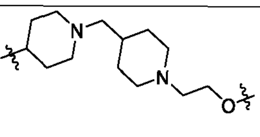 | 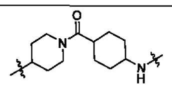 |

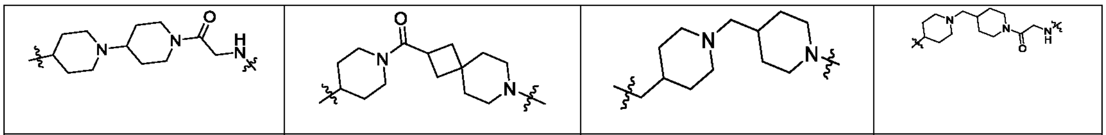

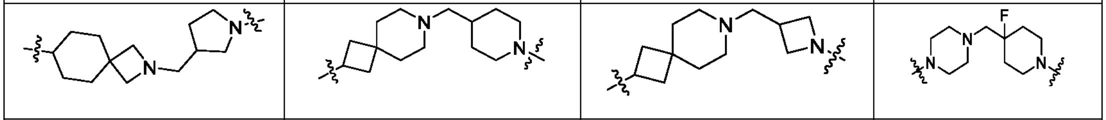

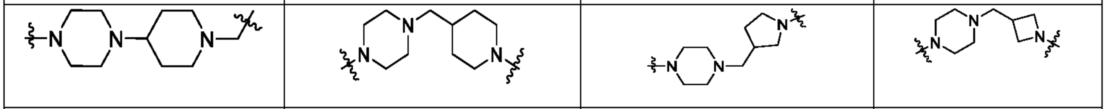

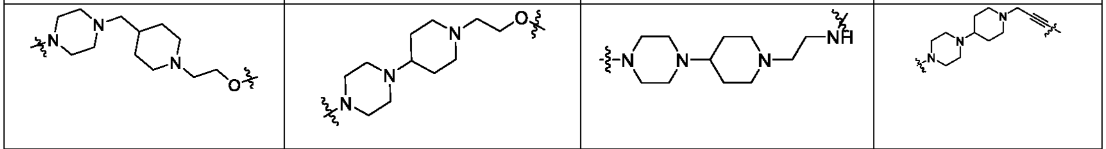

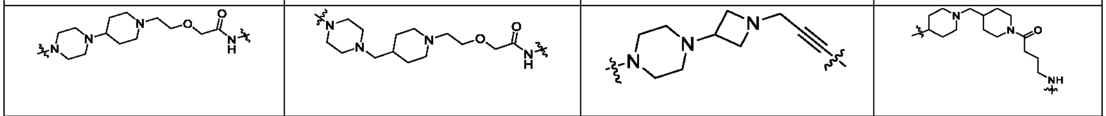

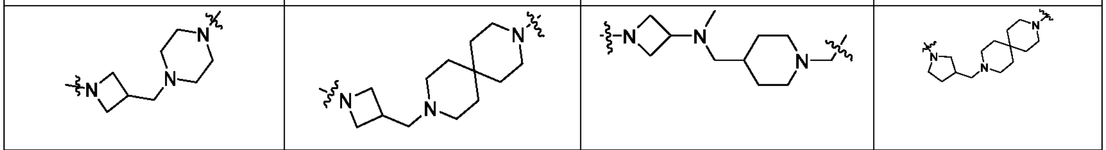

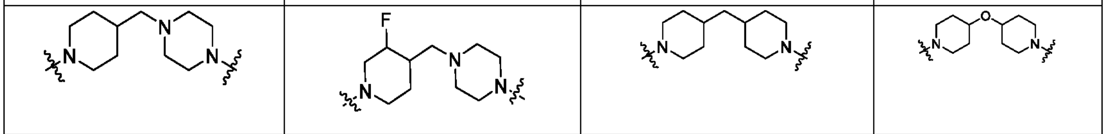

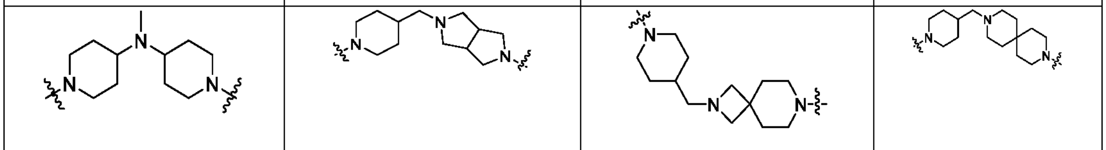

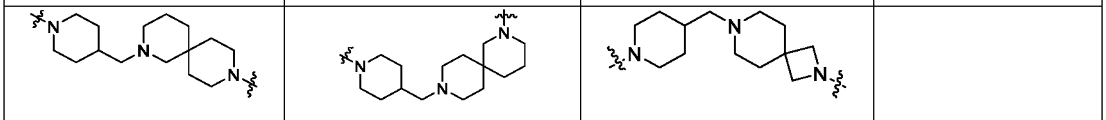

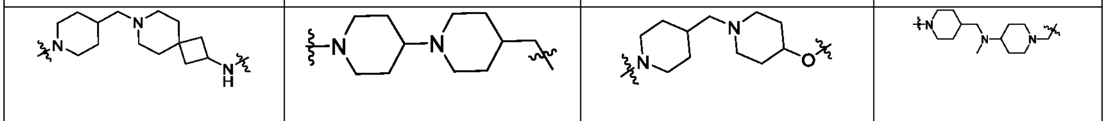

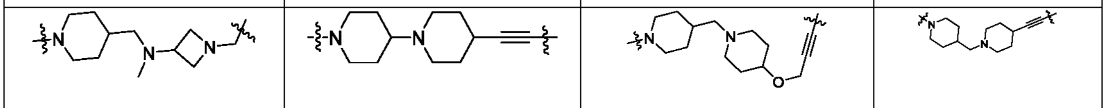

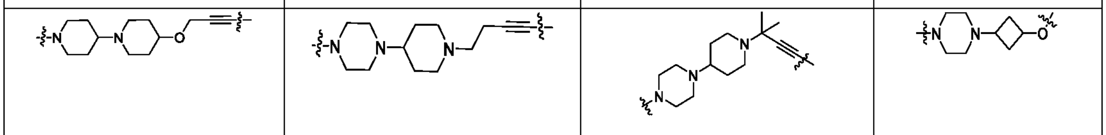

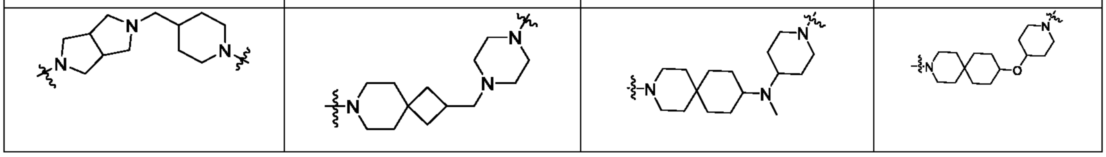

| | | | |
|---|---|---|---|
| 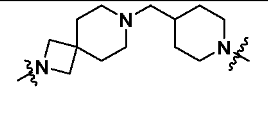 | 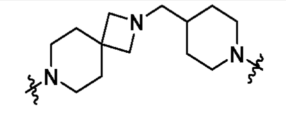 | 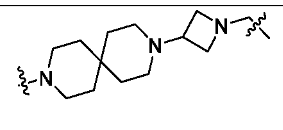 | 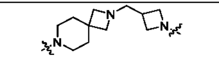 |
| 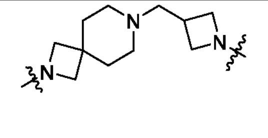 | 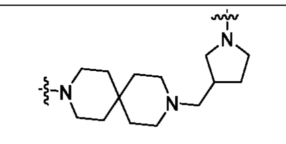 | 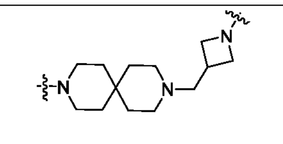 | 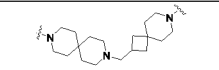 |
| 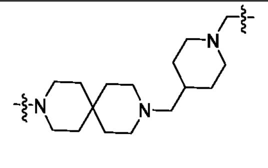 | 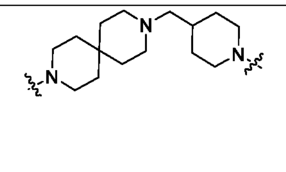 | 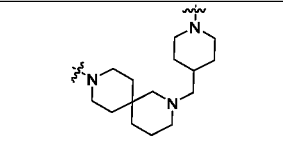 | 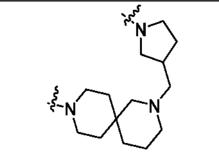 |
| 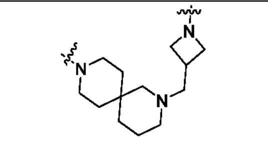 | 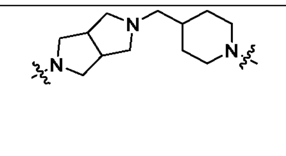 | 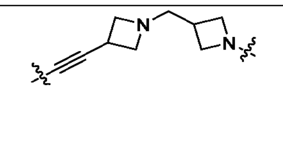 | 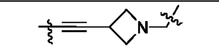 |
| 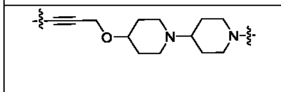 | 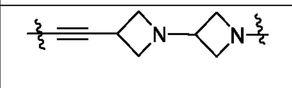 | 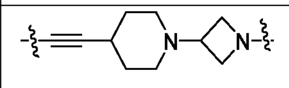 | 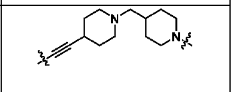 |
| 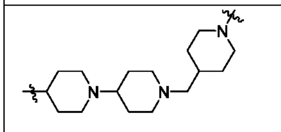 | 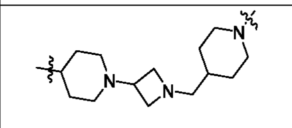 | 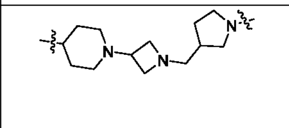 | |
| 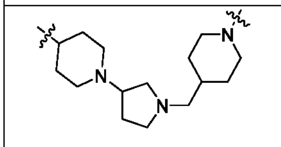 | 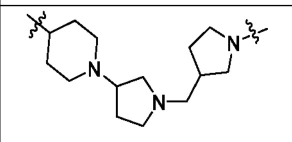 | 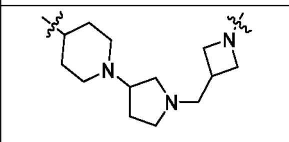 | 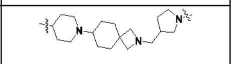 |
| 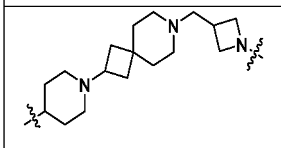 | 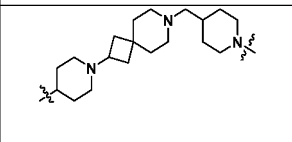 | 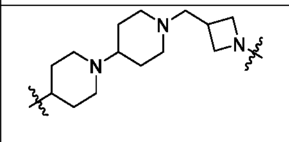 | 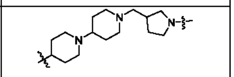 |
| 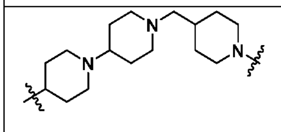 | 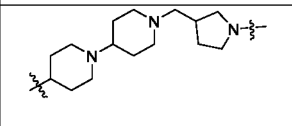 | 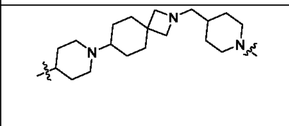 | 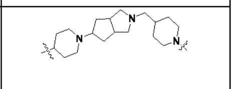 |
| 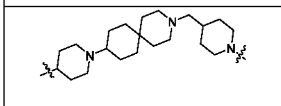 | 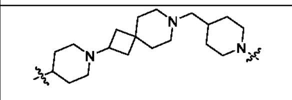 | 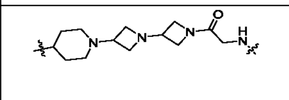 | 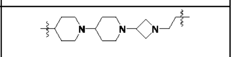 |
| 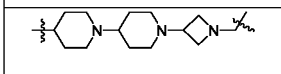 | | | |
| | | | |
| | | | |

| | | | |
|---|---|---|---|
| 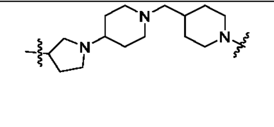 | 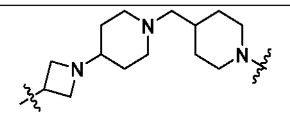 | | |
| 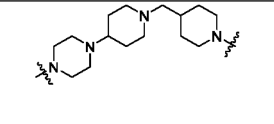 | 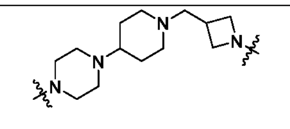 | 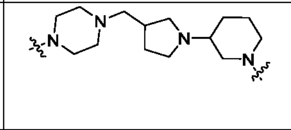 | 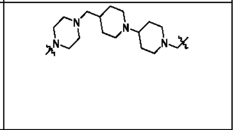 |
| 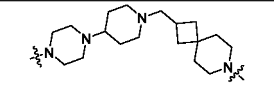 | 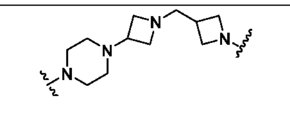 | 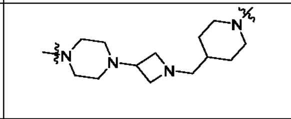 | 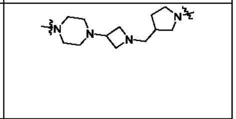 |
| 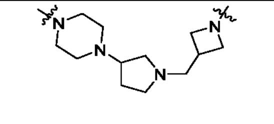 | 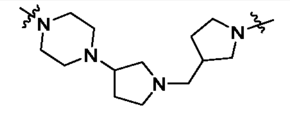 | 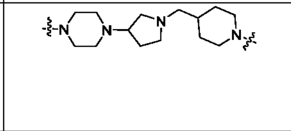 | 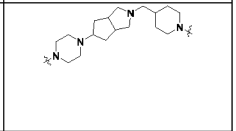 |
| 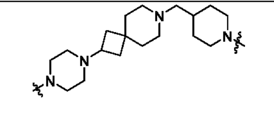 | 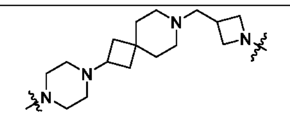 | 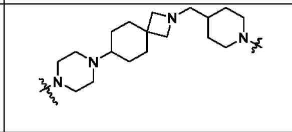 | 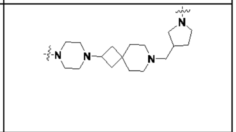 |
| 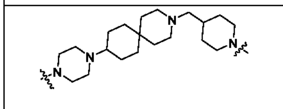 | 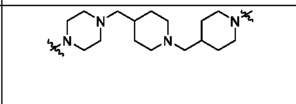 | 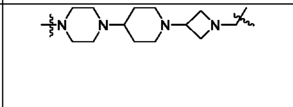 | 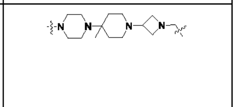 |
| 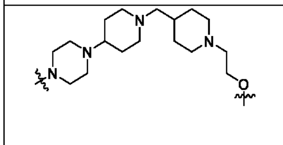 | 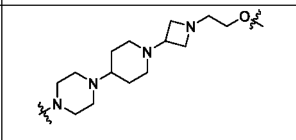 | 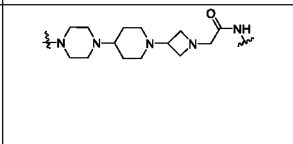 | 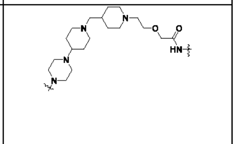 |
| 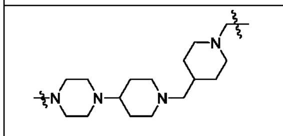 | 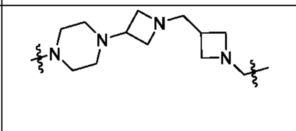 | 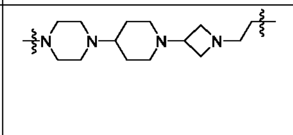 | 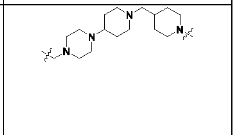 |
| 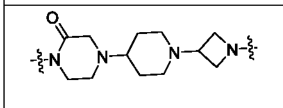 | 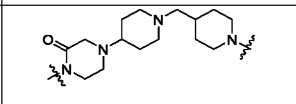 | 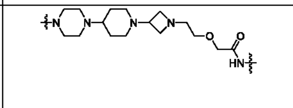 | 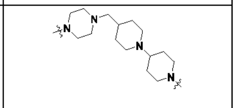 |
| 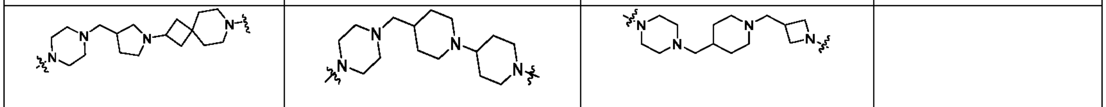 | | | |
| 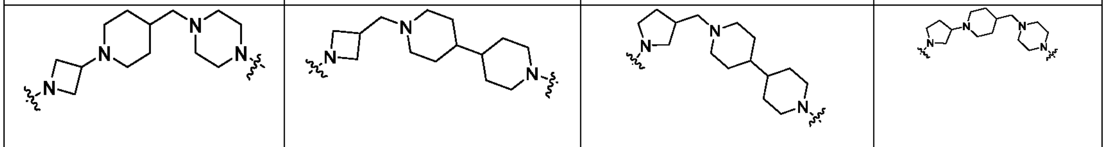 | | | |
| 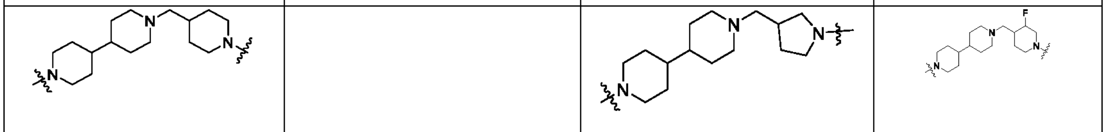 | | | |
| 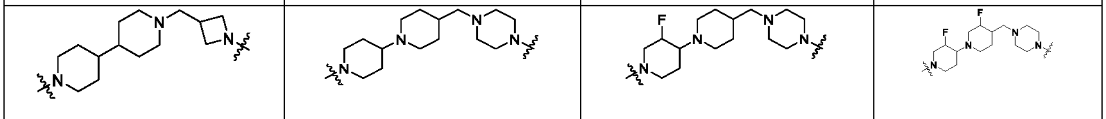 | | | |
| 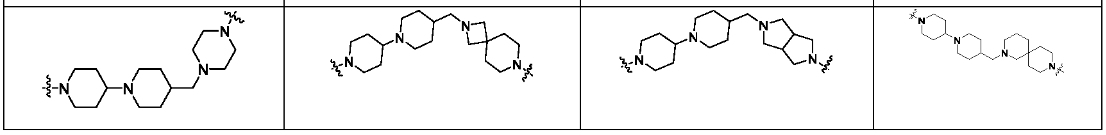 | | | |

| | | | |
|---|---|---|---|
| 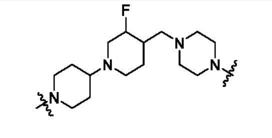 | 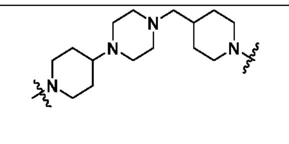 | 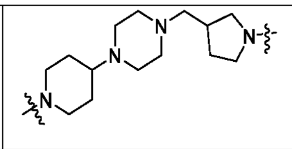 | 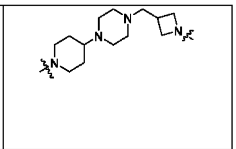 |
| 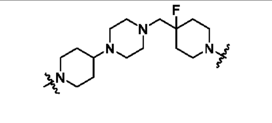 | 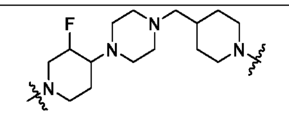 | 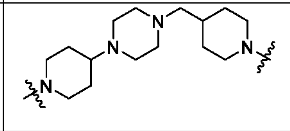 | 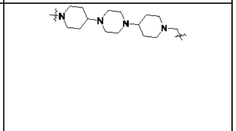 |
| 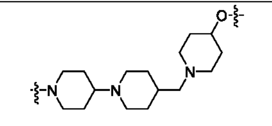 | 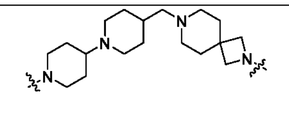 | 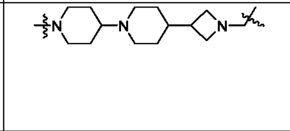 | 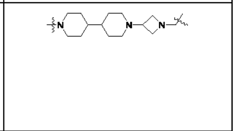 |
| 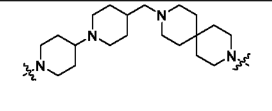 | 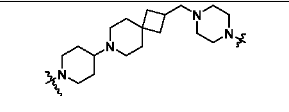 | 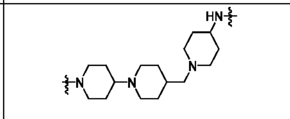 | 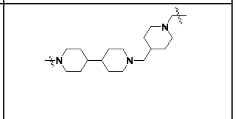 |
| 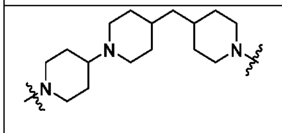 | 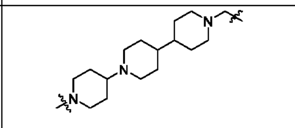 | 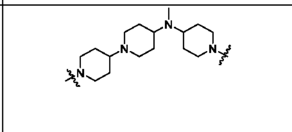 | 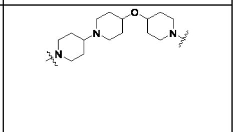 |
| 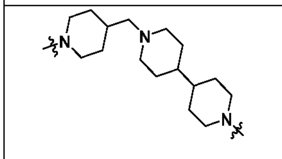 | 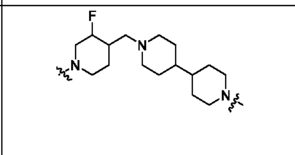 | 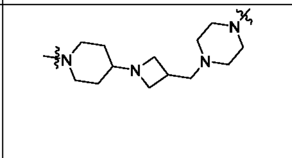 | 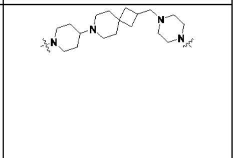 |
| 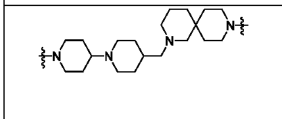 | 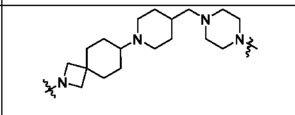 | 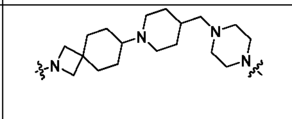 | 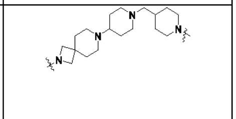 |
| 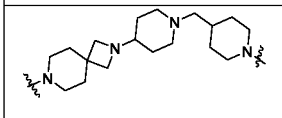 | 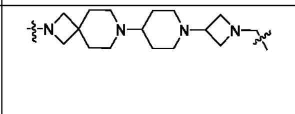 | 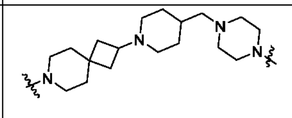 | 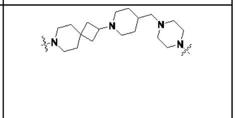 |
| 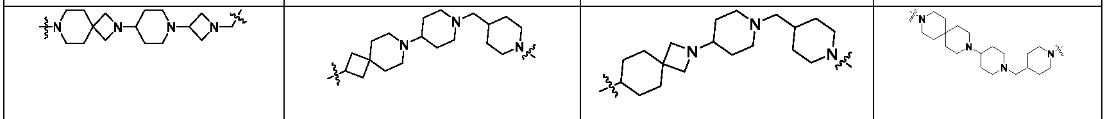 | | | |
| 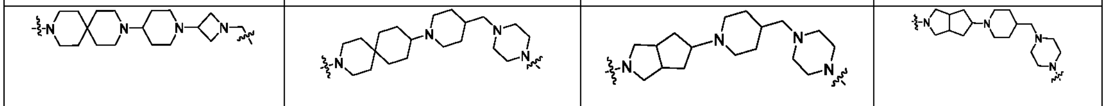 | | | |
| 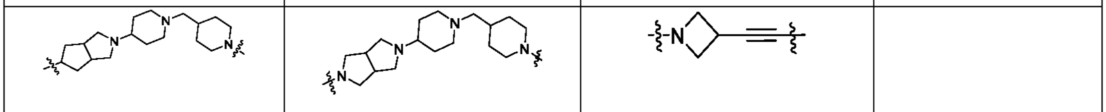 | | | |
| 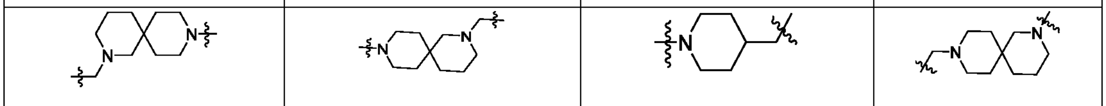 | | | |
| 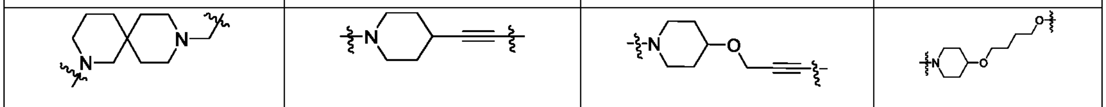 | | | |
| 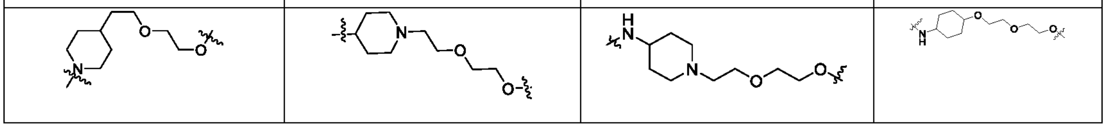 | | | |

| | | | |
|---|---|---|---|
| 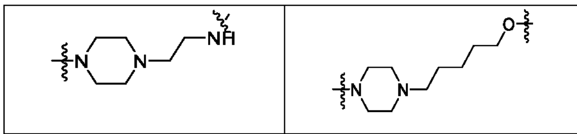 | | 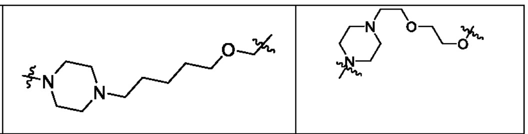 | |
| 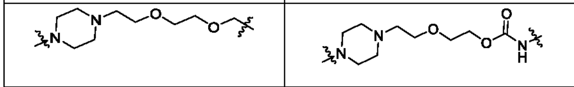 | | 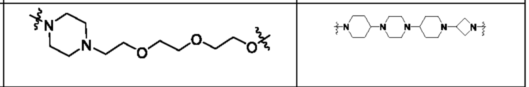 | |
| 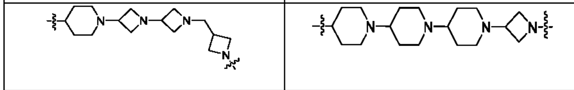 | | 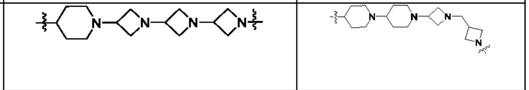 | |
| 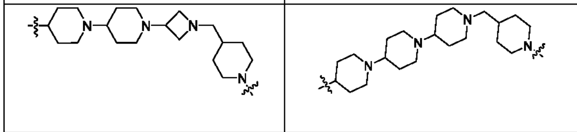 | | 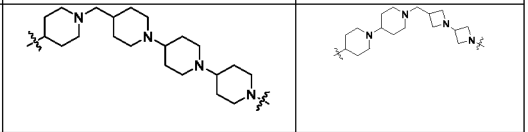 | |
| 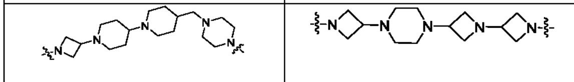 | | 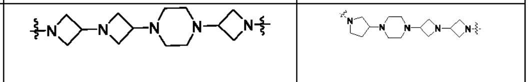 | |
| 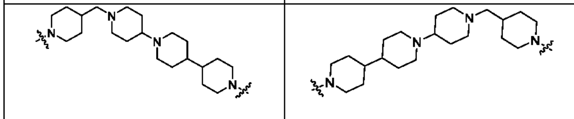 | | 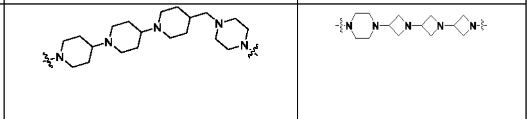 | |
| 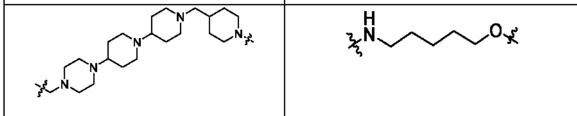 | | 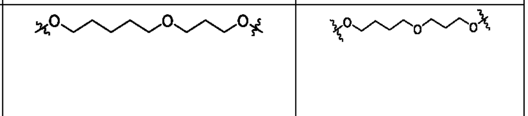 | |
| 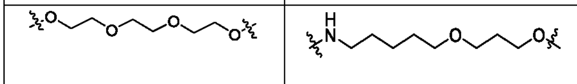 | | 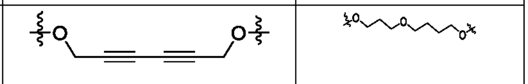 | |
| 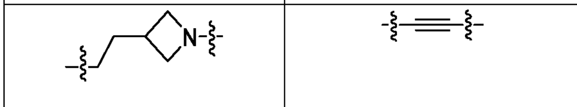 | | 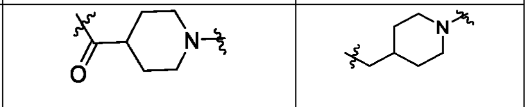 | |
| 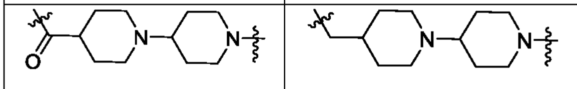 | | | |
| 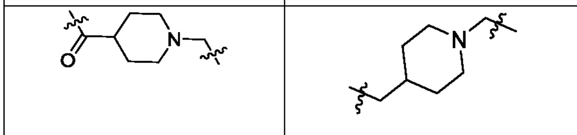 | | 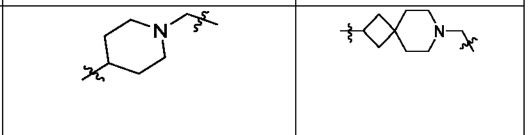 | |
| 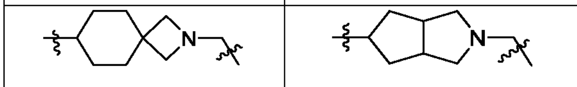 | | 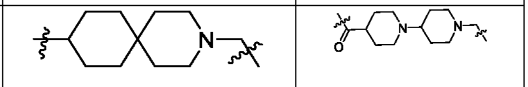 | |
| 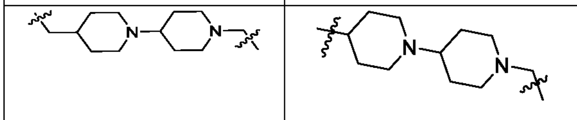 | | 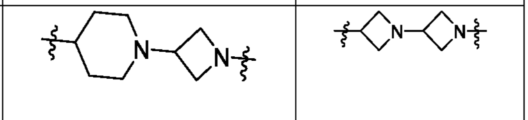 | |
| 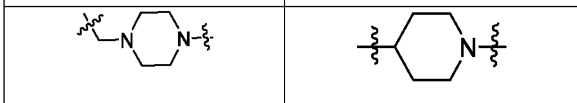 | | 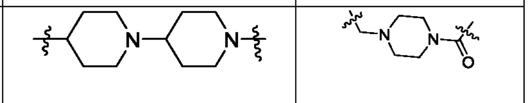 | |
| 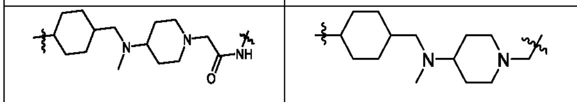 | | 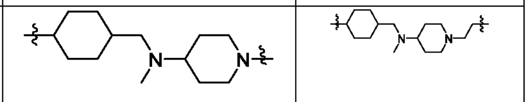 | |
| 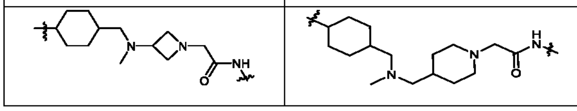 | | 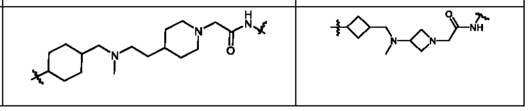 | |

| | | | |
|---|---|---|---|
| 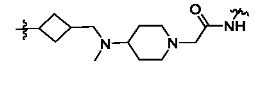 | 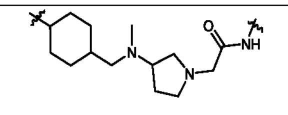 | 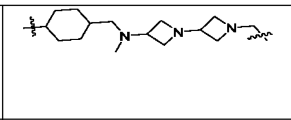 | 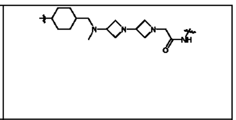 |
| 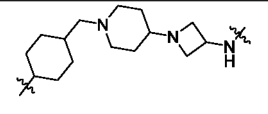 | 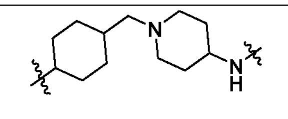 | 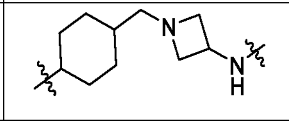 | 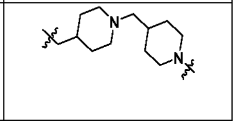 |
| 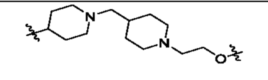 | 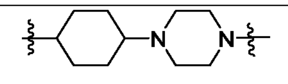 | 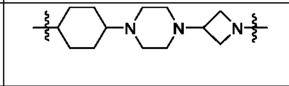 | 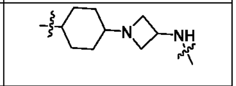 |
| 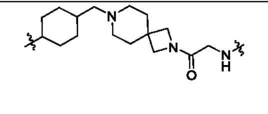 | 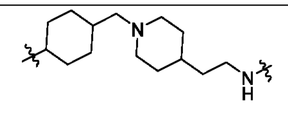 | 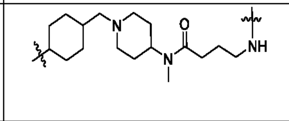 | 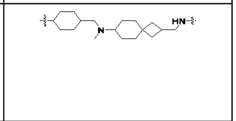 |
| 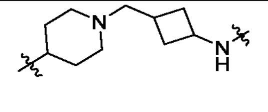 | 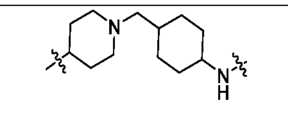 | 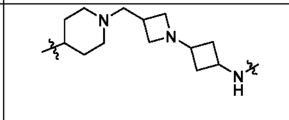 | 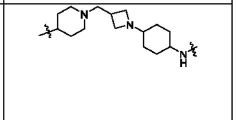 |
| 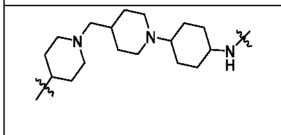 | 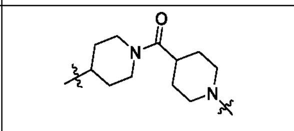 | 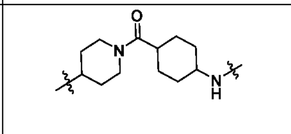 | 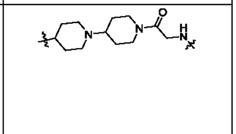 |
| 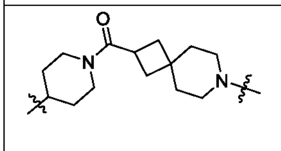 | 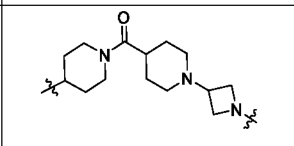 | 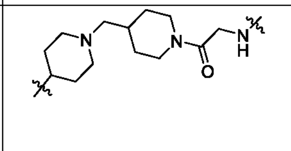 | 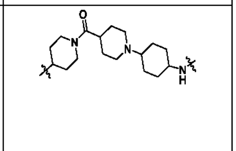 |
| 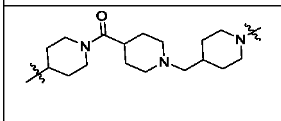 | 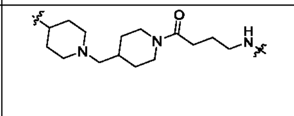 | 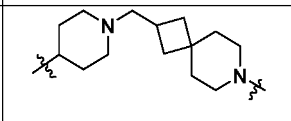 | 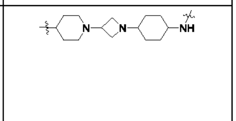 |
| 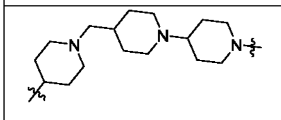 | 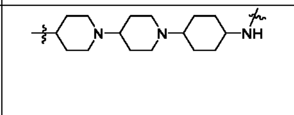 | 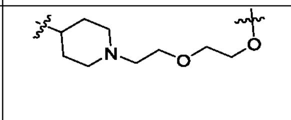 | 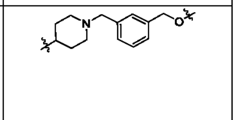 |
| 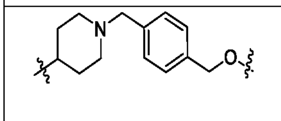 | 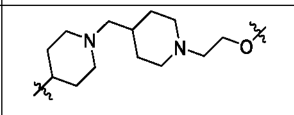 | 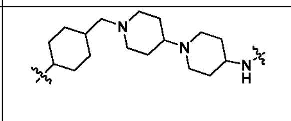 | 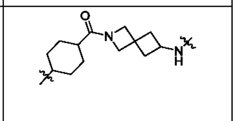 |
| | | | |
| | | | |
| | | | |

| | | | |
|---|---|---|---|
| 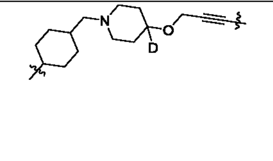 | 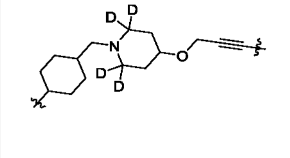 | 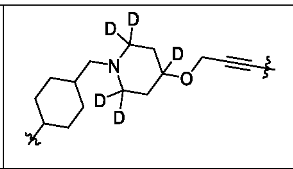 | 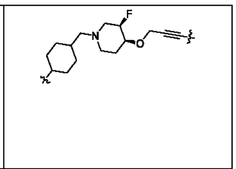 |
| 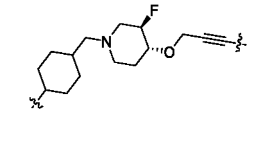 | 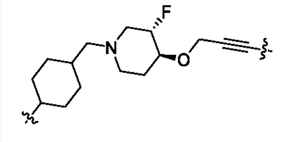 | 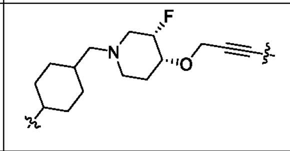 | 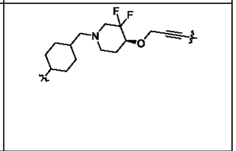 |
| 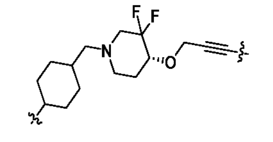 | 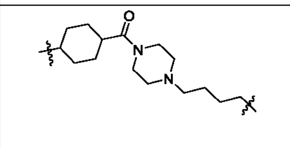 | 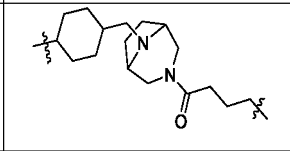 | 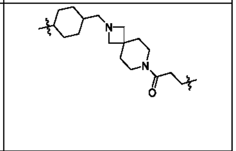 |
| 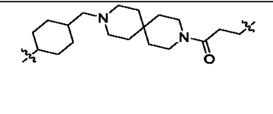 | 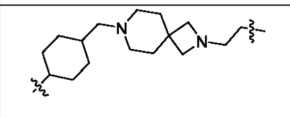 | 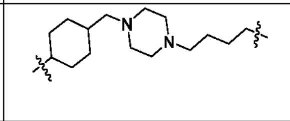 | 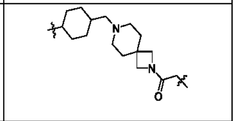 |
| 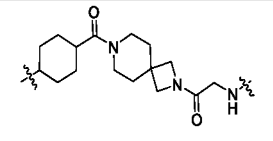 | 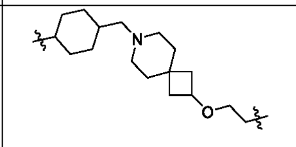 | 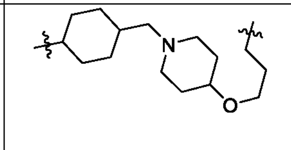 | 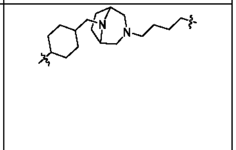 |
| 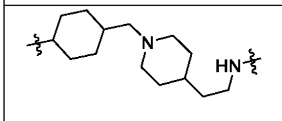 | 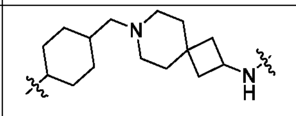 | 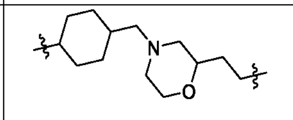 | 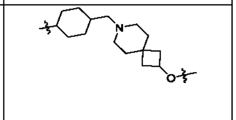 |
| 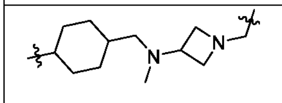 | 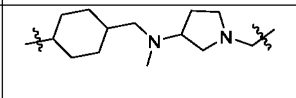 | 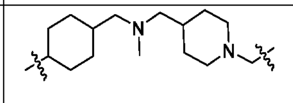 | 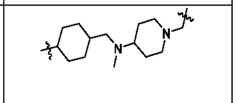 |
| 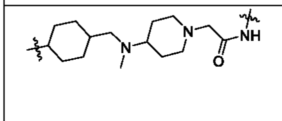 | 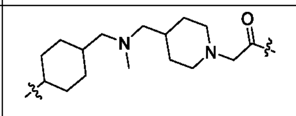 | 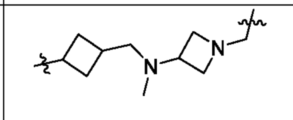 | 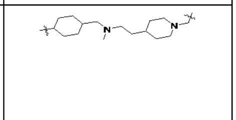 |
| 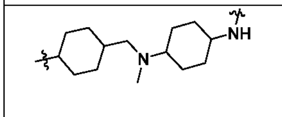 | 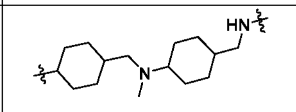 | 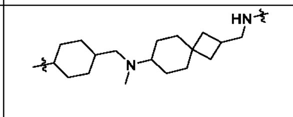 | 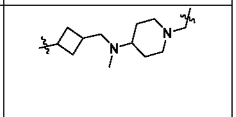 |
| 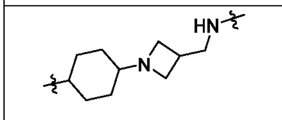 | 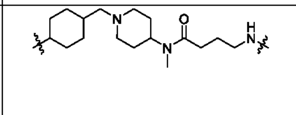 | 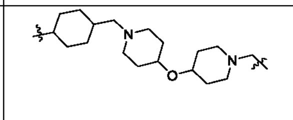 | |
| | | | |
| | | | |

;

in certain embodiments, L is selected from

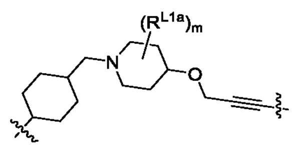

,

each $R^{L1a}$ is independently selected from halogen, CN, OH, $C_{1-4}$ alkyl, or $C_{1-4}$ alkoxy, preferably F, Cl, Br, CN, OH, methyl, ethyl, hydroxymethyl, methoxy or ethoxy, and m is selected from 0, 1, 2, 3 or 4;
in certain embodiments, L is selected from

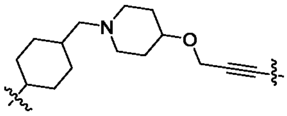, 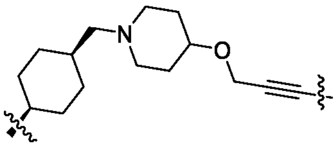,

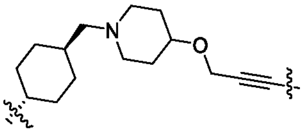, 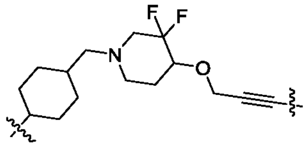, 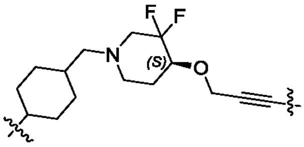,

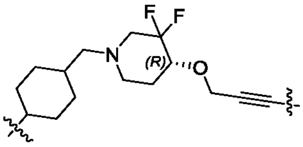, 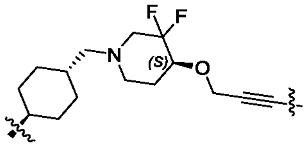, 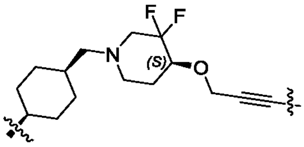,

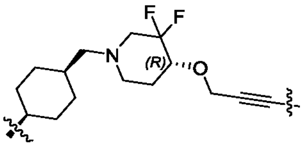, 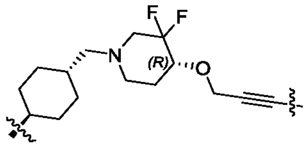, 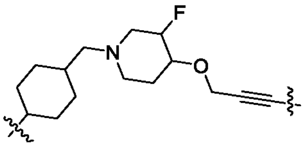,

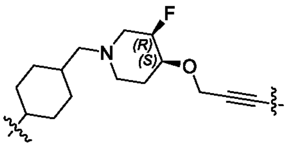, 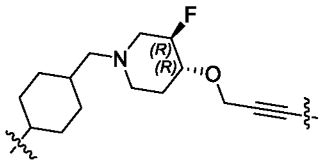, 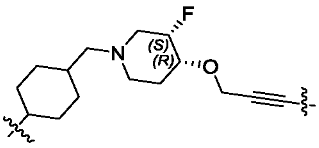,

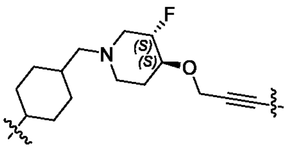, 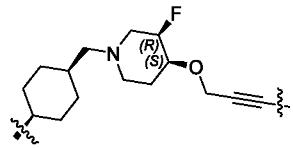, 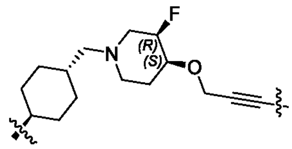,

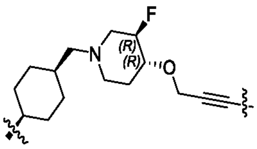, 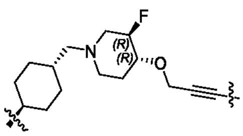, 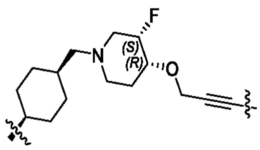, 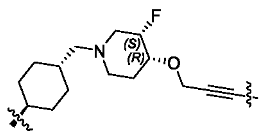,

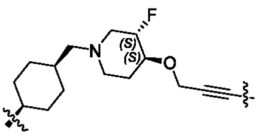 or 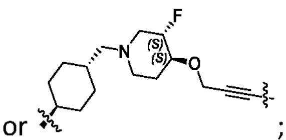;

in certain embodiments, K is selected from

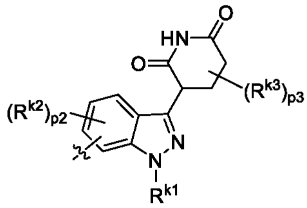

,

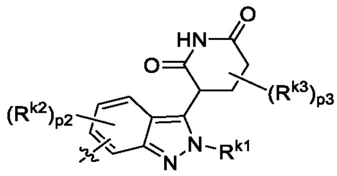

,

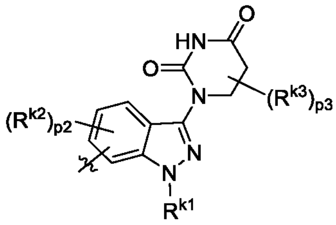

or

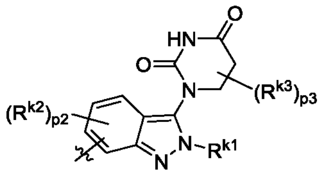

;

in certain embodiments, each $R^{k1}$ is independently selected from H, $C_{1-4}$ alkyl, $C_{2-6}$ alkenyl, $C_{2-6}$ alkynyl, $C_{3-6}$ cycloalkyl or 3- to 6-membered heterocycloalkyl, wherein the alkyl, cycloalkyl or heterocycloalkyl is optionally further substituted with 0 to 4 substituents selected from H, F, Cl, Br, I, OH, $NH_2$, CN, $CF_3$, $C_{1-6}$ alkyl, $C_{1-6}$ alkoxy, $C_{2-6}$ alkenyl, $C_{2-6}$ alkynyl or $C_{3-6}$cycloalkyl;

in certain embodiments, each $R^{k1}$ is independently selected from H, methyl, ethyl, propyl, isopropyl, ethenyl, propenyl, allyl, ethynyl, propynyl, propargyl, cyclopropyl, cyclobutyl, cyclopentyl, cyclohexyl, azetidinyl, azacyclopentyl, piperidyl, oxacyclobutyl, oxacyclopentyl or oxacyclohexyl, wherein the methyl, ethyl, propyl, isopropyl, cyclopropyl, cyclobutyl, cyclopentyl, cyclohexyl, azetidinyl, azacyclopentyl, piperidyl, oxacyclobutyl, oxacyclopentyl or oxacyclohexyl is optionally further substituted with 0 to 4 substituents selected from H, F, Cl, Br, I, OH, CN, $CF_3$, $C_{1-4}$ alkyl, $C_{1-4}$ alkoxy, ethenyl, propenyl, allyl, ethynyl, propynyl, propargyl or $C_{3-6}$ cycloalkyl;

each $R^{k1}$ is independently selected from H, methyl, ethyl, isopropyl, cyclopropyl, oxacyclobutyl, oxacyclohexyl, $-CH_2CF_3$, $-CH(CH_3)CF_3$, $-CH(CH_3)$-cyclopropyl, $-CH_2$-cyclopropyl, $-CH_2$-ethenyl, $-CH_2$-ethynyl, $-CH_2CH_2$-methoxy, or $-CD_3$;

in certain embodiments, $R^{k2}$ and $R^{k3}$ are each independently selected from H, F, Cl, Br, I, OH, =O, $NH_2$, $CF_3$, CN, C(=O)OH, C(=O)$NH_2$, $C_{1-4}$ alkyl or $C_{1-4}$ alkoxy, wherein the alkyl or alkoxy is optionally further substituted with 0 to 4 substituents selected from H, F, Cl, Br, I, OH, or $NH_2$;

or two $R^{k3}$ together with the carbon atoms or ring backbones to which they are directly attached form 3- to 6-membered carbocycle or 3- to 7-membered heterocycle, wherein the carbocycle or heterocycle is optionally further substituted with 0 to 4 substituents selected from H, F, Cl, Br, I, OH, =O, $NH_2$, CN, C(=O)OH, C(=O)$NH_2$, $C_{1-4}$ alkyl or $C_{1-4}$ alkoxy, and the heterocycle contains 1 to 4 heteroatoms selected from O, S or N;

in certain embodiments, $R^{k2}$ and $R^{k3}$ are each independently selected from H, F, Cl, Br, I, OH, =O, $NH_2$, $CF_3$, CN, C(=O)OH, C(=O)$NH_2$, methyl, ethyl, methoxy or ethoxy, wherein the methyl, ethyl, methoxy or ethoxy is optionally further substituted with 0 to 4 substituents selected from H, F, Cl, Br, I, OH or $NH_2$;

in certain embodiments, $R^{k2}$ and $R^{k3}$ are each independently selected from H or F;

in certain embodiments, K is selected from

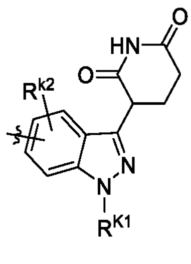

;

in certain embodiments, K is selected from

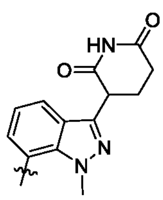 , 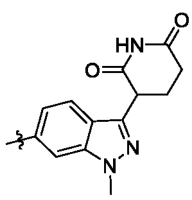 , 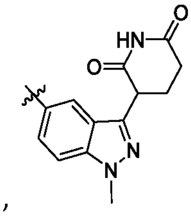 ,

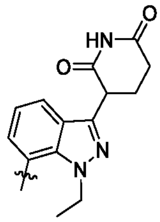 , 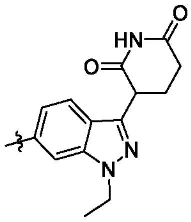 , 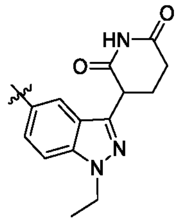 , 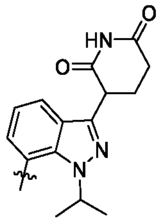 , 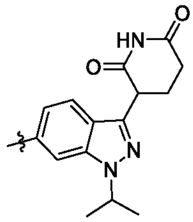 , 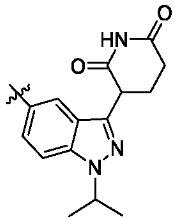 ,

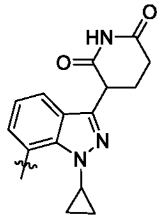 , 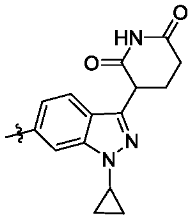 , 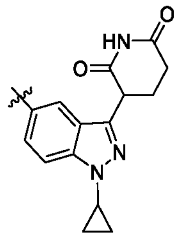 , 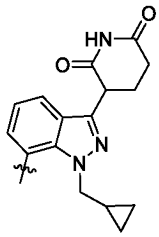 , 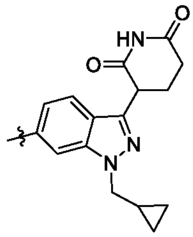 , 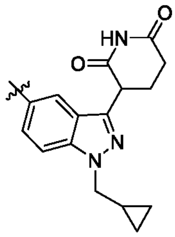 ,

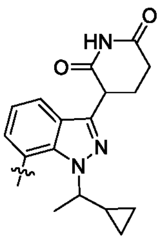 , 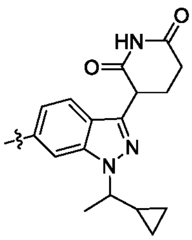 , 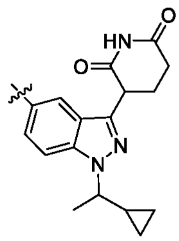 , 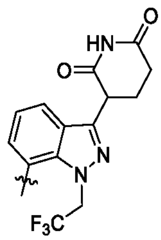 , 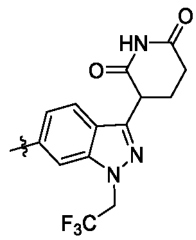 , 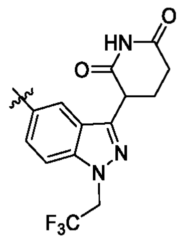 ,

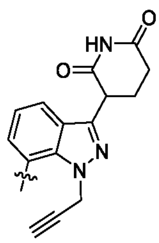 , 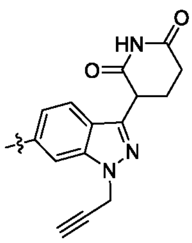 , 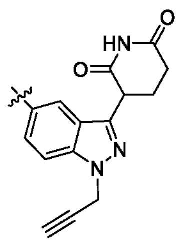 , 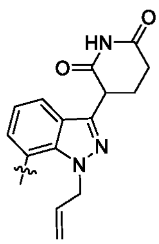 , 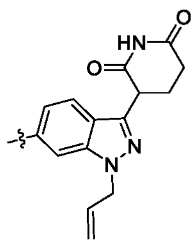 , 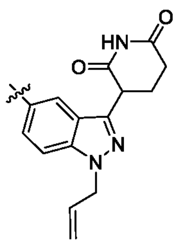 ,

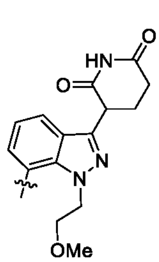 , 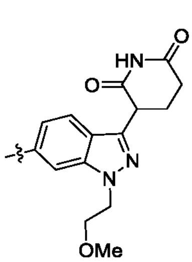 , 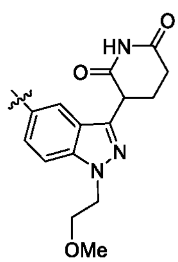 , 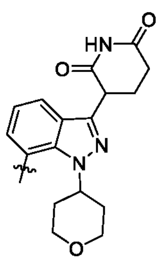 , 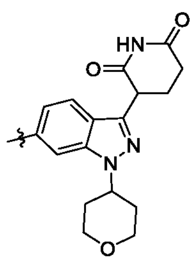 , 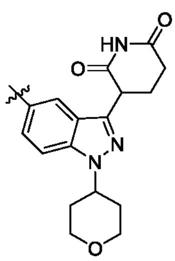 ,

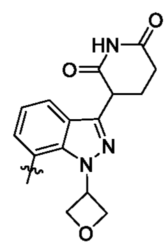, 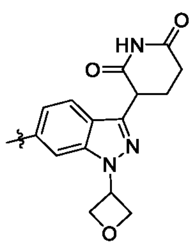, 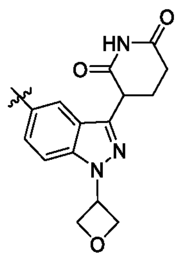, 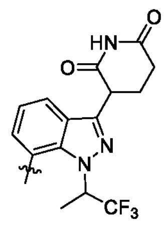, 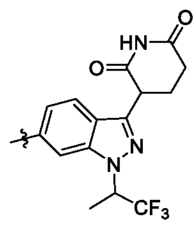, 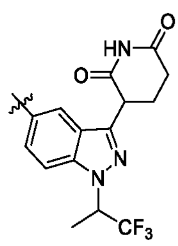,

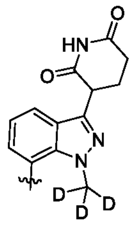, 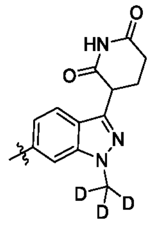, 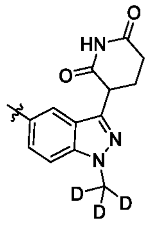, 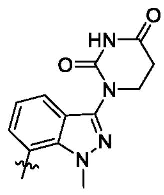, 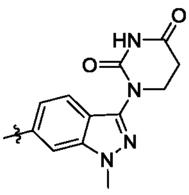, 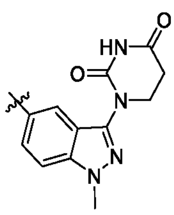,

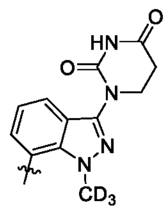, 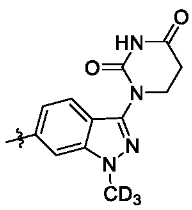, 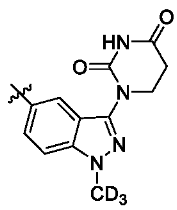, 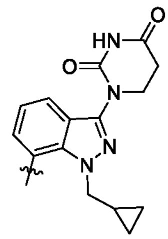, 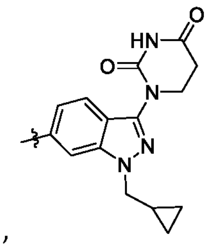, 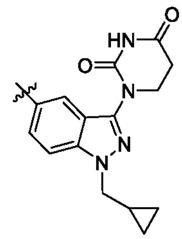,

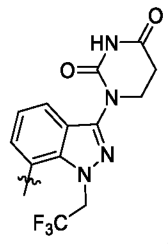, 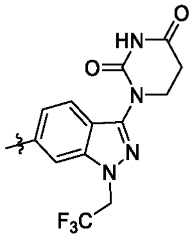, 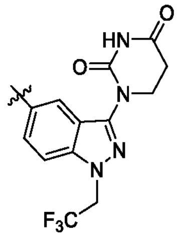, 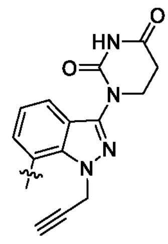, 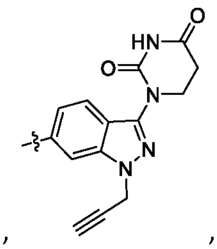, 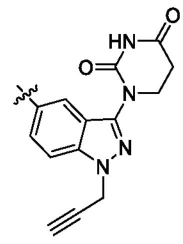,

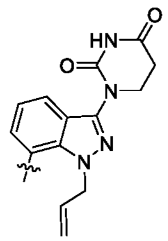, 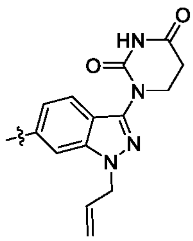, 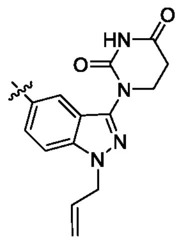, 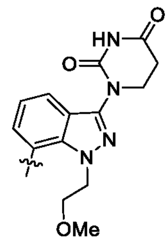, 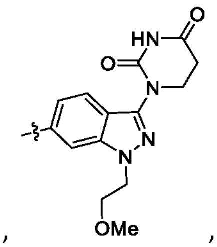, 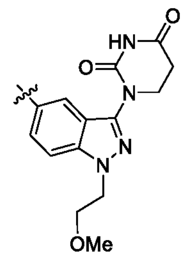,

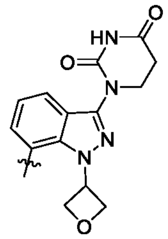, 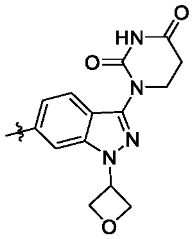, 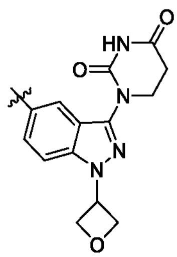, 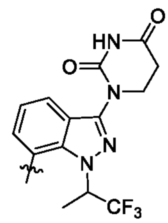, 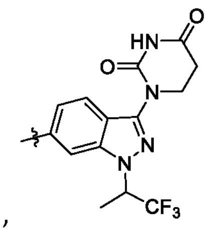, 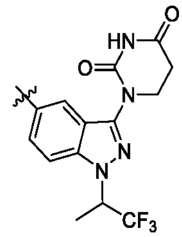,

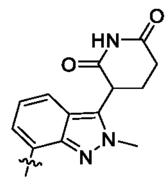, 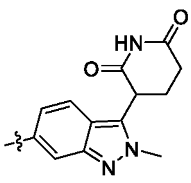, 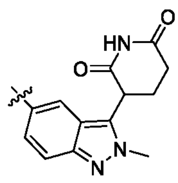, 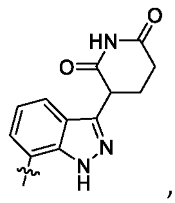, 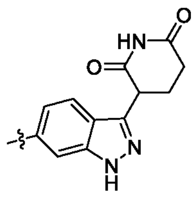, 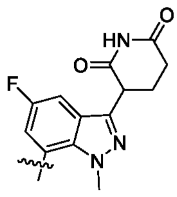

or

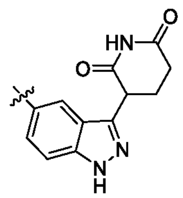;

in certain embodiments, 0 to 50 H of the compound represented by general formula (I) are optionally replaced by 0 to 50 D;
in certain embodiments, 0 to 30 H of the compound represented by general formula (I) are optionally replaced by 0 to 30 D;
in certain embodiments, 0 to 20 H of the compound represented by general formula (I) are optionally replaced by 0 to 20 D;
in certain embodiments, 0 to 20 H of L are optionally replaced by 0 to 20 D;
in certain embodiments, 0 to 10 H of the compound represented by general formula (I) are optionally replaced by 0 to 10 D;
in certain embodiments, 0 to 10 H of -L-K are optionally replaced by 0 to 10 D;
in certain embodiments, 0 to 10 H of L are optionally replaced by 0 to 10 D;
in certain embodiments, 0 to 10 H of K are optionally replaced by 0 to 10 D;
in certain embodiments, 0 to 20 H of L are optionally replaced by 0, 1, 2, 3, 4, 5, 6, 7, 8, 9, 10, 11, 12, 13, 14, 15, 16, 17, 18, 19, or 20 D;
in certain embodiments, 0 to 10 H of L are optionally replaced by 0, 1, 2, 3, 4, 5, 6, 7, 8, 9, or 10 D;
in certain embodiments, 0 to 10 H of K are optionally replaced by 0, 1, 2, 3, 4, 5, 6, 7, 8, 9, or 10 D;
in certain embodiments, 0 to 10 H of -L-K are optionally replaced by 0, 1, 2, 3, 4, 5, 6, 7, 8, 9, or 10 D;
in certain embodiments, n is selected from 0, 1, 2, 3 or 4;
in certain embodiments, q is selected from 0, 1, 2, 3 or 4;
in certain embodiments, n1, n2 and n6 are each independently selected from 0, 1, 2 or 3;
in certain embodiments, p2 and p3 are each independently selected from 0, 1, 2, 3 or 4;
in certain embodiments, p2 or p3 are each independently selected from 0, 1 or 2.

As a first embodiment of the present invention, the above-mentioned compound represented by general formula (I) or the stereoisomer, deuterated compound, solvate, prodrug, metabolite, pharmaceutically acceptable salt or eutectic crystal thereof, wherein

L is selected from -Ak1-Cy1-Ak2-Cy2-Ak3-Cy3-Ak4-Cy4-Ak5-;
Ak1, Ak2, Ak3, Ak4 and Ak5 are each independently selected from $-(CH_2)_q-$, O, $-(CH_2)_qNR^L-$, NR‘C=O, $C{=}ONR^L$, C=O, $-R^LC{=}CR^L-$, C≡C or a bond;
$R^L$ is selected from H or $C_{1-6}$ alkyl;
Cy1, Cy2, Cy3 and Cy4 are each independently selected from a bond, 4- to 7-membered mono-heterocyclic ring, 4- to 10-membered fused-heterocyclic ring, 5- to 12-membered spiro-heterocyclic ring, 7- to 10-membered bridged-heterocyclic ring, 3- to 7-membered monocycloalkyl, 4- to 10-membered fused cycloalkyl, 5- to 12-membered spiro cycloalkyl, 7- to 10-membered bridged cycloalkyl, 5- to 10-membered heteroaryl or 6- to 10-membered aryl, wherein the aryl, heteroaryl, cycloalkyl, mono-heterocyclic ring, fused-heterocyclic ring, spiro-heterocyclic ring or bridged-heterocyclic ring is optionally further substituted with 0 to 4 substituents selected from H, F, Cl, Br, I, OH, C(=O)OH, CN, $NH_2$, =O, $C_{1-4}$ alkyl, halogen-substituted $C_{1-4}$ alkyl, hydroxyl-substituted $C_{1-4}$ alkyl or $C_{1-4}$ alkoxy, and the heteroaryl, mono-heterocyclic ring, fused-heterocyclic ring, spiro-heterocyclic ring or bridged-heterocyclic ring contains 1 to 4 heteroatoms selected from O, S, or N;
B is selected from

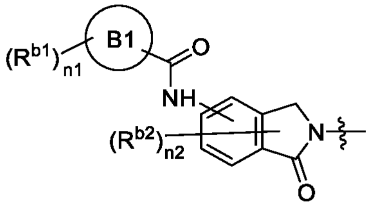 or 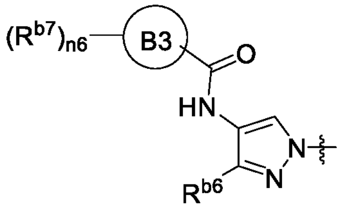 ;

B1 and B3 are each independently selected from $C_{6-10}$ aryl, 5- to 10-membered heteroaryl or 4-to 10-membered heterocyclyl, wherein the heteroaryl or heterocyclyl contains 1 to 4 heteroatoms selected from O, S or N;

$R^{b1}$ and $R^{b7}$ are each independently selected from H, F, Cl, Br, I, =O, OH, $NH_2$, CN, $CF_3$, C(=O)OH, $CHF_2$, $C_{1-4}$ alkyl, $C_{1-4}$ alkoxy, $C_{3-6}$ cycloalkyl, $-(CH_2)_n-R^{b21}$, $-OR^{b21}$, $-N(R^{b21})_2$, $C_{6-10}$ aryl, 5- to 10-membered heteroaryl or 4- to 10-membered heterocyclyl, wherein the alkyl, alkoxy, cycloalkyl, heterocyclyl, aryl or heteroaryl is optionally further substituted with 0 to 4 substituents selected from H, F, Cl, Br, I, OH, =O, $-N(R^{b21})_2$, CN, $CF_3$, C(=O)OH, $C_{1-4}$ alkyl, $C_{1-4}$ alkoxy, $C_{3-6}$ cycloalkyl, 5- to 10-membered heteroaryl, 4- to 10-membered heterocyclyl or $R^{b7a}$, and the heteroaryl or heterocyclyl contains 1 to 4 heteroatoms selected from O, S, or N;

$R^{b7a}$ is selected from $C_{1-4}$ alkyl, $-C_{3-6}$ cycloalkyl, 4- to 10-membered heterocyclyl, $-C_{1-4}$ alkylene $-C_{3-6}$ cycloalkyl, $-C_{1-4}$ alkylene 4- to 10-membered heterocyclyl, $-O-C_{3-6}$ cycloalkyl, -O-4- to 10-membered heterocyclyl, $-NH-C_{3-6}$ cycloalkyl, -NH-4- to 10-membered heterocyclyl, $-N(C_{1-4}$ alkyl$)-C_{3-6}$ cycloalkyl or $-N(C_{1-4}$ alkyl)-4- to 10-membered heterocyclyl, wherein the $R^{b7a}$ is optionally substituted with 1 to 4 substituents selected from H, F, Cl, Br, I, OH, =O, $-N(R^{b21})_2$, CN, $CF_3$, C(=O)OH, $C_{1-4}$ alkyl, $C_{1-4}$ alkoxy, $C_{3-6}$ cycloalkyl or 4- to 10-membered heterocyclyl, and the heterocyclyl contains 1 to 4 heteroatoms selected from O, S, or N;

$R^{b2}$ and $R^{b6}$ are each independently selected from H, F, Cl, Br, I, =O, OH, $-C(=O)N(R^{b21})_2$, $-N(R^{b21})_2$, CN, $CF_3$, C(=O)OH, $CHF_2$, $C_{1-4}$ alkyl, $C_{1-4}$ alkoxy, $C_{3-6}$ cycloalkyl, $-(CH_2)_n-R^{b21}$, $-OR^{b21}$, $C_{6-10}$ aryl, 5- to 10-membered heteroaryl or 4- to 10-membered heterocyclyl, wherein the alkyl, alkoxy, cycloalkyl, aryl, heteroaryl or heterocyclyl is optionally further substituted with 0 to 4 substituents selected from H, F, Cl, Br, I, OH, =O, $NH_2$, CN, $CF_3$, C(=O)OH, $C_{1-4}$ alkyl, $C_{1-4}$ alkoxy, $C_{3-6}$ cycloalkyl, 5- to 10-membered heteroaryl or 4- to 10-membered heterocyclyl, and the heteroaryl or heterocyclyl contains 1 to 4 heteroatoms selected from O, S, or N;

each $R^{b21}$ is independently selected from H, $C_{1-6}$ alkyl, $C_{1-4}$ alkoxy, $C_{3-6}$ cycloalkyl, $C_{6-10}$ aryl, 5- to 10-membered heteroaryl or 4- to 10-membered heterocyclyl, wherein the alkyl, alkoxy, cycloalkyl, aryl, heteroaryl or heterocyclyl is optionally further substituted with 0 to 4 substituents selected from H, F, Cl, Br, I, OH, =O, $NH_2$, CN, $CF_3$, C(=O)OH, $C_{1-4}$ alkyl, $C_{3-6}$ cycloalkyl or $C_{1-4}$ alkoxy, and the heteroaryl or heterocyclyl contains 1 to 4 heteroatoms selected from O, S, or N;

n is selected from 0, 1, 2, 3 or 4;

K is selected from

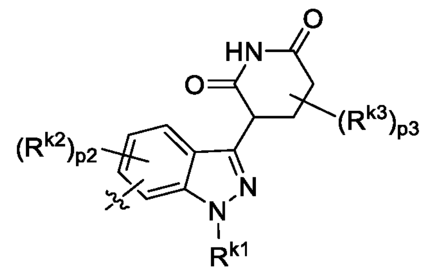 , 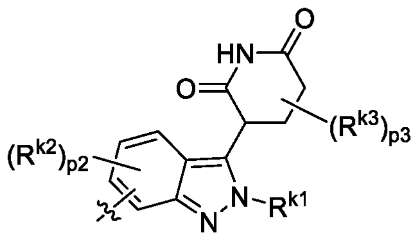 ,

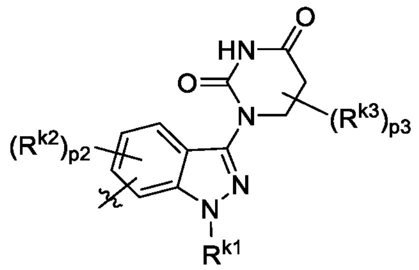 or 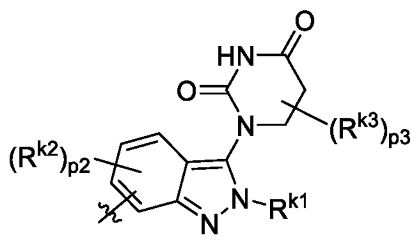 ;

each $R^{k1}$ is independently selected from H, $C_{1-4}$ alkyl, $C_{2-6}$ alkenyl, $C_{2-6}$ alkynyl, $C_{3-6}$ cycloalkyl or 3-to 6-membered heterocycloalkyl, wherein the alkyl, cycloalkyl or heterocycloalkyl is optionally further substituted with 0 to 4

substituents selected from H, F, Cl, Br, I, OH, $NH_2$, CN, $CF_3$, $C_{1-6}$ alkyl, $C_{1-6}$ alkoxy, $C_{2-6}$ alkenyl, $C_{2-6}$ alkynyl or $C_{3-6}$cycloalkyl; ;

$R^{k2}$ and $R^{k3}$ are each independently selected from H, F, Cl, Br, I, OH, =O, $NH_2$, $CF_3$, CN, C(=O)OH, C(=O)$NH_2$, $C_{1-4}$ alkyl or $C_{1-4}$ alkoxy, wherein the alkyl or alkoxy is optionally further substituted with 0 to 4 substituents selected from H, F, Cl, Br, I, OH, or $NH_2$;

or two $R^{k3}$ together with the carbon atoms or ring backbones to which they are directly attached form 3- to 6-membered carbocycle or 3- to 7-membered heterocycle, wherein the carbocycle or heterocycle is optionally further substituted with 0 to 4 substituents selected from H, F, Cl, Br, I, OH, =O, $NH_2$, CN, C(=O)OH, C(=O)$NH_2$, $C_{1-4}$ alkyl or $C_{1-4}$ alkoxy, and the heterocycle contains 1 to 4 heteroatoms selected from O, S or N;

q is selected from 0, 1, 2, 3 or 4;

n1, n2 and n6 are each independently selected from 0, 1, 2 or 3;

p2 and p3 are each independently selected from 0, 1, 2, 3 or 4;

optionally, 0 to 50 H of the compound represented by general formula (I) are replaced by 0 to 50 D.

As a second embodiment of the present invention, the above-mentioned compound represented by general formula (I) or the stereoisomer, deuterated compound, solvate, prodrug, metabolite, pharmaceutically acceptable salt or eutectic crystal thereof, wherein Cy1, Cy2, Cy3 and Cy4 are each independently selected from a bond, 4- to 7-membered nitrogen-containing mono-heterocyclic ring, 4- to 10-membered nitrogen-containing fused-heterocyclic ring, 5- to 12-membered nitrogen-containing spiro-heterocyclic ring, 7- to 10-membered nitrogen-containing bridged-heterocyclic ring, 3- to 7-membered monocycloalkyl, 4-to 10-membered fused cycloalkyl, 5- to 12-membered spiro cycloalkyl, 7- to 10-membered bridged cycloalkyl, 5- to 10-membered heteroaryl or 6- to 10-membered aryl, wherein the mono-heterocyclic ring, fused-heterocyclic ring, bridged-heterocyclic ring, spiro-heterocyclic ring, cycloalkyl, aryl or heteroaryl is optionally further substituted with 0 to 4 substituents selected from H, F, Cl, Br, I, OH, C(=O)OH, CN, $NH_2$, =O, $C_{1-4}$ alkyl, halogen-substituted $C_{1-4}$ alkyl, hydroxyl-substituted $C_{1-4}$ alkyl or $C_{1-4}$ alkoxy, and the mono-heterocyclic ring, fused-heterocyclic ring, bridged-heterocyclic ring, spiro-heterocyclic ring or heteroaryl contains 1 to 4 heteroatoms selected from O, S, or N; the remaining definitions are the same as those in the first embodiment of the present invention.

As a third embodiment of the present invention, the above-mentioned compound represented by general formula (I) or the stereoisomer, deuterated compound, solvate, prodrug, metabolite, pharmaceutically acceptable salt or eutectic crystal thereof, wherein $R^L$ is selected from H, methyl or ethyl;

Cy1, Cy2, Cy3 and Cy4 are each independently selected from a bond or one of the following substituted or unsubstituted groups: cyclopropyl, cyclobutyl, cyclopentyl, cyclohexyl, azetidinyl, azacyclopentyl, piperidyl, morpholinyl, piperazinyl, phenyl, cyclopropyl-fused-cyclopropyl, cyclopropyl-fused-cyclobutyl, cyclopropyl-fused-cyclopentyl, cyclopropyl-fused-cyclohexyl, cyclobutyl-fused-cyclobutyl, cyclobutyl-fused-cyclopentyl, cyclobutyl-fused-cyclohexyl, cyclopentyl-fused-cyclopentyl, cyclopentyl-fused-cyclohexyl, cyclohexyl-fused-cyclohexyl, cyclopropyl-spiro-cyclopropyl, cyclopropyl-spiro-cyclobutyl, cyclopropyl-spiro-cyclopentyl, cyclopropyl-spiro-cyclohexyl, cyclobutyl-spiro-cyclobutyl, cyclobutyl-spiro-cyclopentyl, cyclobutyl-spiro-cyclohexyl, cyclopentyl-spiro-cyclopentyl, cyclopentyl-spiro-cyclohexyl, cyclohexyl-spiro-cyclohexyl, cyclopropyl-fused-azetidinyl, cyclopropyl-fused-azacyclopentyl, cyclopropyl-fused-azacyclohexyl, cyclobutyl-fused-azetidinyl, cyclobutyl-fused-azacyclopentyl, cyclobutyl-fused-azacyclohexyl, cyclopentyl-fused-azetidinyl, cyclopentyl-fused-azacyclopentyl, cyclopentyl-fused-azacyclohexyl, cyclohexyl-fused-azetidinyl, cyclohexyl-fused-azacyclopentyl, cyclohexyl-fused-azacyclohexyl, azetidinyl-fused-azetidinyl, azetidinyl-fused-azacyclopentyl, azetidinyl-fused-azacyclohexyl, azacyclopentyl-fused-azacyclopentyl, azacyclopentyl-fused-azacyclohexyl, azacyclohexyl-fused-azacyclohexyl, cyclobutyl-spiro-azetidinyl, cyclobutyl-spiro-azacyclopentyl, cyclobutyl-spiro-azacyclohexyl, cyclopentyl-spiro-azetidinyl, cyclopentyl-spiro-azacyclopentyl, cyclopentyl-spiro-azacyclohexyl, cyclohexyl-spiro-azetidinyl, cyclohexyl-spiro-azacyclopentyl, cyclohexyl-spiro-azacyclohexyl, azetidinyl-spiro-azetidinyl, azetidinyl-spiro-azacyclopentyl, azetidinyl-spiro-azacyclohexyl, azacyclopentyl-spiro-azacyclopentyl, azacyclopentyl-spiro-azacyclohexyl, azacyclohexyl-spiro-azacyclohexyl,

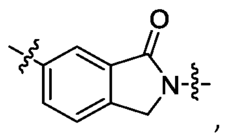,

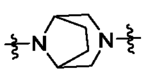, 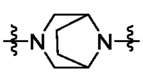, 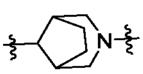, 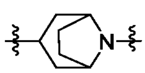, 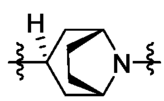, 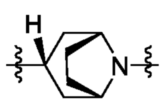, 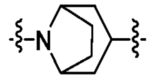,

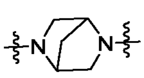, 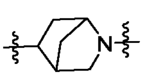, 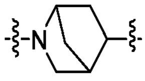, 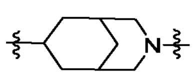, 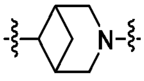, 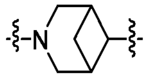, 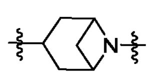,

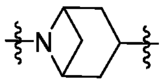, 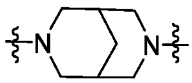, 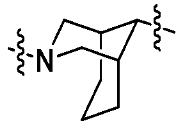, 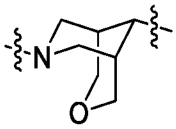 or 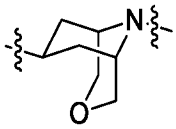, 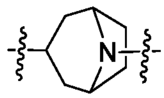,

which, when substituted, is optionally further substituted with 0 to 4 substituents selected from H, F, Cl, Br, I, OH, $NH_2$, C(=O)OH, CN, =O, $C_{1-4}$ alkyl, halogen-substituted $C_{1-4}$ alkyl, hydroxyl-substituted $C_{1-4}$ alkyl or $C_{1-4}$ alkoxy;

B1 and B3 are each independently selected from pyrazolyl, oxazolyl, dioxazolyl, oxadiazolyl, triazolyl, imidazolyl, tetrazolyl, pyrrolyl, thienyl, thiazolyl, thiadiazolyl, pyridyl, phenyl, pyrazinyl, pyrimidyl, pyridazinyl, thienopyrazinyl, benzimidazolyl, pyridotriazolyl, pyrimidopyrazolyl, imidazopyridazinyl, pyridopyrazolyl, pyrrolopyridazinyl or

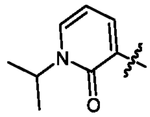;

$R^{b1}$ and $R^{b7}$ are each independently selected from H, F, Cl, Br, I, =O, OH, $NH_2$, CN, $CF_3$, $CHF_2$, $CH_2F$, methyl, ethyl, methoxy, ethoxy, phenyl, pyrrolyl, pyridyl, morpholinyl,

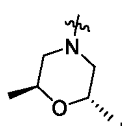, 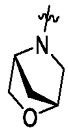, 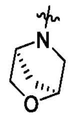,

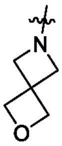, 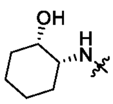, 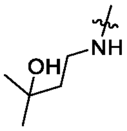, 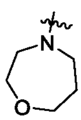, 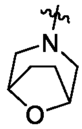, 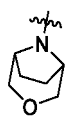, 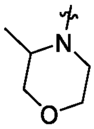, 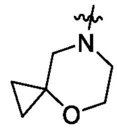, 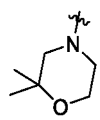, 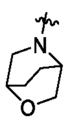, 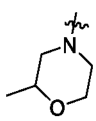,

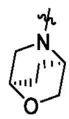, 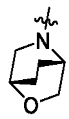, 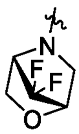, 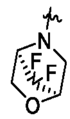 or 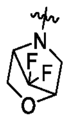,

wherein the methyl, ethyl, methoxy, ethoxy, phenyl, pyrrolyl, pyridyl or morpholinyl is optionally further substituted with 0 to 4 substituents selected from H, F, Cl, Br, I, OH, CN, $CF_3$, $NH_2$, $NHC_{1-4}$ alkyl, $N(C_{1-4}$ alkyl$)_2$, $NHCH_2C_{3-6}$ cycloalkyl, $C_{1-4}$ alkyl, $C_{1-4}$ alkoxy, $C_{3-6}$ cycloalkyl or $R^{b7a}$;

or $R^{b1}$ and $R^{b7}$ are each independently selected from azetidinyl, azacyclopentyl, piperidyl, piperazinyl, morpholinyl or 2-oxa-5-azabicyclo[2.2.1]heptanyl, wherein the $R^{b1}$ and $R^{b7}$ are optionally substituted with 1 to 4 substituents selected from F, Cl, Br, I, OH, =O, CN, $CF_3$, $NH_2$, $NHC_{1-4}$ alkyl, $N(C_{1-4}$ alkyl$)_2$, $NHCH_2C_{3-6}$ cycloalkyl, halogen-substituted $C_{1-4}$ alkyl, cyano-substituted $C_{1-4}$ alkyl, -$C_{1-4}$ alkylene -OH, $C_{1-4}$ alkyl, $C_{1-4}$ alkoxy, -$CH_2$-O-$C_{1-4}$ alkyl, -$CH_2$-$C_{3-6}$ cycloalkyl, -O-$C_{3-6}$ cycloalkyl, -NH-$C_{3-6}$ cycloalkyl, $C_{3-6}$ cycloalkyl, -$CH_2$-4- to 7-membered heterocycloalkyl, -O-4- to 7-membered heterocycloalkyl, -NH-4- to 7-membered heterocycloalkyl, or 4- to 7-membered heterocycloalkyl, and the heterocyclyl contains 1 to 4 heteroatoms selected from O, S, or N;

$R^{b2}$ and $R^{b6}$ are each independently selected from H, F, Cl, Br, I, =O, $CF_3$, $CHF_2$, OH, $NH_2$, NH(methyl), NH(ethyl), NH(propyl), NH(isopropyl), N(methyl$)_2$, N(ethyl$)_2$, CN, methyl, ethyl, methoxy, ethoxy, propoxy, isopropyloxy, morpholinyl, piperazinyl, pyrrolidyl, piperidyl or oxazolidinyl, wherein the methyl, ethyl, methoxy, ethoxy, propoxy, isopropyloxy, morpholinyl, piperazinyl, pyrrolidyl, piperidyl or oxazolidinyl is optionally further substituted with 0 to 4 substituents selected from H, F, Cl, Br, I, OH, CN, $CF_3$, $C_{1-4}$ alkyl, $C_{1-4}$ alkoxy or $C_{3-6}$ cycloalkyl;

each $R^{k1}$ is independently selected from H, methyl, ethyl, propyl, isopropyl, ethenyl, propenyl, allyl, ethynyl, propynyl, propargyl, cyclopropyl, cyclobutyl, cyclopentyl, cyclohexyl, azetidinyl, azacyclopentyl, piperidyl, oxacyclobutyl, oxacyclopentyl or oxacyclohexyl, wherein the methyl, ethyl, propyl, isopropyl, cyclopropyl, cyclobutyl, cyclopentyl, cyclohexyl, azetidinyl, azacyclopentyl, piperidyl, oxacyclobutyl, oxacyclopentyl or oxacyclohexyl is optionally further substituted with 0 to 4 substituents selected from H, F, Cl, Br, I, OH, CN, $CF_3$, $C_{1-4}$ alkyl, $C_{1-4}$ alkoxy, ethenyl, propenyl, allyl, ethynyl, propynyl, propargyl or $C_{3-6}$ cycloalkyl;

$R^{k2}$ and $R^{k3}$ are each independently selected from H, F, Cl, Br, I, OH, =O, $NH_2$, $CF_3$, CN, C(=O)OH, C(=O)$NH_2$, methyl, ethyl, methoxy or ethoxy, wherein the methyl, ethyl, methoxy or ethoxy is optionally further substituted with 0 to 4 substituents selected from H, F, Cl, Br, I, OH or $NH_2$;

each p2 or p3 is independently selected from 0, 1 or 2;

the remaining definitions are the same as those in the first or second embodiment of the present invention.

As a fourth embodiment of the present invention, the above-mentioned compound represented by general formula (I) or the stereoisomer, deuterated compound, solvate, prodrug, metabolite, pharmaceutically acceptable salt or eutectic crystal thereof, wherein

Cy1, Cy2, Cy3 and Cy4 are each independently selected from a bond or one of the following substituted or unsubstituted groups:

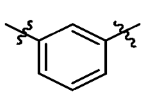, 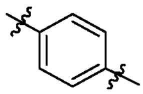, 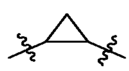, 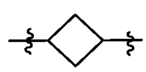,

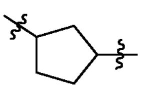, 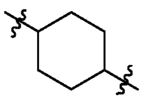, 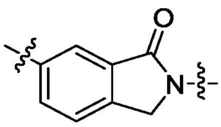, 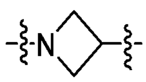, 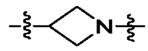, 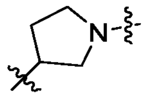,

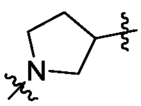, 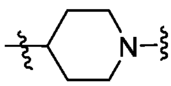, 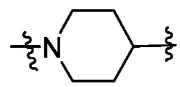, 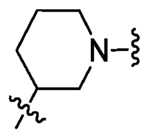, 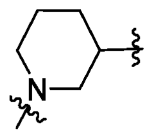, 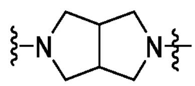,

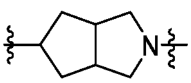, 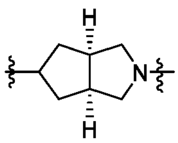, 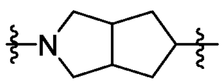, 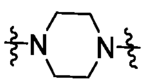, 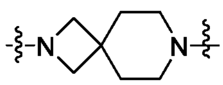,

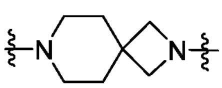, 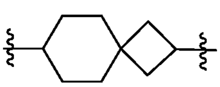, 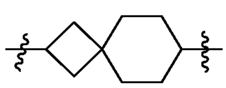, 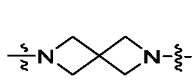, 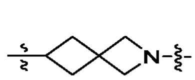,

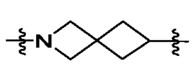, 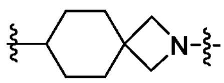, 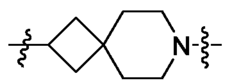, 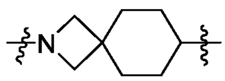, 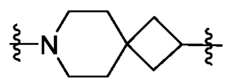,

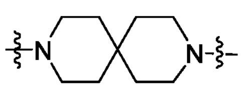, 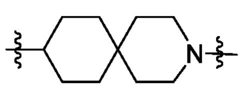, 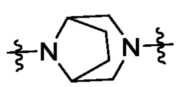, 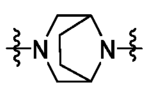, 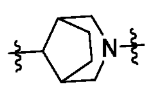,

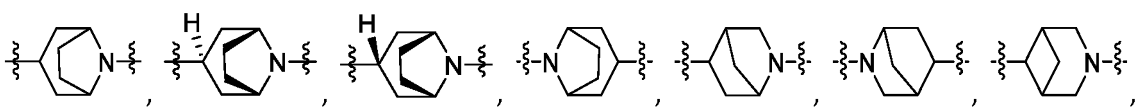

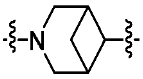, 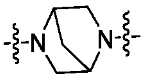, 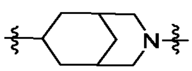, 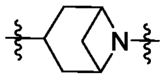, 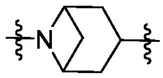, 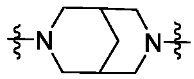,

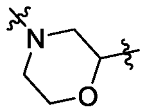, 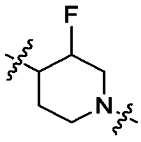, 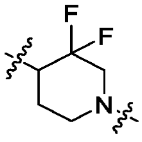, 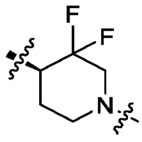, 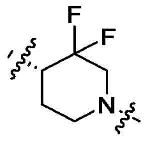, 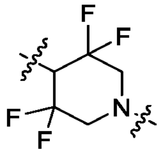, 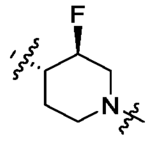,

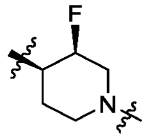, 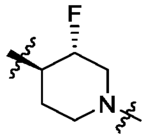, 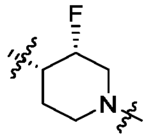, 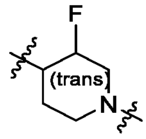 or 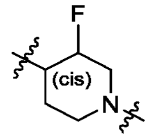,

which, when substituted, is optionally further substituted with 0 to 4 substituents selected from H, F, $CF_3$, methyl, =O, hydroxymethyl, C(=O)OH, CN or $NH_2$;

B is selected from

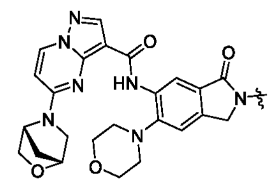, 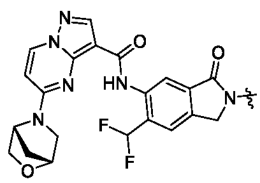, 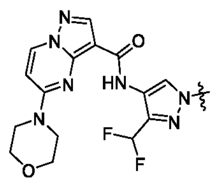,

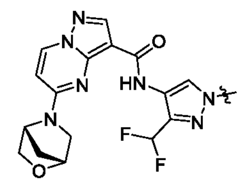, 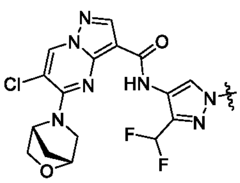, 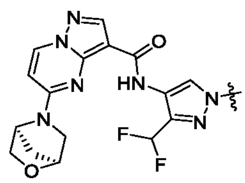, 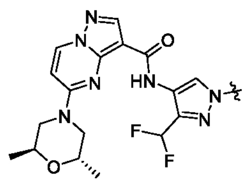,

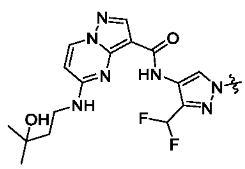, 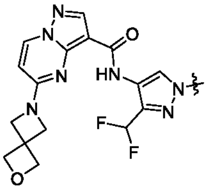, 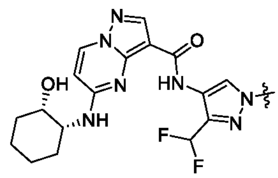, 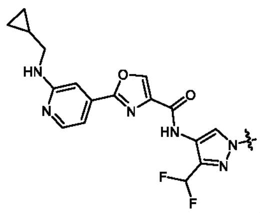,

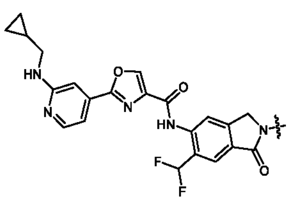, 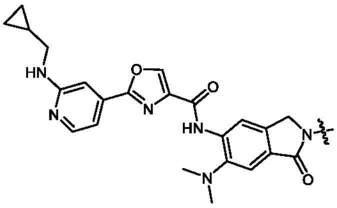, 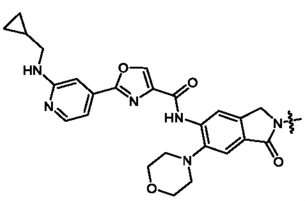,

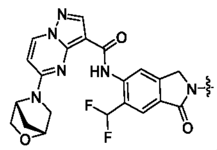, 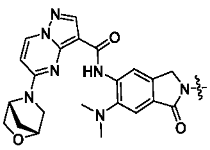, 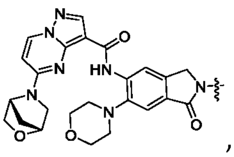, 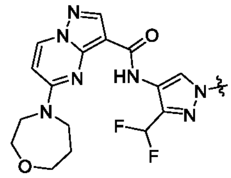, 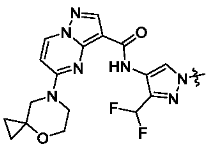,

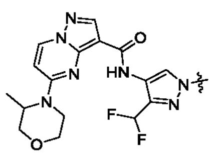, 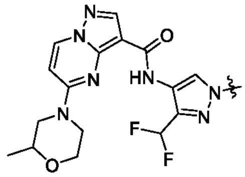, 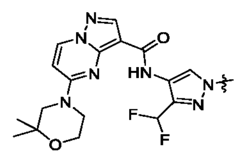, 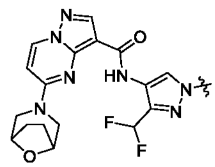, 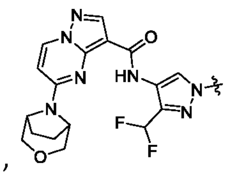,

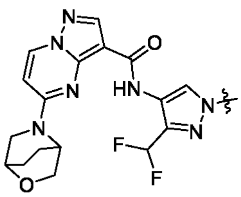, 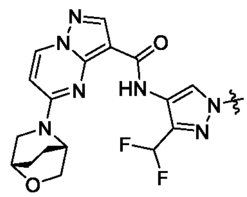, 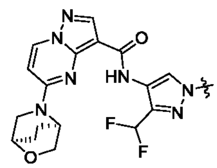, 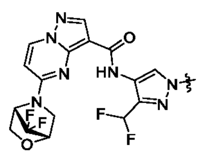,

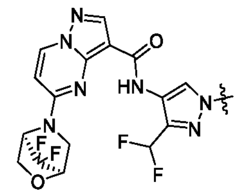, 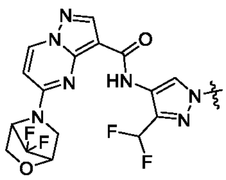, 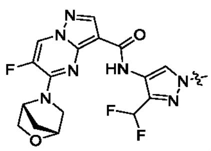, 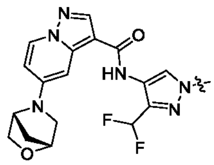, 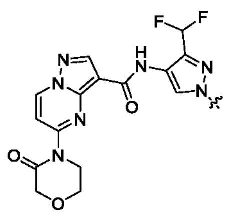,

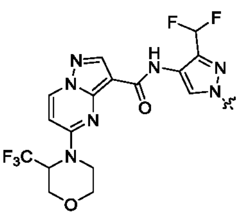, 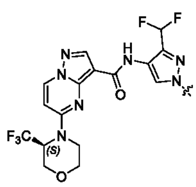, 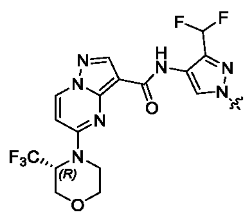, 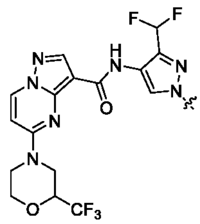, 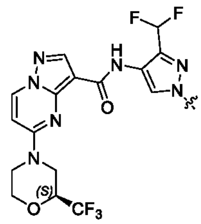,

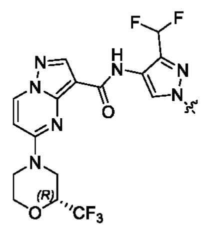, 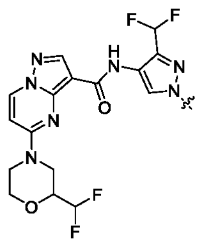, 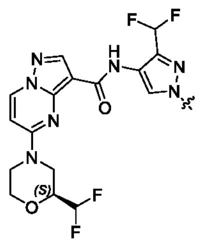, 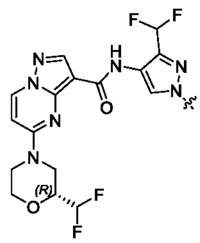, 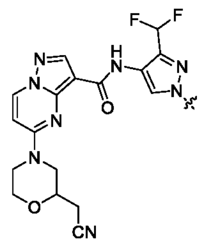,

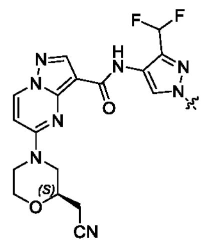, 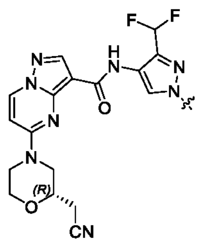, 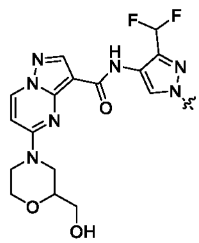, 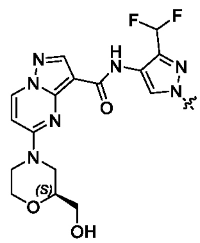, 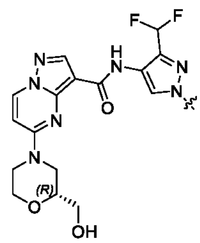,

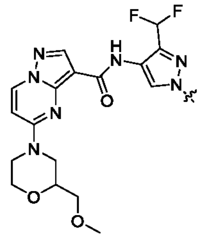, 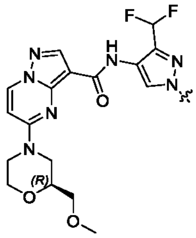, 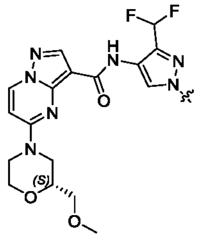, 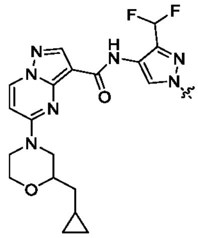, 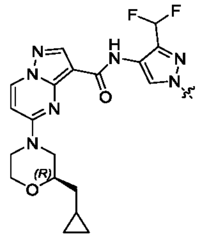,

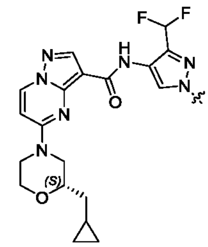, 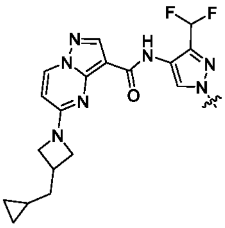, 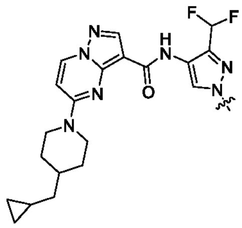, 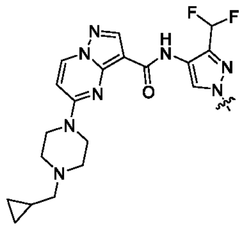,

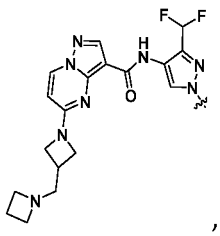, 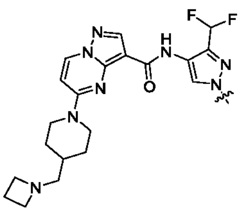, 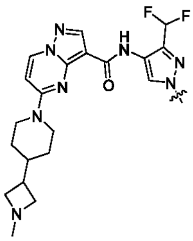, 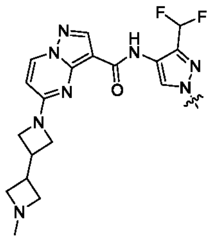, 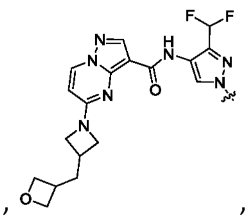,

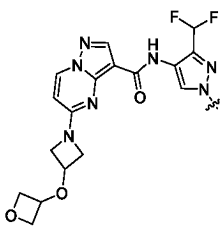, 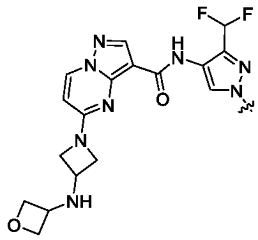, 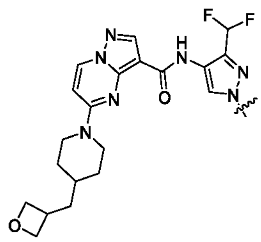, 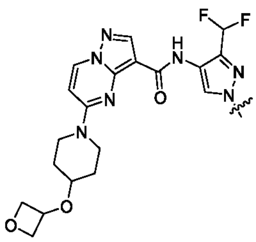,

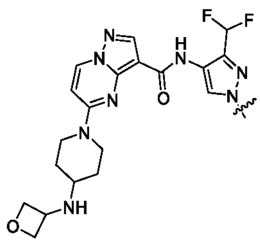, 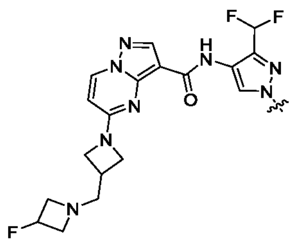 or 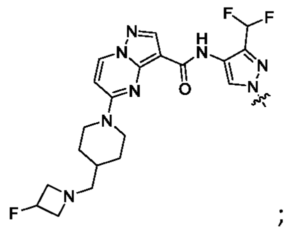;

the remaining definitions are the same as those of the first, second, or third embodiment of the present invention.

As a fifth embodiment of the present invention, the above-mentioned compound represented by general formula (I) or the stereoisomer, deuterated compound, solvate, prodrug, metabolite, pharmaceutically acceptable salt or eutectic crystal thereof, wherein

L is selected from a bond, -Cy1-, -Cy1-Ak2-, -Cy1-Ak2-Ak3-, -Cy1-Ak2-Ak3-Ak4-Ak5-, -Cy1-Cy2-, - Cy1-Ak2-Cy2-, -Cy1-Cy2-Ak3-, -Cy1-Cy2-Ak3-Cy4-, -Cy1-Ak2-Cy2-Ak3-, -Cy1-Ak2-Cy2-Ak3-Ak4-, - Cy1-Ak2-Cy2-Cy3-Ak4-, -Cy1-Cy2-Ak3-Ak4-, -Cy1-Cy2-Ak3-Ak4-Ak5-, -Cy1-Ak2-Cy2-Ak3-Ak4-Ak5-, -Cy1-Ak2-Ak3-Cy3-Ak4-, -Cy1-Ak2-Ak3-Cy3-Ak4-Ak5-, -Cy1-Ak2-Ak3-Ak4-Cy4-Ak5-, -Cy1-Cy2-Cy3-, -Cy1-Ak2-Cy2-Cy3-, -Cy1-Cy2-Cy3-Ak4-, -Cy1-Ak2-Cy2-Cy3-Ak4-, -Cy1-Ak2-Cy2-Ak3-Cy3-, -Cy1-Ak2-Cy2-Cy3-Ak4-Ak5, -Cy1-Ak2 -Cy2 -Ak3-Cy3-Ak4-Ak5-, -Cy1-Cy2-Cy3-Cy4-, -Cy1-Cy2-Ak3-Cy3-Cy4-, -Cy1-Cy2-Cy3-Ak4-Cy4-, -Ak1-Cy1-Ak2-Cy2-, -Ak1-Cy1-Ak2-Cy2-Ak3-, -Ak1-Ak2-Cy2-Ak3-, - Ak1-Ak2-Cy2-, -Ak1-Ak2-Cy2-Cy3-Ak4-, -Ak1-Ak2-Ak3-Cy3-Ak4-, -Ak1-Cy1-Ak2-,

-Ak1-Cy1-Cy2-Ak3-Ak4-, -Ak1-Cy1-Cy2-Ak3-, -Ak1-Cy1-Ak2-Ak3-Ak4-, -Ak1-Cy1-, -Ak1-Cy1-Ak2-Ak3-, -Ak1-Ak2-Cy2-Ak3-Ak4-, -Ak1-Cy1-Ak2-Cy2-Ak3-Ak4-, -Cy1-Ak2-Ak3-Cy3-Cy4-Ak5-, -Ak1-Cy1-Ak2-Ak3-Ak4-Ak5-, -Cy1-Ak1-Ak2-Ak3-, -Ak1-Cy1-Cy2-, -Ak1-Ak2-Ak3-Ak4- or -Cy1-Ak2-Cy2-Ak3-Cy3-Ak4-; Ak1, Ak2, Ak3, Ak4 and Ak5 are each independently selected from O, C=C, $CH_2$, $CH_2CH_2$, $CH_2CH_2CH_2$, $CH_2CH_2CH_2CH_2$, $CH_2N(CH_3)$, $CH_2CH_2N(CH_3)$, $N(CH_3)$, NH, C(=O), $C(=O)N(CH_3)$, $N(CH_3)C(=O)$, C(=O)NH or NHC(=O);

the remaining definitions are the same as those of the first, second, third or fourth embodiment of the present invention.

As a sixth embodiment of the present invention, the above-mentioned compound represented by general formula (I) or the stereoisomer, deuterated compound, solvate, prodrug, metabolite, pharmaceutically acceptable salt or eutectic crystal thereof, wherein

L is selected from

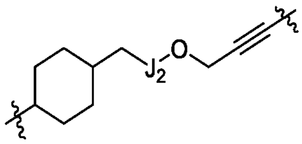 , 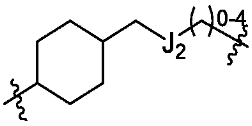 , 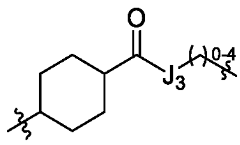 ,

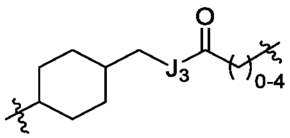 , 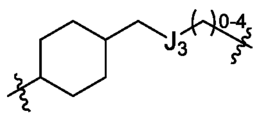 , 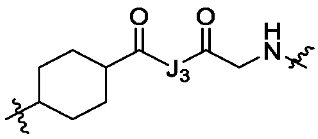 ,

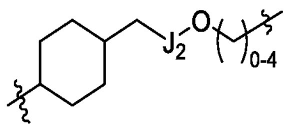 , 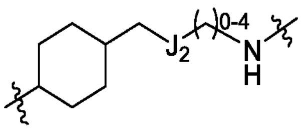 , 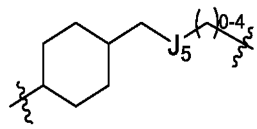 ,

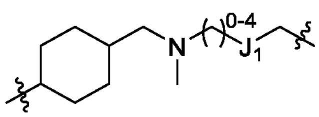 , 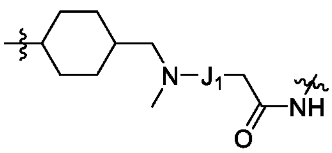 , 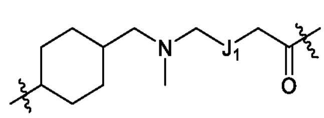 ,

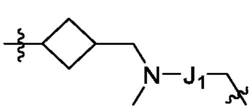 , 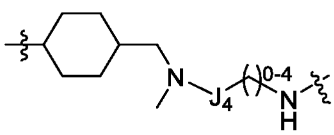 , 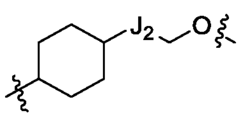 , 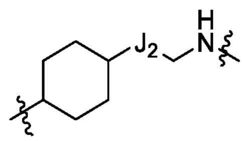 ,

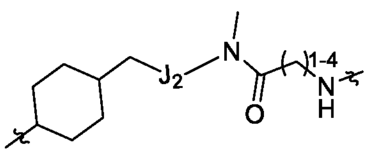 , 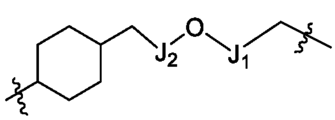 , 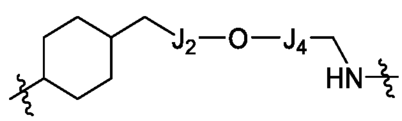 ,

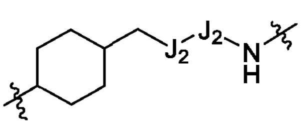 , 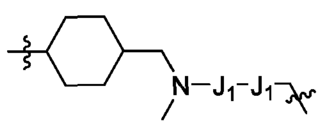 , 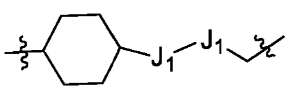 ,

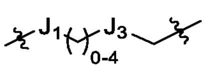 , 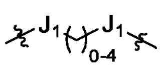 , 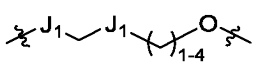 , 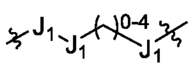 ,

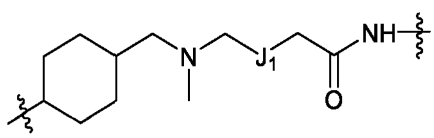, 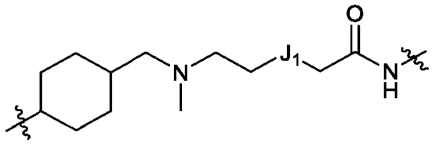,

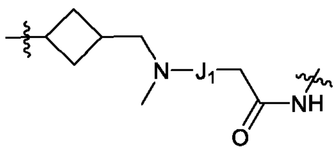, 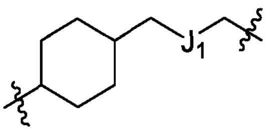, 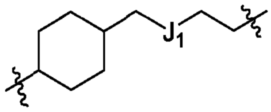,

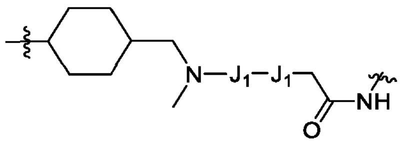, 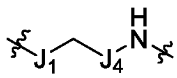, 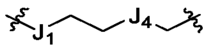, 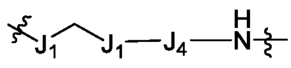,

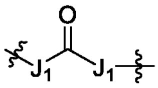, 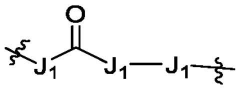, 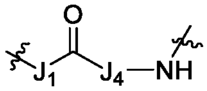, 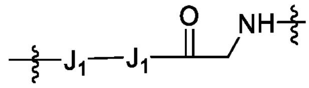,

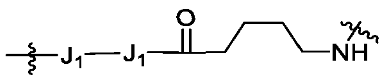, 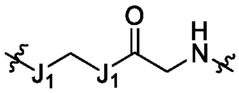, 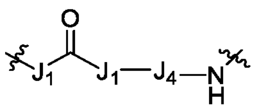,

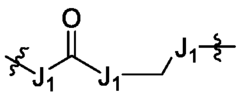, 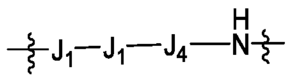, 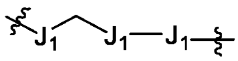, 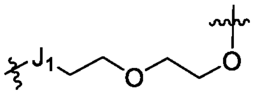,

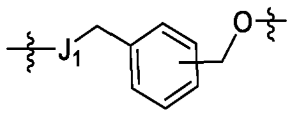, 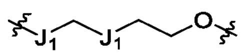, 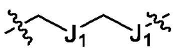, 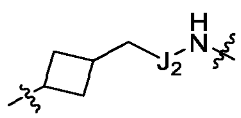,

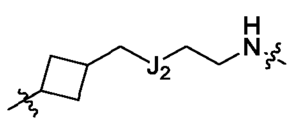,, 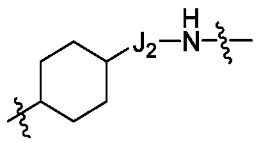, 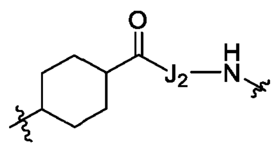,

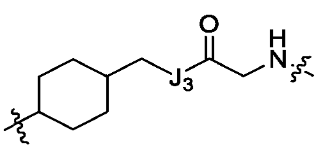, 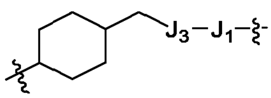, 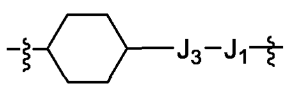,

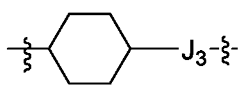, 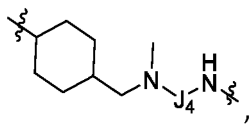, 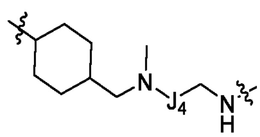, 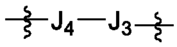,

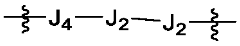, 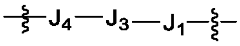, or 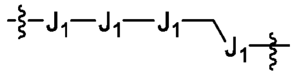;

each $J_1$ is independently selected from

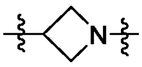, 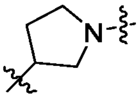, or 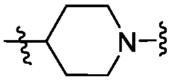;

each $J_2$ is independently selected from

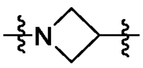, 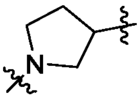, 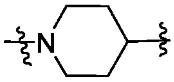, 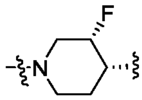,

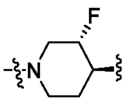, 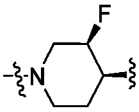, 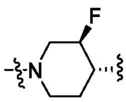, 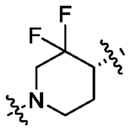, 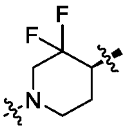, 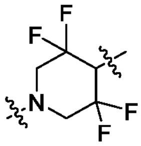, 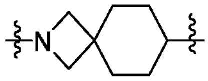,

or

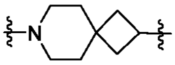;

each $J_3$ is independently selected from

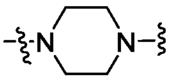, 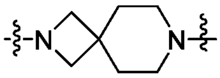, 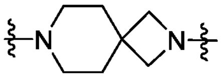,

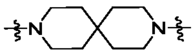, 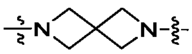, or 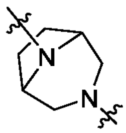;

each $J_4$ is independently selected from

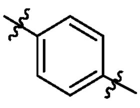, 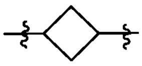, 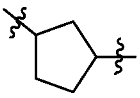, 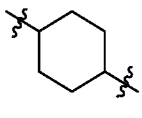,

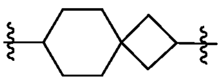, or 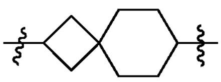;

each $J_5$ is independently selected from

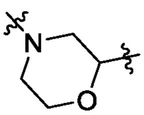;

or, L is selected from

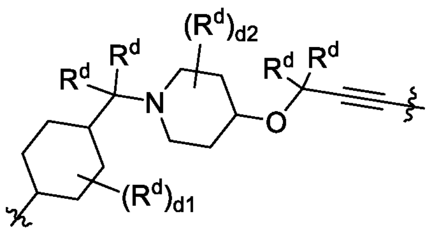
;

$R^d$ is selected from H or D, and at least one of $R^d$ is selected from D;

d1 is selected from 0, 1, 2, 3, 4, 5, 6, 7, 8, 9 or 10;

d2 is selected from 0, 1, 2, 3, 4, 5, 6, 7, 8 or 9;

the remaining definitions are the same as those of the first, second, third, fourth or fifth embodiment of the present invention.

As a seventh embodiment of the present invention, the above-mentioned compound represented by general formula (I) or the stereoisomer, deuterated compound, solvate, prodrug, metabolite, pharmaceutically acceptable salt or eutectic crystal thereof, wherein L is selected from a group shown in Table L-1, wherein the left side of the group is linked to B; the remaining definitions are the same as those of the first, second, third, fourth, fifth or sixth embodiment of the present invention.

As an eighth embodiment of the present invention, the above-mentioned compound represented by general formula (I) or the stereoisomer, deuterated compound, solvate, prodrug, metabolite, pharmaceutically acceptable salt or eutectic crystal thereof, wherein

K is selected from

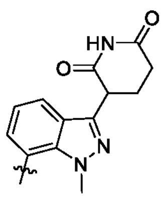 , 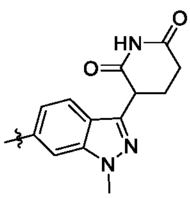 , 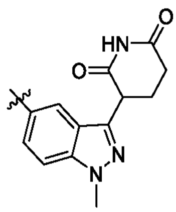 , 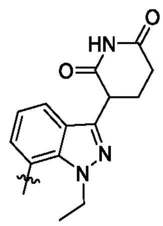 ,

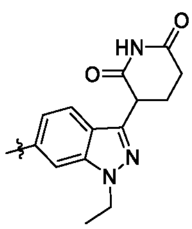 , 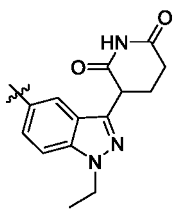 , 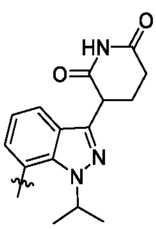 , 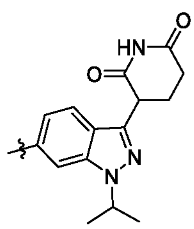 , 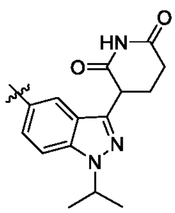 , 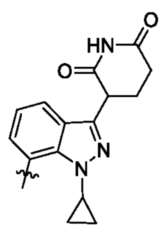 ,

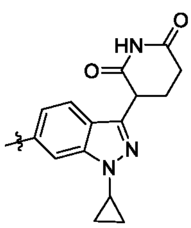 , 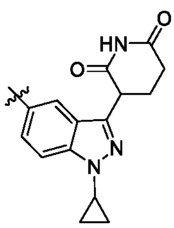 , 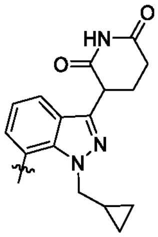 , 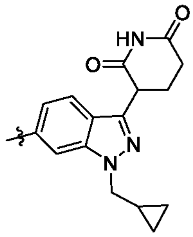 , 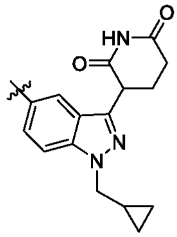 , 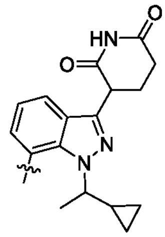 ,

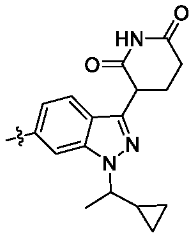, 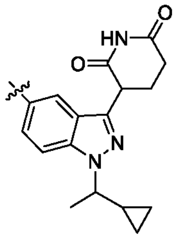, 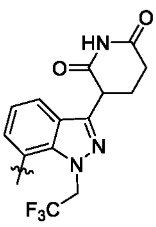, 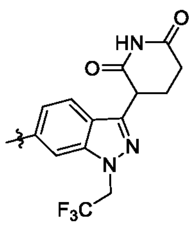, 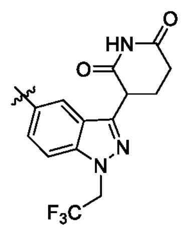, 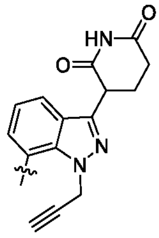,

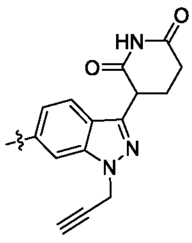, 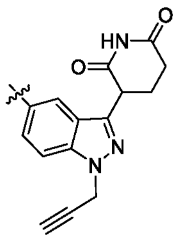, 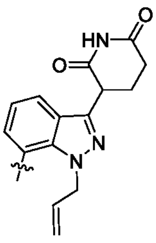, 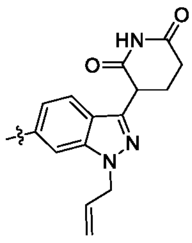, 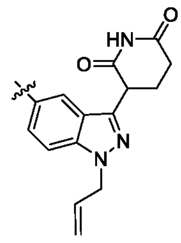, 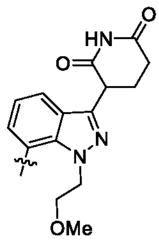,

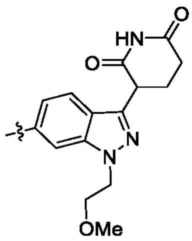, 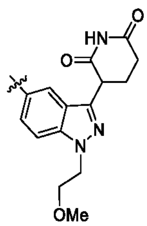, 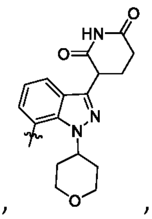, 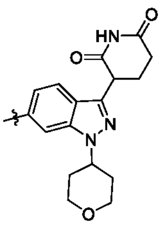, 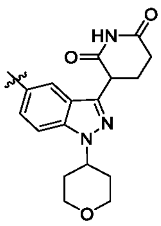, 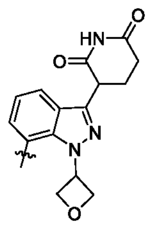,

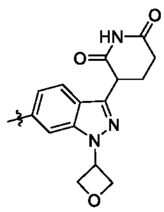, 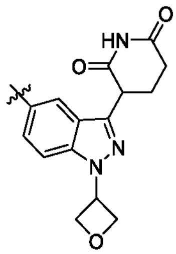, 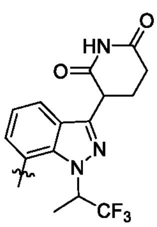, 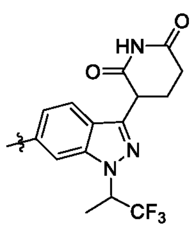, 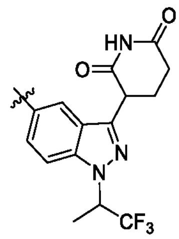, 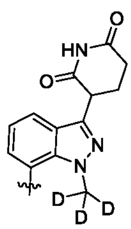,

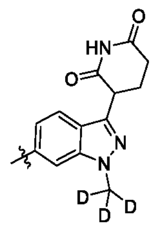, 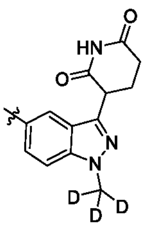, 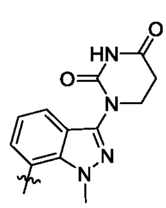, 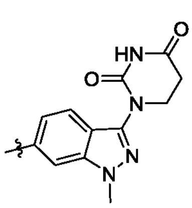, 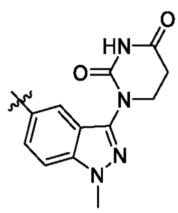, 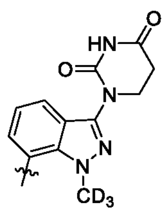,

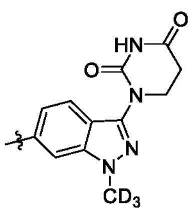, 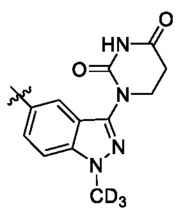, 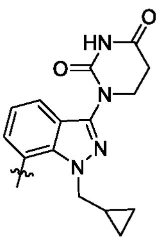, 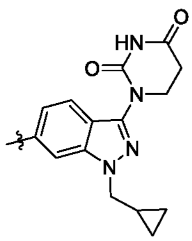, 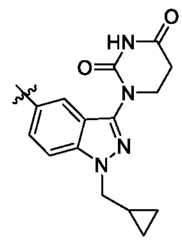, 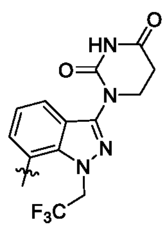,

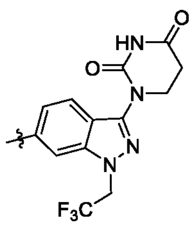 , 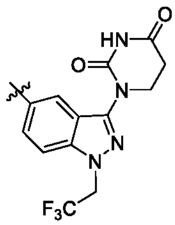 , 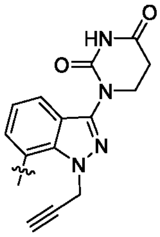 , 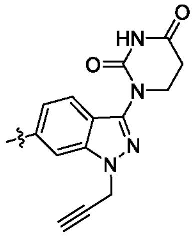 , 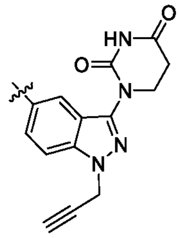 , 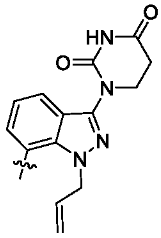 ,

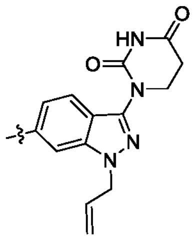 , 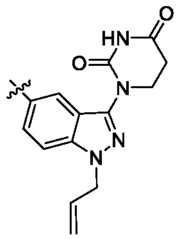 , 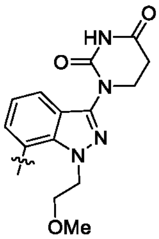 , 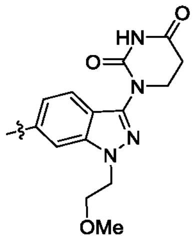 , 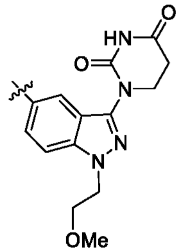 , 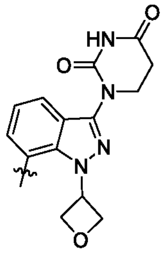 ,

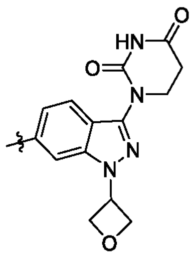 , 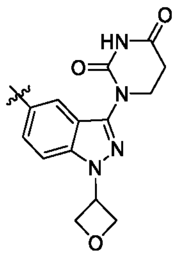 , 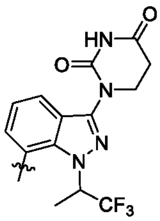 , 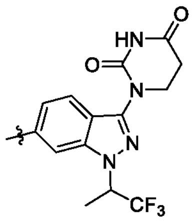 , 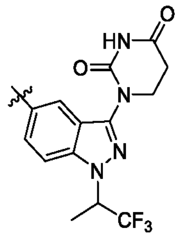 , 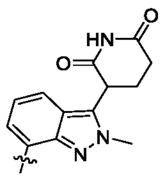 ,

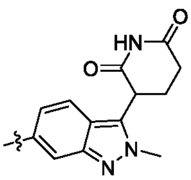 , 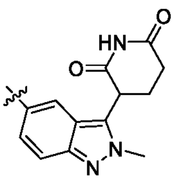 , 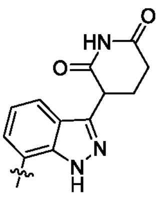 , 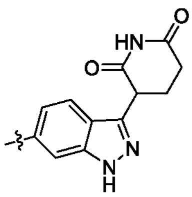 , 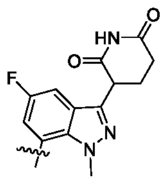

or

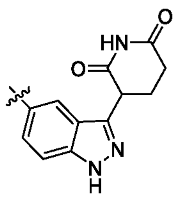 ;

the remaining definitions are the same as those of the first, second, third, fourth, fifth, sixth or seventh embodiment of the present invention.

The present invention relates to a compound as described below or a stereoisomer, deuterated compound, solvate, prodrug, metabolite, pharmaceutically acceptable salt, or eutectic crystal thereof, wherein the compound is selected from one of the structures shown in Table P-1 below:

Table P-1 Compound structure

| | |
|---|---|
| 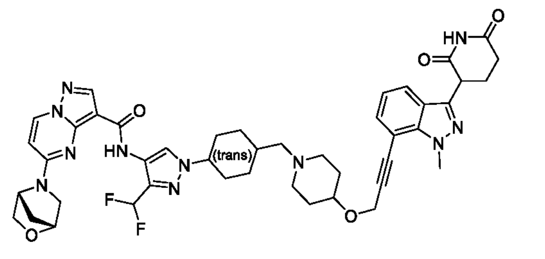 | 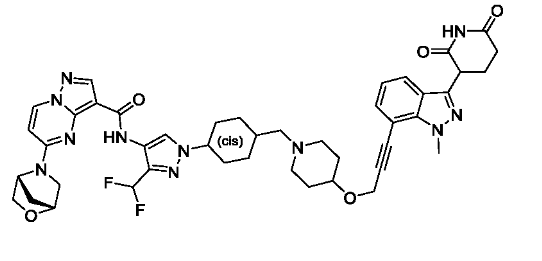 |

| | |
|---|---|
| 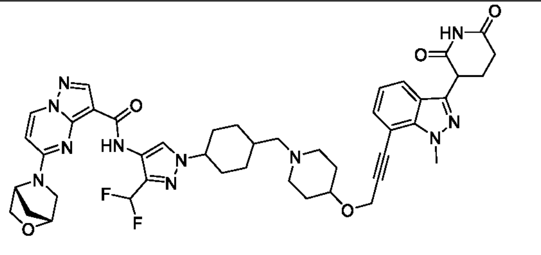 | 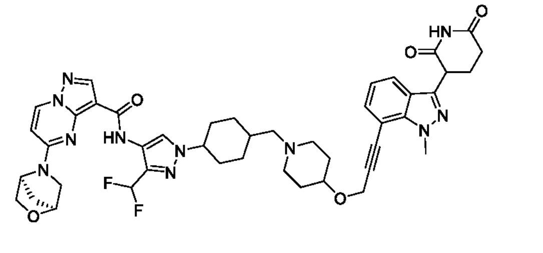 |
| 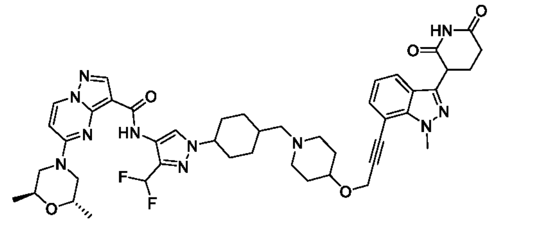 | 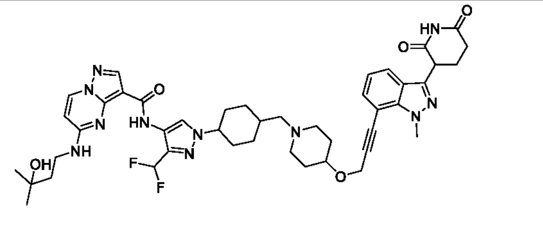 |
| 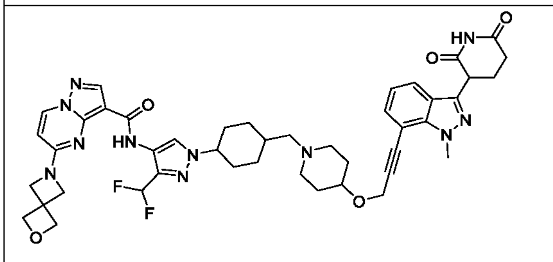 | 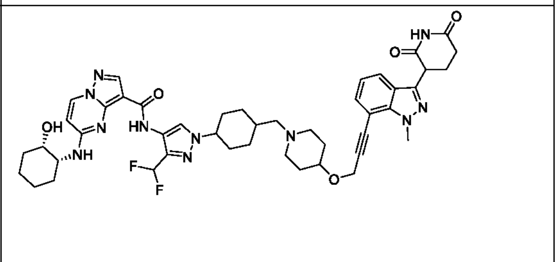 |
| 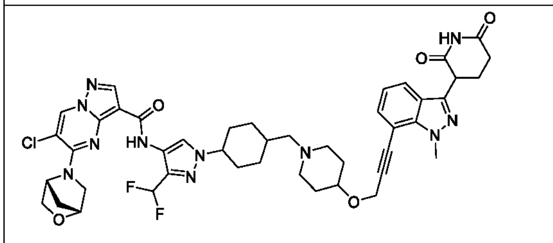 | 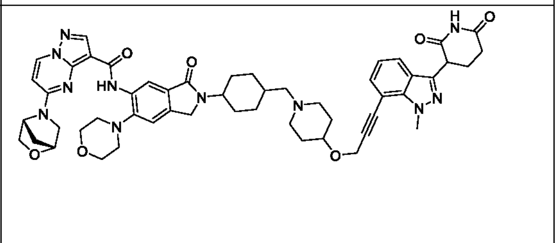 |
| 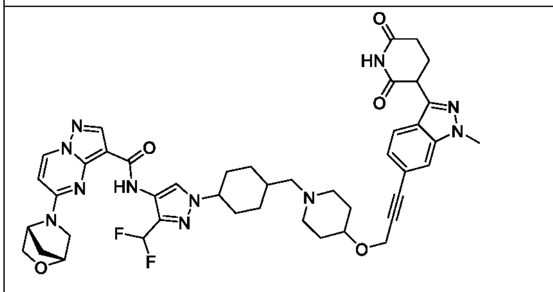 | 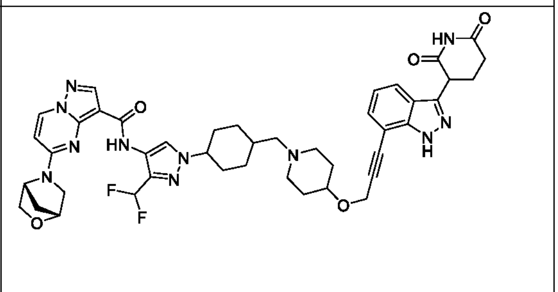 |
| 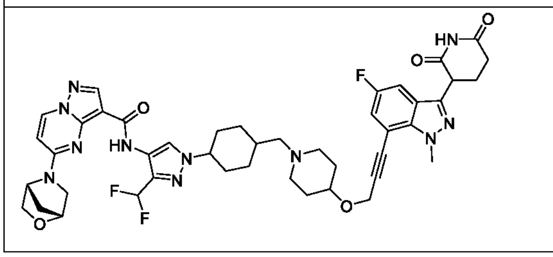 | 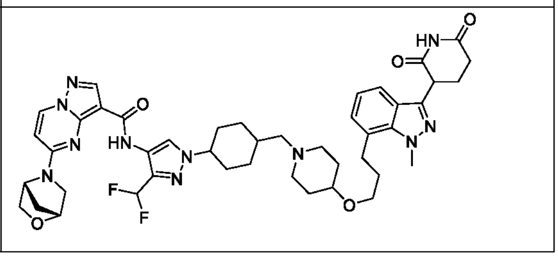 |

| | |
|---|---|
| 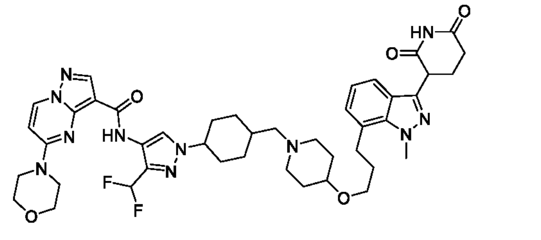 | 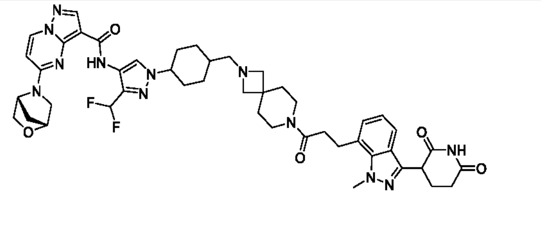 |
| 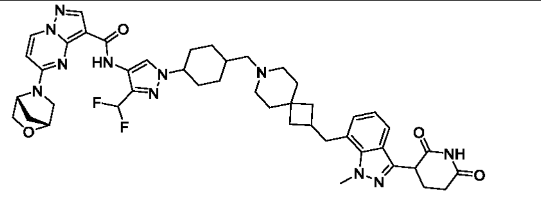 | 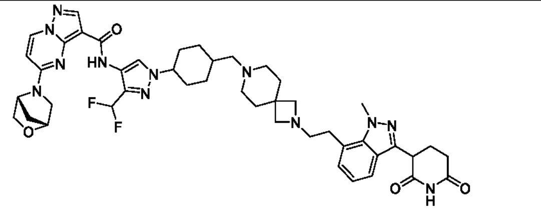 |
| 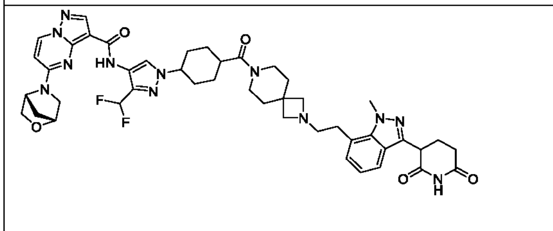 | 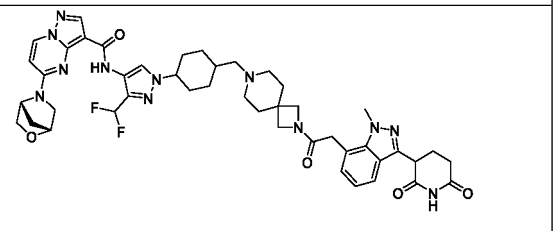 |
| 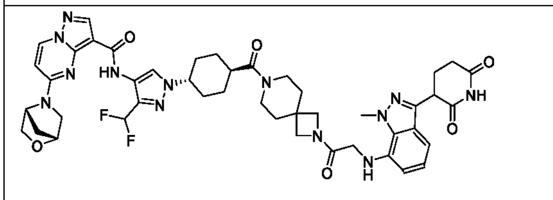 | 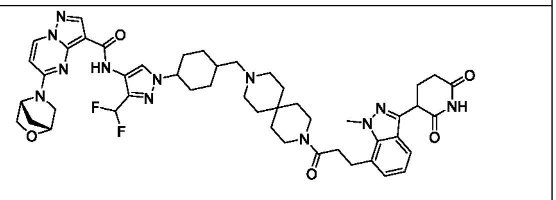 |
| 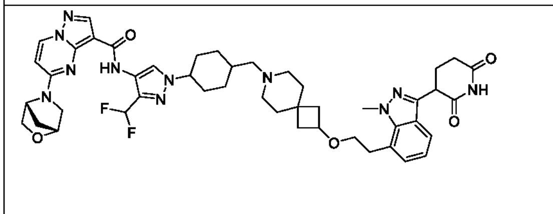 | 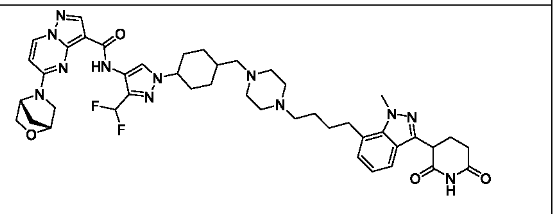 |
| 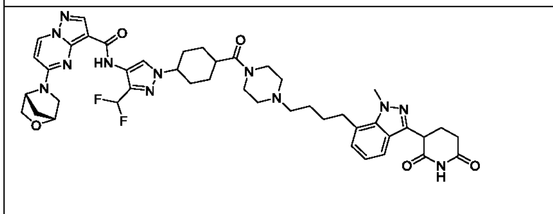 | 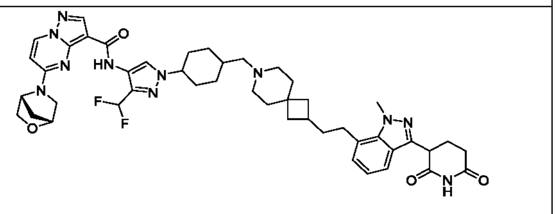 |
| 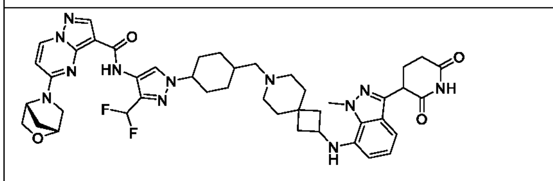 | 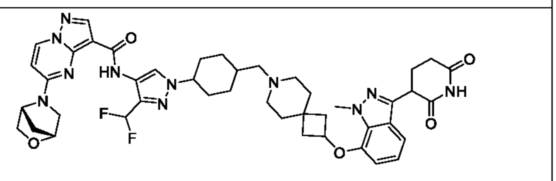 |
| 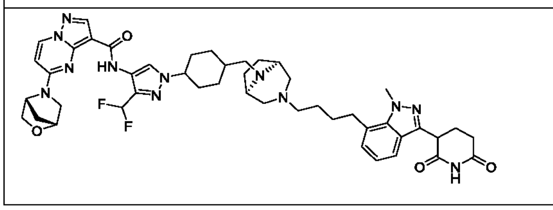 | 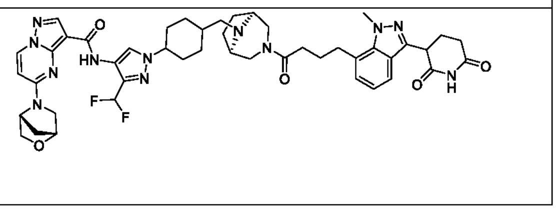 |

| | |
|---|---|
| 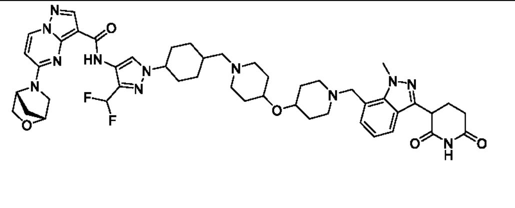 | 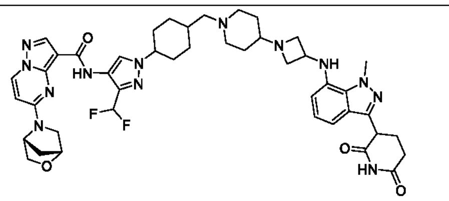 |
| 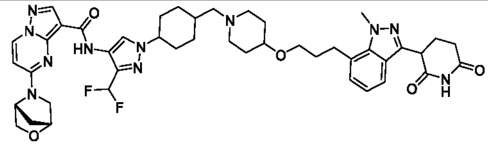 | 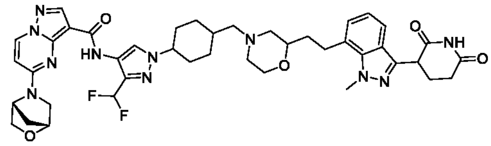 |
| 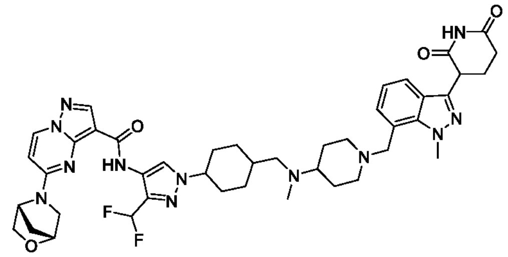 | 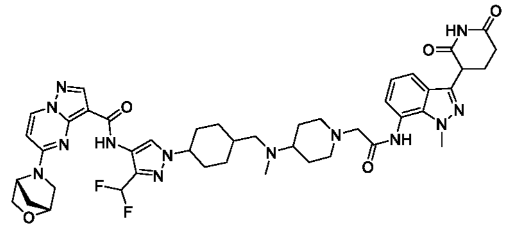 |
| 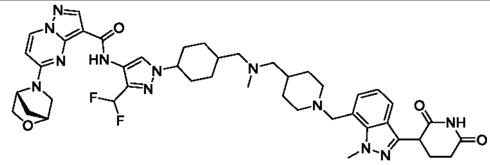 | 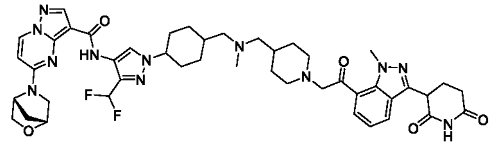 |
| 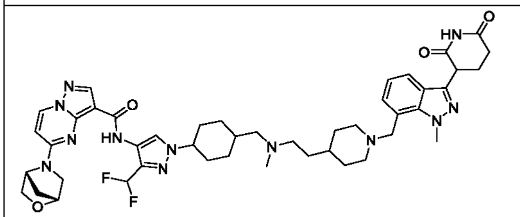 | 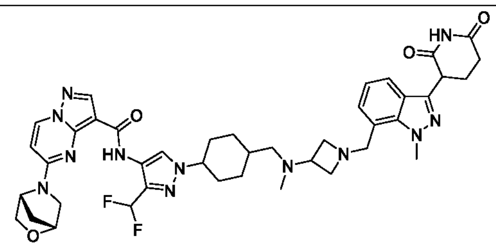 |
| 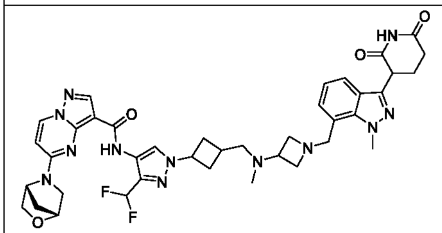 | 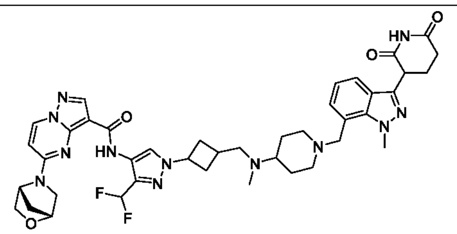 |
| 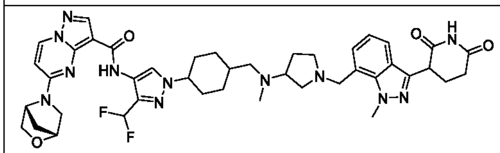 | 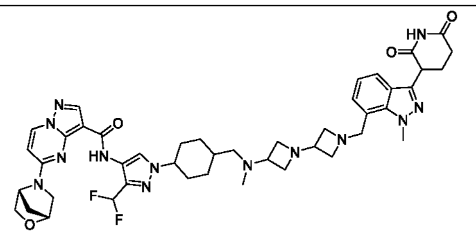 |

| | |
|---|---|
| 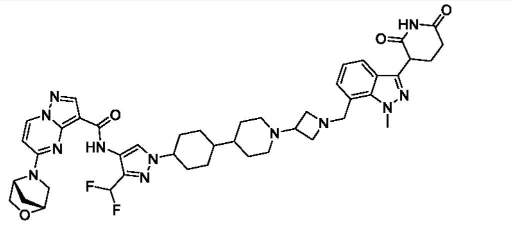 | 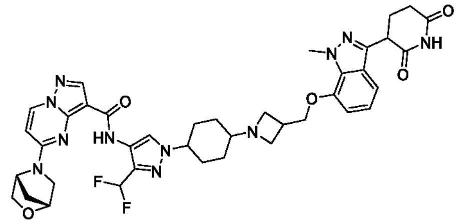 |
| 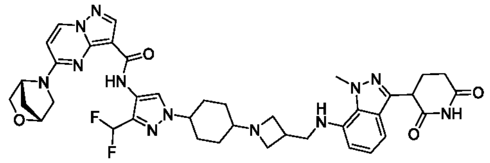 | 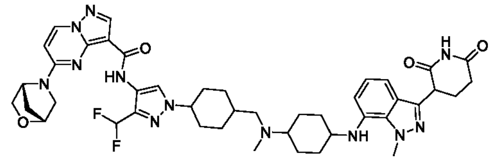 |
| 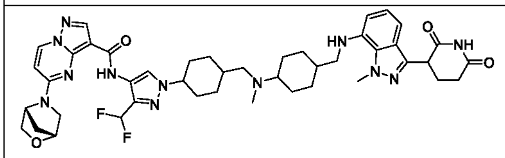 | 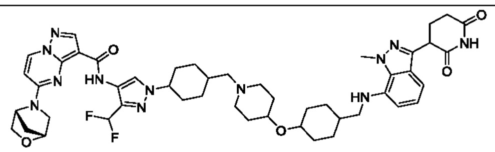 |
| 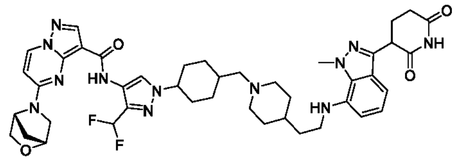 | 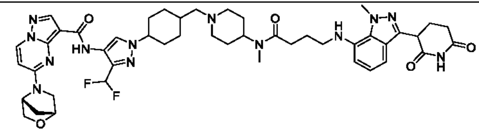 |
| 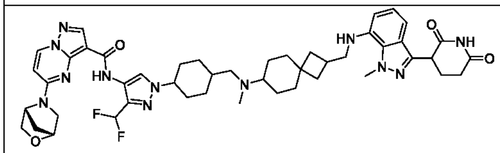 | 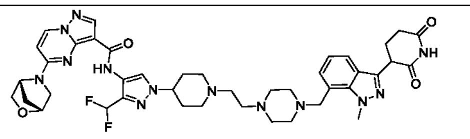 |
| 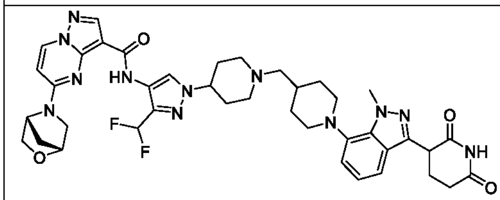 | 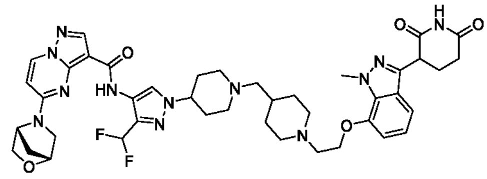 |
| 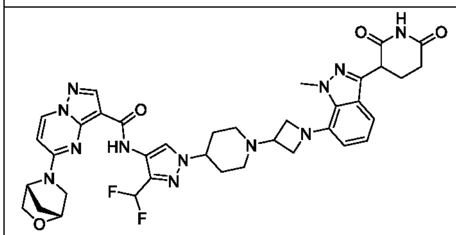 | 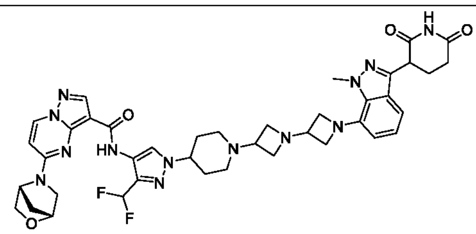 |
| 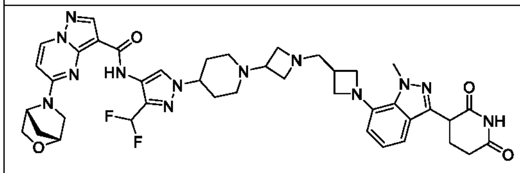 | 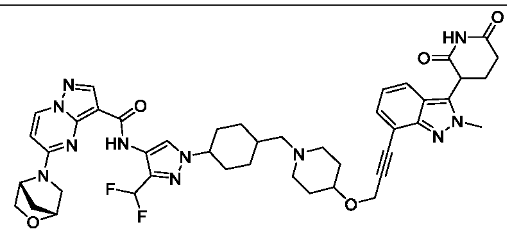 |

| | |
|---|---|
| 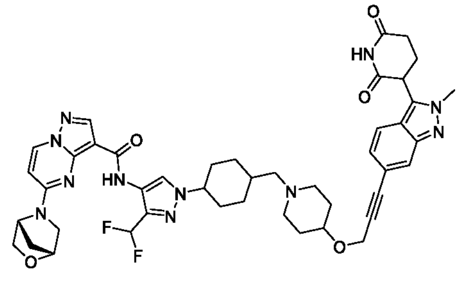 | 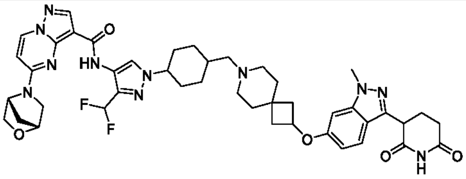 |
| 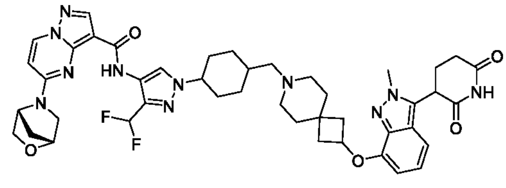 | 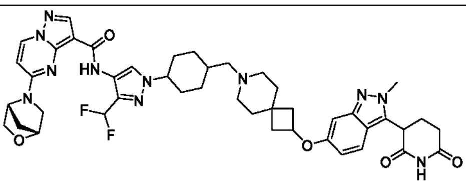 |
| 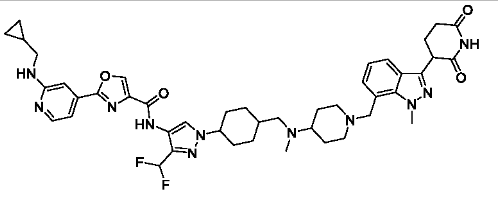 | 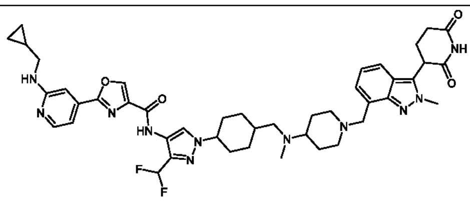 |
| 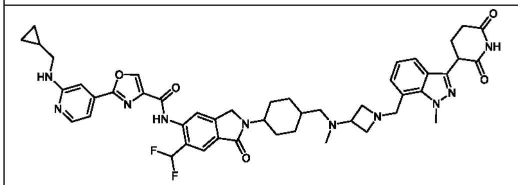 | 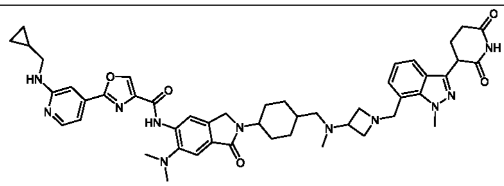 |
| 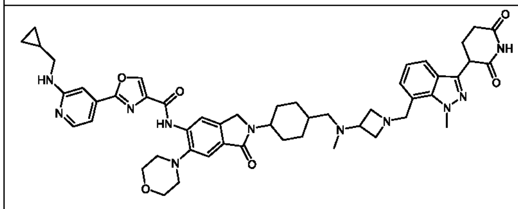 | 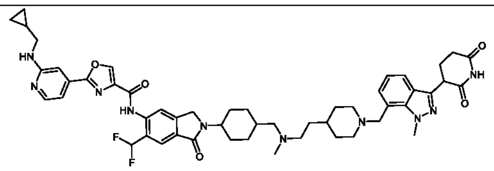 |
| 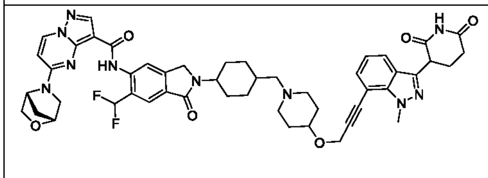 | 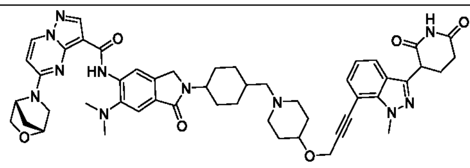 |
| 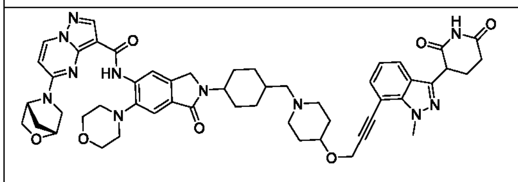 | 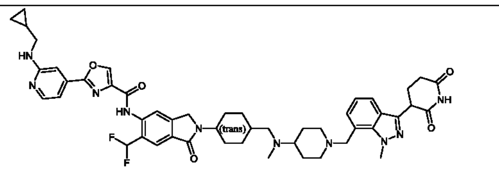 |
| 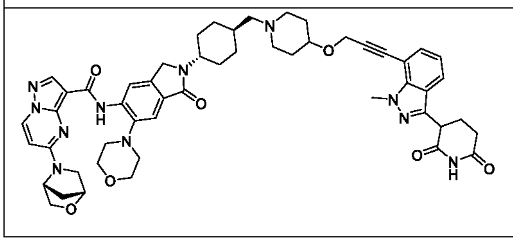 | 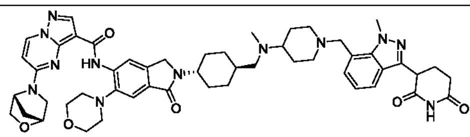 |

| | |
|---|---|
| | 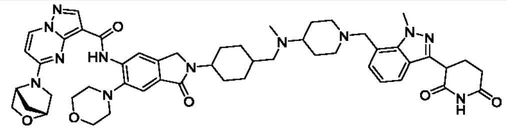 |
| 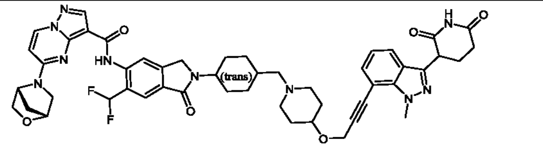 | 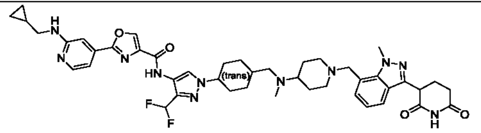 |
| 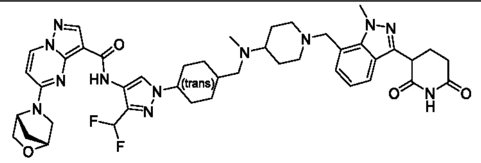 | 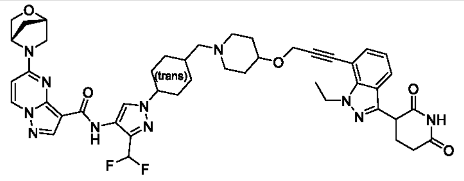 |
| 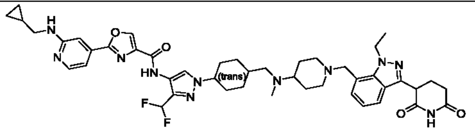 | 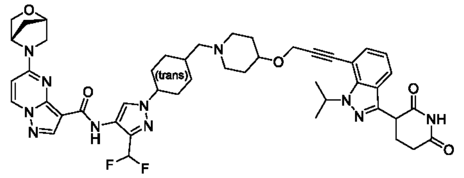 |
| 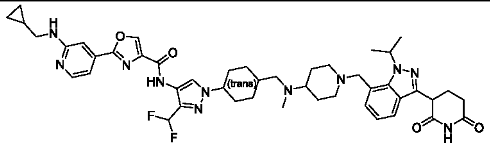 | 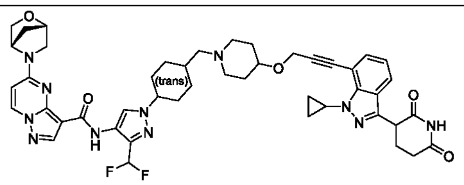 |
| 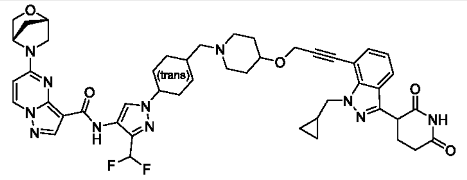 | 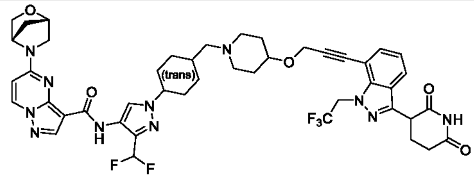 |
| 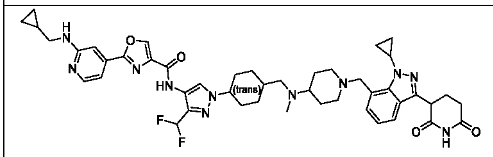 | 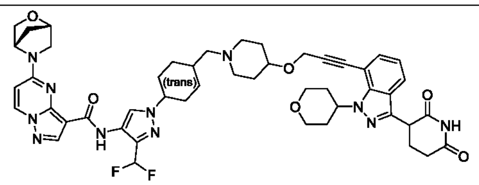 |
| 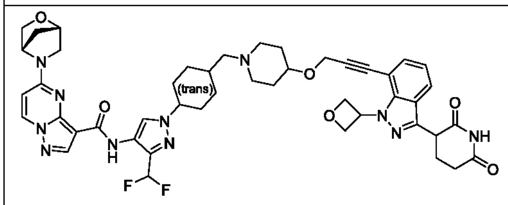 | 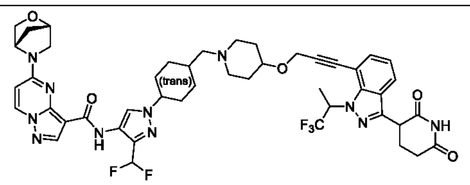 |
| 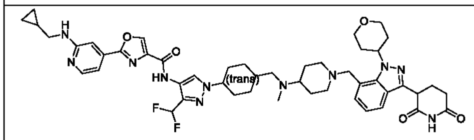 | 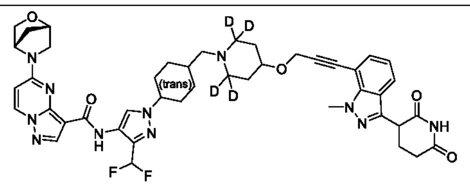 |

| | |
|---|---|
| 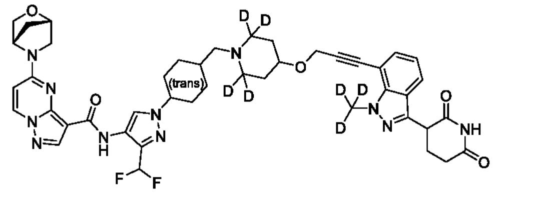 | 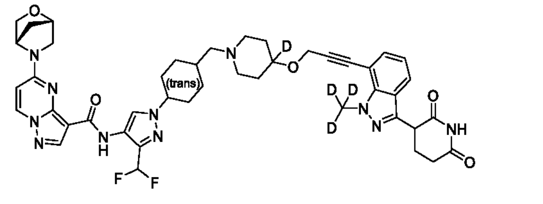 |
| 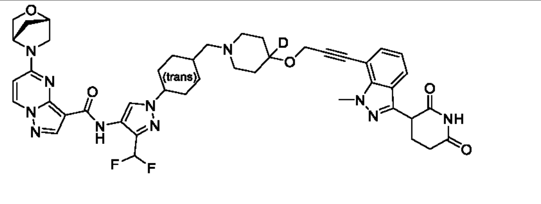 | 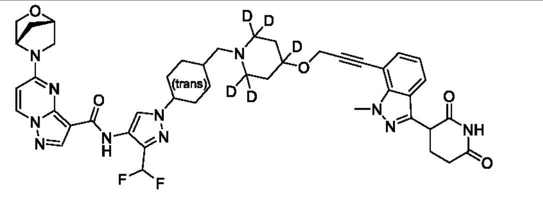 |
| 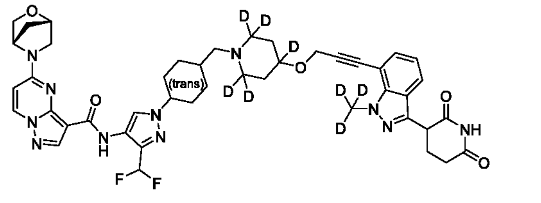 | 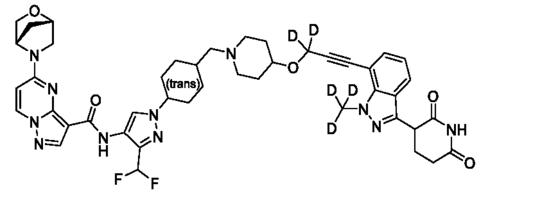 |
| 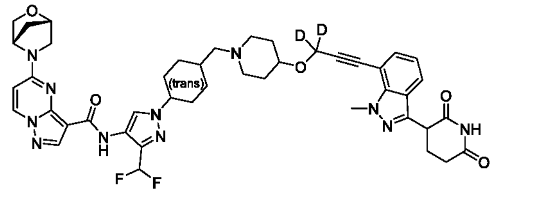 | 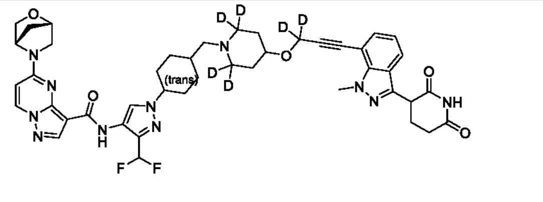 |
| 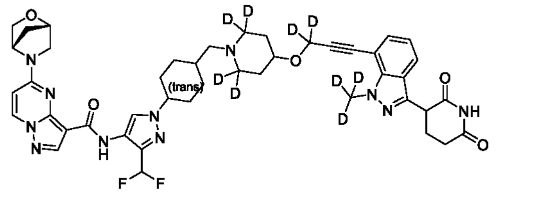 | 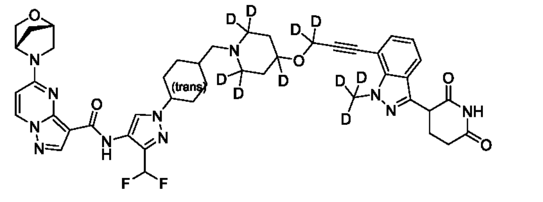 |
| 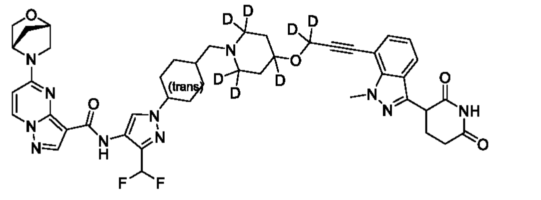 | 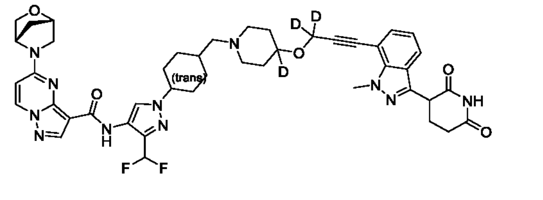 |
| 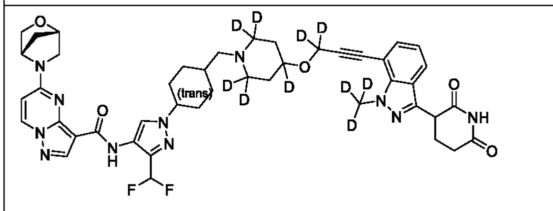 | 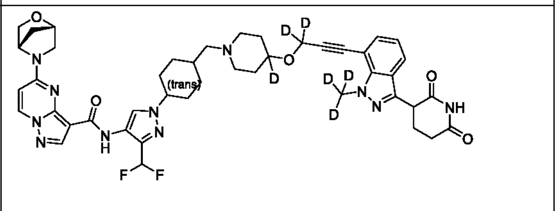 |
| 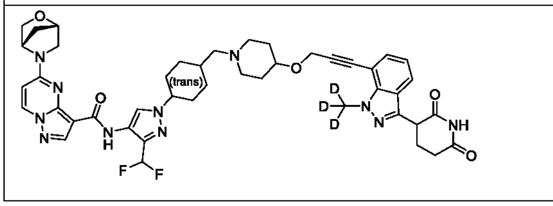 | 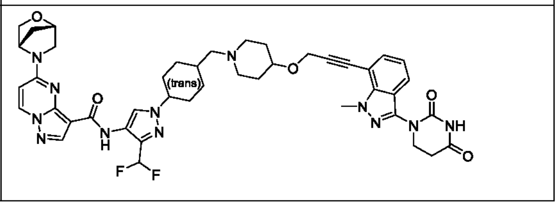 |

| | |
|---|---|
| 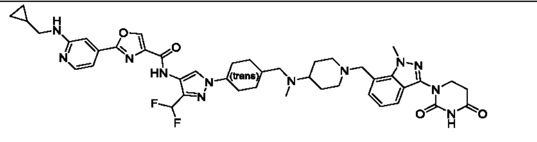 | 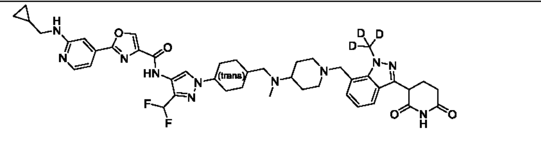 |
| 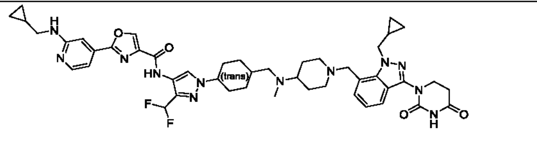 | 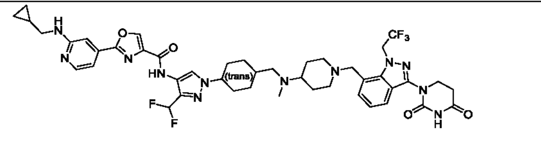 |
| 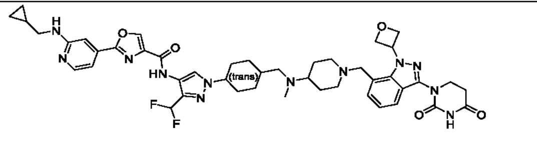 | 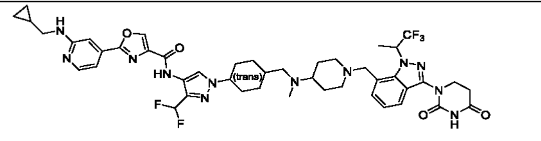 |
| 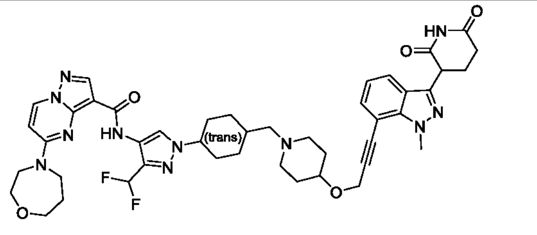 | 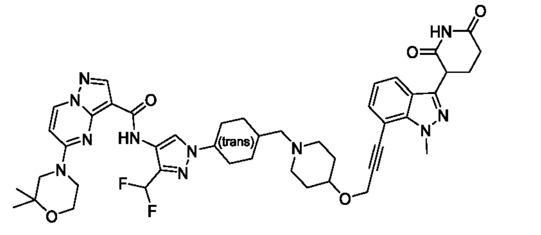 |
| 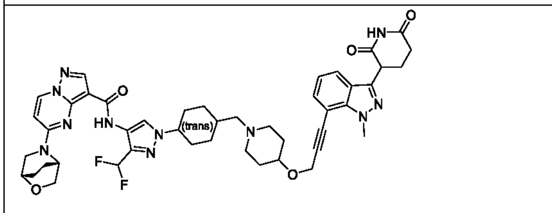 | 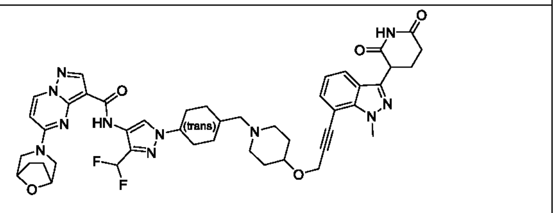 |
| 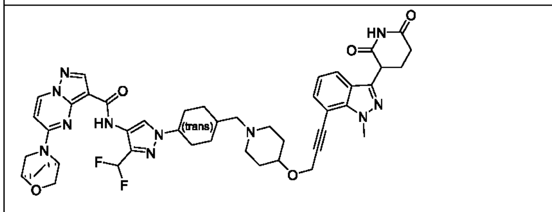 | 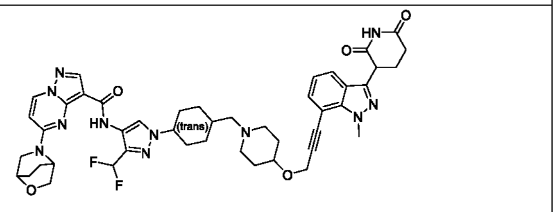 |
| 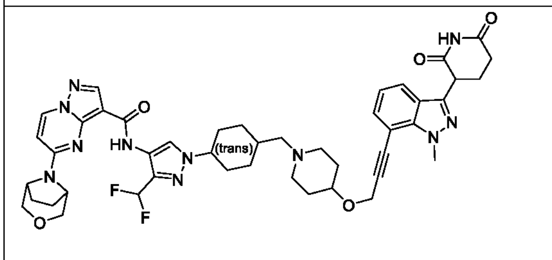 | 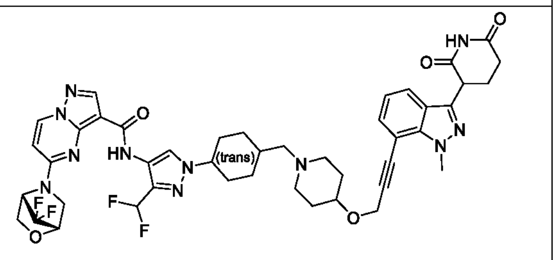 |
| 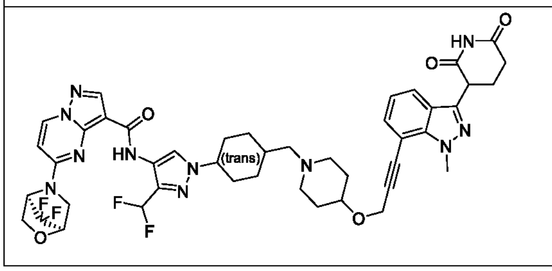 | 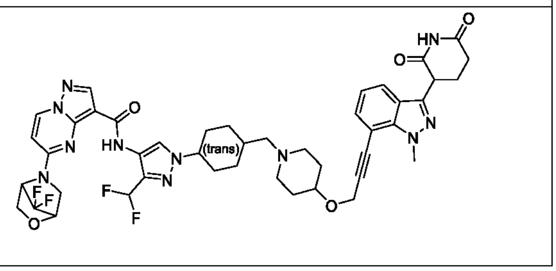 |

| | |
|---|---|
| 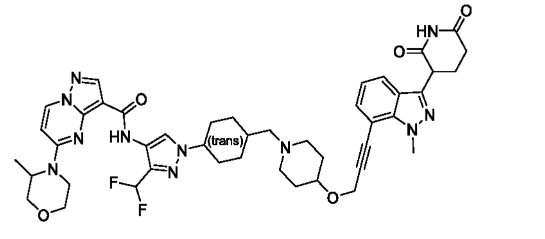 | 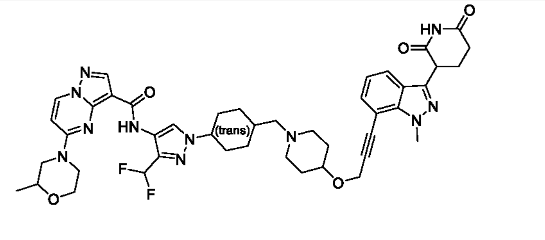 |
| 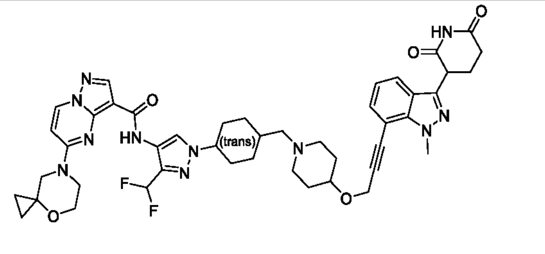 | 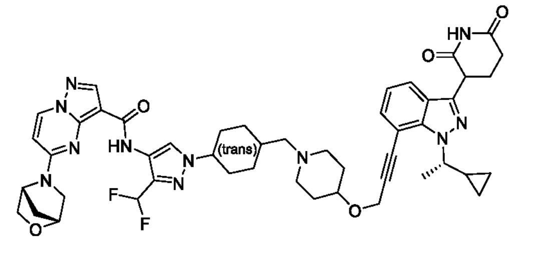 |
| 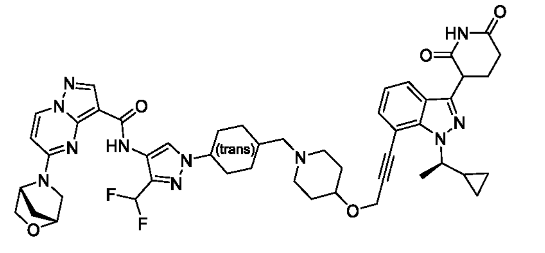 | 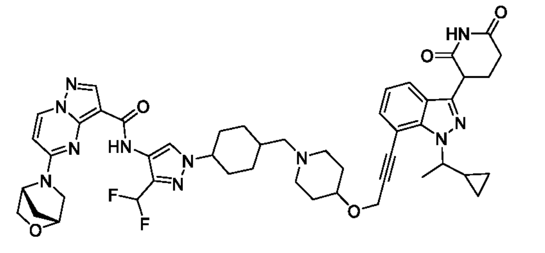 |
| 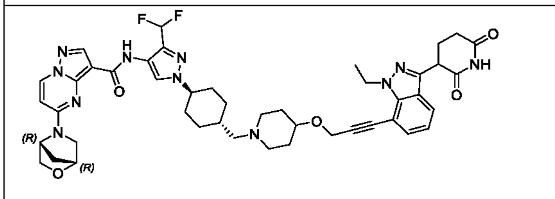 | 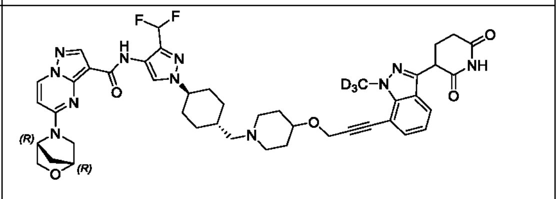 |
| 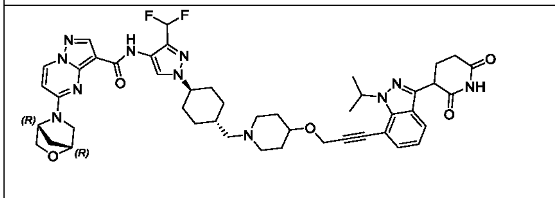 | 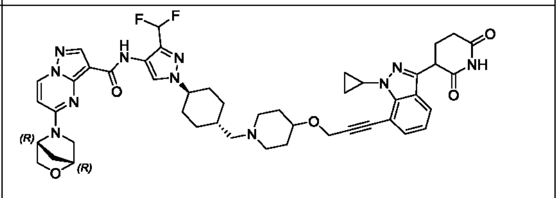 |
| 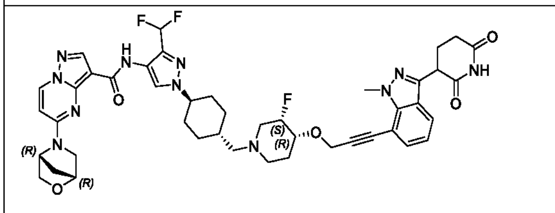 | 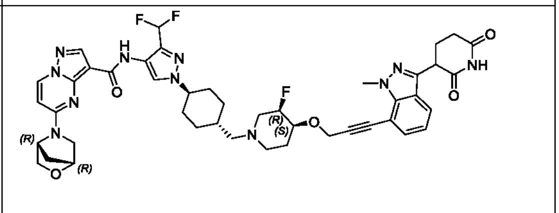 |
| 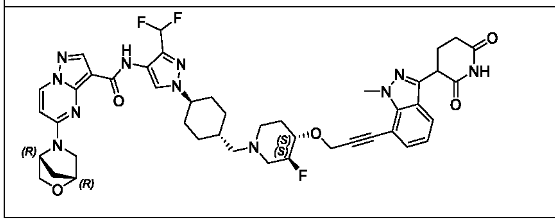 | 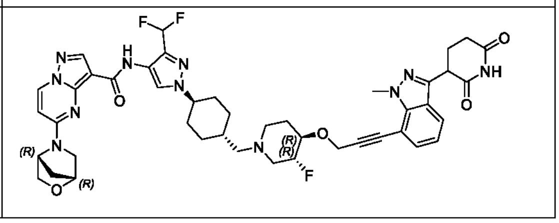 |

| | |
|---|---|
| 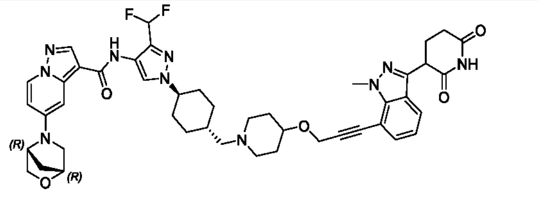 | 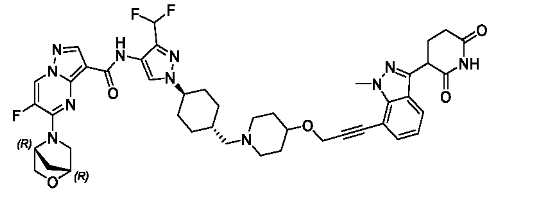 |
| 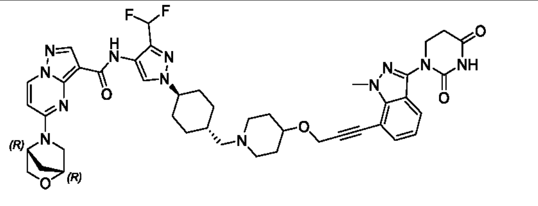 | 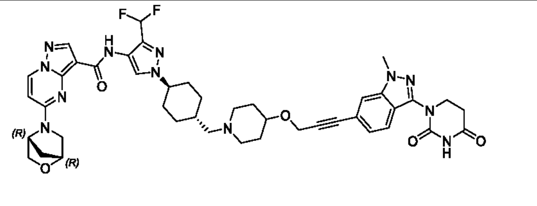 |
| 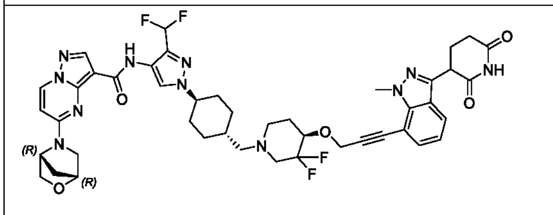 | 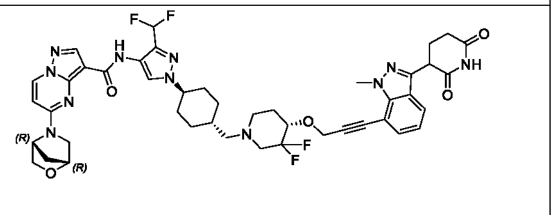 |
| 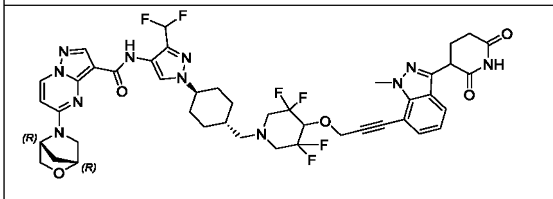 | |
| 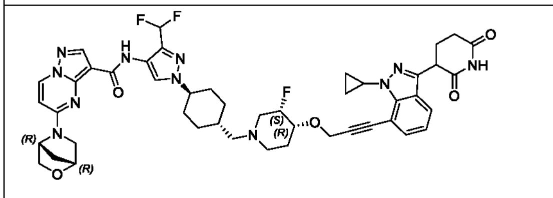 | 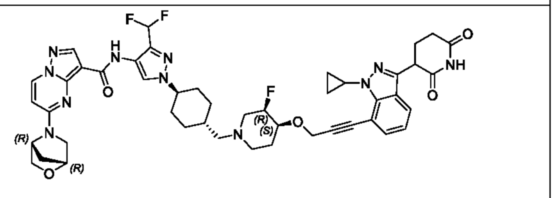 |
| 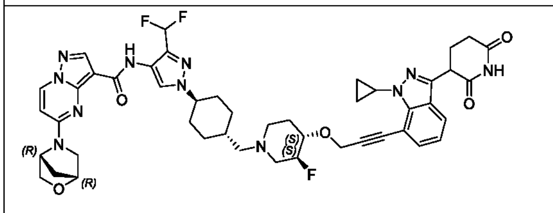 | 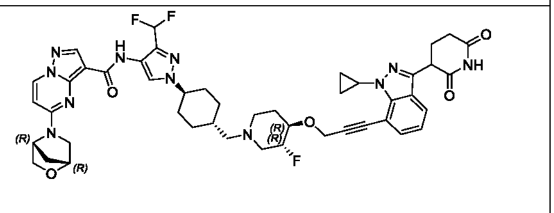 |
| 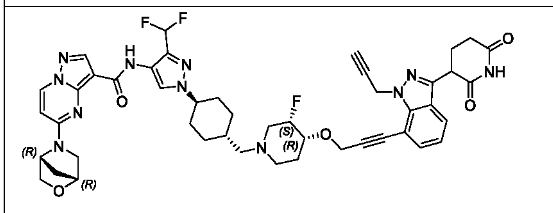 | 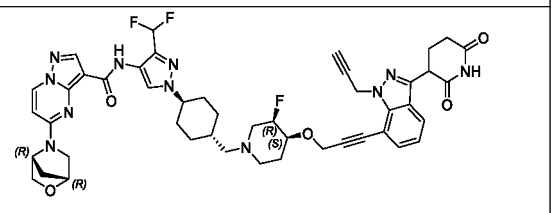 |
| 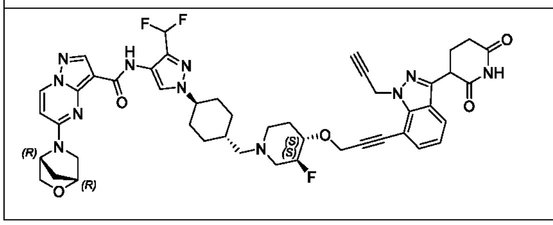 | 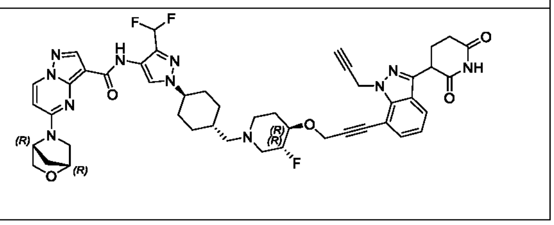 |

| | |
|---|---|
| 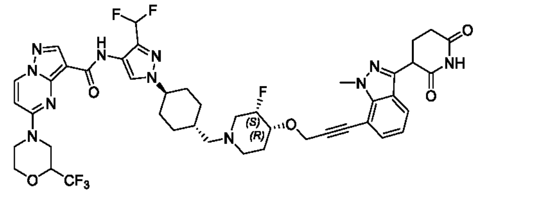 | 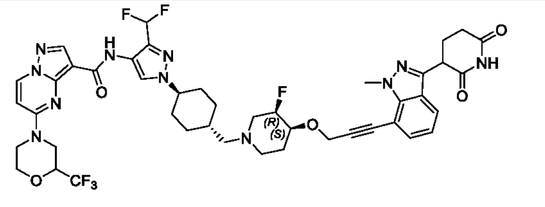 |
| 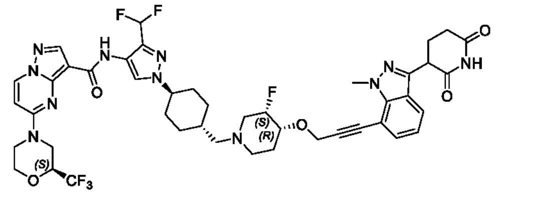 | 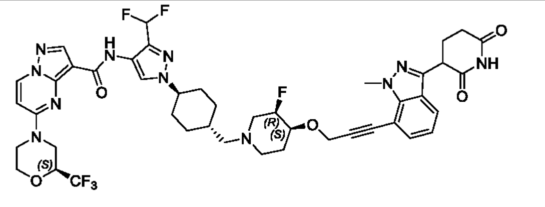 |
| 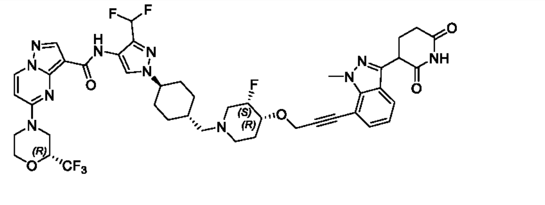 | 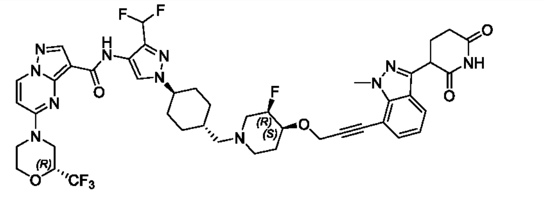 |
| 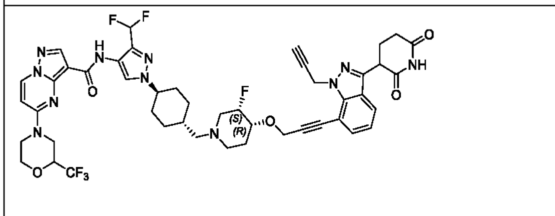 | 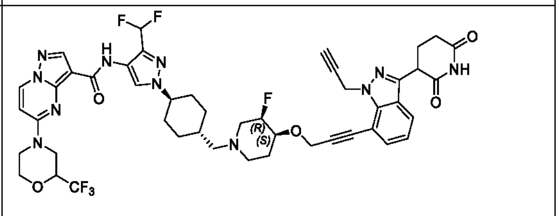 |
| 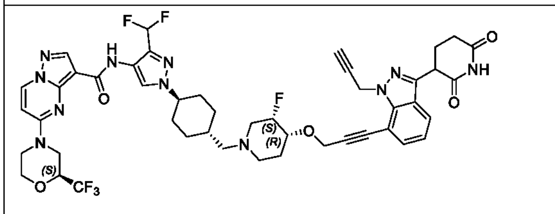 | 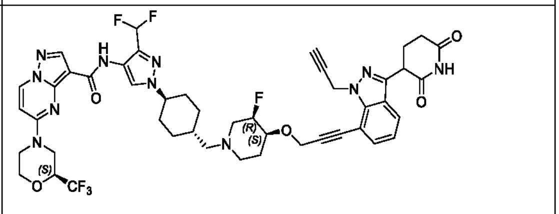 |
| 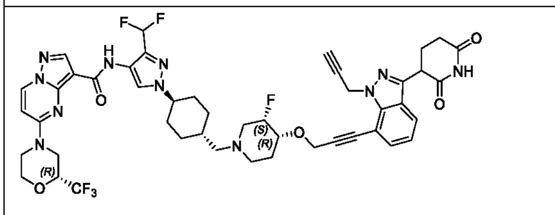 | 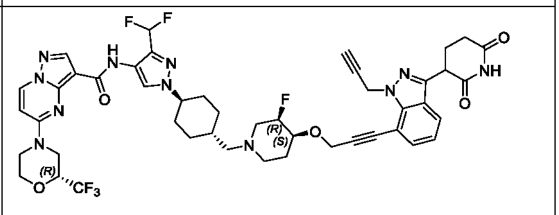 |
| 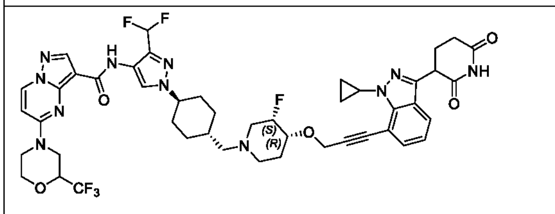 | 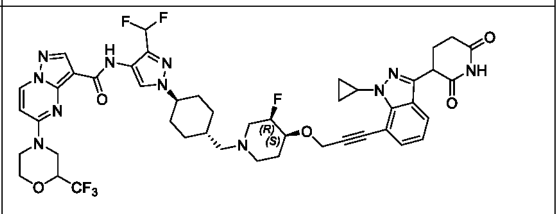 |
| 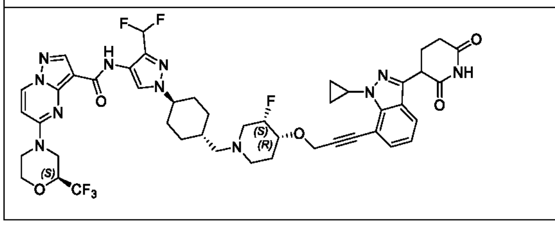 | 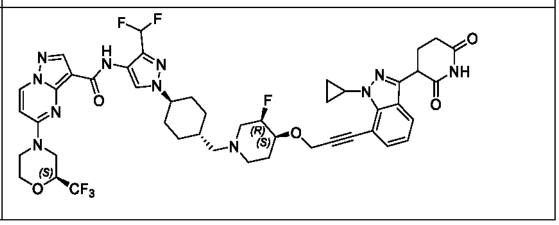 |

| | |
|---|---|
| 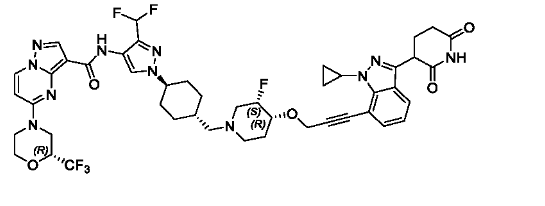 | 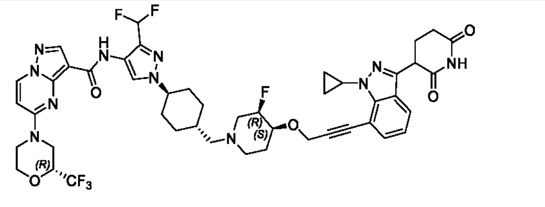 |
| 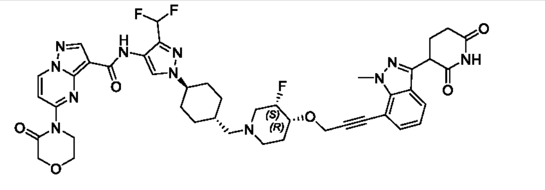 | 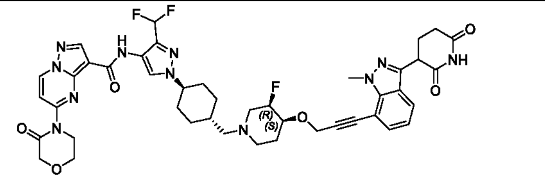 |
| 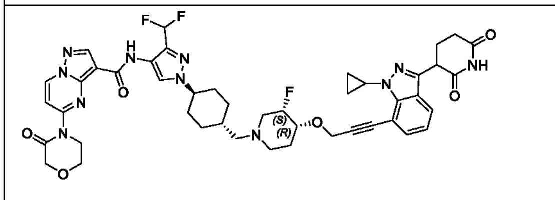 | 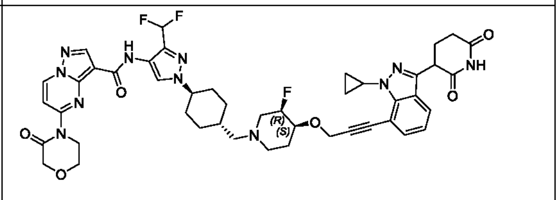 |
| 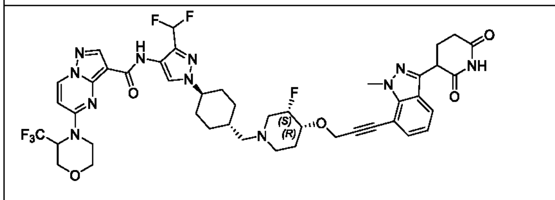 | 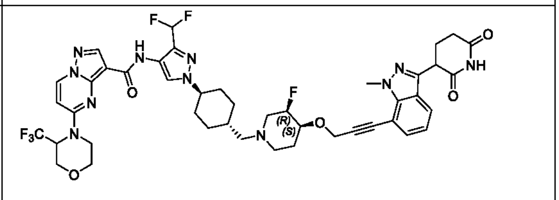 |
| 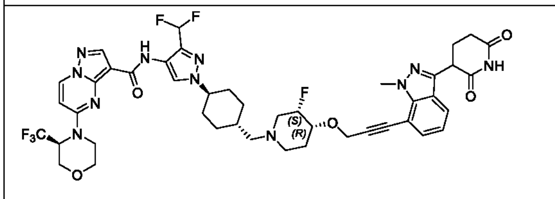 | 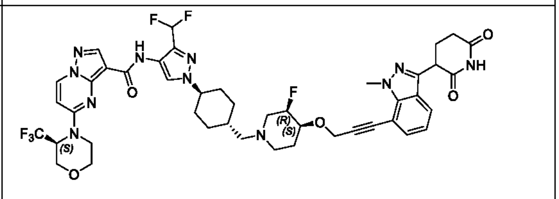 |
| 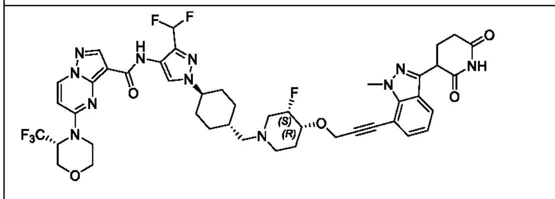 | 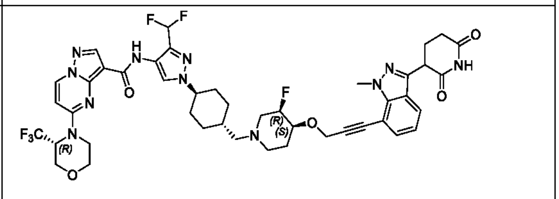 |
| 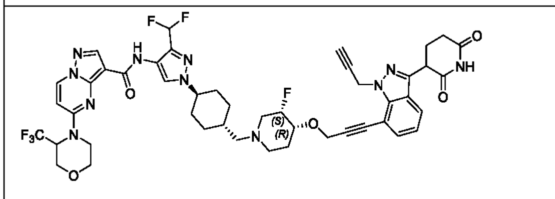 | 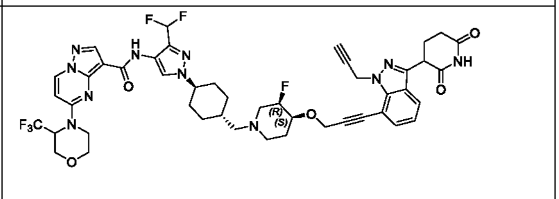 |
| 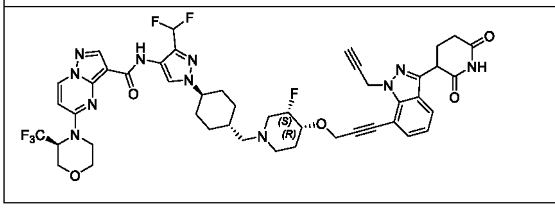 | 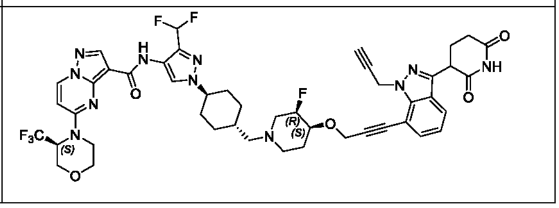 |

| | |
|---|---|
| 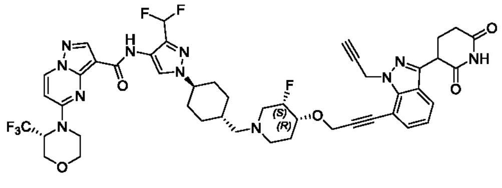 | 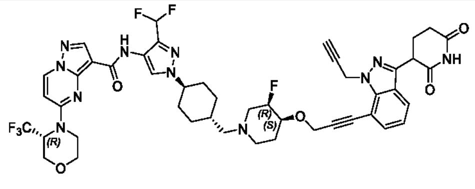 |
| 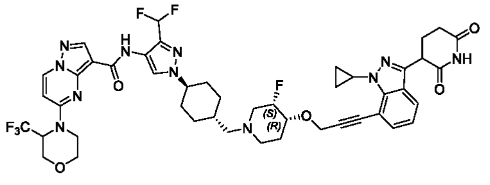 | 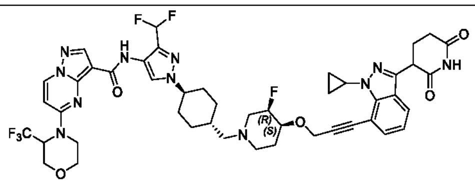 |
| 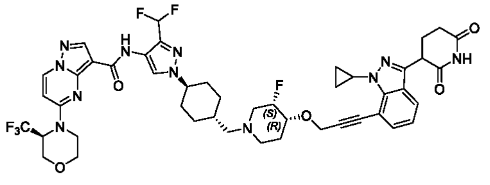 | 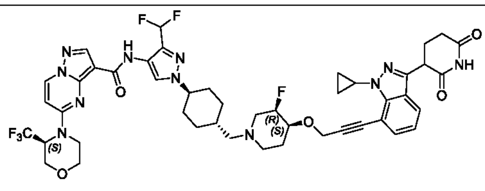 |
| 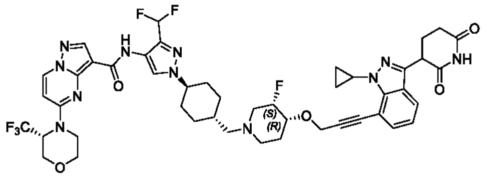 | 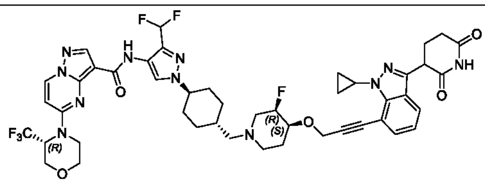 |
| 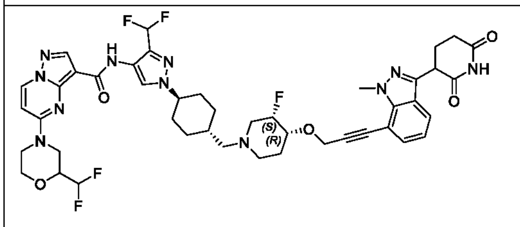 | 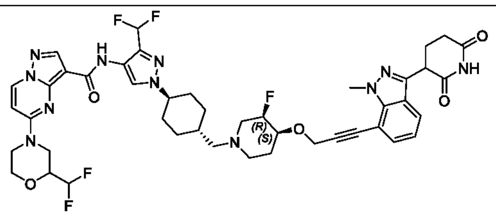 |
| 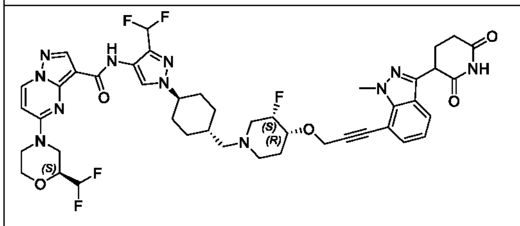 | 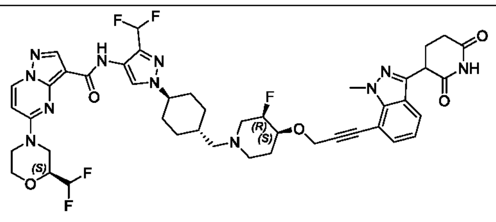 |
| 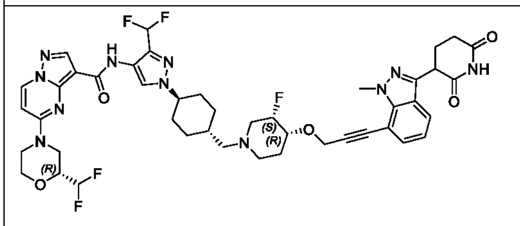 | 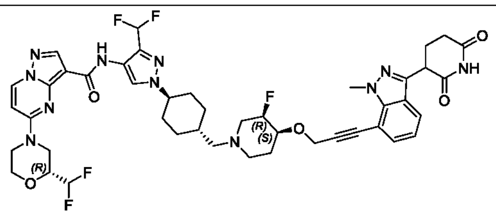 |
| 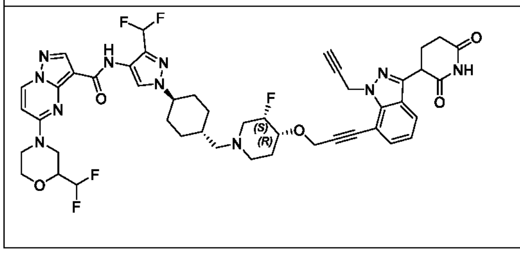 | 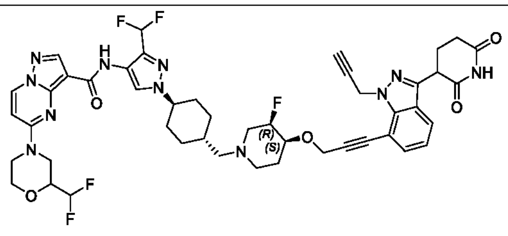 |

| | |
|---|---|
| 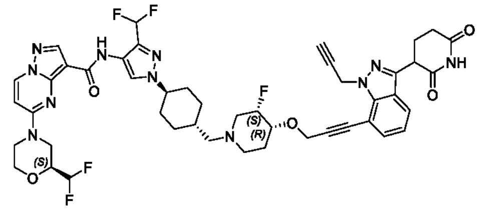 | 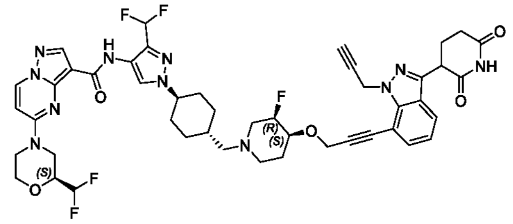 |
| 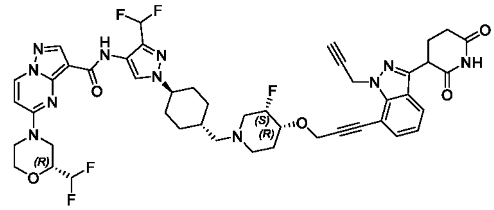 | 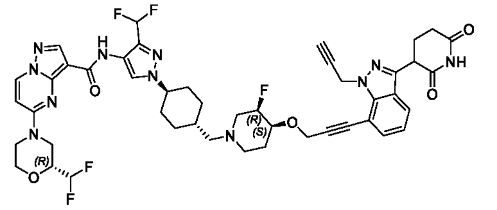 |
| 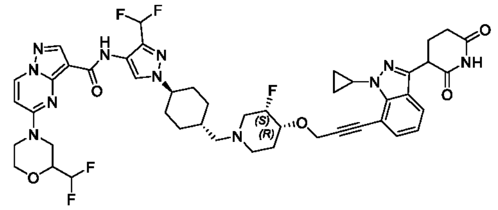 | 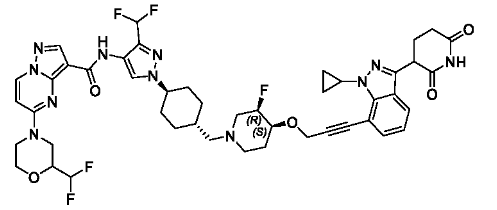 |
| 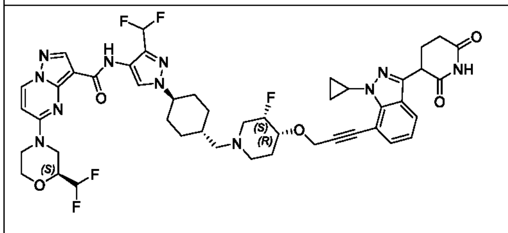 | 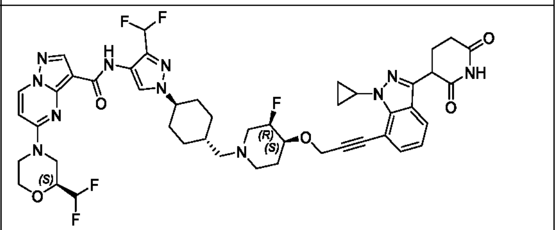 |
| 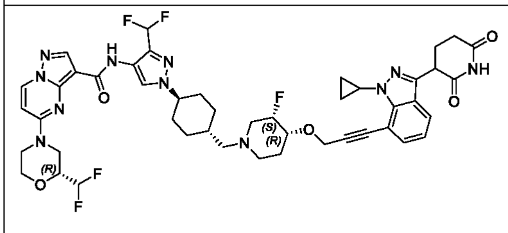 | 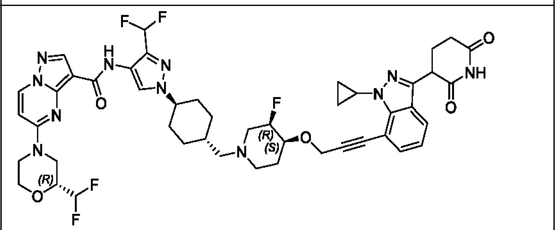 |
| 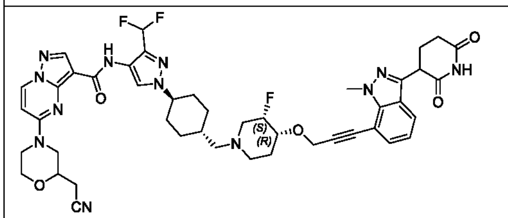 | 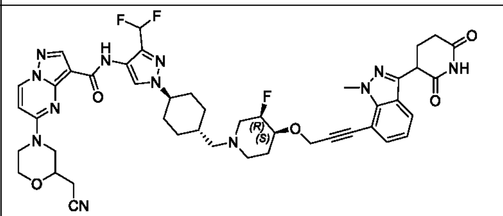 |
| 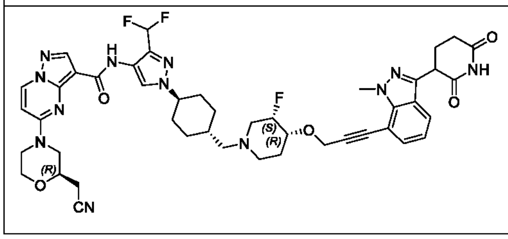 | 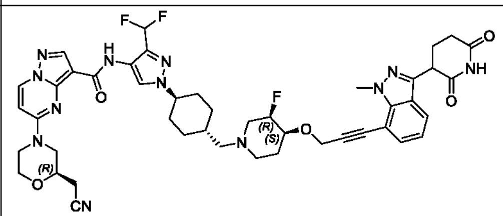 |

| | |
|---|---|
| 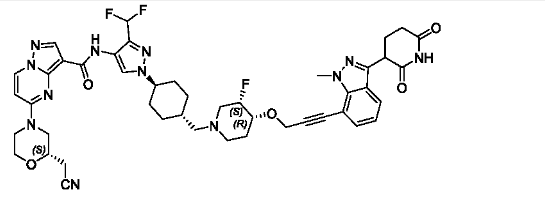 | 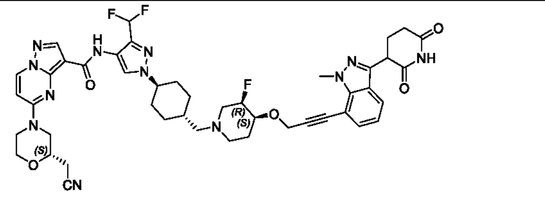 |
| 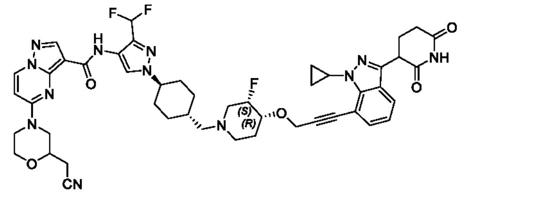 | 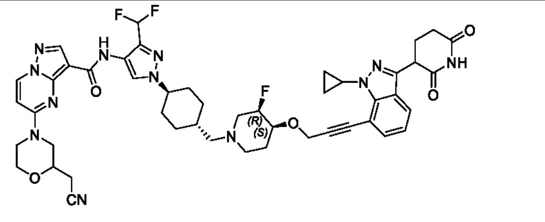 |
| 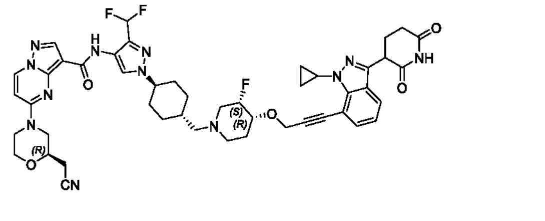 | 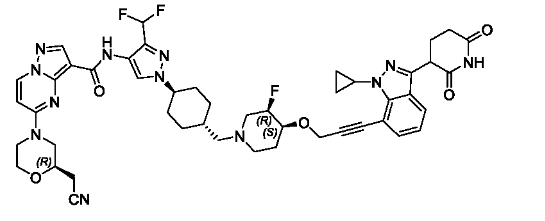 |
| 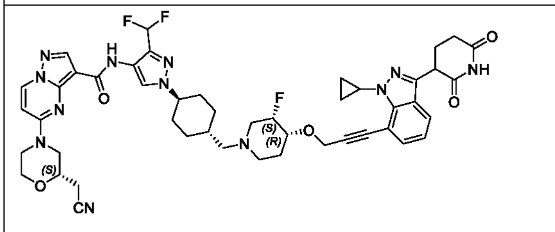 | 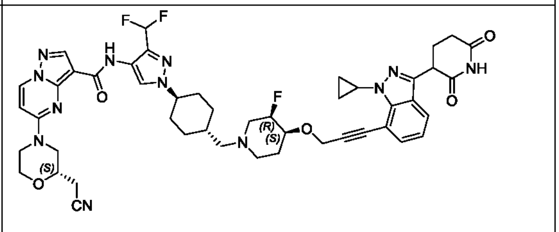 |
| 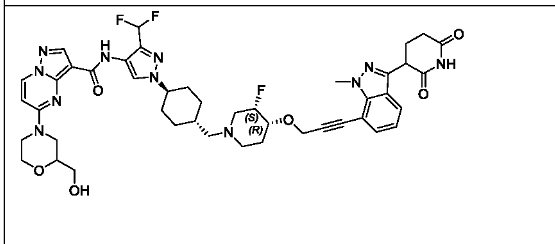 | 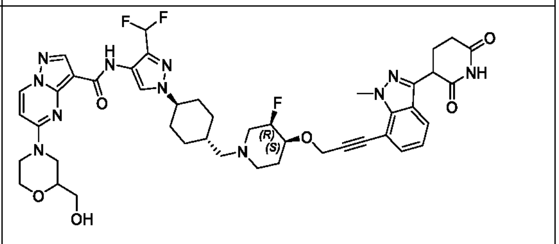 |
| 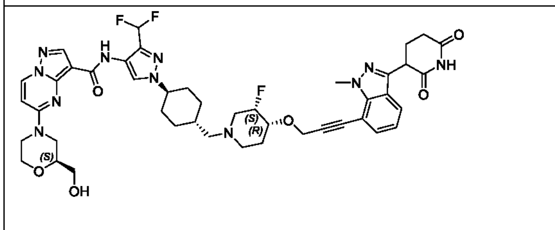 | 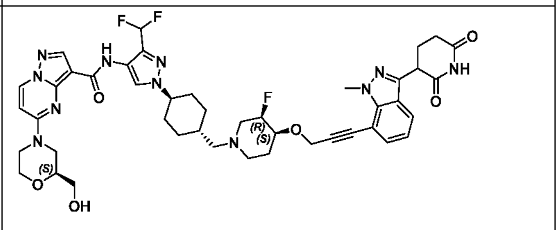 |
| 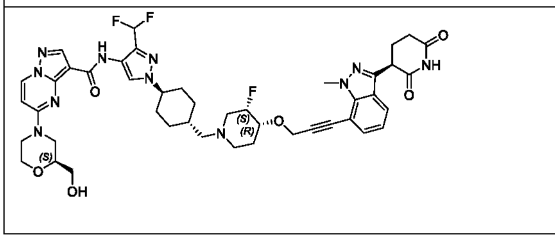 | 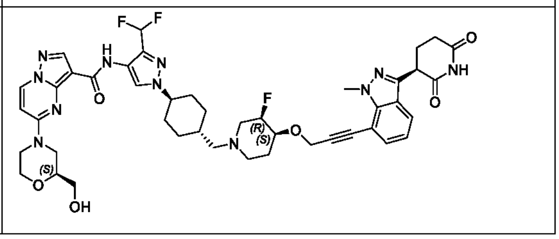 |

| | |
|---|---|
| 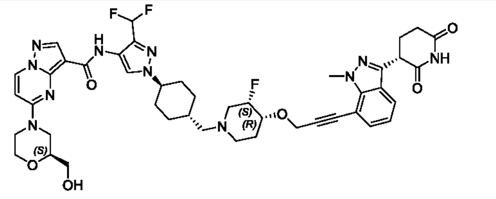 | 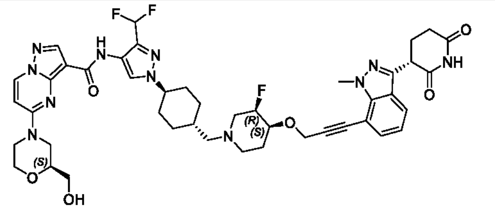 |
| 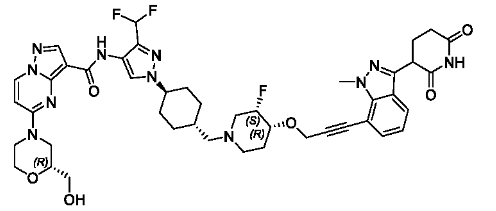 | 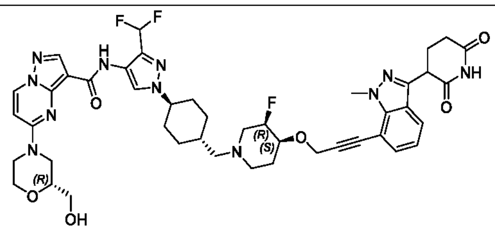 |
| 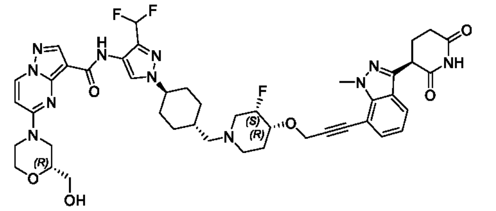 | 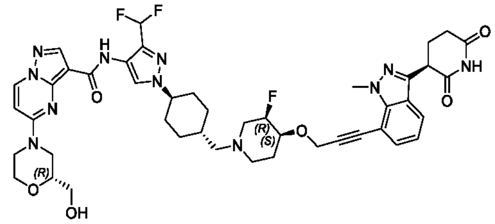 |
| 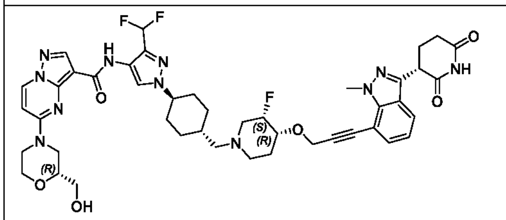 | 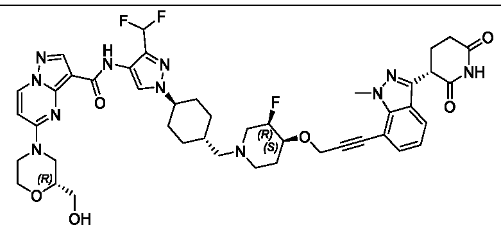 |
| 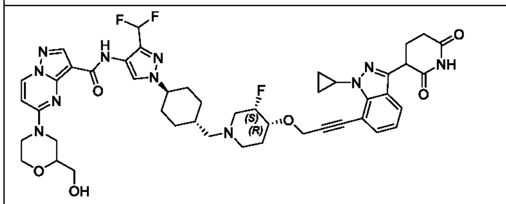 | 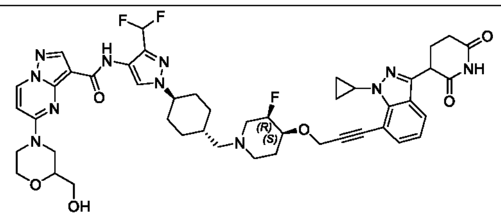 |
| 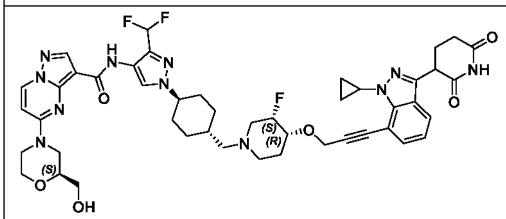 | 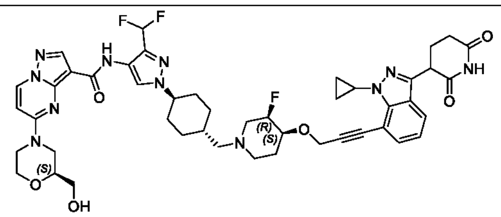 |
| 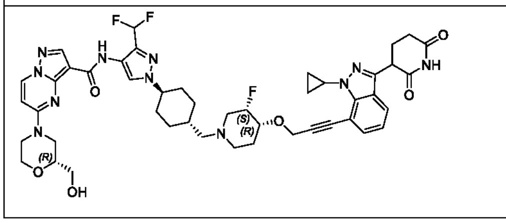 | 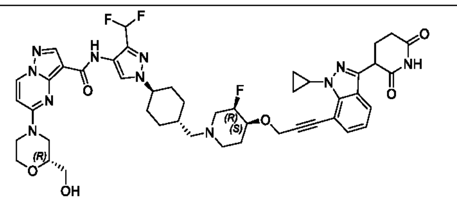 |

| | |
|---|---|
| 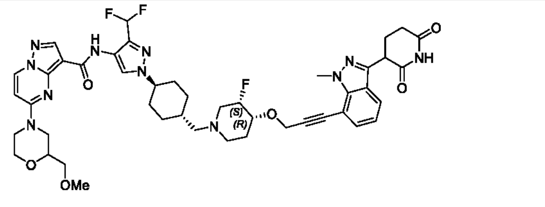 | 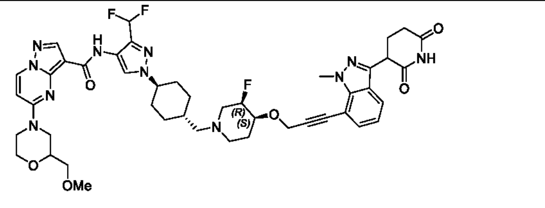 |
| 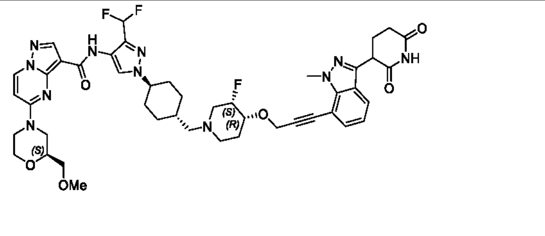 | 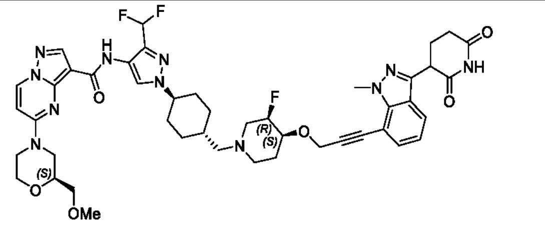 |
| 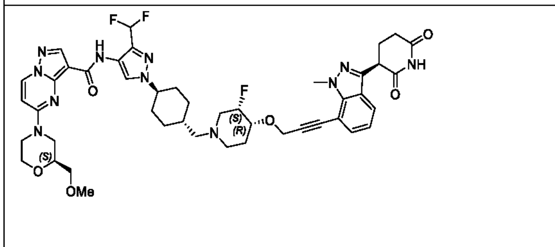 | 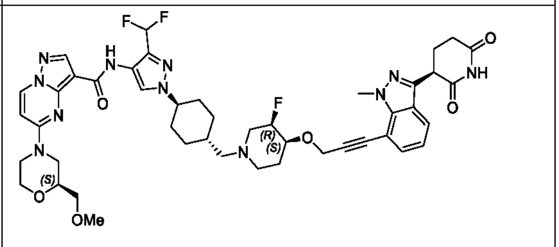 |
| 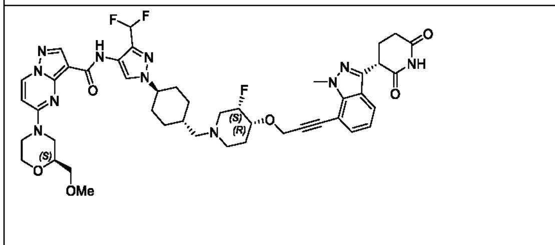 | 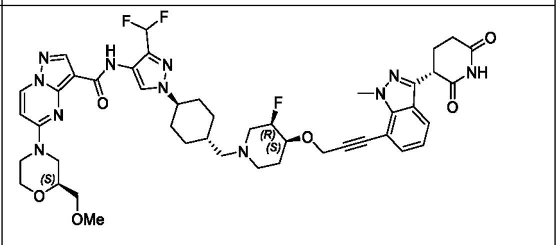 |
| 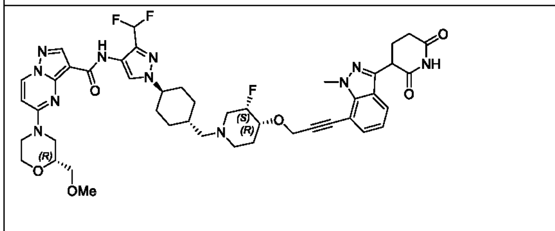 | 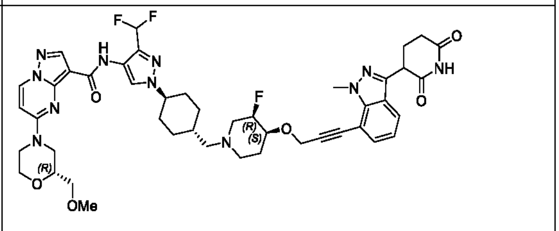 |
| 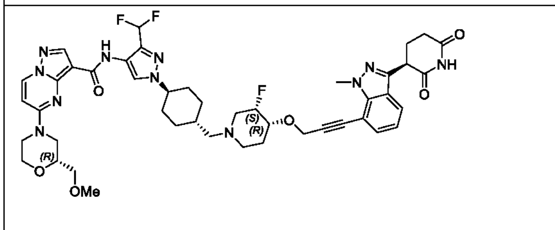 | 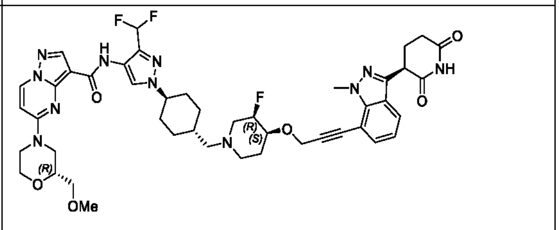 |
| 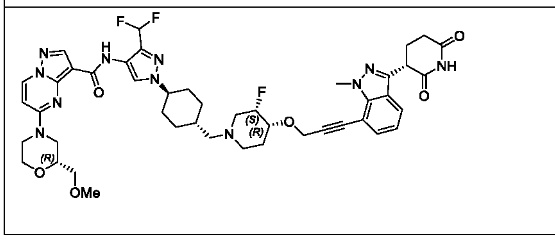 | 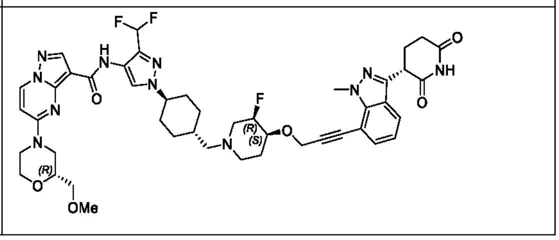 |

| | |
|---|---|
| 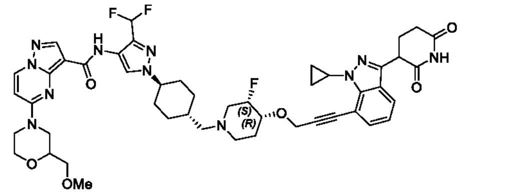 | 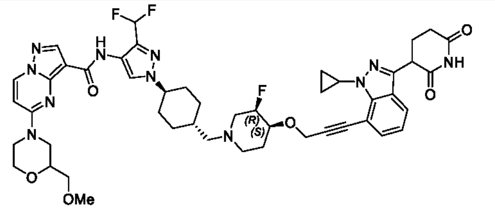 |
| 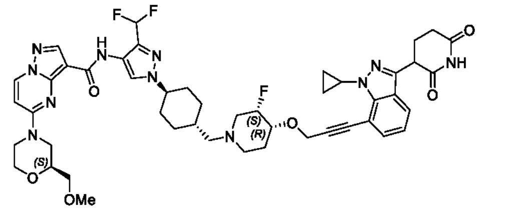 | 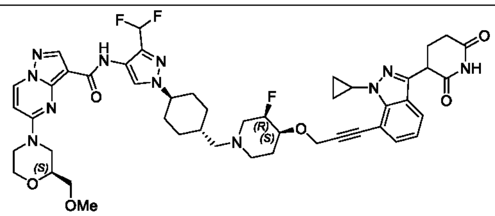 |
| 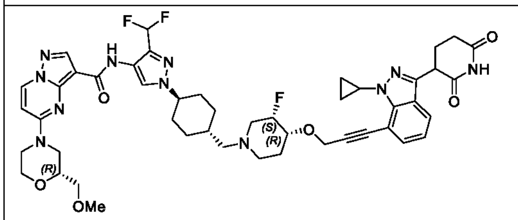 | 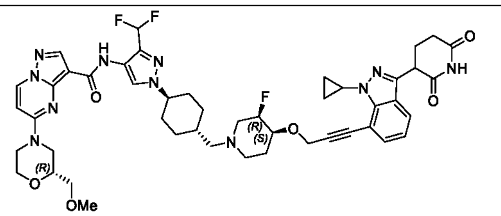 |
| 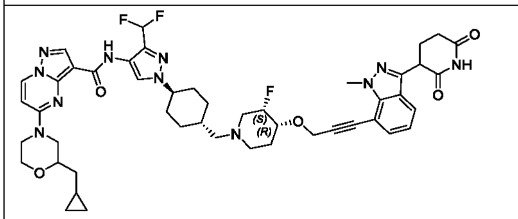 | 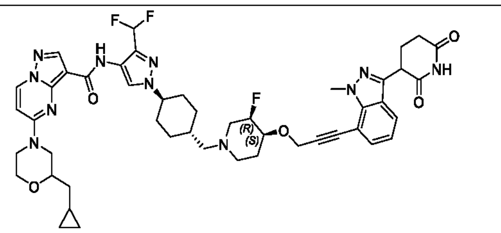 |
| 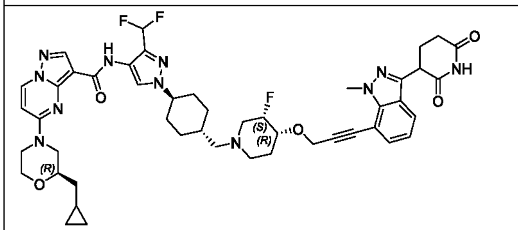 | 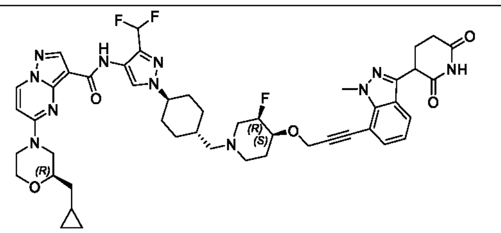 |
| 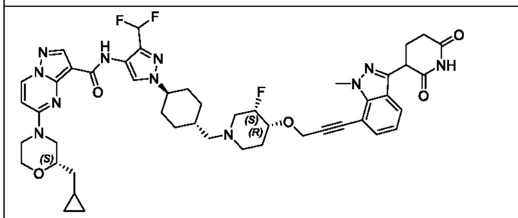 | 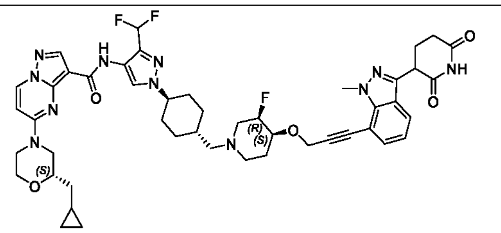 |
| 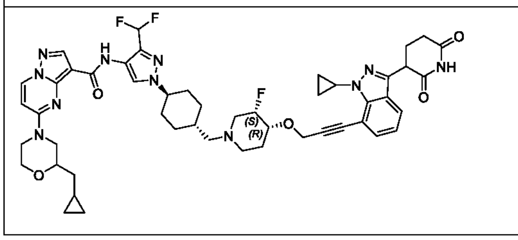 | 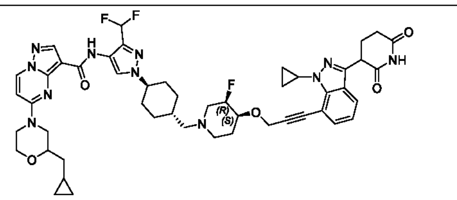 |

| | |
|---|---|
| 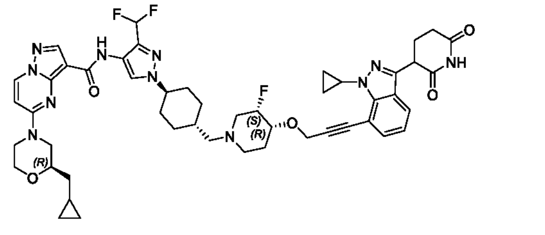 | 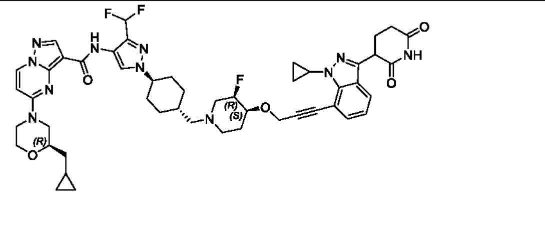 |
| 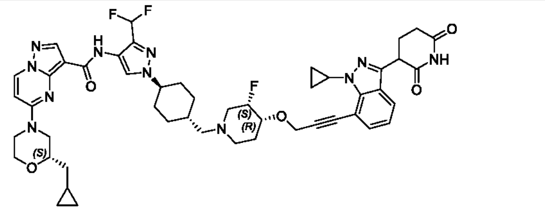 | 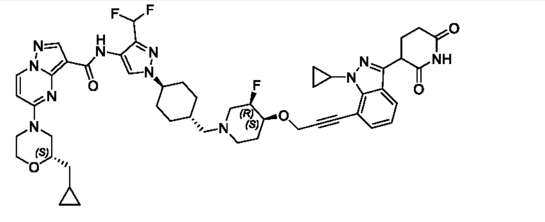 |
| 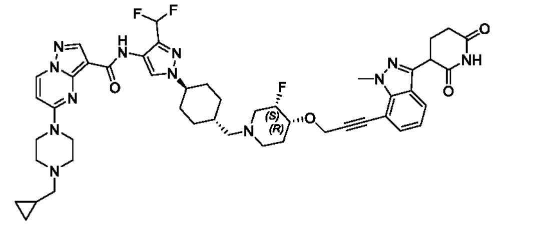 | 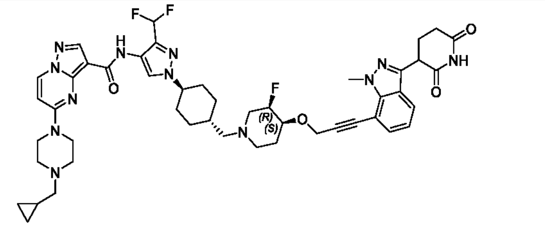 |
| 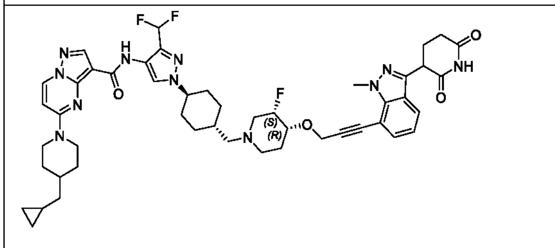 | 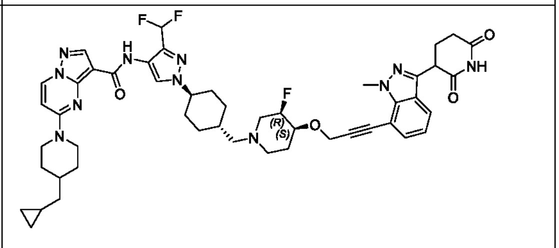 |
| 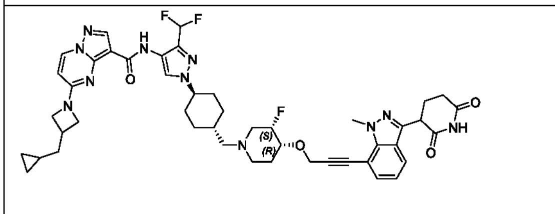 | 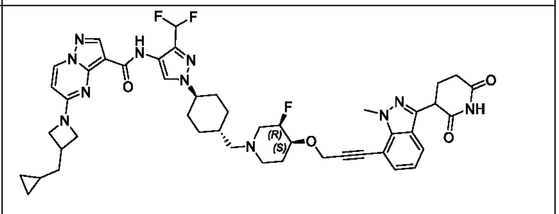 |
| 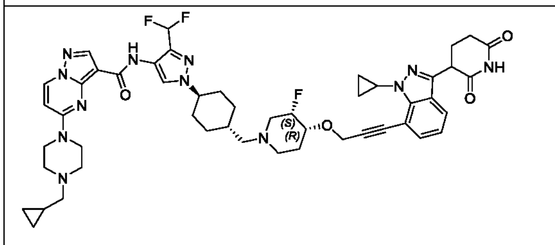 | 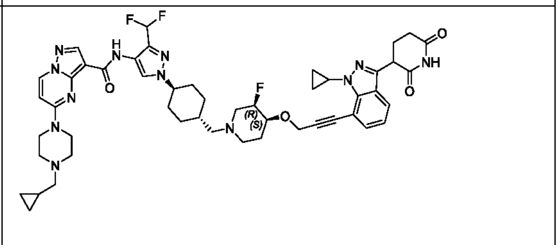 |
| 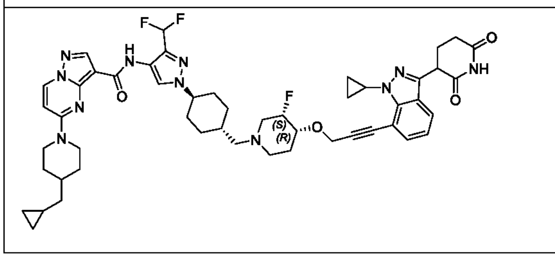 | 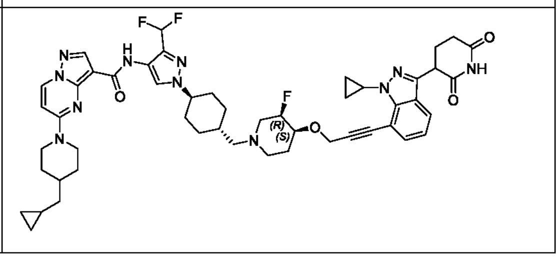 |

| | |
|---|---|
| 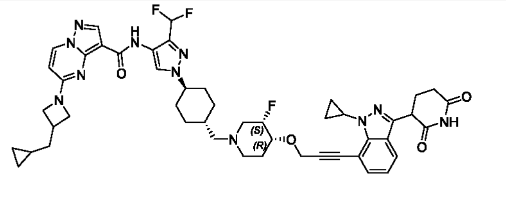 | 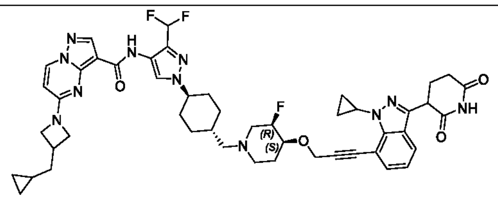 |
| 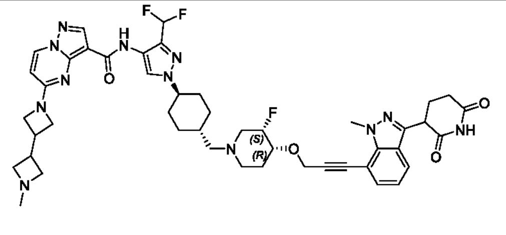 | 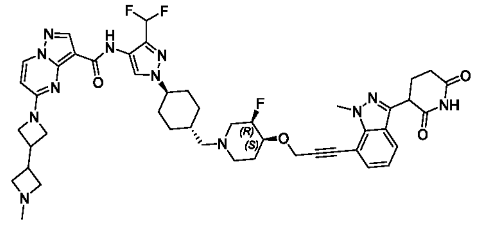 |
| 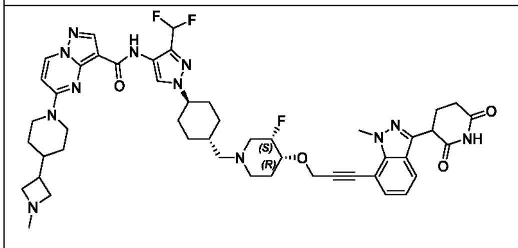 | 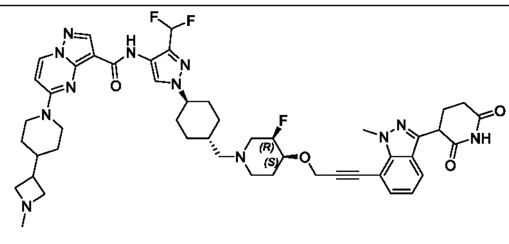 |
| 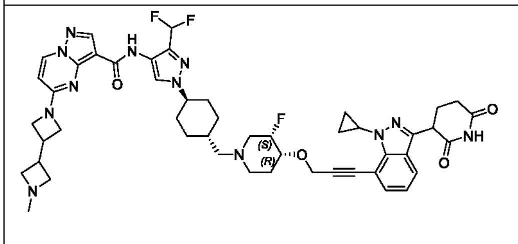 | 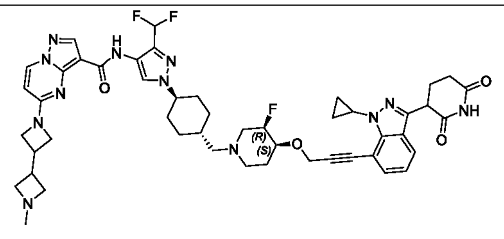 |
| 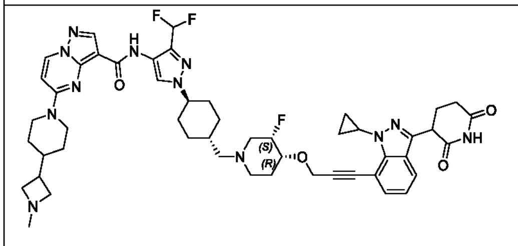 | 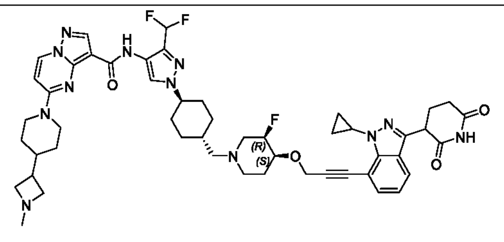 |
| 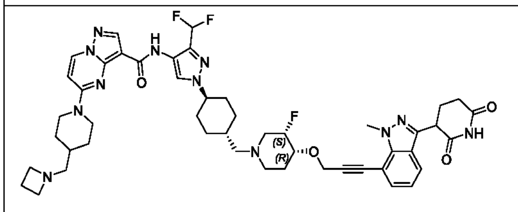 | 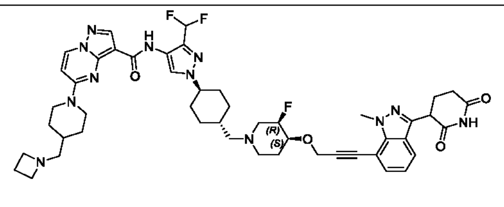 |
| 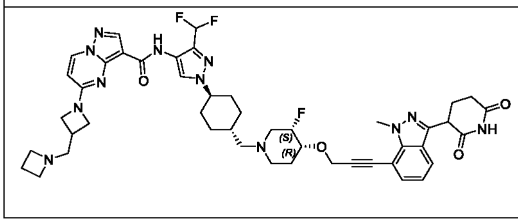 | 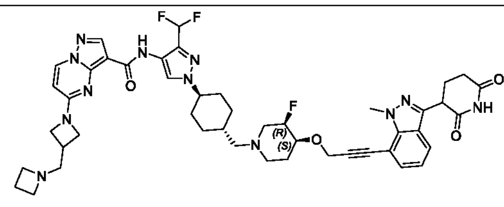 |

| | |
|---|---|
| 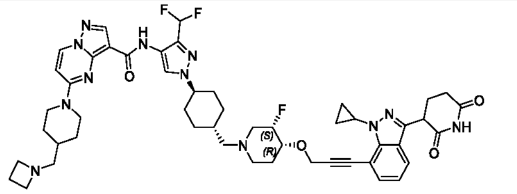 | 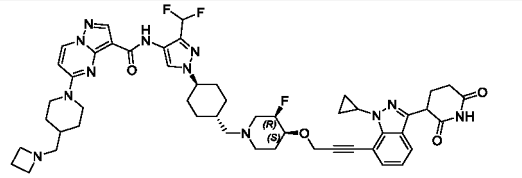 |
| 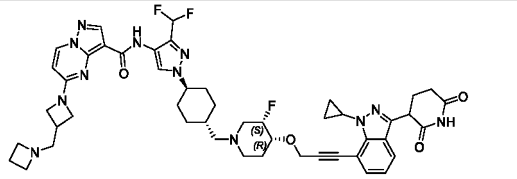 | 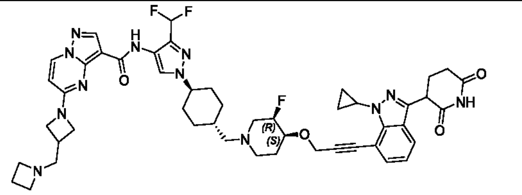 |
| 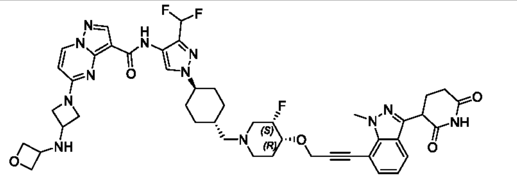 | 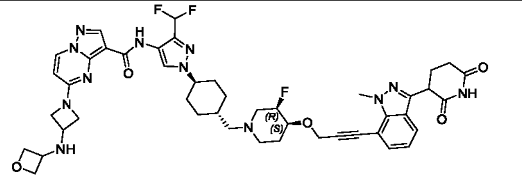 |
| 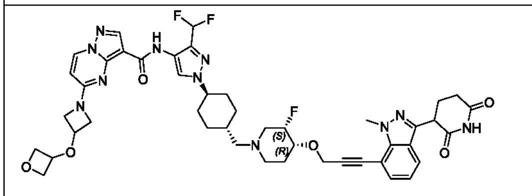 | 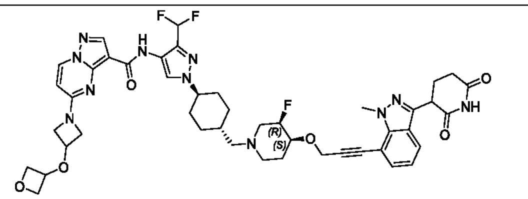 |
| 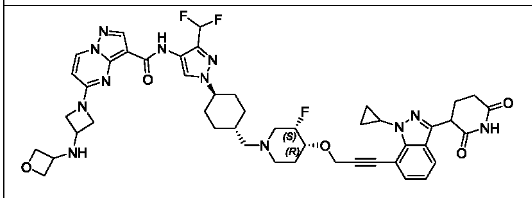 | 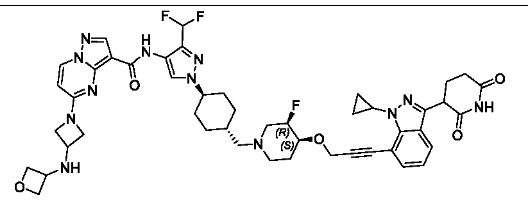 |
| 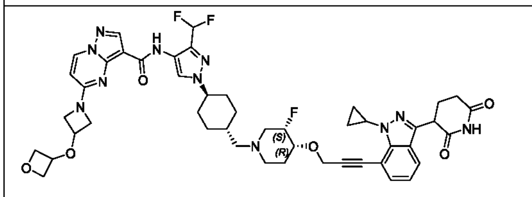 | 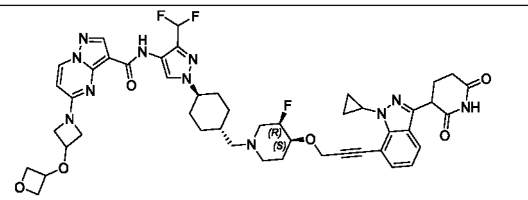 |
| 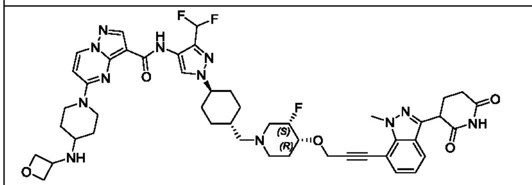 | 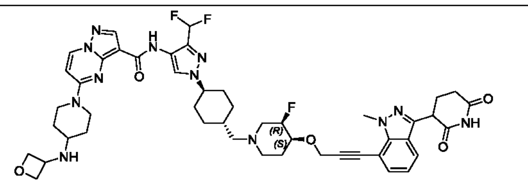 |
| 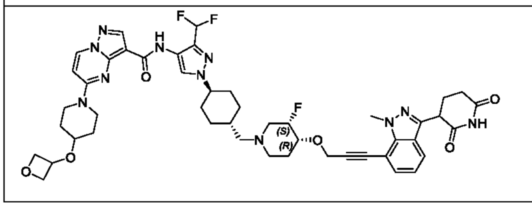 | 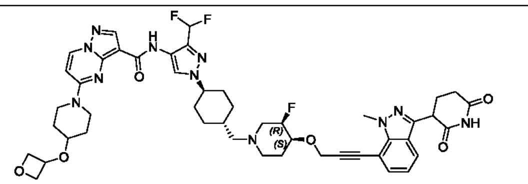 |

| | |
|---|---|
| 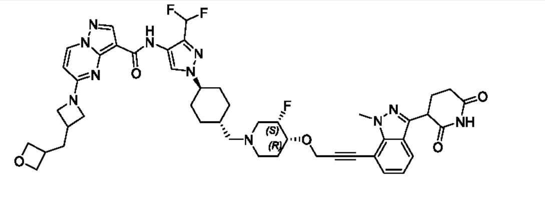 | 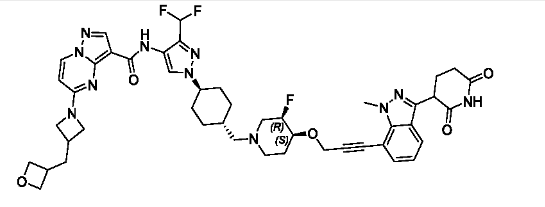 |
| 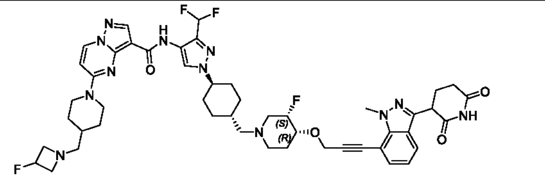 | 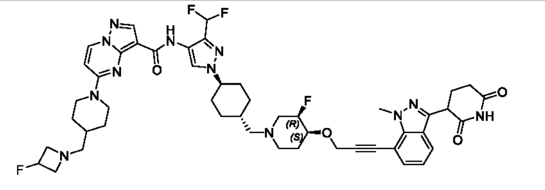 |
| 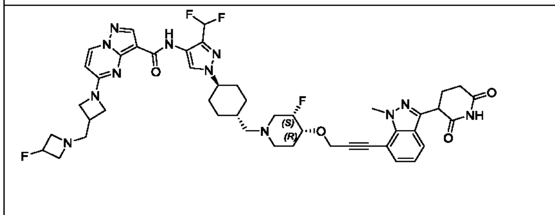 | 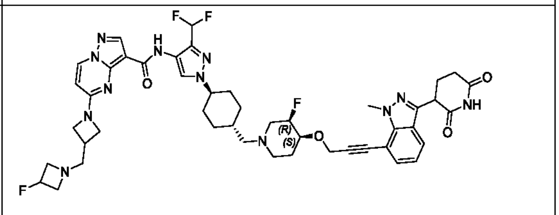 |
| 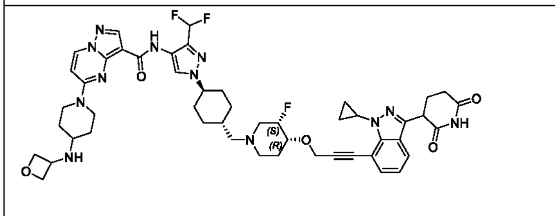 | 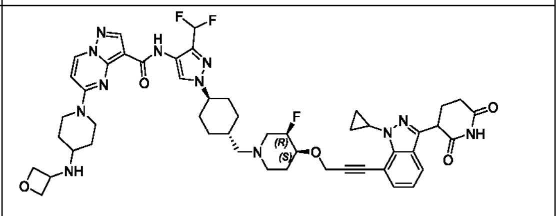 |
| 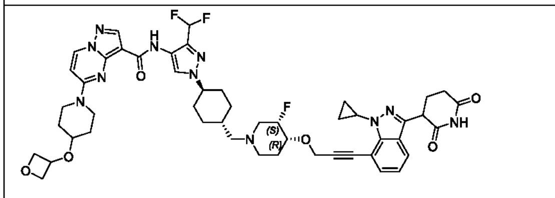 | 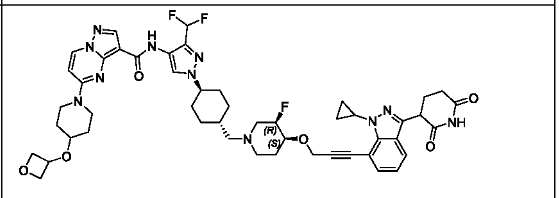 |
| 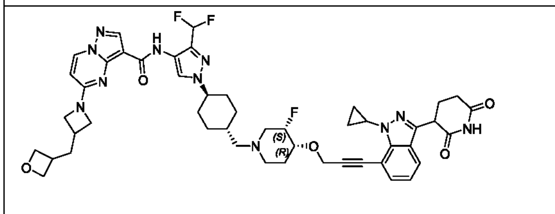 | 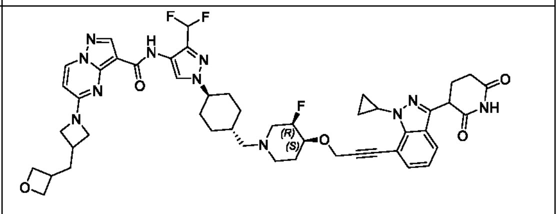 |
| 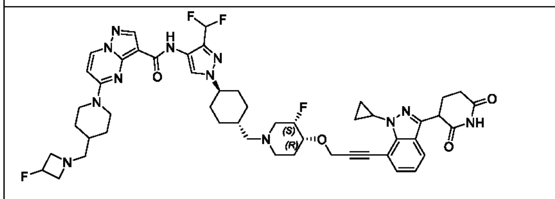 | 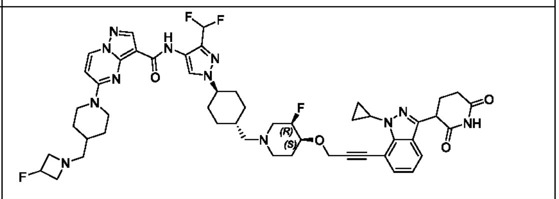 |
| 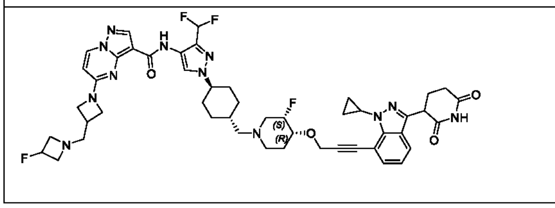 | 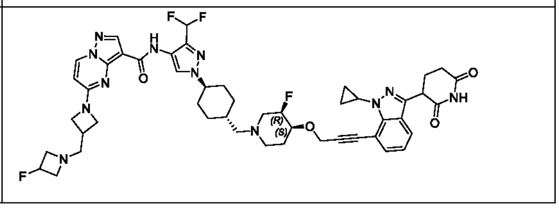 |

The present invention relates to a pharmaceutical composition, comprising the above-mentioned compound or the stereoisomer, deuterated compound, solvate, prodrug, metabolite, pharmaceutically acceptable salt or eutectic crystal thereof according to the present invention, and a pharmaceutically acceptable carrier.

The present invention relates to the use of the above-mentioned compound in the present invention or the stereoisomer, deuterated compound, solvate, prodrug, metabolite, pharmaceutically acceptable salt or eutectic crystal

thereof, or the pharmaceutical composition in the preparation of a medicament for the treatment of a disease related to IRAK4 activity or expression level.

The present invention relates to the use of the above-mentioned compound in the present invention or the stereoisomer, deuterated compound, solvate, prodrug, metabolite, pharmaceutically acceptable salt or eutectic crystal thereof, or the pharmaceutical composition in the preparation of a medicament for the treatment of a disease related to the inhibition or degradation of IRAK4.

The present invention relates to the use of the above-mentioned compound or the stereoisomer, deuterated compound, solvate, prodrug, metabolite, pharmaceutically acceptable salt or eutectic crystal thereof according to the present invention, wherein the disease is selected from an autoimmune disease, an inflammatory disease or cancer.

The present invention relates to a pharmaceutical composition or pharmaceutical preparation comprising a therapeutically effective amount of the compound, or the stereoisomer, deuterated compound, solvate, prodrug, metabolite, pharmaceutically acceptable salt or eutectic crystal thereof according to the present invention, and a pharmaceutically acceptable excipient. The pharmaceutical composition can be in a unit preparation form (the amount of the active drug in the unit preparation is also referred to as the "preparation specification").

The present invention further provides a method for treating a disease in a mammal, the method comprising administering to the mammal a therapeutically effective amount of the compound, or the stereoisomer, deuterated compound, solvate, prodrug, metabolite, pharmaceutically acceptable salt or eutectic crystal thereof or the pharmaceutical composition according to the present invention. In some embodiments, the mammal according to the present invention comprises humans.

The term "effective amount" or "therapeutically effective amount" according to the present application refers to a sufficient amount of the compound disclosed in the present application that is administered to ameliorate, to some extent, one or more symptoms of a disease or condition (such as an autoimmune disease, an inflammatory disease or cancer) being treated. In some embodiments, the outcome is the reduction and/or remission of signs, symptoms or causes of the disease, or any other desired change in the biological system. For example, an "effective amount" in terms of the therapeutic use is an amount of the composition comprising the compound disclosed in the present application that is required to provide clinically significant reduction of the symptoms of the disease. Examples of the therapeutically effective amount include, but are limited to 1-1500 mg, 1-1000 mg, 1-900 mg, 1-800 mg, 1-700 mg, 1-600 mg, 2-600 mg, 3-600 mg, 4-600 mg, 5-600 mg, 6-600 mg, 10-600 mg, 20-600 mg, 25-600 mg, 30-600 mg, 40-600 mg, 50-600 mg, 60-600 mg, 70-600 mg, 75-600 mg, 80-600 mg, 90-600 mg, 100-600 mg, 200-600 mg, 1-500 mg, 2-500 mg, 3-500 mg, 4-500 mg, 5-500 mg, 6-500 mg, 10-500 mg, 20-500 mg, 25-500 mg, 30-500 mg, 40-500 mg, 50-500 mg, 60-500 mg, 70-500 mg, 75-500 mg, 80-500 mg, 90-500 mg, 100-500 mg, 125-500 mg, 150-500 mg, 200-500 mg, 250-500 mg, 300-500 mg, 400-500 mg, 5-400 mg, 10-400 mg, 20-400 mg, 25-400 mg, 30-400 mg, 40-400 mg, 50-400 mg, 60-400 mg, 70-400 mg, 75-400 mg, 80-400 mg, 90-400 mg, 100-400 mg, 125-400 mg, 150-400 mg, 200-400 mg, 250-400 mg, 300-400 mg, 1-300 mg, 2-300 mg, 5-300 mg, 10-300 mg, 20-300 mg, 25-300 mg, 30-300 mg, 40-300 mg, 50-300 mg, 60-300 mg, 70-300 mg, 75-300 mg, 80-300 mg, 90-300 mg, 100-300 mg, 125-300 mg, 150-300 mg, 200-300 mg, 250-300 mg, 1-200 mg, 2-200 mg, 5-200 mg, 10-200 mg, 20-200 mg, 25-200 mg, 30-200 mg, 40-200 mg, 50-200 mg, 60-200 mg, 70-200 mg, 75-200 mg, 80-200 mg, 90-200 mg, 100-200 mg, 125-200 mg, 150-200 mg, 80-1500 mg, 80-1000 mg, and 80-800 mg;

in some embodiments, the pharmaceutical composition comprises the compound, or the stereoisomer, deuterated compound, solvate, prodrug, metabolite, pharmaceutically acceptable salt or eutectic crystal thereof according to the present invention in an amount including but not limited to 1-1500 mg, 1-1000 mg, 20-800 mg, 40-800 mg, 40-400 mg, 25-200 mg, 1 mg, 5 mg, 10 mg, 15 mg, 20 mg, 25 mg, 30 mg, 35 mg, 40 mg, 45 mg, 50 mg, 55 mg, 65 mg, 70 mg, 75 mg, 80 mg, 85 mg, 90 mg, 95 mg, 100 mg, 110 mg, 120 mg, 125 mg, 130 mg, 140 mg, 150 mg, 160 mg, 170 mg, 180 mg, 190 mg, 200 mg, 210 mg, 220 mg, 230 mg, 240 mg, 250 mg, 300 mg, 320 mg, 400 mg, 480 mg, 500 mg, 600 mg, 640 mg, 840 mg, and 1000 mg.

The present invention further provides a method for treating a disease in a mammal, the method comprising administering to a subject a therapeutically effective amount of the compound, or the stereoisomer, deuterated compound, solvate, prodrug, metabolite, pharmaceutically acceptable salt, or eutectic crystal thereof according to the present invention, wherein the therapeutically effective amount is preferably 1-1500 mg, and the disease is preferably an autoimmune disease, an inflammatory disease or cancer.

The present invention further provides a method for treating a disease in a mammal, the method comprising administering to a subject a medicament, i.e., the compound, or the stereoisomer, deuterated compound, solvate, prodrug, metabolite, pharmaceutically acceptable salt, or eutectic crystal thereof according to the present invention in a daily dose of 1-1500 mg/day, wherein the daily dose can be a single dose or divided doses; in some embodiments, the daily dose includes, but is not limited to 10-1500 mg/day, 10-1000 mg/day, 10-800 mg/day, 25-800 mg/day, 50-800 mg/day, 100-800 mg/day, 200-800 mg/day, 25-400 mg/day, 50-400 mg/day, 100-400 mg/day, or 200-400 mg/day; in some embodiments, the daily dose includes, but is not limited to 10 mg/day, 20 mg/day, 25 mg/day, 50 mg/day, 80 mg/day, 100 mg/day, 125 mg/day, 150 mg/day, 160 mg/day, 200 mg/day, 300 mg/day, 320 mg/day, 400 mg/day, 480 mg/day, 600 mg/day, 640 mg/day, 800 mg/day, 1000 mg/day, or 1500 mg/day.

The present invention relates to a kit, wherein the kit can comprise a composition in the form of a single dose or multiple doses and comprises the compound, or the stereoisomer, deuterated compound, solvate, prodrug, metabolite, pharmaceutically acceptable salt, or eutectic crystal thereof according to the present invention, and the amount of the compound or the stereoisomer, deuterated compound, solvate, prodrug, metabolite, pharmaceutically acceptable salt, or eutectic crystal thereof according to the present invention is identical to the amount of same in the above-mentioned pharmaceutical composition.

In the present invention, the amount of the compound, or the stereoisomer, deuterated compound, solvate, prodrug, metabolite, pharmaceutically acceptable salt or eutectic crystal thereof according to the present invention is calculated in the form of a free base in each case.

Unless stated to the contrary, the terms used in the description and claims have the following meanings.

The carbon, hydrogen, oxygen, sulfur, nitrogen or F, Cl, Br, I involved in the groups and compounds of the present invention all comprise their isotopes, and the carbon, hydrogen, oxygen, sulfur or nitrogen involved in the groups and compounds of the present invention is optionally further substituted with one or more of their corresponding isotopes, wherein the isotopes of carbon comprise $^{12}C$, $^{13}C$ and $^{14}C$, the isotopes of hydrogen comprise protium (H), deuterium (D, also known as heavy hydrogen), tritium (T, also known as superheavy hydrogen), the isotopes of oxygen comprise $^{16}O$, $^{17}O$ and $^{18}O$, the isotopes of sulfur comprise $^{32}S$, $^{33}S$, $^{34}S$ and $^{36}S$, the isotopes of nitrogen comprise $^{14}N$ and $^{15}N$, the isotopes of fluorine comprise $^{17}F$ and $^{19}F$, the isotopes of chlorine comprise $^{35}Cl$ and $^{37}Cl$, and the isotopes of bromine comprise $^{79}Br$ and $^{81}Br$.

"CN" refers to cyano.

"Halogen" refers to F, Cl, Br or I.

"Halogen-substituted" refers to F, Cl, Br or I substitution, including but not limited to a substitution with 1 to 10 substituents selected from F, Cl, Br or I, a substitution with 1 to 6 substituents selected from F, Cl, Br or I, or a substitution with 1 to 4 substituents selected from F, Cl, Br or I. "Halogen-substituted" is referred to simply as "halo".

"Alkyl" refers to a substituted or unsubstituted linear or branched saturated aliphatic hydrocarbyl group, including but not limited to an alkyl group of 1 to 20 carbon atoms, an alkyl group of 1 to 8 carbon atoms, an alkyl group of 1 to 6 carbon atoms, or an alkyl group of 1 to 4 carbon atoms. Non-limiting examples include methyl, ethyl, n-propyl, isopropyl, n-butyl, sec-butyl, neobutyl, tert-butyl, n-pentyl, isoamyl, neopentyl, n-hexyl and various branched isomers thereof. The definition of the "alkyl" herein is consistent with this definition. Alkyl can be monovalent, divalent, trivalent or tetravalent.

"Alkylene" refers to a substituted or unsubstituted linear or branched divalent saturated hydrocarbyl group, including $-(CH_2)_v-$ (v is an integer from 1 to 10), and examples of alkylene include, but are not limited to, methylene, ethylene, propylene, butylene, *etc.*

"Cycloalkyl" refers to a substituted or unsubstituted saturated carbocyclic hydrocarbyl group, usually having from 3 to 10 carbon atoms, and non-limiting examples include cyclopropyl, cyclobutyl, cyclopentyl, cyclohexyl, cycloheptyl, etc. The "cycloalkyl" herein is as defined above. Cycloalkyl can be monovalent, divalent, trivalent or tetravalent.

"Heterocycloalkyl" refers to a substituted or unsubstituted saturated heteroatom-containing cyclic hydrocarbyl group, including but not limited to 3 to 10 atoms, 3 to 8 atoms, or 1 to 3 heteroatoms selected from N, O or S. N and S selectively substituted in the heterocycloalkyl ring can be oxidized to various oxidation states. Heterocycloalkyl can be connected to a heteroatom or a carbon atom; heterocycloalkyl can be connected to an aromatic ring or a non-aromatic ring; and heterocycloalkyl can be connected to a bridged ring or a spiro ring. Non-limiting examples include oxiranyl, azacyclopropyl, oxacyclobutyl, azetidinyl, tetrahydrofuryl, tetrahydro-2H-pyranyl, dioxolanyl, dioxanyl, pyrrolidyl, piperidyl, imidazolidinyl, oxazolidinyl, oxazinanyl, morpholinyl, hexahydropyrimidyl or piperazinyl. Heterocycloalkyl group can be monovalent, divalent, trivalent or tetravalent.

"Alkenyl" refers to a substituted or unsubstituted linear or branched unsaturated hydrocarbyl group, having at least 1, usually 1, 2 or 3 carbon-carbon double bonds, with a main chain including but not limited to 2 to 10, 2 to 6, or 2 to 4 carbon atoms. Examples of alkenyl include, but are not limited to, ethenyl, allyl, 1-propenyl, 2-propenyl, 1-butenyl, 2-butenyl, 3-butenyl, 1-pentenyl, 2-pentenyl, 3-pentenyl, 4-pentenyl, 1-methyl-1-butenyl, 2-methyl-1-butenyl, 2-methyl-3-butenyl, 1-hexenyl, 2-hexenyl, 3-hexenyl, 4-hexenyl, 5-hexenyl, 1-methyl-1-pentenyl, 2-methyl-1-pentenyl, 1-heptenyl, 2-heptenyl, 3-heptenyl, 4-heptenyl, 1-octenyl, 3-octenyl, 1-nonenyl, 3-nonenyl, 1-decenyl, 4-decenyl, 1,3-butadiene, 1,3-pentadiene, 1,4-pentadiene, 1,4-hexadiene, etc. The definition of the "alkenyl" herein is consistent with this definition. Alkenyl can be monovalent, divalent, trivalent or tetravalent.

"Alkynyl" refers to a substituted or unsubstituted linear or branched unsaturated hydrocarbyl group, having at least 1, usually 1, 2 or 3 carbon-carbon triple bonds, with a main chain including 2 to 10 carbon atoms, including but not limited to a main chain including 2 to 6 carbon atoms, or a main chain including 2 to 4 carbon atoms. Examples of alkynyl include, but are not limited to, ethynyl, propargyl, 1-propynyl, 2-propynyl, 1-butynyl, 2-butynyl, 3-butynyl, 1-pentynyl, 2-pentynyl, 3-pentynyl, 4-pentynyl, 1-methyl-1-butynyl, 2-methyl-1-butynyl, 2-methyl-3-butynyl, 1-hexynyl, 2-hexynyl, 3-hexynyl, 4-hexynyl, 5-hexynyl, 1-methyl-1-pentynyl, 2-methyl-1-pentynyl, 1-heptynyl, 2-heptynyl, 3-heptynyl, 4-heptynyl, 1-octynyl, 3-octynyl, 1-nonynyl, 3-nonynyl, 1-decynyl, 4-decynyl, *etc.* Alkynyl can be monovalent, divalent, trivalent or tetravalent.

"Alkoxy" refers to a substituted or unsubstituted -O-alkyl group. Non-limiting examples include methoxy, ethoxy, n-propoxy, isopropoxy, n-butoxy, sec-butoxy, tert-butoxy, n-pentoxy, n-hexyloxy, cyclopropoxy and cyclobutoxy.

"Carbocyclyl" or "carbocycle" refers to a substituted or unsubstituted saturated or unsaturated aromatic ring or non-aromatic ring, wherein the aromatic ring or non-aromatic ring can be a 3-to 8-membered monocyclic ring, a 4- to 12-membered bicyclic ring or a 10- to 15-membered tricyclic ring system. Carbocyclyl can be connected to an aromatic ring or a non-aromatic ring, wherein the aromatic ring or non-aromatic ring is optionally a monocyclic ring, a bridged ring or a spiro ring. Non-limiting examples include cyclopropane, cyclobutane, cyclopentane, cyclohexane, cycloheptane, 1-cyclopentyl-1-enyl, 1-cyclopentyl-2-enyl, 1-cyclopentyl-3-enyl, cyclohexyl, 1-cyclohexyl-2-enyl, 1-cyclohexyl-3-enyl, cyclohexenyl, a benzene ring, a naphthalene ring,

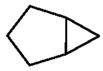, 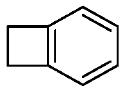, 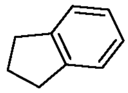 or 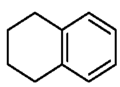

"Carbocyclyl" or "carbocycle" can be monovalent, divalent, trivalent or tetravalent.

"Heterocyclyl" or "heterocycle" refers to a substituted or unsubstituted saturated or unsaturated aromatic ring or non-aromatic ring, wherein the aromatic ring or non-aromatic ring can be a 3- to 8-membered monocyclic ring, a 4- to 12-membered bicyclic ring or a 10- to 15-membered tricyclic ring system, and contains one or more (including but not limited to 2, 3, 4 or 5) heteroatoms selected from N, O or S, and the selectively substituted N and S in the heterocyclyl ring can be oxidized to various oxidation states. Heterocyclyl can be connected to a heteroatom or a carbon atom; heterocyclyl can be connected to an aromatic ring or a non-aromatic ring; and heterocyclyl can be connected to a bridged ring or a spiro ring. Non-limiting examples include oxiranyl, azacyclopropyl, oxacyclobutyl, azetidinyl, 1,3-dioxolanyl, 1,4-dioxolanyl, 1,3-dioxanyl, azacycloheptyl, pyridyl, furyl, thienyl, pyranyl, N-alkylpyrrolyl, pyrimidyl, pyrazinyl, pyridazinyl, imidazolyl, piperidyl, morpholinyl, thiomorpholinyl, 1,3-dithianyl, dihydrofuryl, dihydropyranyl, dithiolanyl, tetrahydrofuryl, tetrahydropyrrolyl, tetrahydroimidazolyl, tetrahydrothiazolyl, tetrahydropyranyl, benzopyridyl, pyrrolopyridyl, benzodihydrofuryl, pyrrolyl, pyrazolyl, thiazolyl, oxazolyl, pyrazinyl, indazolyl, benzothienyl, benzofuryl, benzopyrrolyl, benzimidazolyl, benzothiazolyl, benzoxazolyl, benzopyridyl, benzopyrimidyl, benzopyrazinyl, piperazinyl, azabicyclo[3.2.1]octanyl, azabicyclo[5.2.0]nonanyl, oxatricyclo[5.3.1.1]dodecyl, azaadamantyl, oxaspiro[3.3]heptanyl,

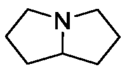, 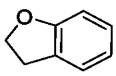,

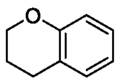, 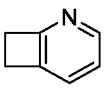, 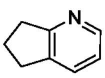, 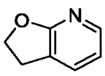, 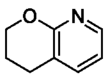, 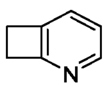, 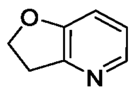, 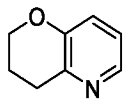, 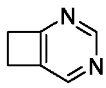,

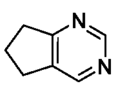, 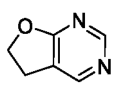 or 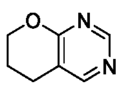

"Heterocyclyl" or "heterocycle" can be monovalent, divalent, trivalent or tetravalent.

"Spiro ring" or "spiro ring group" refers to a polycyclic group that shares one atom (called a spiro atom) between substituted or unsubstituted monocyclic rings. The number of ring atoms in the spiro ring system includes but is not limited to 5 to 20, 6 to 14, 6 to 12, or 6 to 10, wherein one or more rings may contain 0 or more (including but not limited to 1, 2, 3 or 4) double bonds, and may optionally contain 0 to 5 heteroatoms selected from N, O or $S(=O)_n$ (n is 0, 1, or 2). Non-limiting examples include:

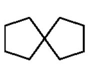 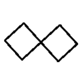 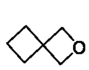 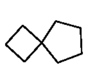 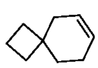 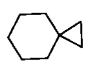 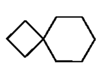

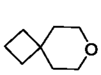 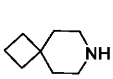 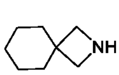 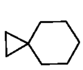 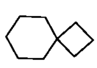 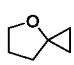 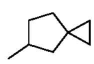

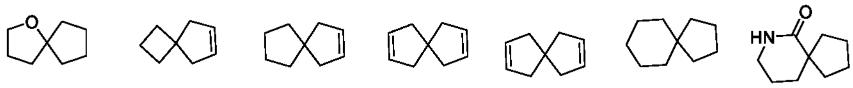

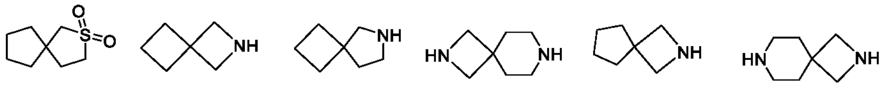

"Spiro ring" or "spiro ring group" can be monovalent, divalent, trivalent or tetravalent.

"Fused ring" or "fused ring group" refers to a polycyclic group in which each ring in the system shares an adjacent pair of atoms with other rings in the system, wherein one or more rings may contain 0 or more (including but not limited to 1, 2, 3 or 4) double bonds, and may be substituted or unsubstituted, and each ring in the fused ring system may contain 0 to 5 heteroatoms or groups containing heteroatoms (including but not limited to N, $S(=O)_n$ or O, wherein n is 0, 1 or 2). The number of ring atoms in the fused ring system includes but is not limited to 5 to 20, 5 to 14, 5 to 12, or 5 to 10. Non-limiting examples include:

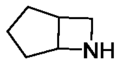,

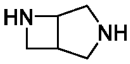, 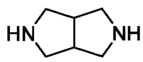,

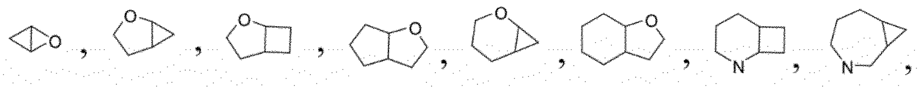

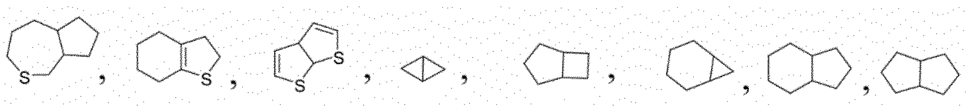

or

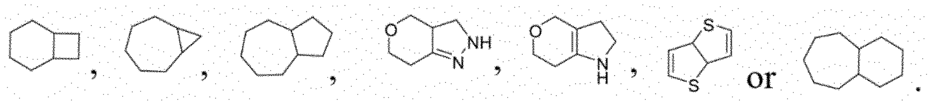

"Fused ring" or "fused ring group" can be monovalent, divalent, trivalent or tetravalent.

"Bridged ring" or "bridged ring group" refers to a substituted or unsubstituted polycyclic group containing any two atoms that are not directly connected, and may contain 0 or more double bonds. Any ring in the fused ring system may contain 0 to 5 groups selected from heteroatoms or groups containing heteroatoms (including but not limited to N, S(=O)n or O, wherein n is 0, 1 or 2). The number of ring atoms includes but is not limited to 5 to 20, 5 to 14, 5 to 12 or 5 to 10. Non-limiting examples include

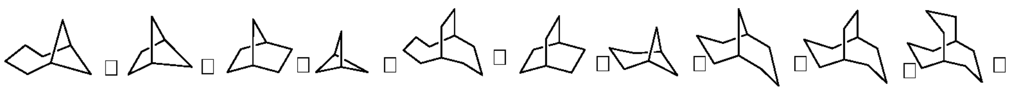

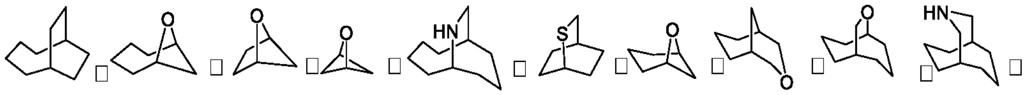

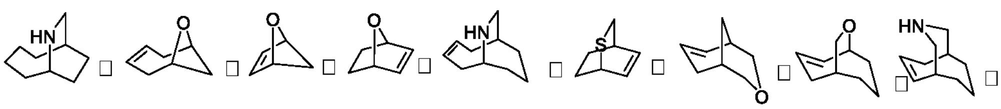

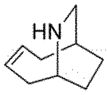, 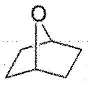, 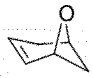, 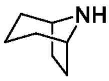, 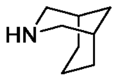, 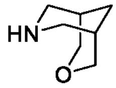, 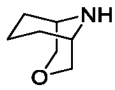,

cubane or adamantane. "Bridged ring" or "bridged ring group" can be monovalent, divalent, trivalent or tetravalent.

"Carbospiro ring", "spiro ring carbocyclyl", "spirocarbocyclyl" or "carbospiro ring group" refers to a "spiro ring" with a ring system consisting only of carbon atoms. The definition of the "carbospiro ring", "spiro ring carbocyclyl", "spirocarbocyclyl" or "carbospiro ring group" herein is consistent with that of a spiro ring.

"Carbo-fused ring", "fused ring carbocyclyl", "fused carbocyclyl" or "carbo-fused ring group" refers to a "fused ring" with a ring system consisting only of carbon atoms. The definition of the "carbo-fused ring", "fused ring carbocyclyl", "fused carbocyclyl" or "carbo-fused ring group" herein is consistent with that of a fused ring.

"Carbo-bridged ring", "bridged ring carbocyclyl", "bridged carbocyclyl" or "carbo-bridged ring group" refers to a "bridged ring" with a ring system consisting only of carbon atoms. The definition of the "carbo-bridged ring", "bridged ring carbocyclyl", "bridged carbocyclyl" or "carbo-bridged ring group" herein is consistent with that of a bridged ring.

"Mono-heterocyclic ring", "monocyclic heterocyclyl" or "mono-heterocyclic ring group" refers to "heterocyclyl" or "heterocycle" with a monocyclic system. The definition of the "heterocyclyl", "monocyclic heterocyclyl" or "mono-heterocyclic ring group" herein is consistent with that of heterocycle.

"Fused-heterocyclic ring", "fused-heterocyclic ring group", "fused ring heterocyclyl" or "fused-heterocyclic ring group" refers to a "fused ring" containing a heteroatom. The definition of the "fused-heterocyclic ring", "fused-heterocyclic ring group", "fused ring heterocyclyl" or "fused-heterocyclic ring group" herein is consistent with that of a fused ring.

"Spiro-heterocyclic ring", "spiro-heterocyclic ring group", "spiro ring heterocyclyl" or "spiro-heterocyclic ring group" refers to a "spiro ring" containing a heteroatom. The definition of the "spiro-heterocyclic ring", "spiro-heterocyclic ring group", "spiro ring heterocyclyl" or "spiro-heterocyclic ring group" herein is consistent with that of a spiro ring.

"Bridged-heterocyclic ring", "bridged-heterocyclic ring group", "bridged ring heterocyclyl" or "bridged-heterocyclic ring group" refers to a "bridged ring" containing a heteroatom. The definition of the "bridged-heterocyclic ring", "bridged-heterocyclic ring group", "bridged ring heterocyclyl" or "bridged-heterocyclic ring group" herein is consistent with that of a bridged ring.

"Aryl" or "aromatic ring" refers to a substituted or unsubstituted aromatic hydrocarbyl group with a monocyclic ring or a fused ring, wherein the number of ring atoms in the aromatic ring includes but is not limited to 6 to 18, 6 to 12 or 6 to 10 carbon atoms. The aryl ring can be fused to a saturated or unsaturated carbocycle or heterocycle, wherein the ring connected to the parent structure is an aryl ring. Non-limiting examples include a benzene ring, a naphthalene ring, or

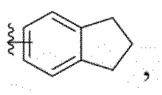, 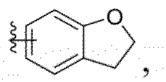, 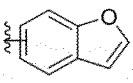, 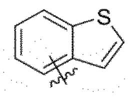, 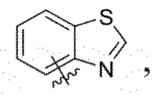,

and "aryl" or "aromatic ring" may be monovalent, divalent, trivalent or tetravalent. When divalent, trivalent or tetravalent, the point of connection is on the aryl ring.

"Heteroaryl" or "heteroaromatic ring" refers to a substituted or unsubstituted aromatic hydrocarbyl group containing 1 to 5 heteroatoms or groups containing heteroatoms (including but not limited to N, O or S(=O)n, wherein n is 0, 1 or 2), wherein the number of ring atoms in the heteroaromatic ring includes but is not limited to 5-15, 5-10 or 5-6. Non-limiting examples of heteroaryl include, but are not limited to pyridyl, furyl, thienyl, pyridyl, pyranyl, N-alkylpyrrolyl, pyrimidyl, pyrazinyl, pyridazinyl, imidazolyl, benzopyrazole, benzoimidazole, benzopyridine, pyrrolopyridine, etc. The heteroaryl ring may be fused to a saturated or unsaturated carbocycle or heterocycle, wherein the ring connected to the parent structure is a heteroaryl ring. Non-limiting examples include

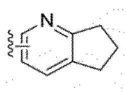, 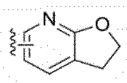, 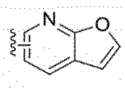, 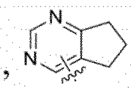, 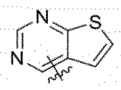, 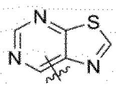, 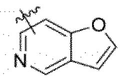

and

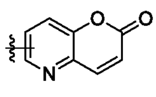

The definition of the "heteroaryl" herein is consistent with this definition. Heteroaryl can be monovalent, divalent, trivalent

or tetravalent. When divalent, trivalent or tetravalent, the point of connection is on the heteroaryl ring.

"Substitution" or "substituted" refers to a substitution with 1 or more (including but not limited to 2, 3, 4 or 5) substituents including but not limited to H, F, Cl, Br, I, alkyl, cycloalkyl, alkoxy, haloalkyl, mercaptan, hydroxyl, nitro, mercapto, amino, cyano, isocyano, aryl, heteroaryl, heterocyclyl, bridged ring group, spiro ring group, fused ring group, hydroxyalkyl, =O, carbonyl, aldehyde, carboxylic acid, carboxylate, $-(CH_2)_m-C(=O)-R^a$, $-O-(CH_2)_m-C(=O)-R^a$, $-(CH_2)_m-C(=O)-NR^bR^c$, $-(CH_2)_mS(=O)_nR^a$, $-(CH_2)_m$-alkenyl-$R^a$, $OR^d$ or $-(CH_2)_m$-alkynyl-$R^a$ (wherein m and n are 0, 1 or 2), arylthio, thiocarbonyl, silyl or $-NR^bR^c$ and the like, wherein $R^b$ and $R^c$ are independently selected from H, hydroxyl, amino, carbonyl, alkyl, alkoxy, cycloalkyl, heterocyclyl, aryl, heteroaryl, sulfonyl, or trifluoromethylsulfonyl. Alternatively, $R^b$ and $R^c$ may form a five- or six-membered cycloalkyl or heterocyclyl; each $R^a$ or $R^d$ is independently selected from aryl, heteroaryl, alkyl, alkoxy, cycloalkyl, heterocyclyl, carbonyl, ester group, bridged ring group, spiro ring group or fused ring group.

"Containing 1 to 4 heteroatoms selected from O, S or N" refers to contains 1, 2, 3 or 4 heteroatoms selected from O, S or N.

"Substituted with 0 to X substituents" refers to substituted with 0, 1, 2, 3 ... X substituents, wherein X is selected from any integer between 1 and 10. For example, "substituted with 0 to 4 substituents" refers to substituted with 0, 1, 2, 3 or 4 substituents. For example, "substituted with 0 to 5 substituents" refers to substituted with 0, 1, 2, 3, 4 or 5 substituents. For example, "bridged-heterocyclic ring is optionally further substituted with 0 to 4 substituents selected from H or F" means that the bridged-heterocyclic ring is optionally further substituted with 0, 1, 2, 3 or 4 substituents selected from H or F.

An X- to Y-membered ring (X is selected from an integer less than Y and greater than or equal to 3, and Y is selected from any integer between 4 and 12) includes X-, X+1-, X+2-, X+3-, X+4-, ..., to Y-membered rings. Rings include heterocycle, carbocycle, an aromatic ring, aryl, heteroaryl, cycloalkyl, a mono-heterocyclic ring, a fused-heterocyclic ring, a spiro-heterocyclic ring or a bridged-heterocyclic ring. For example, a "4- to 7-membered mono-heterocyclic ring" refers to a 4-, 5-, 6- or 7-membered mono-heterocyclic ring, and a "5- to 10-membered fused-heterocyclic ring" refers to a 5-, 6-, 7-, 8-, 9- or 10-membered fused-heterocyclic ring.

The term "optional" or "optionally" refers to that the events or circumstances subsequently described may but not necessarily occur, and the description includes the occasions where the events or circumstances occur or do not occur. For example, "alkyl optionally substituted with F" means that the alkyl may but not necessarily be substituted with F, and the description includes the case where the alkyl is substituted with F and the case where the alkyl is not substituted with F.

"Pharmaceutically acceptable salt" or "pharmaceutically acceptable salt thereof" refers to a salt of the compound according to the present invention, which salt maintains the biological effectiveness and characteristics of a free acid or a free base, and is obtained by reacting the free acid with a non-toxic inorganic base or organic base, or reacting the free base with a non-toxic inorganic acid or organic acid.

"Pharmaceutical composition" refers to a mixture of one or more compounds of the present invention, or stereoisomers, tautomers, deuterated compounds, solvates, prodrugs, metabolites, pharmaceutically acceptable salts or eutectic crystals thereof and other chemical components, wherein "other chemical components" refer to pharmaceutically acceptable carriers, excipients and/or one or more other therapeutic agents.

The term "preparation specification" refers to the weight of the active drug contained in each vial, tablet or other unit preparation.

"Carrier" refers to a material that does not cause significant irritation to an organism and does not eliminate the biological activity and characteristics of a compound administered.

"Prodrug" refers to a compound that can be converted into the compound of the present invention with the biological activity by metabolism *in vivo.* The prodrug of the present invention is prepared by modifying an amino or carboxyl group in the compound of the present invention, and the modification can be removed by conventional operations or *in vivo* to obtain a parent compound. When the prodrug of the present invention is administered to a mammalian individual, the prodrug is split to form a free amino or carboxyl group.

The term "eutectic crystal" refers to a crystal formed by the combination of active pharmaceutical ingredient (API) and eutectic crystal former (CCF) under the action of hydrogen bonds or other non-covalent bonds. The pure state of API and CCF are both solid at room temperature, and there is a fixed stoichiometric ratio between various components. The eutectic crystal is a multi-component crystal, which includes both a binary eutectic crystal formed between two neutral solids and a multi-element eutectic crystal formed between a neutral solid and a salt or solvate.

"Animal" is meant to include mammals, such as humans, companion animals, zoo animals, and domestic animals, preferably humans, horses, or dogs.

The term "stereoisomer" refers to an isomer produced as a result of different spatial arrangement of atoms in molecules, including cis-trans isomers, enantiomers and conformational isomers.

"Tautomer" refers to a functional group isomer produced by the rapid movement of an atom in two positions in a molecule, such as keto-enol isomerization and amide-imino alcohol isomerization.

"$IC_{5O}$" refers to the concentration of a medicament or inhibitor required to inhibit half of a given biological process (or a component of the process such as an enzyme, a receptor and a cell).

Detailed Description of Embodiments

The technical solutions of the present invention will be described in detail below in conjunction with examples, but the protection scope of the present invention includes but is not limited thereto.

The compounds used in the reactions described herein are prepared according to organic synthesis techniques known to those skilled in the art, and starting from commercially available chemicals and(or) compounds described in chemical documents. "Commercially available chemicals" are obtained from regular commercial sources, and suppliers include: Titan Technology Co., Ltd., Energy Chemical Co., Ltd., Shanghai Demo Co., Ltd., Chengdu Kelong Chemical Co., Ltd., Accela ChemBio Co., Ltd., PharmaBlock Sciences (Nanjing), Inc., WuXi Apptec Co., Ltd., J&K Scientific Co., Ltd., etc.

The structures of the compounds are determined by nuclear magnetic resonance (NMR) or (and) mass spectrometry (MS). The NMR shift (δ) is given in the unit of $10^{-6}$ (ppm). NMR is determined with (Bruker Avance III 400 and Bruker Avance 300) nuclear magnetic resonance instrument; the solvents for determination are deuterated dimethyl sulfoxide (DMSO-$d_6$), deuterated chloroform ($CDCl_3$) and deuterated methanol ($CD_3OD$); and the internal standard is tetramethylsilane (TMS).

MS is determined with Agilent 6120B (ESI) and Agilent 6120B (APCI);
HPLC is determined with Agilent 1260DAD high pressure liquid chromatograph (Zorbax SB-C18 100 × 4.6 mm, 3.5 μM);
Yantai Huanghai HSGF254 or Qingdao GF254 silica gel plate is used as a thin layer chromatography silica plate, and the silica gel plate for the thin layer chromatography (TLC) is of the specification of 0.15 mm-0.20 mm, and the specification when separating and purifying a product by thin layer chromatography is 0.4 mm - 0.5 mm;
and for the column chromatography, Yantai Huanghai silica gel of 200-300 mesh silica gel is generally used as a carrier.

Reagent and solvent abbreviations:
Dess-Martin Oxidant: (1,1,1-Triacetoxy)-1,1-dihydro-1,2-benziodoxol-3(1H)-one (CAS No. 87413-09-0); TBSOTf: tert-Butyldimethylsilyl trifluoromethanesulfonate; rac-BINAP: 1,1'-Binaphthyl-2,2'-bis(diphenylphosphine) (CAS No. 98327-87-8); $Pd_2(dba)_3$: Tris(dibenzylidene acetone)dipalladium (CAS No. 51364-51-3); DMA: N,N-dimethylacetamide; DMF: N,N-dimethylformamide; DCM: dichloromethane; MeOH: methanol; NMI: N-methylimidazole; TCFH: N,N,N',N'-tetramethylchloroformamidinium hexafluorophosphate.

**Preparation of intermediate 1**

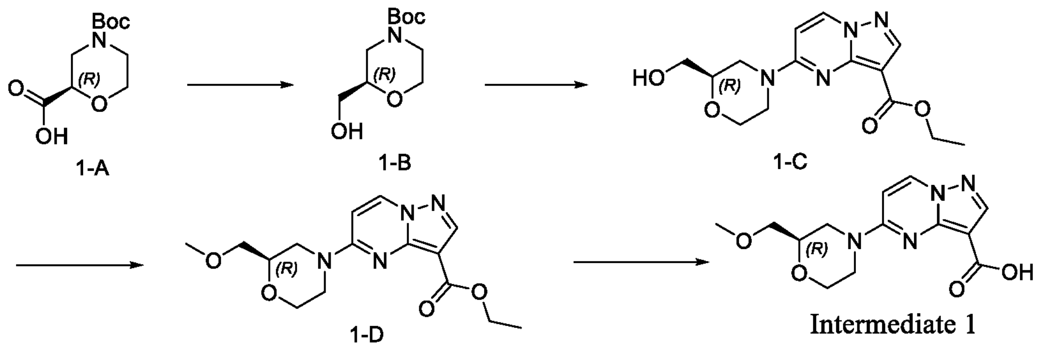

1-A 1-B 1-C 1-D Intermediate 1

Step 1: Preparation of 1-B

1-A (7.5 g, 32.4 mmol) was dissolved in THF (120 mL), and the solution was cooled to 0°C. 1 mol/L borane in tetrahydrofuran (60 mL, 60 mmol) was slowly added under nitrogen atmosphere, and reacted at room temperature for 16 h. The reaction system was cooled to 0°C, 30 mL of a mixed solution of methanol and acetic acid (v/v) = 9 : 1 was added, and the mixture was concentrated under reduced pressure. 130 mL of water and 150 mL of ethyl acetate were added, and liquid separation was carried out. The aqueous phase was extracted with ethyl acetate (100 mL×2), and the organic phase was combined. The organic phase was washed with saturated brine (190 mL×2), dried over anhydrous sodium sulfate, and concentrated under reduced pressure. The crude was separated and purified by silica gel column chromatography (petroleum ether/ethyl acetate (v/v) = 23:77) to obtain 1-B (5.5 g, yield: 78%).

LCMS m/z = 218.1 $[M+1]^+$.

Step 2: Preparation of 1-C

1-B (5.5 g, 25.3 mmol) was added into a 250 mL single-necked flask, and dichloromethane (80 mL) and trifluoroacetic acid (20 mL) were added. The mixture was reacted at room temperature for 4 h. The reaction system was concentrated under reduced pressure, acetonitrile (100 mL) was added to the residue, N,N-diisopropylethylamine (9.8 g, 75.8 mmol) and ethyl 5-chloropyrazolo[1,5-a]pyrimidine-3-carboxylate (5.3 g, 23.49 mmol) were added, and the mixture was reacted at 60°C for 12 h. The reaction system was cooled to room temperature and concentrated under reduced pressure. The crude was separated and purified by silica gel column chromatography (petroleum ether:ethyl acetate (v/v) = 41:59) to obtain 1-C (4.6 g, yield: 59%).
LCMS m/z = 307.1 $[M+1]^+$.

Step 3: Preparation of 1-D

1-C (2.4 g, 7.8 mmol) was added into a 100 mL single-necked flask, and dry DMF (30 mL) and potassium carbonate (3.2 g, 23.15 mmol) were added. The mixture was cooled to 0°C, and iodomethane (2.2 g, 15.5 mmol) was added. The mixture was reacted at room temperature for 16 h. Water (300 mL) was added to the reaction system, and the mixture was extracted with ethyl acetate (60 mL × 3). The organic phase was washed with aqueous saturated sodium chloride solution (80 mL × 2), dried over anhydrous sodium sulfate, and concentrated under reduced pressure. The crude was separated and purified by silica gel column chromatography (petroleum ether: ethyl acetate (v/v) = 22 : 78) to obtain 1-D (1.3 g, yield: 52%).
LCMS m/z = 321.1 $[M+1]^+$.

Step 4: Preparation of intermediate 1

1-D (1.2 g, 3.75 mmol) was added into a 100 mL single-necked flask, and methanol (15 mL), water (5 mL), and sodium hydroxide (760 mg, 19 mmol) were added, and the mixture was reacted at 50°C for 16 h. The reaction system was cooled to room temperature, the pH was adjusted to 5 with 1 mol/L aqueous hydrochloric acid solution, and extracted with a mixed solvent of dichloromethane and methanol (v/v) = 10 : 1 (60 mL × 3), the organic phase was washed with aqueous saturated sodium chloride solution (80 mL × 2), dried over anhydrous sodium sulfate, and concentrated under reduced pressure to obtain a crude intermediate 1 (1.0 g).
LCMS m/z = 293.1 $[M+1]^+$.

**Preparation of intermediate 2**

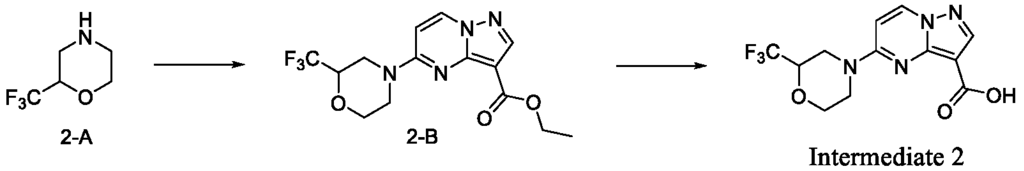

Step 1: Preparation of 2-B

The hydrochloride salt of 2-A (1.8 g, 9.4 mmol) was added into a 100 mL single-necked flask, and acetonitrile (30 mL), N,N-diisopropylethylamine (3.6 g, 27.9 mmol) and ethyl 5-chloropyrazolo[1,5-a]pyrimidine-3-carboxylate (2.1 g, 9.31 mmol) were added, and the mixture was reacted at 60°C for 12 h. The reaction system was cooled to room temperature and concentrated under reduced pressure. The crude was separated and purified by silica gel column chromatography (petroleum ether: ethyl acetate (v/v) = 68 : 32) to obtain 2-B (2.3 g, yield: 71%).
LCMS m/z = 345.1 $[M+1]^+$.

Step 2: Preparation of intermediate 2

2-B (2.3 g, 6.68 mmol) was added into a 250 mL single-necked flask, and methanol (30 mL), water (10 mL), and sodium hydroxide (1.3 g, 32.5 mmol) were added, and the mixture was reacted at 50°C for 16 h. The reaction system was cooled to room temperature, the pH was adjusted to 5 with 1 mol/L aqueous hydrochloric acid solution, and extracted with a mixed solvent of dichloromethane and methanol (v/v) = 10 : 1 (80 mL × 3), the organic phase was washed with aqueous

saturated sodium chloride solution (80 mL × 2), dried over anhydrous sodium sulfate, and concentrated under reduced pressure to obtain a crude intermediate 2 (1.8 g).

LCMS m/z = 317.1 $[M+1]^+$.

**Preparation of intermediate 3**

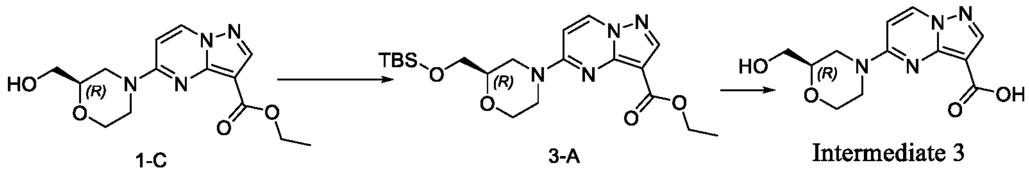

Step 1: Preparation of 3-A

1-C (2.3 g, 7.51 mmol) was dissolved in 20 mL of dichloromethane, and triethylamine (2.28 g, 22.53 mmol) was added. The mixture was cooled to 0°C, and TBSCl (1.70 g, 11.28 mmol) was added. The mixture was reacted at room temperature for 16 h. The reaction system was concentrated under reduced pressure, and the crude was separated and purified by silica gel column chromatography (petroleum ether/ethyl acetate (v/v) = 65 : 35) to obtain 3-A (1.4 g, yield: 44%).

Step 2: Preparation of intermediate 3

3-A (1.4 g, 3.33 mmol) was dissolved in a mixed solvent of tetrahydrofuran/water (v/v) = 4: 1, lithium hydroxide monohydrate (699 mg, 16.66 mmol) was added, and the mixture was reacted at room temperature for 16 h. The reaction system was adjusted to pH 5 with 0.5 mol/L aqueous hydrochloric acid solution, extracted with a mixed solvent of dichloromethane/methanol (v/v) = 10 : 1 (50 mL × 3), and the organic phase was washed with saturated brine (30 mL × 3), dried over anhydrous sodium sulfate, and concentrated under reduced pressure to obtain a crude intermediate 3 (0.57 g).

**Preparation of intermediate 4**

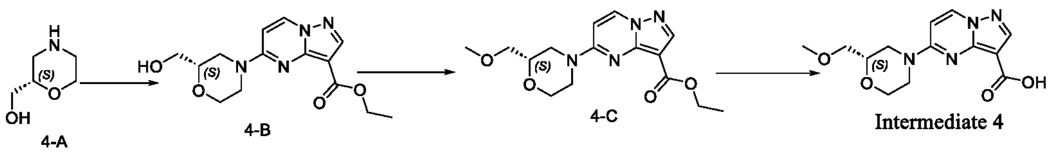

Step 1: Preparation of 4-B

4-A (10 g, 85.4 mmol), ethyl 5-chloropyrazolo[1,5-a]pyrimidine-3-carboxylate (10.49 g, 46.49 mmol) and DIPEA (36.04 g, 278.8 mmol) were dissolved in 100 mL of acetonitrile and reacted at 60°C for 12 h. The reaction system was cooled to room temperature and concentrated under reduced pressure. The crude was separated and purified by silica gel column chromatography (petroleum ether: ethyl acetate (v/v) = 2 : 3) to obtain 4-B (10 g, yield: 70%).

LCMS m/z = 307.1 $[M+1]^+$.

Step 2: Preparation of 4-C

4-B (6 g, 19.59 mmol) was dissolved in DMF (60 mL), and 0.95 g of 60% NaH was added at 0°C. The mixture was stirred at room temperature for 1 h, and then iodomethane (4.2 g, 29.59 mmol) was added at 0°C. The mixture was reacted at room temperature for 16 h. Water (50 mL) was added to the reaction system, and the mixture was extracted with dichloromethane (100 mL × 3). The organic phase was dried over anhydrous sodium sulfate and concentrated under reduced pressure. The crude was separated and purified by silica gel column chromatography (petroleum ether: ethyl acetate (v/v) = 1 : 4) to obtain 4-C (2 g, yield: 32%).

LCMS m/z = 321.1 $[M+1]^+$.

Step 3: Preparation of intermediate 4

4-C (2 g, 6.25 mmol) and lithium hydroxide (0.47 g, 19.62 mmol) were added into a 100 mL single-necked flask, methanol (10 mL) and water (5 mL) were added, and the mixture was reacted at 60°C for 4 h. The reaction system was cooled to room temperature, added to 50 mL of water, adjusted to pH 2 with 6 mol/L aqueous hydrochloric acid solution, and extracted with ethyl acetate (60 mL × 3), and the organic phase was dried over anhydrous sodium sulfate and concentrated under reduced pressure to obtain a crude intermediate 4 (2 g).

**Preparation of intermediate 5**

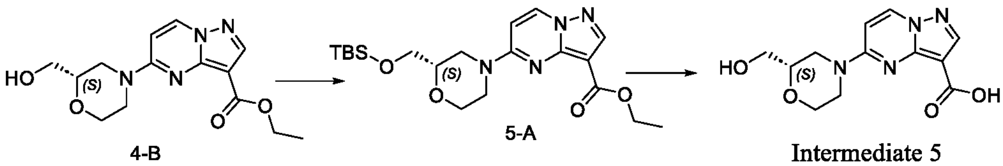

4-B 5-A Intermediate 5

Step 1: Preparation of 5-A

4-B (4 g, 13.06 mmol) and triethylamine (5.29 g, 52.28 mmol) were dissolved in 50 mL of dichloromethane, TBSCl (3.94 g, 26.14 mmol) was added, and the mixture was reacted at room temperature for 16 h. 50 mL of water was added to the reaction system, and liquid separation was carried out. The organic phase was dried over anhydrous sodium sulfate, and concentrated under reduced pressure. The crude was separated and purified by silica gel column chromatography (petroleum ether/ethyl acetate (v/v) = 65 : 35) to obtain 5-A (2 g, yield: 36%). Step 2: Preparation of intermediate 5

5-A (1.8 g, 4.28 mmol) and lithium hydroxide (0.32 g, 13.36 mmol) were added into a 100 mL single-necked flask, and 20 mL of methanol and 10 mL of water were added, and the mixture was reacted at 60°C for 4 h. The reaction system was cooled to room temperature, and 50 mL of water was added. The pH was adjusted to 2 with 1 mol/L aqueous hydrochloric acid solution. The mixture was extracted with ethyl acetate (50 mL × 3). The organic phase was dried over anhydrous sodium sulfate and concentrated under reduced pressure to obtain a crude intermediate 5 (2 g).

**Preparation of the hydrochloride salt of intermediate 6 (trans)**

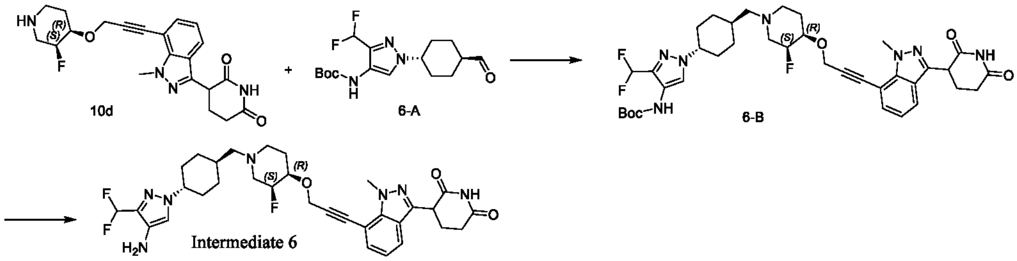

10d 6-A 6-B Intermediate 6

Step 1: Preparation of 6-B

The trifluoroacetate salt of the crude 10d (5.57 g) was dissolved in 40 mL DMA, and sodium bicarbonate (1.49 g, 17.74 mmol) was added. After stirring at room temperature for 15 min, 6-A (3.66 g, 10.66 mmol) (synthesis method, see WO 2021158634), 1.2 mL acetic acid and 4 Å molecular sieves (5 g) were added. After stirring at room temperature for 2 h, sodium triacetoxyborohydride (3.77 g, 17.79 mmol) was added and reacted at room temperature for 16 h. 150 mL of aqueous saturated sodium bicarbonate solution was added to the reaction system, extracted with 100 mL of dichloromethane, the organic phase was dried over anhydrous sodium sulfate, concentrated under reduced pressure, and the crude was separated and purified by silica gel column chromatography (dichloromethane/ethyl acetate (v/v) = 1 : 1) to obtain 6-B (3.1 g, yield: 40%).

Step 2: Synthesis of the hydrochloride salt of intermediate 6

6-B (600 mg, 0.83 mmol) was dissolved in 10 mL of 1,4-dioxane, and 10 mL of 4 mol/L hydrogen chloride 1,4-dioxane solution was added. The mixture was reacted at room temperature for 2 h. The reaction system was concentrated under reduced pressure to obtain the hydrochloride salt of a crude intermediate 6 (0.65 g).

LCMS m/z = 626.3 $[M+1]^+$.

**Preparation of intermediate 7 (trans)**

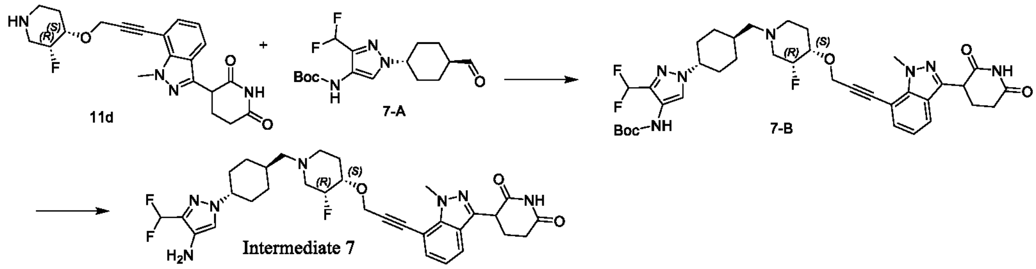


Step 1: Preparation of 7-B

The trifluoroacetate salt of the crude 11d (5.0 g) was dissolved in 60 mL DMA, and sodium bicarbonate (1.68 g, 20.0 mmol) was added. After stirring at room temperature for 15 min, 7-A (3.29 g, 9.58 mmol) (synthesis method, see WO 2021158634), 1 mL acetic acid and 4 Å molecular sieves (5 g) were added. After stirring at room temperature for 2 h, sodium triacetoxyborohydride (3.38 g, 15.95 mmol) was added and reacted at room temperature for 16 h. 150 mL of aqueous saturated sodium bicarbonate solution was added to the reaction system, extracted with 100 mL of dichloromethane, the organic phase was dried over anhydrous sodium sulfate, concentrated under reduced pressure, and the crude was separated and purified by silica gel column chromatography (dichloromethane/ethyl acetate (v/v) = 1 : 1) to obtain 7-B (1.0 g, yield: 14%).

Step 2: Preparation of intermediate 7

7-B (1.1 g, 1.52 mmol) was added to 30 mL of acetonitrile, and p-toluenesulfonic acid monohydrate (0.86 g, 4.52 mmol) was added, and the mixture was reacted at room temperature for 3 h. The reaction solution was concentrated under reduced pressure, 100 mL of ethyl acetate was added, and the pH was adjusted to 9 with saturated sodium bicarbonate solution. The organic phase was separated, dried over anhydrous sodium sulfate, and concentrated under reduced pressure to obtain a crude intermediate 7 (0.9 g).

**Example 1: Preparation of compound 1 (trans)**

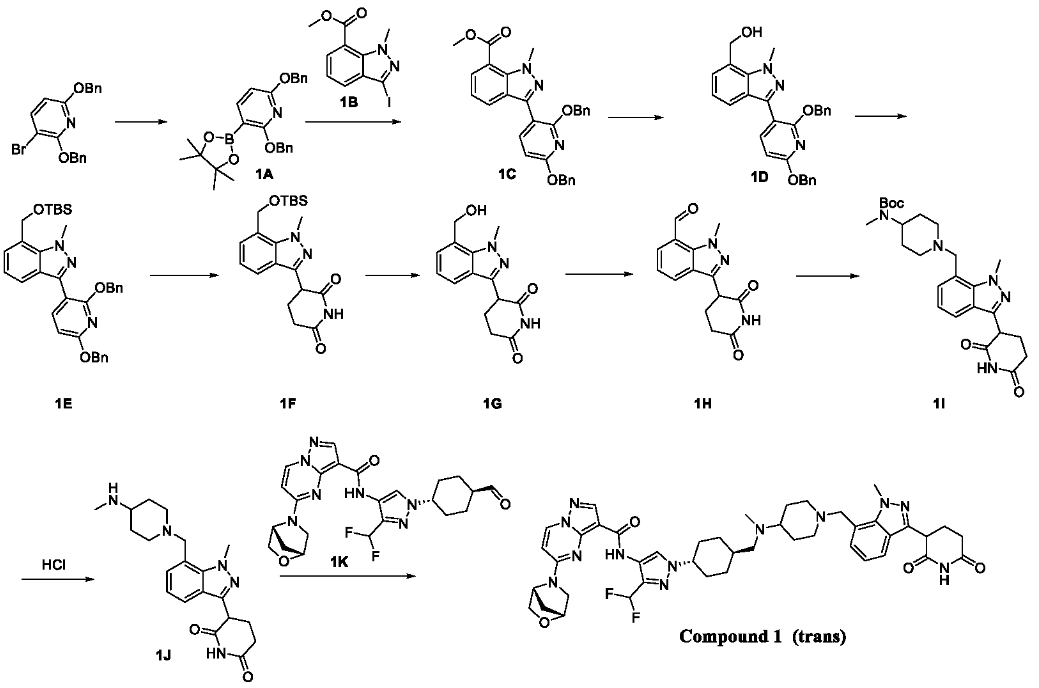


Step 1: **Preparation of 1A**

2,6-Bis(benzyloxy)-3-bromopyridine (5.0 g, 13.50 mmol), bis(pinacolato)diboron (5.14 g, 20.24 mmol) and potassium acetate (2.65 g, 27.0 mmol) were added to dry 1,4-dioxane (20 mL), and Pd(dppf)$Cl_2$.DCM (1.10 g, 1.35 mmol) was added, and the mixture was reacted at 100°C under nitrogen protection overnight. The mixture was cooled to room temperature, and the reaction liquid was subjected to suction filtration over celite, extracted with ethyl acetate (50 mL × 3), dried over anhydrous sodium sulfate, filtered, concentrated, and purified by silica gel column chromatography (ethyl acetate/petroleum ether (V/V) = 1/20) to obtain **1A** (4 g, yield: 71%).

LCMS m/z = 418.2 $[M+H]^+$.

Step 2: **Preparation of 1C**

Under nitrogen protection, **18** (1.5 g, 4.75 mmol) (synthesized according to patent CN113512025), **1A** (2.97 g, 7.12 mmol), Pd(dppf)$Cl_2$.DCM (CAS: 95464-05-4) (0.78 g, 0.96 mmol), and cesium carbonate (3.10 g, 9.5 mmol) were added to 1,4-dioxane (45 mL) and water (10 mL), and the mixture was reacted at 105°C for 3 h. After the reaction was completed, the reaction liquid was cooled to room temperature, water (50 mL) was added, and the mixture was extracted with ethyl acetate (30 mL × 3), dried over anhydrous sodium sulfate, filtered, concentrated, and purified by silica gel column chromatography (ethyl acetate/petroleum ether (V/V) = 1/5) to obtain **1C** (1.06 g, yield: 46.5%).

LCMS m/z = 480.1 $[M+H]^+$.

Step 3: **Preparation of 1D**

**1C** (700 mg, 1.46 mmol) was dissolved in anhydrous THF (20 mL), lithium aluminum tetrahydride (166 mg, 4.37 mmol) was added, and the mixture was reacted at room temperature for 1 h. After the reaction was completed, water (2 mL) was added to quench the reaction, and the reaction liquid was subjected to suction filtration over celite, dried over anhydrous sodium sulfate, filtered, concentrated, and purified by silica gel column chromatography (ethyl acetate/petroleum ether (V/V) = 1/1) to obtain **1D** (580 mg, yield: 88%)

LCMS m/z = 452.2 $[M+H]^+$.

Step 4: **Preparation of 1E**

**1D** (600 mg, 1.33 mmol) was dissolved in THF (30 mL), triethylamine (673 mg, 6.65 mmol) was added, and the mixture was stirred at room temperature for 10 min. TBSOTf (879 mg, 3.33 mmol) was added, and the mixture was reacted at room temperature for 1 h. After the reaction was completed, the mixture was concentrated and purified by silica gel column chromatography (ethyl acetate/petroleum ether (V/V) = 1/5) to obtain **1E** (630 mg, yield: 84%).

$^1$H NMR (400 MHz, $CDCl_3$) δ 7.89 (d, 1H), 7.66 - 7.61 (m, 1H), 7.48 - 7.42 (m, 2H), 7.41 - 7.34 (m, 2H), 7.34 - 7.29 (m, 3H), 7.27 - 7.21 (m, 4H), 6.96 (dd, 1H), 6.53 (d, 1H), 5.45 (s, 2H), 5.41 (s, 2H), 5.07 (s, 2H), 4.43 (s, 3H), 0.90 (s, 9H), 0.06 (s, 6H).

Step 5: **Preparation of 1F**

**1E** (630 mg, 1.11 mmol) was dissolved in ethanol (30 mL), and palladium on carbon (10%) (1 g) was added. The mixture was subjected to hydrogen replacement three times, and reacted overnight at room temperature. The reaction liquid was subjected to suction filtration over celite, concentrated, and purified by silica gel column chromatography (ethyl acetate/petroleum ether (V/V) = 1/1) to obtain **IF** (197 mg, yield: 46%).
LCMS m/z = 388.2 $[M+H]^+$.

Step 6: **Preparation of 1G**

**IF** (197 mg, 0.51 mmol) was dissolved in THF (5 mL), and tetrabutylammonium fluoride in THF (2.55 mL, 1 mol/L) was added. The mixture was reacted at room temperature for 1 h. After the reaction was completed, water (20 mL) was added, and the mixture was extracted with ethyl acetate (20 mL × 3). The mixture was dried over anhydrous sodium sulfate, filtered, and concentrated to obtain **1G.**
LCMS m/z = 274.1 $[M+H]^+$.

Step 7: **Preparation of 1H**

The **1G** from the previous step was dissolved in dichloromethane (5 mL), and Dess-Martin periodinane (432 mg, 1.02 mmol) was added and the mixture was reacted at room temperature for 1 h. The reaction liquid was subjected to suction filtration over celite, concentrated, and purified by silica gel column chromatography (methanol/dichloromethane (V/V) = 1/20) to obtain **1H** (100 mg, two-step yield: 72%).
LCMS m/z = 272.1 $[M+H]^+$.

Step 8: **Preparation of 1I**

**1H** (50 mg, 0.18 mmol), tert-butyl methyl(piperidin-4-yl)aminocarboxylate (77 mg, 0.36 mmol) and glacial acetic acid (11 mg, 0.18 mmol) were dissolved in anhydrous dichloroethane (5 mL), a 4Å molecular sieve (1 g) was added, and the mixture was reacted at room temperature for 2 h. Sodium triacetoxyborohydride (76 mg, 0.36 mmol) was added and the mixture was reacted at room temperature overnight. After the reaction was completed, the reaction liquid was subjected to suction filtration over celite, concentrated, and purified by a preparative silica gel plate (methanol/dichloromethane (V/V) = 1/20) to obtain **1I** (54 mg, yield: 64%).
LCMS m/z = 470.3 $[M+H]^+$.

Step 9: **Preparation of 1J**

**1I** (52 mg, 0.11 mmol) was dissolved in hydrogen chloride in dioxane (5 mL, 4 mol/L) and the mixture was reacted at room temperature for 1 h. After the reaction was completed, the mixture was concentrated and the residue was redissolved in 5 mL of dioxane. Triethylamine (0.5 mL) was added and concentrated to obtain a crude **1J.**

Step 10: **Preparation of compound 1**

**1K** (54 mg, 0.11 mmol) (synthesized according to patent WO 2020113233), the crude **1J** and glacial acetic acid (7 mg, 0.11 mmol) were dissolved in anhydrous DMA (5 mL), a 4Å molecular sieve (1 g) was added, and the mixture was reacted at room temperature for 2 h. Sodium triacetoxyborohydride (35 mg, 0.17 mmol) was added and the mixture was reacted at room temperature overnight. The mixture was filtered, and the filtrate was purified by preparative HPLC (instrument: waters 2767 preparative liquid chromatography; chromatographic column: XBridge@Prep C18 (30mm × 150mm); composition of mobile phases: mobile phase A: acetonitrile, and mobile phase B: water (containing 0.1% trifluoroacetic acid)), and lyophilized. The obtained solid was added to dichloromethane (10 mL) for redissolution, and water (5 mL) and saturated sodium bicarbonate solution (1 mL) were added. The organic layer was separated, dried over anhydrous sodium sulfate, filtered, and concentrated to obtain **Compound 1** (20 mg, yield: 22%).
$^1$H NMR (400 MHz, DMSO-$d_6$) δ 10.87 (s, 1H), 9.49(d, 1H), 8.78(d, 1H), 8.37(d, 1H), 8.25(d, 1H), 7.67 - 7.60 (m, 1H), 7.25 - 6.94 (m, 3H), 6.89 - 6.40 (m, 1H), 5.30 - 5.02 (m, 1H), 4.83 - 4.71 (m, 1H), 4.41 - 4.33 (m, 1H), 4.30 (s, 3H), 4.20 - 4.04 (m, 2H), 3.86 - 3.66 (m, 4H), 3.67 - 3.40 (m, 3H), 3.17(d, 2H), 2.94 - 2.83 (m, 2H), 2.70 - 2.61 (m, 2H), .2.37 - 2.28 (m,

2H), 2.21 - 2.16 (m, 3H), 2.07 - 1.81 (m, 8H), 1.75 - 1.56 (m, 4H), 1.41 - 1.32 (m, 2H), 1.08 - 0.91 (m, 2H).

LCMS m/z = 839.4 $[M+H]^+$.

**Example 2: Preparation of compound 2 (trans)**

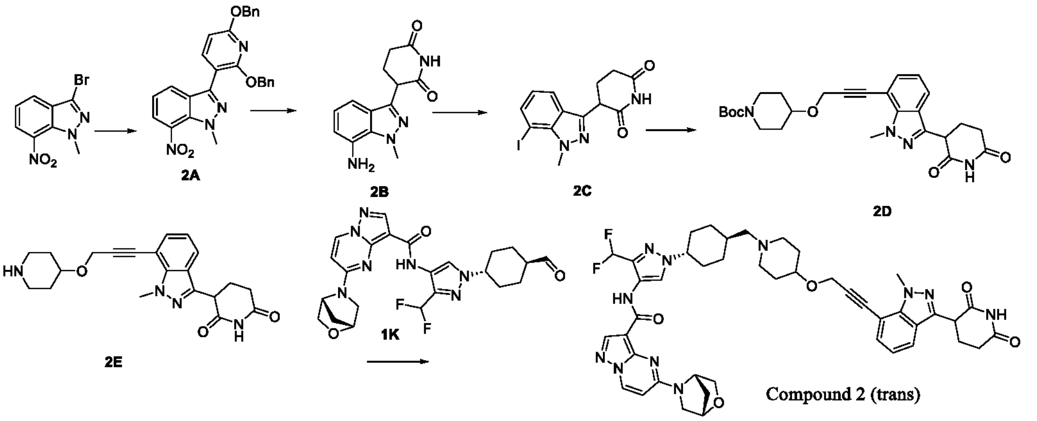

2A
2B
2C
2D
2E
1K
Compound 2 (trans)

Step 1: **Preparation of 2A**

Under nitrogen protection, 3-bromo-1-methyl -7-nitro-1H-indazole (3 g, 11.72 mmol), 1A (7.3 g, 17.5 mmol), dioxane (80 mL), water (20 mL), Pd(dppf)$Cl_2$.DCM (1.4 g, 1.71 mmol) and cesium carbonate (11.5 g, 35.3 mmol) were sequentially added to a 50 mL single-necked flask, and the mixture was subjected to nitrogen replacement three times, and reacted at 100°C for 12h. After the reaction was completed, the reaction liquid was cooled to room temperature, poured into water, and extracted with ethyl acetate three times. The organic phases were combined, dried over anhydrous sodium sulfate and concentrated under reduced pressure, and the residue was purified by silica gel column chromatography to obtain **2A** (3.3 g, yield: 60%).

LCMS m/z = 467.1 $[M+H]^+$.

Step 2: **Preparation of 2B**

**2A** (1.6 g, 3.43 mmol), 10% palladium on carbon (0.9 g), and 10% palladium hydroxide on carbon (1.2 g) were dissolved in 20 mL of tetrahydrofuran and 20 mL of methanol. The mixture was subjected to hydrogen replacement three times, and reacted at 30°C overnight. The mixture was cooled to room temperature and filtered with celite. The filter cake was washed with methanol three times. The organic phases were combined, dried over anhydrous sodium sulfate, and spun to dryness under reduced pressure to obtain **2B** (650 mg, yield 73%).

LCMS m/z = 259.2 $[M+H]^+$.

Step 3: **Preparation of 2C**

**2B** (250 mg, 0.97 mmol), KI (242 mg, 1.46 mmol), iodine (371 mg, 1.46 mmol) were dissolved in acetonitrile (5 mL) and cooled to 0°C. Then, isoamyl nitrite (171 mg, 1.46 mmol) was slowly added and the mixture was reacted at 30°C for 8 h under nitrogen protection. 10 mL of saturated ammonium chloride solution was added, and the mixture was extracted with dichloromethane. The organic layer was concentrated under reduced pressure, and the residue was purified by silica gel column chromatography to obtain 2C (130 mg, yield 36%).

LCMS m/z = 370.0 $[M+H]^+$.

Step 4: **Preparation of 2D**

**2C** (130 mg, 0.35 mmol), tert-butyl 4-(prop-2-yn-1-yloxy)piperidine-1-carboxylate (101 mg, 0.42 mmol), CuI (13 mg, 0.07 mmol), $PdCl_2(PPh_3)_2$ (25 mg, 0.036 mmol) and triethylamine (1 mL) were dissolved in DMF (4 mL), and the mixture was subjected to nitrogen replacement three times and reacted at 60°C for 6h. The mixture was cooled to room temperature and diluted with 5 mL of an aqueous solution, and the mixture was extracted with dichloromethane. The organic layer was concentrated under reduced pressure, and the residue was purified by silica gel column chromatography to obtain **2D** (91 mg, yield 54%).

LCMS m/z = 381.2 [M-Boc+H]$^+$.

Step 5: **Preparation of 2E**

**2D** (91 mg, 0.19 mmol) was dissolved in methanol (1 mL), 4*N* hydrogen chloride dioxane solution (3 mL) was added, and the mixture was reacted at room temperature for 1h, and concentrated under reduced pressure. 5 mL of dichloromethane and 1 mL of methanol were added to redissolve the residue, 2 mL of triethylamine was added, and the mixture was concentrated under reduced pressure to obtain the crude **2E**.

Step 6: **Preparation of compound 2**

The crude **2E** from the previous step, **1K** (102 mg, 0.21 mmol), acetic acid (12.6 mg, 0.21mmol) were sequentially dissolved in DMA (5 mL), and a 4Å molecular sieve (2 g) was added. The mixture was stirred at room temperature for 30 min, sodium triacetoxyborohydride (57 mg, 0.27 mmol) was then added, and the resulting mixture was reacted overnight at room temperature. The mixture was extracted with 20 mL of aqueous saturated sodium bicarbonate solution and 20 mL of dichloromethane. The organic layer was concentrated under reduced pressure, and the residue was purified by silica gel column chromatography (mobile phase: DCM/MeOH(V/V)=100/1-20/1) to obtain **compound 2** (30 mg, two-step yield: 19%).

LCMS m/z =850.4 [M+H]$^+$.

$^1$H NMR (400 MHz, DMSO-$d_6$) $\delta$10.90 (s, 1H), 9.56 - 9.42 (m, 1H), 8.77 (d, 1H), 8.42 - 8.35 (m, 1H), 8.28 - 8.22 (m,1H), 7.78 (d, 1H), 7.51 (d, 1H), 7.27 - 6.94 (m, 2H), 6.89 - 6.42 (m, 1H), 5.31 - 5.03 (m, 1H), 4.82 - 4.70 (m, 1H), 4.54 (s, 2H), 4.41 (dd, 1H), 4.29 (s, 3H), 4.23 - 4.11 (m, 1H), 3.87 - 3.40 (m, 5H), 2.80 - 2.56 (m, 4H), 2.43 - 2.31 (m, 1H), 2.27 - 1.82 (m, 13H), 1.80 - 1.66 (m, 2H), 1.66 - 1.41 (m, 3H), 1.14 - 0.96 (m, 2H).

**Example 3: Preparation of compound 3** (trans)

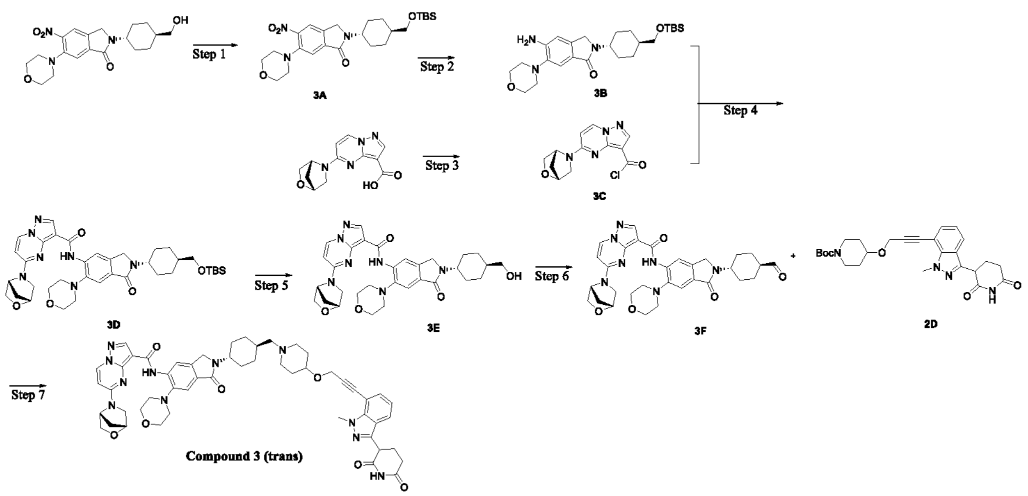


Step 1: **Preparation of 3A**

2-(4-(Hydroxymethyl)cyclohexyl)-6-morpholinyl-5-nitrosoisoindolin-1-one (500 mg, 1.33 mmol) (for synthesis step thereof, reference was made to patent WO 2020264499) and triethylamine (540 mg, 5.34 mmol) were dissolved in anhydrous tetrahydrofuran (10 mL) and cooled to 0°C; TBSOTf (700 mg, 2.65 mmol) was slowly added dropwise, and the mixture was reacted for 2 h at room temperature under nitrogen protection. The reaction liquid was concentrated under reduced pressure and the residue was purified by silica gel column chromatography to obtain **3A** (521 mg, yield 80%).

LCMS m/z = 490.2 [M+H]$^+$.

Step 2: **Preparation of 3B**

**3A** (521 mg, 1.06 mmol ) was dissolved in ethanol (10 mL) and water (2 mL), and the mixture was warmed to 80°C.

A mixture of ammonium chloride (280 mg, 5.23 mmol) and iron powder (300 mg,5.37 mmol ) was added, and stirred at 80°C for 1 h, cooled to room temperature, and filtered. The filter cake was washed with 50 mL of dichloromethane. 10 mL of saturated brine was added to the filtrate. Liquid separation was carried out and the organic layer was dried over anhydrous sodium sulfate, and concentrated under reduced pressure to obtain the crude **3B** (440 mg).
LCMS m/z =460.3 $[M+H]^+$.

Step 3: **Preparation of 3C**

5-((1R,4R)-2-oxa-5-azabicyclo[2.2.1]heptan-5-yl)pyrazolo[1,5-a]pyrimidine-3-carboxylic acid (1.0 g, 3.84 mmol) was dissolved in thionyl chloride (30 mL) and stirred at 70°C for 2 h. The reaction solution was cooled to room temperature and concentrated under reduced pressure to obtain the crude **3C** (1.1 g).

Step 4: **Preparation of 3D**

Under nitrogen atmosphere, **3B** (440 mg, 0.96 mmol) and pyridine (150 mg, 1.9 mmol) were dissolved in anhydrous dichloromethane (10 mL) and cooled to 0°C. The crude **3C** (500 mg) was dissolved in anhydrous dichloromethane (4 mL), slowly added dropwise to the system, and reacted at room temperature for 2 h. The reaction liquid was concentrated under reduced pressure and the residue was purified by silica gel column chromatography to obtain **3D** (600 mg, yield 89%).
LCMS m/z = 702.3 $[M+H]^+$.

Step 5: **Preparation of 3E**

**3D** (600 mg, 0.85 mmol) was dissolved in tetrahydrofuran (10 mL) and cooled to 0°C, and TBAF (1M, 1.71 mL) was slowly added and reacted at room temperature for 4 h. The reaction liquid was concentrated under reduced pressure and the residue was purified by silica gel column chromatography to obtain **3E** (372 mg, yield 74%).
LCMS m/z = 588.2 $[M+H]^+$.

Step 6: **Preparation of 3F**

**3E** (370 mg, 0.63 mmol) and Dess-Martin periodinane (530 mg, 1.25 mmol) were added to tetrahydrofuran (10 mL) and stirred at room temperature for 1 h. The reaction liquid was concentrated under reduced pressure and the residue was purified by silica gel column chromatography to obtain 3F (350 mg, yield 95%).
LCMS m/z = 586.3 $[M+H]^+$.

Step 7: **Preparation of compound 3**

**2D** (100 mg, 0.21 mmol) was dissolved in dichloromethane (4 mL), and trifluoroacetic acid (4 mL) was added. The mixture was reacted at room temperature for 1 h. The mixture was concentrated under reduced pressure and 10 mL of dichloromethane was added to redissolve the residue. 3 mL of triethylamine was added to adjust the solution to alkalinity. The mixture was concentrated under reduced pressure to obtain the compound. The residue was dissolved in DMA (6mL), and **3F** (120 mg, 0.20 mmol) and a 4Å molecular sieve (2 g) were added. The mixture was stirred at room temperature for 30 min, and then sodium triacetoxyborohydride (58 mg, 0.27 mmol) was added; and the resulting mixture was reacted overnight at room temperature. The mixture was extracted with 20 mL of aqueous saturated sodium bicarbonate solution and 20 mL of dichloromethane, and the organic layer was concentrated under reduced pressure, and the residue was purified by thin layer chromatography on silica gel plate (mobile phase: DCM/MeOH(V/V)= 20/1) to obtain **compound 3** (93 mg, yield: 49%).
LCMS m/z =950.4 $[M+H]^+$.

**Example 4:** Preparation of the trifluoroacetate salt of **compound 4** (trans)

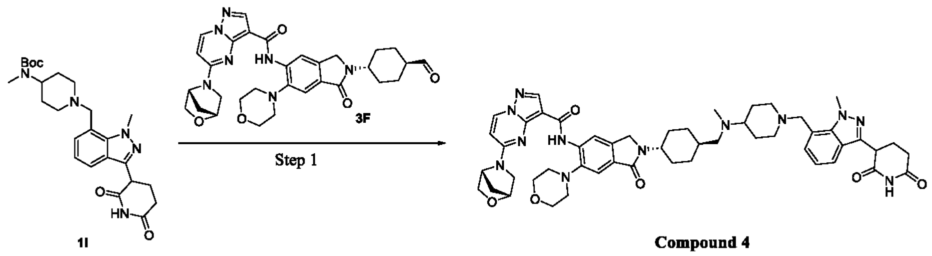


1l 3F Compound 4

**1l** (131 mg, 0.28 mmol) was dissolved in hydrogen chloride in dioxane (5 mL, 4 mol/L), and reacted at room temperature for 1 h. After the reaction was completed, the reaction mixture was concentrated, and the residue was redissolved in 5 mL of dioxane. Triethylamine (0.5 mL) was added and the mixture was concentrated in vacuo. **3F,** glacial acetic acid (17 mg, 0.28 mmol), anhydrous DMA (5 mL), and molecular sieves (2 g) were added to this concentrate. After reaction at room temperature for 2 h, sodium triacetoxyborohydride (119 mg, 0.56 mmol) was added, and the mixture was reacted overnight at room temperature. The mixture was filtered, and saturated sodium bicarbonate solution (30 mL) was added to quench the reaction. The reaction liquid was extracted with ethyl acetate (3 × 30 mL), washed with saturated brine (2 × 20 mL), dried over anhydrous sodium sulfate, filtered, concentrated, and purified with a preparative silica gel plate (DCM: MeOH=10:1). The crude is further purified by preparative HPLC (instrument: waters 2767 preparative liquid chromatography; chromatographic column: XBridge@PrepC18 (30mm × 150mm); composition of mobile phases: mobile phase A: acetonitrile, and mobile phase B: water (containing 0.1% trifluoroacetic acid)), and lyophilized to obtain the trifluoroacetate of **compound 4** (17 mg).

LCMS m/z = 470.4 [(M+2H)/2]$^+$.

**Example 6: Preparation of compound 6 (trans)**

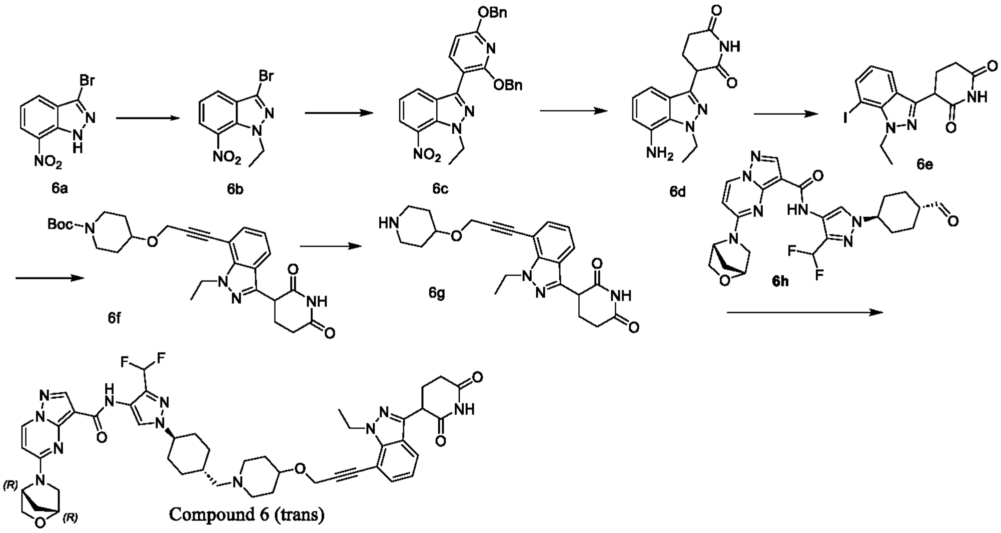

6a 6b 6c 6d 6e 6f 6g 6h

Compound 6 (trans)

Step 1: Preparation of 6b

6a (6 g, 24.79 mmol) was dissolved in 30 mL of acetone; potassium hydroxide (2.09 g, 37.25 mmol) was added at 0°C, and stirred at 0°C for 15 min; iodoethane (3.87 g, 24.81 mmol) was added dropwise, and reacted at 20°C for 16 h. The reaction system was concentrated under reduced pressure, and 50 mL of ethyl acetate and 100 mL of water were added. Liquid separation was carried out and the organic phase was dried over anhydrous sodium sulfate and concentrated under reduced pressure. The crude was separated and purified by silica gel column chromatography (ethyl acetate /petroleum ether (v/v) = 1:20) to obtain 6b (1.40 g, yield 21%).

LCMS m/z = 270.0 [M+1]$^+$.

Step 2: **Preparation** of 6c

Under nitrogen protection, 6b (1.3 g, 4.81 mmol), 1A (3.01 g, 7.21 mmol), 1,4-dioxane (60 mL), water (20 mL), Pd(dppf)$Cl_2$.DCM (0.39 g, 0.48 mmol) and cesium carbonate (4.70 g, 14.43 mmol) were sequentially added to a 50 mL single-necked flask, and the mixture was subjected to nitrogen replacement three times, and reacted at 100°C for 12 h. The reaction liquid was cooled to room temperature and poured into 30 mL of water. The mixture was extracted with 200 mL of ethyl acetate three times, and the organic phases were combined, dried over anhydrous sodium sulfate, and concentrated under reduced pressure. The crude was separated and purified by silica gel column chromatography (ethyl acetate /petroleum ether (v/v) = 1:5) to obtain 6c (1.5 g, yield 65%).
LCMS m/z = 481.1 $[M+1]^+$.

Step 3: **Preparation** of 6d

6c (1.5 g, 3.12 mmol), 10% palladium on carbon (0.8 g), and 10% palladium hydroxide on carbon (1.0 g) were dissolved in 30 mL of tetrahydrofuran and 30 mL of methanol. The mixture was subjected to hydrogen replacement three times, and reacted at 30°C under the atmosphere of hydrogen balloon for 16 h. The reaction liquid was cooled to room temperature and filtered with celite. The filter cake was washed with 50 mL of methanol three times, and the organic phases were combined, dried over anhydrous sodium sulfate, and concentrated under reduced pressure to obtain the crude 6d (800 mg).
LCMS m/z = 273.2 $[M+1]^+$.

Step 4: **Preparation** of 6e

The above-mentioned crude 6d (400 mg), KI (0.37 g, 2.23 mmol), CuI (0.406 g, 2.13 mmol) and iodine (560 mg, 2.21 mmol) were dissolved in acetonitrile (5 mL), and cooled to 0°C. Isoamyl nitrite (260 mg, 2.21 mmol) was slowly added. The mixture was reacted at 30°C for 6 h under nitrogen protection. To the reaction liquid was added 10 mL of saturated ammonium chloride solution, and the mixture was extracted with 50 mL of dichloromethane. Liquid separation was carried out and the organic phase was dried over anhydrous sodium sulfate and concentrated under reduced pressure. The crude was separated and purified by silica gel column chromatography (petroleum ether /ethyl acetate (v/v) =2 : 1-1 : 1) to obtain 6e (200 mg, two-step yield from 6c: 33%).
LCMS m/z = 384.1 $[M+1]^+$.

Step 5: **Preparation** of 6f

6e (100 mg, 0.26 mmol), tert-butyl 4-(prop-2-yn-1-yloxy)piperidine-1-carboxylate (for the synthesis method, see WO 2021247899) (75 mg, 0.313 mmol), CuI (10 mg, 0.0525 mmol), $PdCl_2(PPh_3)_2$ (26 mg, 0.037 mmol) and triethylamine (0.13 g, 1.28 mmol) were dissolved in DMF (5 mL), the mixture was subjected to nitrogen replacement three times, and the mixture was reacted at 60°C for 6 h. The reaction solution was cooled to room temperature, 5 mL of water was added, and the mixture was extracted with 50 mL of dichloromethane. Liquid separation was carried out, and the organic phase was concentrated under reduced pressure. The crude was separated and purified by silica gel column chromatography (petroleum ether/ethyl acetate (v/v) = 2 : 1-1 : 1) to obtain 6f (90 mg, yield: 70%).

Step 6: 6 g of trifluoroacetate salt

6f (90 mg, 0.18 mmol) was dissolved in 5 mL of dichloromethane, 2 mL of trifluoroacetic acid was added, and the mixture was reacted at room temperature for 2 h. The reaction liquid was concentrated under reduced pressure to obtain 6 g of a crude trifluoroacetate salt (0.1 g).
LCMS m/z = 395.2 $[M+1]^+$.

Step 7: **Preparation** of compound 6

The above-mentioned 6 g of the crude trifluoroacetate salt (100 mg) was dissolved in 5 mL of DMA, and sodium bicarbonate (30 mg, 0.36 mmol) was added. After stirring at room temperature for 15 min, 6h (100 mg, 0.21 mmol), 0.04 mL of acetic acid and a 4 Å molecular sieve (2 g) were added. After stirring at room temperature for 30 min, sodium triacetoxyborohydride (76 mg, 0.36 mmol) was added and the mixture was reacted at room temperature for 16 h. 20 mL of aqueous saturated sodium bicarbonate solution and 20 mL of dichloromethane were added, liquid separation was carried out, and the organic phase was concentrated under reduced pressure. The crude was separated and purified by silica gel column chromatography (dichloromethane/methanol (v/v) = 100 : 1-20 : 1). The obtained crude was then subjected to

chiral preparative chromatography and lyophilized to obtain compound 6 (78.5 mg, yield: 43%).

Chiral preparation method:

instrument and preparative column: Waters 150 SFC preparative liquid chromatography,
preparative column model Chiralpak Column;
Mobile phase system: $sCO_2$ (supercritical $CO_2$)/a mixed solvent of isopropanol and acetonitrile,
isocratic elution: $sCO_2$/a mixed solvent of isopropanol and acetonitrile = 2 : 3; flow rate: 100 mL/min.

Chiral analysis method for target compounds:

instrument: SHIMADZU LC-30AD sf; chromatographic column: Chiralpak AD-3; Specification: 50 × 4.6 mm I.D., 3 μm ; mobile phase A: $sCO_2$ (supercritical $CO_2$); mobile phase B: a mixed solvent of isopropanol and acetonitrile (containing 0.05% diethylamine); column temperature: 35°C; flow rate: 3 mL/min; wavelength: 220 nm; Elution program: Isocratic elution: mobile phase A: B = 2 : 3; retention time of target compounds: 0.875 min.

$^1$H NMR (400 MHz, DMSO-$d_6$) δ 10.90 (s, 1H), 9.55 - 9.45 (m, 1H), 8.78 (d, 1H), 8.42 - 8.34 (m, 1H), 8.29 - 8.21 (m, 1H), 7.84 - 7.73 (m, 1H), 7.57 - 7.46 (m, 1H), 7.30 - 6.93 (m, 2H), 6.92 - 6.40 (m, 1H), 5.35 - 5.00 (m, 1H), 4.84 - 4.66 (m, 3H), 4.53 (s, 2H), 4.46 - 4.37 (m, 1H), 4.25 - 4.08 (m, 1H), 3.90 - 3.38 (m, 5H), 2.80 - 2.56 (m, 4H), 2.45 - 2.30 (m, 1H), 2.25 - 1.83 (m, 13H), 1.83 - 1.65 (m, 2H), 1.64 - 1.44 (m, 3H), 1.40 (t, 3H), 1.13 - 0.94 (m, 2H).

LCMS m/z = 864.3 [M+1]$^+$.

**Example 7: Preparation of compound 7** (Trans) Trans-5-((1R,4R)-2-oxa-5-azabicyclo[2.2.1]heptan-5-yl)-N-(3-(difluoromethyl)-1-(4-((4-((3-(3-(2,6-dioxopiperidin-3-yl)-1-(methyl-d3)-1H-indazol-7-yl)prop-2-yn-1-yl)oxy)piperidin-1-yl)methyl)cyclohexyl)-1H-pyrazol-4-yl)pyrazolo[1,5-a]pyrimidine-3-carboxamide

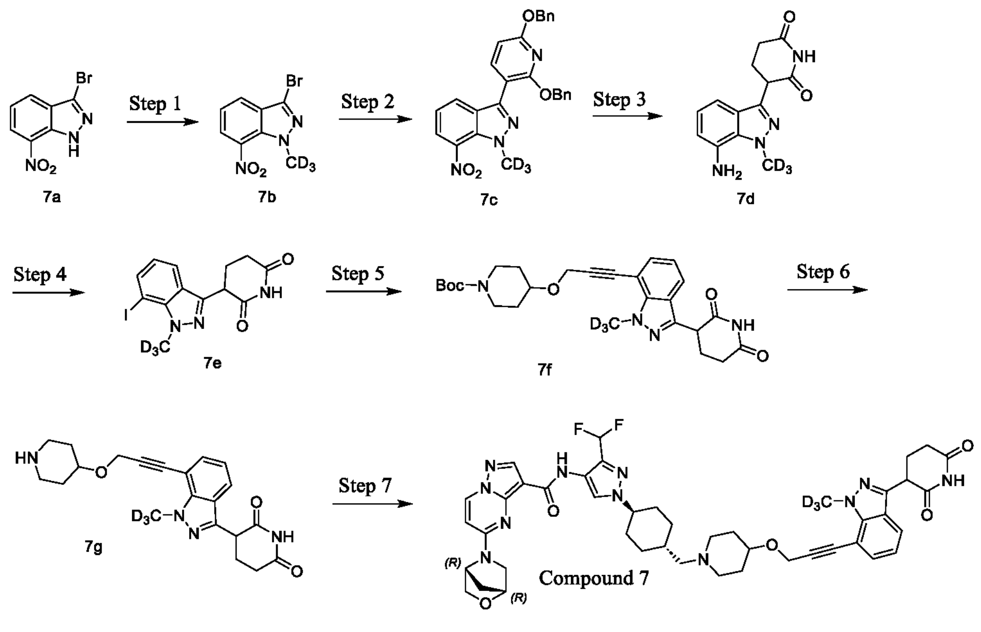

7a 7b 7c 7d 7e 7f 7g Compound 7

Compound 7 was prepared using deuterated iodomethane as the starting material and referring to the synthesis method of example 6 to obtain compound 7.

Step 1: Preparation of 7b

7a (6.00 g, 24.79 mmol) was dissolved in 30 mL of acetone; potassium hydroxide (2.09 g, 37.25 mmol) was added at 0°C, and stirred at 0°C for 15 min; deuterated iodomethane (3.60 g, 24.83 mmol) was added dropwise, and reacted at 20°C for 16 h. The reaction system was concentrated under reduced pressure, and 50 mL of ethyl acetate and 100 mL of

water were added. Liquid separation was carried out and the organic phase was dried over anhydrous sodium sulfate and concentrated under reduced pressure. The crude was separated and purified by silica gel column chromatography (ethyl acetate /petroleum ether (v/v) = 1:20) to obtain 7b (4.90 g, yield 76%).

Step 2: Preparation of 7c

Under nitrogen protection, 7b (1.5 g, 5.79 mmol), 1A (3.62 g, 8.67 mmol), 1,4-dioxane (60 mL), water (20 mL), $Pd(dppf)Cl_2$.DCM (0.47 g, 0.578 mmol) and cesium carbonate (5.66 g, 17.37 mmol) were sequentially added to a 50 mL single-necked flask, and the mixture was subjected to nitrogen replacement three times, and reacted at 100°C for 12 h. The reaction liquid was cooled to room temperature and poured into 30 mL of water. The mixture was extracted with 200 mL of ethyl acetate three times, and the organic phases were combined, dried over anhydrous sodium sulfate, and concentrated under reduced pressure. The crude was separated and purified by silica gel column chromatography (ethyl acetate /petroleum ether (v/v) = 1:10) to obtain 7c (1.6 g, yield 59%).

LCMS m/z = 470.2 $[M+1]^+$.

Step 3: Preparation of 7d

7c (1.6 g, 3.41 mmol), 10% palladium on carbon (0.8 g), and 10% palladium hydroxide on carbon (1.0 g) were dissolved in 30 mL of tetrahydrofuran and 30 mL of methanol. The mixture was subjected to hydrogen replacement three times, and reacted at 30°C under the atmosphere of hydrogen balloon for 16 h. The reaction liquid was cooled to room temperature, and 50 mL of dichloromethane was added. The mixture was stirred for 5 min and filtered with celite. The filter cake was washed with 50 mL of methanol three times, and the organic phases were combined, dried over anhydrous sodium sulfate, and concentrated under reduced pressure to obtain the crude 7d (850 mg).

LCMS m/z = 262.1 $[M+1]^+$.

Step 4: Preparation of 7e

The above-mentioned crude 7d (380 mg), KI (0.37 g, 2.23 mmol), CuI (0.406 g, 2.13 mmol) and iodine (560 mg, 2.21 mmol) were dissolved in acetonitrile (5 mL), and cooled to 0°C. Isoamyl nitrite (260 mg, 2.21 mmol) was slowly added. The mixture was reacted at 30°C for 6 h under nitrogen protection. To the reaction liquid was added 10 mL of saturated ammonium chloride solution, and the mixture was extracted with 50 mL of dichloromethane. Liquid separation was carried out and the organic phase was dried over anhydrous sodium sulfate and concentrated under reduced pressure. The crude was separated and purified by silica gel column chromatography (petroleum ether /ethyl acetate (v/v) =2:1-1:1) to obtain 7e (220 mg, two-step yield from 7c: 39%).

Step 5: Preparation of 7f

7e (100 mg, 0.27 mmol), tert-butyl 4-(prop-2-yn-1-yloxy)piperidine-1-carboxylate (78 mg, 0.326 mmol), CuI (10 mg, 0.0525 mmol), $PdCl_2(PPh_3)_2$ (20 mg, 0.0285 mmol) and triethylamine (0.14 g, 1.38 mmol) were dissolved in DMF (5 mL), the mixture was subjected to nitrogen replacement three times, and the mixture was reacted at 60°C for 6 h. The reaction solution was cooled to room temperature, 5 mL of water was added, and the mixture was extracted with 50 mL of dichloromethane. Liquid separation was carried out, and the organic phase was concentrated under reduced pressure. The crude was separated and purified by silica gel column chromatography (petroleum ether/ethyl acetate (v/v) = 2 : 1-1 : 1) to obtain 7f (110 mg, yield: 84%).

Step 6 Preparation of 7 g trifluoroacetate salt

7f (110 mg, 0.23 mmol) was dissolved in 5 mL of dichloromethane, 2 mL of trifluoroacetic acid was added, and the mixture was reacted at room temperature for 2 h. The reaction liquid was concentrated under reduced pressure to obtain 7 g of a crude trifluoroacetate salt (0.12 g).

Step 7: Preparation of compound 7

The above-mentioned 7 g of the crude trifluoroacetate salt (120 mg) was dissolved in 5 mL of DMA, and sodium bicarbonate (37 mg, 0.44 mmol) was added. After stirring at room temperature for 15 min, 6h (130 mg, 0.273 mmol), 0.04 mL of acetic acid and a 4Å molecular sieve (2 g) were added. After stirring at room temperature for 30 min, sodium triacetoxyborohydride (97 mg, 0.458 mmol) was added and the mixture was reacted at room temperature for 16 h. 20 mL of aqueous saturated sodium bicarbonate solution and 20 mL of dichloromethane were added to the reaction liquid, liquid

separation was carried out, and the organic phase was concentrated under reduced pressure. The crude was separated and purified by silica gel column chromatography (dichloromethane/methanol (v/v) = 100 : 1-20 : 1). The obtained crude was subjected to chiral preparative chromatography and lyophilized to obtain compound 7 (57.1 mg, yield: 25%).

Chiral preparation method:

instrument and preparative column: Waters 150 SFC preparative liquid chromatography,
preparative column model Chiralpak Column;
Mobile phase system: $sCO_2$ (supercritical $CO_2$)/a mixed solvent of isopropanol and acetonitrile,
isocratic elution: $sCO_2$/a mixed solvent of isopropanol and acetonitrile = 2:3; flow rate: 100 mL/min.

Chiral analysis method for compound 7:

instrument: SHIMADZU LC-30AD sf; chromatographic column: Chiralpak AD-3; Specification: 50 × 4.6 mm I.D., 3 μm ; mobile phase A: $sCO_2$ (supercritical $CO_2$); mobile phase B: a mixed solvent of isopropanol and acetonitrile (containing 0.05% diethylamine); column temperature: 35°C; flow rate: 3 mL/min; wavelength: 220 nm; Elution program: Isocratic elution: mobile phase A: B = 2 : 3; retention time of compound 7: 0.989 min.
$^1$H NMR (400 MHz, DMSO-$d_6$) δ 10.90 (s, 1H), 9.56 - 9.42 (m, 1H), 8.77 (d, 1H), 8.42 - 8.35 (m, 1H), 8.29 - 8.22 (m,1H), 7.78 (d, 1H), 7.51 (d, 1H), 7.29 - 6.93 (m, 2H), 6.90 - 6.40 (m, 1H), 5.33 - 5.02 (m, 1H), 4.84 - 4.70 (m, 1H), 4.53 (s, 2H), 4.41 (dd, 1H), 4.24 - 4.10 (m, 1H), 3.97 - 3.40 (m, 5H), 2.80 - 2.56 (m, 4H), 2.43 - 2.31 (m, 1H), 2.27 - 1.82 (m, 13H), 1.80 - 1.66 (m, 2H), 1.66 - 1.41 (m, 3H), 1.14 - 0.96 (m, 2H).
LCMS m/z = 853.4 $[M+1]^+$.

**Example 8: Preparation of compound 8** (Trans)

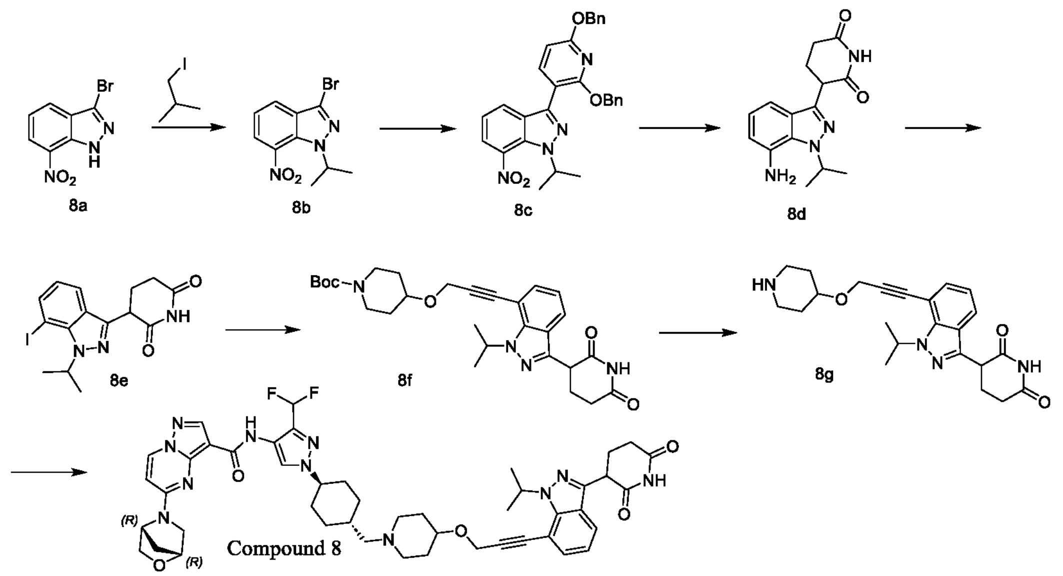
8a
8b
8c
8d
8e
8f
8g
Compound 8

**Compound 8** was prepared using 8a+iodoisopropyl as the starting material and referring to the synthesis method of example 6.
Purification method of crude compound 8: The crude was first subjected to chiral preparation and then conventional preparation, and lyophilized to obtain chiral isomer 1 (6.3 mg, yield: 4%) and chiral isomer 2 (9.6 mg, yield: 6%) of compound 8.

Chiral preparation method:

instrument and preparative column: Waters 150 SFC preparative liquid chromatography, preparative column model Chiralpak Column;
Mobile phase system: $sCO_2$ (supercritical $CO_2$)/a mixed solvent of isopropanol and acetonitrile, isocratic elution: $sCO_2$/a mixed solvent of isopropanol and acetonitrile = 65 : 35; flow rate: 140 mL/min.

Analytical methods for target compounds:

instrument: SHIMADZU LC-30AD sf; chromatographic column: Chiralpak AD-3; Specification: 50 × 4.6 mm I.D., 3 μm ; mobile phase A: $sCO_2$ (supercritical $CO_2$); mobile phase B: a mixed solvent of isopropanol and acetonitrile (containing 0.05% diethylamine); column temperature: 35°C; flow rate: 3 mL/min; wavelength: 220 nm; Elution program: Isocratic elution: mobile phase A:B = 3 : 2; retention time of chiral isomer 1 of compound 8: 2.086 min. retention time of chiral isomer 2 of compound 8: 2.687 min.

Conventional preparation method:

instrument and preparative column: SHIMADZU LC-20AP preparative liquid chromatography, preparative column model C18 packing material. Preparation method: the crude was dissolved with acetonitrile and water to prepare into a sample liquid. Mobile phase system: acetonitrile/water (containing 0.225% formic acid). Gradient elution method: a gradient from 30% acetonitrile to 60% acetonitrile (elution time 15 min).

NMR of chiral isomer 1 of compound 8:

$^1$H NMR (400 MHz, DMSO-$d_6$) δ 10.88 (s, 1H), 9.56 - 9.42 (m, 1H), 8.78 (d, 1H), 8.42 - 8.35 (m, 1H), 8.28 - 8.22 (m,1H), 7.78 (d, 1H), 7.51 (d, 1H), 7.27 - 6.94 (m, 2H), 6.89 - 6.42 (m, 1H), 5.80 - 5.63 (m, 1H), 5.33 - 5.01 (m, 1H), 4.83 - 4.70 (m, 1H), 4.66 - 4.48 (m, 2H), 4.42 (dd, 1H), 4.30 - 4.10 (m, 1H), 3.96 - 3.40 (m, 5H), 2.80 - 2.56 (m, 3H), 2.45 - 1.65 (m, 17H), 1.65 - 1.41 (m, 9H), 1.15 - 0.95 (m, 2H).
Chiral isomer 1 of compound 8 with LCMS m/z = 878.4 $[M+1]^+$.

NMR of chiral isomer 2 of compound 8:

$^1$H NMR (400 MHz, DMSO-$d_6$) δ 10.88 (s, 1H), 9.56 - 9.42 (m, 1H), 8.77 (d, 1H), 8.42 - 8.35 (m, 1H), 8.28 - 8.22 (m,1H), 7.78 (d, 1H), 7.50 (d, 1H), 7.28 - 6.94 (m, 2H), 6.89 - 6.40 (m, 1H), 5.80 - 5.63 (m, 1H), 5.33 - 5.01 (m, 1H), 4.83 - 4.70 (m, 1H), 4.66 - 4.48 (m, 2H), 4.42 (dd, 1H), 4.30 - 4.10 (m, 1H), 3.96 - 3.40 (m, 5H), 2.80 - 2.56 (m, 3H), 2.45 - 1.65 (m, 17H), 1.65 - 1.41 (m, 9H), 1.15 - 0.95 (m, 2H).
Chiral isomer 2 of compound 8 with LCMS m/z = 878.4 $[M+1]^+$.

**Example 10:** Preparation of **compound 10** (Trans)

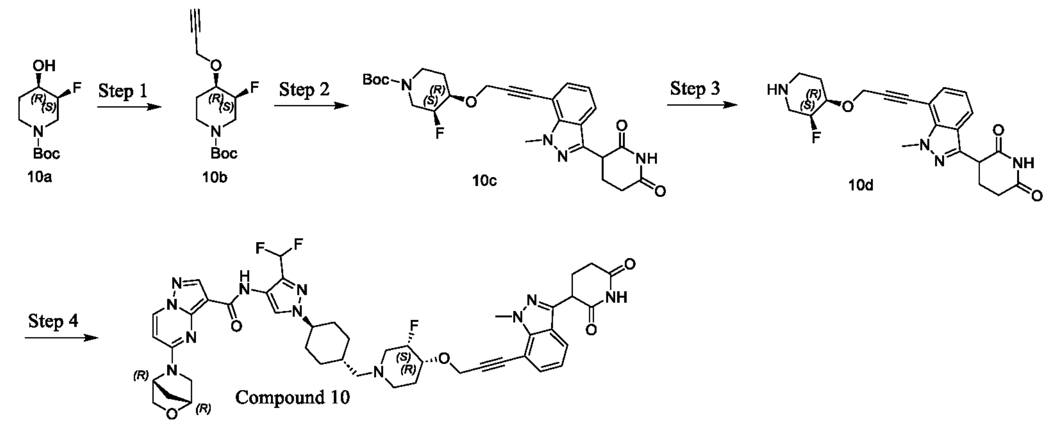


Compound 10 (20.5 mg, yield: 11%) was prepared from tert-butyl (3S,4R)-3-fluoro-4-hydroxypiperidine-1-carboxylate (10a) as the starting material with reference to the synthesis method of example 12.

Chiral preparation method:

instrument and preparative column: Waters 150 SFC preparative liquid chromatography, preparative column model Chiralpak OD Column;
Mobile phase system: $sCO_2$ (supercritical $CO_2$)/a mixed solvent of isopropanol and acetonitrile (containing 0.1% aqueous ammonia), isocratic elution: $sCO_2$/a mixed solvent of isopropanol and acetonitrile (containing 0.1% aqueous ammonia) = 3:7; flow rate: 100 mL/min.

Analytical methods for target compounds:

instrument: SHIMADZU LC-30AD sf; chromatographic column: Chiralpak OD-3; Specification: 50 × 4.6 mm I.D., 3 μm ; mobile phase A: $sCO_2$ (supercritical $CO_2$); mobile phase B: a mixed solvent of isopropanol and acetonitrile (containing 0.05% diethylamine); column temperature: 35°C; flow rate: 3 mL/min; wavelength: 220 nm; Elution program: Isocratic elution: mobile phase A: B = 2:3; retention time of target compounds: 2.329 min.

Conventional preparation method:

instrument and preparative column: SHIMADZU LC-20AP preparative liquid chromatography, preparative column model Phenomenex C18. Preparation method: the crude was dissolved with acetonitrile and water to prepare into a sample liquid. Mobile phase system: acetonitrile/water (containing 10 mmol/L ammonium bicarbonate). Gradient elution method: a gradient from 50% acetonitrile to 80% acetonitrile (elution time 10 min).

$^1$H NMR (400 MHz, DMSO-$d_6$) δ 10.90 (s, 1H), 9.56 - 9.42 (m, 1H), 8.78 (d, 1H), 8.42 - 8.35 (m, 1H), 8.28 - 8.22 (m,1H), 7.79 (d, 1H), 7.52 (d, 1H), 7.27 - 6.94 (m, 2H), 6.89 - 6.42 (m, 1H), 5.33 - 5.01 (m, 1H), 4.95 - 4.70 (m, 2H), 4.61 (s, 2H), 4.46 - 4.36 (m, 1H), 4.29 (s, 3H), 4.24 - 4.10 (m, 1H), 3.88 - 3.40 (m, 5H), 2.95 - 2.55 (m, 4H), 2.45 - 1.65 (m, 16H), 1.65 - 1.45 (m, 1H), 1.11- 0.95 (m, 2H).

LCMS m/z = 868.3 [M+1]$^+$.

**Example 11: Preparation of compound 11** (Trans)

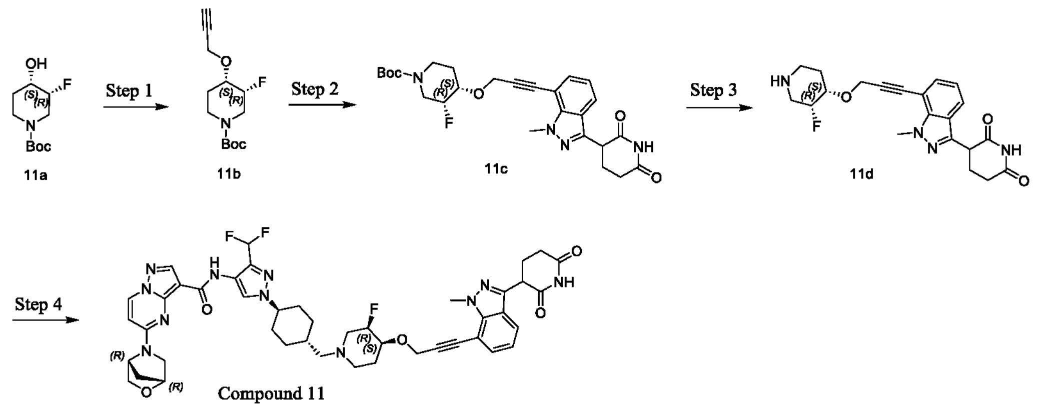

11a
11b
11c
11d
Compound 11

Using 11a and 3-bromoprop-1-yne as starting materials and referring to the synthesis method of example 12, compound 11 (25.7 mg) was obtained.

Chiral preparation method:

instrument and preparative column: Waters 150 SFC preparative liquid chromatography, preparative column model Chiralpak Column;
Mobile phase system: $sCO_2$ (supercritical $CO_2$)/a mixed solvent of isopropanol and acetonitrile (containing 0.1% aqueous ammonia), isocratic elution: $sCO_2$/a mixed solvent of isopropanol and

acetonitrile (containing 0.1% aqueous ammonia) = 3:7; flow rate: 100 mL/min.

Analytical methods for target compounds:

instrument: SHIMADZU LC-30AD sf; chromatographic column: Chiralpak OD-3; Specification: 50 × 4.6 mm I.D., 3 μm ; mobile phase A: $sCO_2$ (supercritical $CO_2$); mobile phase B: a mixed solvent of isopropanol and acetonitrile (containing 0.05% diethylamine); column temperature: 35°C; flow rate: 3 mL/min; wavelength: 220 nm; Elution program: Isocratic elution: mobile phase A: B = 2:3; retention time of target compounds: 2.263 min.

Conventional preparation method:

instrument and preparative column: SHIMADZU LC-20AP preparative liquid chromatography, preparative column model Phenomenex C18. Preparation method: the crude was dissolved with acetonitrile and water to prepare into a sample liquid. Mobile phase system: acetonitrile/water (containing 10 mmol/L ammonium bicarbonate). Gradient elution method: a gradient from 40% acetonitrile to 70% acetonitrile (elution time 10 min).

$^1$H NMR (400 MHz, DMSO-$d_6$) δ 10.90 (s, 1H), 9.56 - 9.42 (m, 1H), 8.78 (d, 1H), 8.42 - 8.35 (m, 1H), 8.28 - 8.22 (m,1H), 7.79 (d, 1H), 7.52 (d, 1H), 7.27 - 6.94 (m, 2H), 6.89 - 6.42 (m, 1H), 5.33 - 5.01 (m, 1H), 4.95 - 4.70 (m, 2H), 4.61 (s, 2H), 4.46 - 4.36 (m, 1H), 4.29 (s, 3H), 4.24 - 4.10 (m, 1H), 3.88 - 3.40 (m, 5H), 2.95 - 2.55 (m, 4H), 2.45 - 1.65 (m, 16H), 1.65 - 1.45 (m, 1H), 1.11- 0.95 (m, 2H).

LCMS m/z = 868.3 $[M+1]^+$.

**Example 12:** Preparation of **compound 12** (Trans)

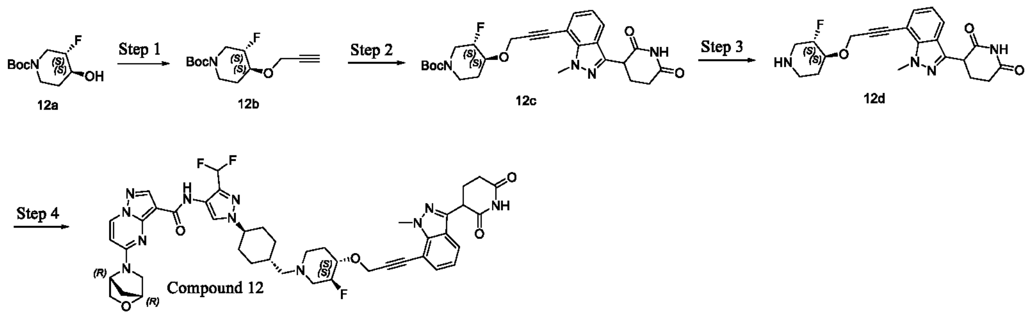


Step 1: Preparation of 12b

12a (1.3 g, 5.93 mmol) was added to 50 mL of tetrahydrofuran, and the mixture was cooled to 0°C. 0.22 g of sodium hydride was added, and the mixture was stirred at 0°C for 30 min. Then, 3-bromoprop-1-yne (0.77 g, 6.48 mmol) was added and the mixture was reacted at room temperature for 12 h. 100 mL of saturated ammonium chloride solution was added to the reaction system, and the mixture was extracted with 100 mL of ethyl acetate. The organic phase was dried over anhydrous sodium sulfate and concentrated under reduced pressure. The crude was separated and purified by silica gel column chromatography (petroleum ether/ethyl acetate (v/v) = 4:1) to obtain 12b (1.1 g, yield: 72%).

Step 2: Preparation of 12c

12b (0.07 g, 0.27 mmol) and 3-(7-iodo-1-methyl-1H-indazol-3-yl)piperidine-2,6-dione (2C) (0.1 g, 0.27 mmol) were added to 5 mL of DMF, and 1 mL of triethylamine, CuI (0.02 g, 0.1 mmol) and $PdCl_2(PPh_3)_2$ (0.035 g, 0.05 mmol) were added. The mixture was subjected to nitrogen replacement three times, and reacted at 60°C for 4 h under nitrogen protection. The reaction liquid was cooled to room temperature, 30 mL of ethyl acetate was added, and the organic phase was washed with 50 mL of purified water. The mixture was dried over anhydrous sodium sulfate, and concentrated under reduced pressure. The crude was separated and purified by silica gel column chromatography (petroleum ether/ethyl acetate (v/v) = 2:1-1:1) to obtain 12c (0.09 g, yield: 67%).

Step 3: Preparation of the trifluoroacetate salt of 12d

Compound 12c (0.09 g, 0.18 mmol) was added to 3 mL of dichloromethane, and 1 mL of trifluoroacetic acid was

added, and the mixture was reacted at room temperature for 2 h. The reaction liquid was concentrated under reduced pressure to obtain the trifluoroacetate salt of a crude 12d (0.1 g).

LCMS m/z = 399.1 $[M+1]^+$.

Step 4: Preparation of compound 12

To the above-mentioned trifluoroacetate salt (0.1 g) of the crude 12d, 2 mL of triethylamine and 5 mL of DMA were added to dissolve same. Then, 6h (0.087 g, 0.18 mmol) was added, and after stirring at room temperature for 3 h, sodium triacetoxyborohydride (0.042 g, 0.2 mmol) was added, and the mixture was reacted at room temperature for 16 h. The reaction liquid was filtered, and the filtrate was concentrated under reduced pressure. The obtained crude was first subjected to chiral preparation and then conventional preparation, and lyophilized to obtain compound 12 (22 mg, yield: 14%).

Chiral preparation method:

instrument and preparative column: Waters 150 SFC preparative liquid chromatography, preparative column model Chiralpak Column;
Mobile phase system: $sCO_2$ (supercritical $CO_2$)/a mixed solvent of isopropanol and acetonitrile, isocratic elution: $sCO_2$/a mixed solvent of isopropanol and acetonitrile = 2:3; flow rate: 100 mL/min.

Analytical methods for target compounds:

instrument: SHIMADZU LC-30AD sf; chromatographic column: Chiralpak AD-3; Specification: 50 × 4.6 mm I.D., 3 μm ; mobile phase A: $sCO_2$ (supercritical $CO_2$); mobile phase B: a mixed solvent of isopropanol and acetonitrile (containing 0.05% diethylamine); column temperature: 35°C; flow rate: 3 mL/min; wavelength: 220 nm; Elution program: Isocratic elution: mobile phase A:B = 2:3; retention time of target compounds: 1.16 min.

Conventional preparation method:

instrument and preparative column: SHIMADZU LC-20AP preparative liquid chromatography, preparative column model Phenomenex C18. Preparation method: the crude was dissolved with acetonitrile and water to prepare into a sample liquid. Mobile phase system: acetonitrile/water (containing 0.05% ammonium bicarbonate). Gradient elution method: a gradient from 45% acetonitrile to 75% acetonitrile (elution time 15 min).

$^1H$ NMR (400 MHz, DMSO-$d_6$) δ 10.90 (s, 1H), 9.56 - 9.42 (m, 1H), 8.77 (d, 1H), 8.42 - 8.35 (m, 1H), 8.28 - 8.22 (m,1H), 7.79 (d, 1H), 7.52 (d, 1H), 7.27 - 6.94 (m, 2H), 6.89 - 6.42 (m, 1H), 5.33 - 5.01 (m, 1H), 4.85 - 4.70 (m, 1H), 4.63 (s, 2H), 4.59 - 4.35 (m, 2H), 4.29 (s, 3H), 4.24 - 4.08 (m, 1H), 3.88 - 3.40 (m, 5H), 3.10 - 2.95 (m, 1H), 2.78 - 2.55 (m, 3H), 2.45 - 1.37 (m, 17H), 1.11- 0.95 (m, 2H).

LCMS m/z = 434.8 $[M/2+1]^+$.

**Example 13:** Preparation of **compound 13** (Trans)

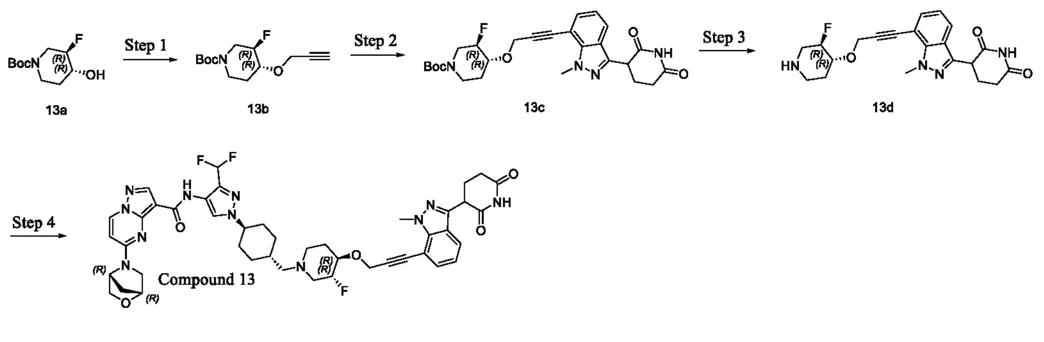

13a 13b 13c 13d Compound 13

Using 13a+3-bromoprop-1-yne as the starting material and referring to the synthesis method of example 12, compound 13 (10.6 mg) was obtained.

Chiral preparation method:

instrument and preparative column: Waters 150 SFC preparative liquid chromatography, preparative column model Chiralpak AD Column;
Mobile phase system: $sCO_2$ (supercritical $CO_2$)/a mixed solvent of isopropanol and acetonitrile (containing 0.1% aqueous ammonia), isocratic elution: $sCO_2$/a mixed solvent of isopropanol and acetonitrile (containing 0.1% aqueous ammonia) = 2:3; flow rate: 100 mL/min.

Analytical methods for target compounds:

instrument: SHIMADZU LC-30AD sf; chromatographic column: Chiralpak AD-3; Specification: 50 × 4.6 mm I.D., 3 μm ; mobile phase A: $sCO_2$ (supercritical $CO_2$); mobile phase B: a mixed solvent of isopropanol and acetonitrile (containing 0.05% diethylamine); column temperature: 35°C; flow rate: 3 mL/min; wavelength: 220 nm; Elution program: Isocratic elution: mobile phase A:B = 1:1; retention time of target compounds: 2.345 min.

Conventional preparation method:

instrument and preparative column: SHIMADZU LC-20AP preparative liquid chromatography, preparative column model Phenomenex C18. Preparation method: the crude was dissolved with acetonitrile and water to prepare into a sample liquid. Mobile phase system: acetonitrile/water (containing 10 mmol/L ammonium bicarbonate). Gradient elution method: a gradient from 40% acetonitrile to 70% acetonitrile (elution time 15 min).
$^1$H NMR (400 MHz, DMSO-$d_6$) δ 10.90 (s, 1H), 9.56 - 9.42 (m, 1H), 8.78 (d, 1H), 8.42 - 8.35 (m, 1H), 8.28 - 8.22 (m,1H), 7.79 (d, 1H), 7.52 (d, 1H), 7.27 - 6.94 (m, 2H), 6.89 - 6.42 (m, 1H), 5.33 - 5.01 (m, 1H), 4.85 - 4.70 (m, 1H), 4.63 (s, 2H), 4.59 - 4.35 (m, 2H), 4.29 (s, 3H), 4.24 - 4.08 (m, 1H), 3.88 - 3.40 (m, 5H), 3.10 - 2.95 (m, 1H), 2.78 - 2.55 (m, 3H), 2.45 - 1.37 (m, 17H), 1.11- 0.95 (m, 2H).
LCMS m/z = 868.3 $[M+1]^+$.

**Example 14:** Preparation of the trifluoroacetate salt of **compound 14** (Trans)

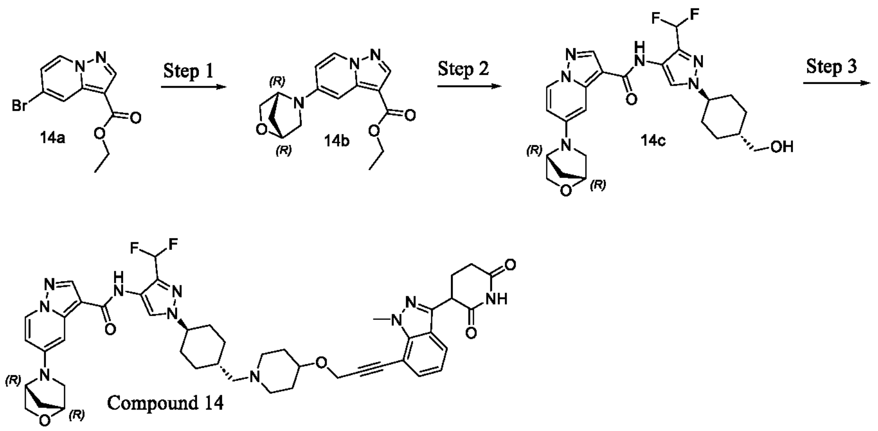

14a
14b
14c
Compound 14

Step 1: Preparation of 14b

14a (1.1 g, 4.09 mmol), (1R,4R)-2-oxa-5-azabicyclo[2.2.1]heptane (0.49 g, 4.94 mmol),*rac*-BINAP (0.25 g, 0.40 mmol), cesium carbonate (4.0 g, 12.3 mmol) and $Pd_2(dba)_3$ (0.75 g, 0.82 mmol) were dissolved in 30 mL of dioxane. The mixture was subjected to nitrogen replacement three times and reacted at 100°C for 20 h. The reaction liquid was cooled to room temperature, 50 mL of water was slowly added, and the mixture was extracted with ethyl acetate (50 mL). The organic phase was dried over anhydrous sodium sulfate and concentrated under reduced pressure. The crude was separated and purified by silica gel column chromatography (ethyl acetate/petroleum ether (v/v) = 0:1-7:3) to obtain 14b (0.40 g, yield: 34%).
LCMS m/z = 288.1 $[M+1]^+$.

Step 2: Preparation of 14c

14b (400 mg, 1.39 mmol) and lithium hydroxide monohydrate (290 mg, 6.91 mmol) were dissolved in methanol (10 mL) and water (20 mL) and the mixture was reacted at 70°C for 20 h. The reaction liquid was cooled to room temperature, and concentrated under reduced pressure. 20 mL of water was added, the pH was adjusted to 5 with 1 mol/L aqueous hydrochloric acid solution, and the mixture was extracted with 50 mL of dichloromethane. The organic phase was dried over anhydrous sodium sulfate, and concentrated under reduced pressure to obtain a crude (200 mg). The above-mentioned crude (170 mg), trans-(4-(4-amino-3-(difluoromethyl)-1H-pyrazol-1-yl)cyclohexyl)methanol (for synthesis method, see WO 2021247897) (160 mg, 0.65 mmol) and N-methylimidazole (190 mg, 2.31 mmol) were dissolved in acetonitrile (5 mL), and then TCFH (270 mg, 0.96 mmol) was added and the mixture was reacted at room temperature for 20 h. The reaction liquid was concentrated under reduced pressure, and the crude was separated and purified by silica gel column chromatography (methanol/dichloromethane (v/v) = 0 : 1-1 : 9) to obtain 14c (150 mg, yield: 47%).

LCMS m/z = 487.2 $[M+1]^+$.

Step 3: Preparation of trifluoroacetate salt of compound 14

Compound 14c (200 mg, 0.41 mmol) was dissolved in dichloromethane (3 mL), and Dess-Martin oxidant (340 mg, 0.80 mmol) was added and the mixture was reacted at room temperature for 2 h. The reaction solution was concentrated under reduced pressure, and the crude was separated and purified by silica gel column chromatography (methanol/dichloromethane (v/v) = 0 : 1-1 : 9) to obtain intermediate 14A (100 mg). The above-mentioned crude **2E** (46 mg) was dissolved in DMA (3 mL), and intermediate 14A (60 mg) and 0.1 mL of acetic acid were added. After stirring at room temperature for 30 min, sodium triacetoxyborohydride (31 mg, 0.146 mmol) was added and the mixture was reacted at room temperature for 16 h. 10 mL of aqueous saturated sodium bicarbonate solution was slowly added to the reaction liquid, and the mixture was extracted twice with 20 mL of ethyl acetate. The organic phases were combined, washed with 30 mL of aqueous saturated sodium chloride solution, dried over anhydrous sodium sulfate, and concentrated under reduced pressure. The crude was separated and purified by silica gel column chromatography (methanol/dichloromethane (v/v) = 0 : 1-1 : 9). The obtained crude was then subjected to conventional preparation and lyophilized to obtain trifluoroacetate salt of compound 14 (9 mg).

Conventional preparation method:

instrument and preparative column: SHIMADZU LC-20AP preparative liquid chromatography, preparative column model Phenomenex C18. Preparation method: the crude was dissolved with acetonitrile and water to prepare into a sample liquid. Mobile phase system: acetonitrile/water (containing 0.1% trifluoroacetic acid). Gradient elution method: a gradient from 25% acetonitrile to 40% acetonitrile (elution time 16 min).

$^1H$ NMR (400 MHz, DMSO-$d_6$) δ 10.90 (s, 1H), 9.34 (s, 1H), 8.58 - 8.42 (m, 2H), 8.11 (s, 1H), 7.80 (d, 1H), 7.52 (d, 1H), 7.33 - 6.93 (m, 3H), 6.78 - 6.66 (m, 1H), 4.82 - 4.66 (m, 2H), 4.60 (s, 2H), 4.42 (dd, 1H), 4.35 - 4.27 (m, 3H), 4.27 - 4.15 (m, 1H), 4.05 - 3.76 (m, 2H), 3.74 - 3.65 (m, 1H), 3.65 - 3.33 (m, 3H), 3.18 - 2.93 (m, 5H), 2.77 - 2.57 (m, 2H), 2.45 - 1.60 (m, 15H), 1.30 - 1.09 (m, 2H). LCMS m/z = 849.2 $[M+1]^+$.

**Example 15:** Preparation of the trifluoroacetate salt of **compound 15** (Trans)

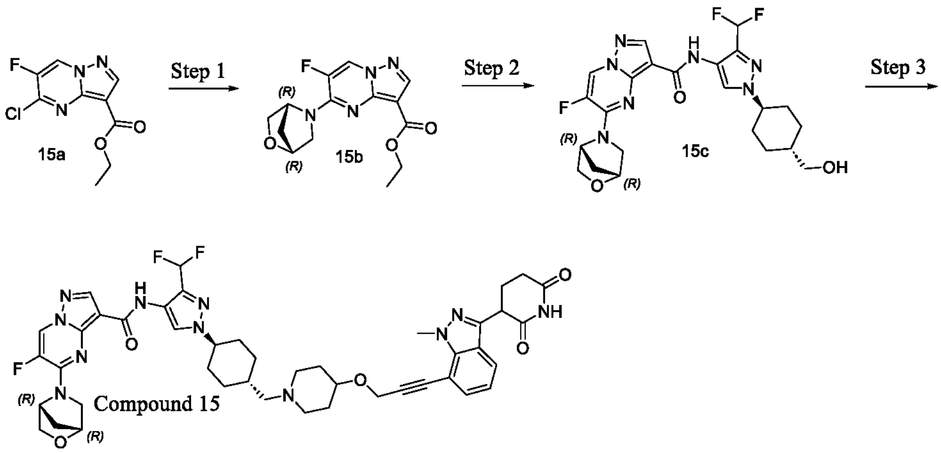


Using 15a+(1R, 4R)-2-oxa-5-azabicyclo[2.2.1]heptane as the starting material and referring to the synthesis method of example 14, trifluoroacetate salt of compound 15 (10 mg) was obtained.

Refining method: the crude was separated and purified by silica gel column chromatography (methanol/dichloromethane (v/v) = 0:1-1:9), and the obtained crude was subjected to conventional preparation, and the preparative liquid was adjusted to acidity with trifluoroacetic acid and lyophilized to obtain the trifluoroacetate salt of compound 15 (10 mg).

Conventional preparation method:

instrument and preparative column: SHIMADZU LC-20AP preparative liquid chromatography, preparative column model Phenomenex C18. Preparation method: the crude was dissolved with acetonitrile and water to prepare into a sample liquid. Mobile phase system: acetonitrile/water (containing 10 mmol/L ammonium bicarbonate). Gradient elution method: a gradient from 30% acetonitrile to 60% acetonitrile (elution time 10 min).

$^1$H NMR (400 MHz, DMSO-$d_6$) δ 10.90 (s, 1H), 9.35 - 9.17 (m, 2H), 8.40 (s, 1H), 8.29 (s, 1H), 7.80 (d, 1H), 7.53 (d, 1H), 7.30 - 6.93 (m, 2H), 5.33 - 5.12 (m, 1H), 4.77 - 4.70 (m, 1H), 4.60 (s, 2H), 4.47 - 4.37 (m, 1H), 4.35 - 4.15 (m, 4H), 4.07 - 3.65 (m, 5H), 3.63 - 3.32 (m, 2H), 3.15 - 2.92 (m, 4H), 2.78 - 2.55 (m, 2H), 2.46 - 1.65 (m, 15H), 1.28 - 1.10 (m, 2H). LCMS m/z = 868.3 [M+1]$^+$.

**Example 16: Preparation of compound 16** (Trans)

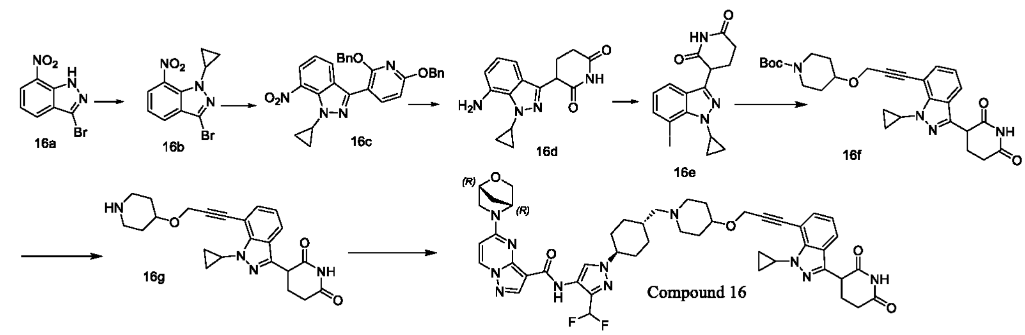


Step 1: Preparation of 16b

16a (4.0 g, 16.53 mmol), cyclopropylboronic acid (4.28 g, 49.83 mmol), sodium carbonate (5.28 g, 49.82 mmol), $Cu(OAc)_2$ (3.00 g, 16.52 mmol) and 2,2'-bipyridine (2.60 g, 16.65 mmol) were dissolved in 60 mL of DCE. The mixture was subjected to nitrogen replacement three times and reacted at 80°C for 6 h under nitrogen protection. The reaction system was cooled to room temperature and filtered, and the filtrate was concentrated under reduced pressure. The crude was separated and purified by silica gel column chromatography (petroleum ether/ethyl acetate (v/v) = 5 : 1) to obtain 16b (1.7 g, yield: 36%).

Step 2: Preparation of 16c

Under nitrogen protection, 16b (1.5 g, 5.32 mmol), 1A (3.33 g, 7.98 mmol), dioxane (60 mL), water (20 mL), $Pd(dppf)Cl_2{\bullet}CH_2Cl_2$ (CAS: 95464-05-4) (0.43 g, 0.53 mmol) and cesium carbonate (5.20 g, 15.96 mmol) were sequentially added to a 100 mL single-necked flask. The mixture was subjected to nitrogen replacement three times, and reacted at 100°C for 12 h under nitrogen protection. The reaction system was cooled to room temperature, poured into 50 mL of water, and extracted with ethyl acetate (100 mL × 3), and the organic phases were combined, dried over anhydrous sodium sulfate, and concentrated under reduced pressure. The crude was separated and purified by silica gel column chromatography (petroleum ether:ethyl acetate (v/v) = 5:1) to obtain 16c (1.8 g, yield: 69%).
LCMS m/z = 493.1 $[M+1]^+$.

Step 3: Preparation of 16d

16c (1.7 g, 3.45 mmol) and 10% Pd/C (0.8 g) were dissolved in 30 mL of tetrahydrofuran and 30 mL of methanol, and the mixture was subjected to hydrogen replacement three times, and reacted at 30°C for 16 h. The reaction system was filtered with celite, the filter cake was washed with methanol (30 mL × 3), the filtrate was dried over anhydrous sodium sulfate, and concentrated under reduced pressure to obtain a crude 16d (850 mg).
LCMS m/z = 285.1 $[M+1]^+$.

Step 4: Preparation of 16e

The above-mentioned crude 16d (400 mg), KI (0.35 g, 2.11 mmol), CuI (0.40 g, 2.10 mmol) and $I_2$ (0.54 g, 2.13 mmol) were dissolved in acetonitrile (10 mL), and cooled to 0°C. Isoamyl nitrite (250 mg, 2.13 mmol) was slowly added. The mixture was reacted at 30°C for 6 h under nitrogen protection. To the reaction system was added 10 mL of aqueous saturated ammonium chloride solution, the mixture was extracted with 50 mL of dichloromethane, and the organic phase was dried over anhydrous sodium sulfate and concentrated under reduced pressure. The crude was separated and purified by silica gel column chromatography (petroleum ether: ethyl acetate (v/v) = 2 : 1-1 : 1) to obtain 16e (220 mg, two-step yield from compound 16c: 34%).
LCMS m/z = 396.0 $[M+1]^+$.

Step 5: Preparation of 16f

16e (150 mg, 0.38 mmol), tert-butyl 4-(prop-2-yn-1-yloxy)piperidine-1-carboxylate (0.11 g, 0.46 mmol), CuI (14 mg, 0.074 mmol), $PdCl_2(PPh_3)_2$ (27 mg, 0.0385 mmol) and TEA (0.19 g, 1.88 mmol) were dissolved in 5 mL of DMF, and the mixture was subjected to nitrogen replacement three times, and reacted 60°C for 6 h. The reaction system was cooled to room temperature, 5 mL of water was added, and the mixture was extracted with 30 mL of dichloromethane. The organic phase was dried over anhydrous sodium sulfate and concentrated under reduced pressure. The crude was separated and purified by silica gel column chromatography (petroleum ether: ethyl acetate (v/v) = 2 : 1-1 : 1) to obtain 16f (130 mg, yield: 68%).

Step 6: Preparation of 16 g of trifluoroacetate salt

16f (120 mg, 0.24 mmol) was dissolved in 5 mL of dichloromethane, and 2 mL of trifluoroacetic acid was added, and the mixture was reacted at room temperature for 2 h. The reaction system was concentrated under reduced pressure to obtain 16 g of a crude trifluoroacetate salt (0.13 g).
LCMS m/z = 407.2 $[M+1]^+$.

Step 7: Preparation of compound 16

The 16 g of the crude trifluoroacetate salt (130 mg) was dissolved in 5 mL of DMA, and sodium bicarbonate (40 mg, 0.48 mmol) was added. After stirring at room temperature for 15 min, 6h (140 mg, 0.29 mmol), 0.04 mL of acetic acid and a 4 Å molecular sieve (2 g) were added. After stirring at room temperature for 30 min, sodium triacetoxyborohydride (102 mg, 0.48 mmol) was added and the mixture was reacted at room temperature for 16 h. 20 mL of aqueous saturated sodium bicarbonate solution and 20 mL of dichloromethane were added to the reaction liquid, liquid separation was carried out, and the organic phase was concentrated under reduced pressure. The crude was separated and purified by silica gel column chromatography (dichloromethane/methanol (v/v) = 100:1-20:1). The obtained crude was subjected to HPLC purification to obtain compound 16 (50.4 mg, yield: 20%).
$^1H$ NMR (400 MHz, DMSO-$d_6$) δ 10.89 (s, 1H), 9.55 - 9.45 (m, 1H), 8.78 (d, 1H), 8.38 (d, 1H), 8.25 (d, 1H), 7.75 (d,

1H), 7.52 (d, 1H), 7.27 - 6.92 (m, 2H), 6.90 - 6.40 (m, 1H), 5.33 - 5.02 (m, 1H), 4.83 - 4.70 (m, 1H), 4.51 (s, 2H), 4.43 - 4.32 (m, 1H), 4.23 - 4.01 (m, 2H), 3.88 - 3.40 (m, 5H), 2.78 - 2.55 (m, 4H), 2.44 - 1.40 (m, 19H), 1.30 - 0.94 (m, 6H).

LCMS m/z = 876.3 $[M+1]^+$.

**Example 17: Preparation of compound 17** (Trans)

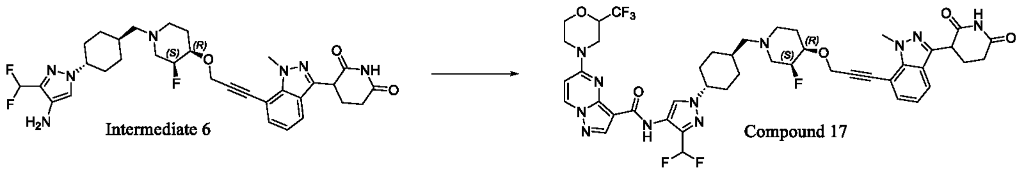


The hydrochloride salt of the above-mentioned crude intermediate 6 (130 mg) was dissolved in 5 mL of acetonitrile, and the above-mentioned crude intermediate 2 (61 mg) and TCFH (67 mg, 0.24 mmol) were added, and NMI (0.11 g, 1.34 mmol) was added, and the mixture was reacted at room temperature for 16 h. The reaction system was concentrated under reduced pressure, 10 mL of water was added to the residue, and the mixture was filtered. The filter cake was washed with 5 mL of water, dissolved in 10 mL of DCM, dried over anhydrous sodium sulfate, and concentrated under reduced pressure. The crude was separated and purified by silica gel column chromatography (dichloromethane/methanol (v/v) = 12:1). The obtained crude was subjected to chiral preparative chromatography to obtain chiral isomer 1 (37.5 mg, two-step yield from compound 2-B: 18%) and chiral isomer 2 (18.4 mg, two-step yield from compound 2-B: 9%) of compound 17, respectively.

Chiral preparation method:

instrument and preparative column: Shimadzu LC-A HPLC preparative liquid chromatography, preparative column model Chiralpak IE, 250 × 30 mm, 10 μm; Mobile phase system: acetonitrile/methanol, isocratic elution: acetonitrile /methanol = 1 : 1; flow rate: 80 mL/min.

Analytical methods for target compounds:

instrument: Shimadzu LC-20AB with PDA detector; chromatographic column: Chiralpak IE-3; Specification: 150 × 4.6 mm I.D., 3 μm ; mobile phase A: acetonitrile; mobile phase B: methanol; column temperature: 35°C; flow rate: 1 mL/min; wavelength: 220 nm; Elution program: Isocratic elution: mobile phase A: B = 3 : 7;

retention time of chiral isomer 1: 3.368 min.

$^1$H NMR (400 MHz, DMSO-$d_6$) δ 10.89 (s, 1H), 9.28 (s, 1H), 8.88 (d, 1H), 8.38 - 8.29 (m, 2H), 7.79 (d, 1H), 7.52 (d, 1H), 7.26 - 6.90 (m, 3H), 4.94 - 4.72 (m, 1H), 4.61 (s, 2H), 4.58 - 4.48 (m, 1H), 4.48 - 4.34 (m, 3H), 4.29 (s, 3H), 4.24 - 4.04 (m, 2H), 3.85 - 3.71 (m, 2H), 3.30 - 3.22 (m, 2H), 2.95 - 2.76 (m, 1H), 2.76 - 2.55 (m, 3H), 2.46 - 2.26 (m, 2H), 2.24 - 2.10 (m, 4H), 2.10 - 1.95 (m, 2H), 1.95 - 1.65 (m, 6H), 1.65 - 1.49 (m, 1H), 1.13 - 0.94 (m, 2H).

LCMS m/z = 924.4 $[M+1]^+$.

retention time of chiral isomer 2: 4.029 min.

$^1$H NMR (400 MHz, DMSO-$d_6$) δ 10.89 (s, 1H), 9.28 (s, 1H), 8.88 (d, 1H), 8.38 - 8.29 (m, 2H), 7.79 (d, 1H), 7.52 (d, 1H), 7.26 - 6.90 (m, 3H), 4.94 - 4.72 (m, 1H), 4.60 (s, 2H), 4.58 - 4.48 (m, 1H), 4.48 - 4.34 (m, 3H), 4.29 (s, 3H), 4.24 - 4.04 (m, 2H), 3.85 - 3.71 (m, 2H), 3.30 - 3.22 (m, 2H), 2.95 - 2.76 (m, 1H), 2.76 - 2.55 (m, 3H), 2.46 - 2.26 (m, 2H), 2.24 - 2.10 (m, 4H), 2.10 - 1.95 (m, 2H), 1.95 - 1.65 (m, 6H), 1.65 - 1.49 (m, 1H), 1.13 - 0.94 (m, 2H).

LCMS m/z = 924.4 $[M+1]^+$.

**Example 18: Preparation of compound 18** (Trans)

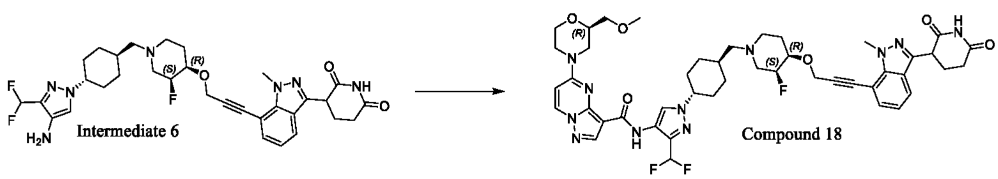

The hydrochloride salt of the above-mentioned crude intermediate 6 (130 mg) was dissolved in 5 mL of acetonitrile, and the above-mentioned crude intermediate 1 (56 mg) and TCFH (67 mg, 0.24 mmol) were added, and NMI (0.11 g, 1.34 mmol) was added, and the mixture was reacted at room temperature for 16 h. The reaction system was concentrated under reduced pressure, 10 mL of water was added to the residue, and the mixture was filtered. The filter cake was washed with 5 mL of water, dissolved in 10 mL of DCM, dried over anhydrous sodium sulfate, and concentrated under reduced pressure. The crude was separated and purified by silica gel column chromatography (dichloromethane/methanol (v/v) = 12 : 1). The obtained crude was subjected to chiral preparative chromatography to obtain chiral isomer 1 (17.8 mg, two-step yield from compound 1-D: 9%) and chiral isomer 2 (16.2 mg, two-step yield from compound 1-D: 9%) of compound 18, respectively.

Chiral preparation method:

instrument and preparative column: Shimadzu LC-A HPLC preparative liquid chromatography, preparative column model Chiralpak IE, 250 × 30 mm, 10 μm; Mobile phase system: acetonitrile/methanol, isocratic elution: acetonitrile /methanol = 1 : 1; flow rate: 80 mL/min.

Analytical methods for target compounds: instrument: Shimadzu LC-20AB with PDA detector; chromatographic column: Chiralpak IE-3; Specification: 150 × 4.6 mm I.D., 3 μm ; mobile phase A: acetonitrile; mobile phase B: methanol; column temperature: 35°C; flow rate: 1 mL/min; wavelength: 220 nm; Elution program: Isocratic elution: mobile phase A: B = 3 : 7;

retention time of chiral isomer 1: 6.469 min.

$^1H$ NMR (400 MHz, DMSO-$d_6$) δ 10.89 (s, 1H), 9.32 (s, 1H), 8.81 (d, 1H), 8.37 (s, 1H), 8.29 (s, 1H), 7.84 - 7.73 (m, 1H), 7.56 - 7.48 (m, 1H), 7.28 - 6.96 (m, 2H), 6.91 (d, 1H), 4.94 - 4.71 (m, 1H), 4.60 (s, 2H), 4.46 - 4.24 (m, 6H), 4.24 - 4.10 (m, 1H), 4.00 - 3.91 (m, 1H), 3.87 - 3.53 (m, 3H), 3.50 - 3.42 (m, 2H), 3.29 (s, 3H), 3.21 - 3.07 (m, 1H), 3.06 - 2.93 (m, 1H), 2.93 - 2.76 (m, 1H), 2.76 - 2.55 (m, 3H), 2.45 - 2.25 (m, 2H), 2.25 - 2.10 (m, 4H), 2.10 - 1.95 (m, 2H), 1.95 - 1.65 (m, 6H), 1.65 - 1.49 (m, 1H), 1.12 - 0.95 (m, 2H).

LCMS m/z = 900.5 $[M+1]^+$.

retention time of chiral isomer 2: 7.967 min.

$^1H$ NMR (400 MHz, DMSO-$d_6$) δ 10.89 (s, 1H), 9.32 (s, 1H), 8.81 (d, 1H), 8.37 (s, 1H), 8.29 (s, 1H), 7.84 - 7.73 (m, 1H), 7.56 - 7.48 (m, 1H), 7.28 - 6.96 (m, 2H), 6.91 (d, 1H), 4.94 - 4.71 (m, 1H), 4.60 (s, 2H), 4.46 - 4.24 (m, 6H), 4.24 - 4.10 (m, 1H), 4.00 - 3.91 (m, 1H), 3.87 - 3.53 (m, 3H), 3.50 - 3.42 (m, 2H), 3.29 (s, 3H), 3.21 - 3.07 (m, 1H), 3.06 - 2.93 (m, 1H), 2.93 - 2.76 (m, 1H), 2.76 - 2.55 (m, 3H), 2.45 - 2.25 (m, 2H), 2.25 - 2.10 (m, 4H), 2.10 - 1.95 (m, 2H), 1.95 - 1.65 (m, 6H), 1.65 - 1.49 (m, 1H), 1.12 - 0.95 (m, 2H).

LCMS m/z = 900.4 $[M+1]^+$.

**Example 19: Preparation of compound 19** (Trans)

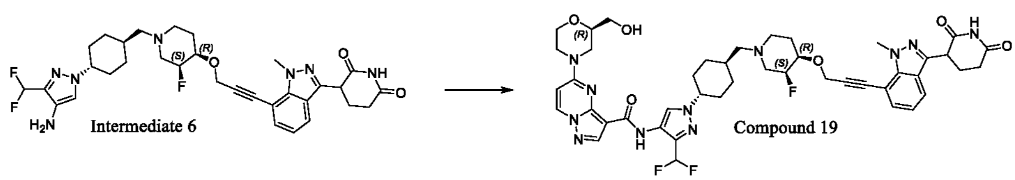


The hydrochloride salt of the above-mentioned crude intermediate 6 (130 mg) was dissolved in 5 mL of acetonitrile, and the above-mentioned crude intermediate 3 (53 mg) and TCFH (67 mg, 0.24 mmol) were added, and NMI (0.11 g, 1.34 mmol) was added, and the mixture was reacted at room temperature for 16 h. The reaction system was concentrated under reduced pressure, 10 mL of water was added to the residue, and the mixture was filtered. The filter cake was washed with 5 mL of water, dissolved in 10 mL of DCM, dried over anhydrous sodium sulfate, and concentrated under reduced pressure. The crude was separated and purified by silica gel column chromatography (dichloromethane/methanol (v/v) = 12 : 1). The obtained crude was subjected to chiral preparative chromatography to obtain chiral isomer 1 (18.2 mg, two-step yield from compound 3-A: 7%) and chiral isomer 2 (12.5 mg, two-step yield from compound 3-A: 5%) of compound 19, respectively.

Chiral preparation method:

instrument and preparative column: Shimadzu LC-A HPLC preparative liquid chromatography, preparative

column model Chiralpak IE, 250 × 30 mm, 10 μm; Mobile phase system: acetonitrile/methanol, isocratic elution: acetonitrile /methanol = 1:1; flow rate: 80 mL/min.

Analytical methods for target compounds:

instrument: Shimadzu LC-20AB with PDA detector; chromatographic column: Chiralpak IE-3; Specification: 150 × 4.6 mm I.D., 3 μm ; mobile phase A: acetonitrile; mobile phase B: methanol; column temperature: 35°C; flow rate: 1 mL/min; wavelength: 220 nm; Elution program: Isocratic elution: mobile phase A: B = 3 : 7;
retention time of chiral isomer 1: 5.313 min.
$^1$H NMR (400 MHz, DMSO-$d_6$) δ 10.90 (s, 1H), 9.34 (s, 1H), 8.81 (d, 1H), 8.36 (s, 1H), 8.29 (s, 1H), 7.79 (d, 1H), 7.52 (d, 1H), 7.27 - 6.94 (m, 2H), 6.90 (d, 1H), 4.93 - 4.72 (m, 2H), 4.61 (s, 2H), 4.52 - 4.10 (m, 7H), 4.03 - 3.92 (m, 1H), 3.87 - 3.70 (m, 1H), 3.67 - 3.46 (m, 4H), 3.20 - 3.06 (m, 1H), 3.05 - 2.93 (m, 1H), 2.93 - 2.76 (m, 1H), 2.75 - 2.56 (m, 3H), 2.44 - 2.30 (m, 2H), 2.25 - 2.10 (m, 4H), 2.09 - 1.95 (m, 2H), 1.95 - 1.66 (m, 6H), 1.65 - 1.50 (m, 1H), 1.12 - 0.95 (m, 2H).
LCMS m/z = 886.4 $[M+1]^+$.
retention time of chiral isomer 2: 6.457 min.
$^1$H NMR (400 MHz, DMSO-$d_6$) δ 10.89 (s, 1H), 9.34 (s, 1H), 8.81 (d, 1H), 8.36 (s, 1H), 8.29 (s, 1H), 7.79 (d, 1H), 7.52 (d, 1H), 7.27 - 6.94 (m, 2H), 6.90 (d, 1H), 4.93 - 4.72 (m, 2H), 4.60 (s, 2H), 4.52 - 4.10 (m, 7H), 4.03 - 3.92 (m, 1H), 3.87 - 3.70 (m, 1H), 3.67 - 3.46 (m, 4H), 3.20 - 3.06 (m, 1H), 3.05 - 2.93 (m, 1H), 2.93 - 2.76 (m, 1H), 2.75 - 2.56 (m, 3H), 2.44 - 2.30 (m, 2H), 2.25 - 2.10 (m, 4H), 2.09 - 1.95 (m, 2H), 1.95 - 1.66 (m, 6H), 1.65 - 1.50 (m, 1H), 1.12 - 0.95 (m, 2H).
LCMS m/z = 886.4 $[M+1]^+$.
The obtained chiral isomer 2 was subjected to acidic preparative chromatography to obtain the trifluoroacetate salt of chiral isomer 2.

Acidic preparation method:

instrument and preparative column: Shimadzu LC-20AP preparative liquid chromatography, preparative column model Welch Xtimate C18; Mobile phase system: water (containing 0.1% trifluoroacetic acid)/acetonitrile, gradient elution: water (containing 0.1% trifluoroacetic acid)/acetonitrile = 18:82-48:52; flow rate: 25 mL/min.
NMR data of trifluoroacetate salt of chiral isomer 2:
$^1$H NMR (400 MHz, DMSO-$d_6$) δ 10.91 (s, 1H), 9.35 (s, 1H), 8.82 (d, 1H), 8.38 (s, 1H), 8.29 (s, 1H), 7.81 (d, 1H), 7.53 (d, 1H), 7.27 - 7.07 (m, 2H), 6.91 (d, 1H), 4.90 - 4.56 (m, 3H), 4.53 - 3.82 (m, 10H), 3.65 - 3.46 (m, 4H), 3.20 - 3.08 (m, 2H), 3.07 - 2.90 (m, 2H), 2.76 - 2.56 (m, 2H), 2.45 - 1.68 (m, 14H), 1.60 - 1.46 (m, 1H), 1.34 - 1.10 (m, 2H).

**Example 20: Preparation of compound 20** (Trans)

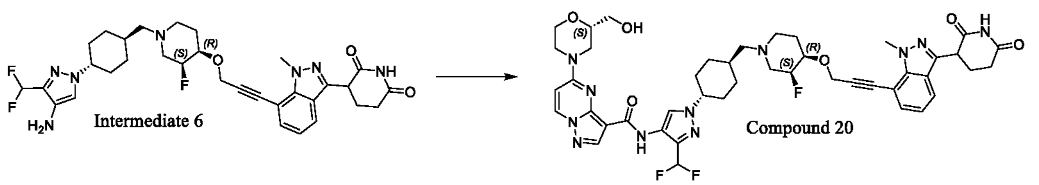


The hydrochloride salt of the above-mentioned crude intermediate 6 (130 mg) was dissolved in 5 mL of acetonitrile, and the above-mentioned crude intermediate 5 (53 mg) and TCFH (67 mg, 0.24 mmol) were added, and NMI (0.11 g, 1.34 mmol) was added, and the mixture was reacted at room temperature for 16 h. The reaction system was concentrated under reduced pressure, 10 mL of water was added to the residue, and the mixture was filtered. The filter cake was washed with 5 mL of water, dissolved in 10 mL of DCM, dried over anhydrous sodium sulfate, and concentrated under reduced pressure. The crude was separated and purified by silica gel column chromatography (dichloromethane/methanol (v/v) = 12 : 1). The obtained crude was subjected to chiral preparative chromatography to obtain chiral isomer 1 (10.3 mg, two-step yield from compound 5-A: 4%) and chiral isomer 2 (9.8 mg, two-step yield from compound 5-A: 4%) of compound 20, respectively.

Chiral preparation method:

instrument and preparative column: Shimadzu LC-A HPLC preparative liquid chromatography, preparative column model Chiralpak IE, 250 × 30 mm, 10 μm; Mobile phase system: acetonitrile/methanol, isocratic elution:

acetonitrile /methanol = 1 : 1; flow rate: 80 mL/min.

Analytical methods for target compounds:

instrument: Shimadzu LC-20AB with PDA detector; chromatographic column: Chiralpak IE-3; Specification: 150 × 4.6 mm I.D., 3 μm ; mobile phase A: acetonitrile; mobile phase B: methanol; column temperature: 35°C; flow rate: 1 mL/min; wavelength: 220 nm; Elution program: Isocratic elution: mobile phase A:B = 3:7;

retention time of chiral isomer 1: 5.229 min.

$^1$H NMR (400 MHz, DMSO-$d_6$) δ 10.89 (s, 1H), 9.34 (s, 1H), 8.81 (d, 1H), 8.36 (s, 1H), 8.29 (s, 1H), 7.79 (d, 1H), 7.52 (d, 1H), 7.27 - 6.94 (m, 2H), 6.89 (d, 1H), 4.93 - 4.72 (m, 2H), 4.61 (s, 2H), 4.52 - 4.10 (m, 7H), 4.03 - 3.92 (m, 1H), 3.87 - 3.70 (m, 1H), 3.67 - 3.46 (m, 4H), 3.20 - 3.06 (m, 1H), 3.05 - 2.93 (m, 1H), 2.93 - 2.76 (m, 1H), 2.75 - 2.56 (m, 3H), 2.44 - 2.30 (m, 2H), 2.25 - 2.10 (m, 4H), 2.09 - 1.95 (m, 2H), 1.95 - 1.66 (m, 6H), 1.65 - 1.50 (m, 1H), 1.12 - 0.94 (m, 2H).

LCMS m/z = 886.4 $[M+1]^+$.

retention time of chiral isomer 2: 6.378 min.

$^1$H NMR (400 MHz, DMSO-$d_6$) δ 10.89 (s, 1H), 9.34 (s, 1H), 8.81 (d, 1H), 8.36 (s, 1H), 8.29 (s, 1H), 7.79 (d, 1H), 7.52 (d, 1H), 7.27 - 6.94 (m, 2H), 6.90 (d, 1H), 4.93 - 4.72 (m, 2H), 4.60 (s, 2H), 4.52 - 4.10 (m, 7H), 4.03 - 3.92 (m, 1H), 3.87 - 3.70 (m, 1H), 3.67 - 3.46 (m, 4H), 3.20 - 3.06 (m, 1H), 3.05 - 2.93 (m, 1H), 2.93 - 2.76 (m, 1H), 2.75 - 2.56 (m, 3H), 2.44 - 2.30 (m, 2H), 2.25 - 2.10 (m, 4H), 2.09 - 1.95 (m, 2H), 1.95 - 1.66 (m, 6H), 1.65 - 1.50 (m, 1H), 1.12 - 0.94 (m, 2H).

LCMS m/z = 886.4 $[M+1]^+$.

**Example 21: Preparation of compound 21** (Trans)

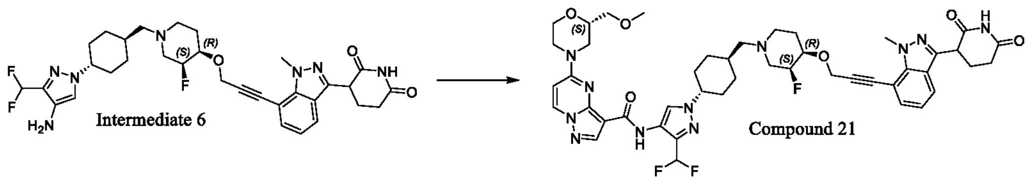


The hydrochloride salt of the above-mentioned crude intermediate 6 (130 mg) was dissolved in 5 mL of acetonitrile, and the above-mentioned crude intermediate 4 (56 mg) and TCFH (67 mg, 0.24 mmol) were added, and NMI (0.11 g, 1.34 mmol) was added, and the mixture was reacted at room temperature for 16 h. The reaction system was concentrated under reduced pressure, 10 mL of water was added to the residue, and the mixture was filtered. The filter cake was washed with 5 mL of water, dissolved in 10 mL of DCM, dried over anhydrous sodium sulfate, and concentrated under reduced pressure. The crude was separated and purified by silica gel column chromatography (dichloromethane/methanol (v/v) = 12 : 1). The obtained crude was subjected to chiral preparative chromatography to obtain chiral isomer 1 (18.3 mg, two-step yield from compound 4-C: 5%) and chiral isomer 2 (20.3 mg, two-step yield from compound 4-C: 6%) of compound 21, respectively.

Chiral preparation method:

instrument and preparative column: Shimadzu LC-A HPLC preparative liquid chromatography, preparative column model Chiralpak IE, 250 × 30 mm, 10 μm; Mobile phase system: acetonitrile/methanol, isocratic elution: acetonitrile /methanol = 1:1; flow rate: 80 mL/min.

Analytical methods for target compounds:

instrument: Shimadzu LC-20AB with PDA detector; chromatographic column: Chiralpak IE-3; Specification: 150 × 4.6 mm I.D., 3 μm ; mobile phase A: acetonitrile; mobile phase B: methanol; column temperature: 35°C; flow rate: 1 mL/min; wavelength: 220 nm; Elution program: Isocratic elution: mobile phase A: B = 3 : 7;

retention time of chiral isomer 1: 6.563 min.

$^1$H NMR (400 MHz, DMSO-$d_6$) δ 10.90 (s, 1H), 9.33 (s, 1H), 8.81 (d, 1H), 8.37 (s, 1H), 8.29 (s, 1H), 7.84 - 7.73 (m, 1H), 7.56 - 7.48 (m, 1H), 7.28 - 6.96 (m, 2H), 6.91 (d, 1H), 4.94 - 4.71 (m, 1H), 4.61 (s, 2H), 4.46 - 4.24 (m, 6H), 4.24 - 4.10 (m, 1H), 4.00 - 3.91 (m, 1H), 3.87 - 3.53 (m, 3H), 3.50 - 3.42 (m, 2H), 3.30 (s, 3H), 3.21 - 3.07 (m, 1H), 3.06 - 2.93 (m, 1H), 2.93 - 2.76 (m, 1H), 2.76 - 2.55 (m, 3H), 2.45 - 2.25 (m, 2H), 2.25 - 2.10 (m, 4H), 2.10 - 1.95 (m, 2H), 1.95 - 1.65 (m, 6H), 1.65 - 1.49 (m, 1H), 1.12 - 0.95 (m, 2H).

LCMS m/z = 900.4 [M+1]+.

retention time of chiral isomer 2: 8.07 min.

$^1$H NMR (400 MHz, DMSO-$d_6$) δ 10.90 (s, 1H), 9.33 (s, 1H), 8.81 (d, 1H), 8.37 (s, 1H), 8.29 (s, 1H), 7.84 - 7.73 (m, 1H), 7.56 - 7.48 (m, 1H), 7.28 - 6.96 (m, 2H), 6.91 (d, 1H), 4.94 - 4.71 (m, 1H), 4.61 (s, 2H), 4.46 - 4.24 (m, 6H), 4.24 - 4.10 (m, 1H), 4.00 - 3.91 (m, 1H), 3.87 - 3.53 (m, 3H), 3.50 - 3.42 (m, 2H), 3.30 (s, 3H), 3.21 - 3.07 (m, 1H), 3.06 - 2.93 (m, 1H), 2.93 - 2.76 (m, 1H), 2.76 - 2.55 (m, 3H), 2.45 - 2.25 (m, 2H), 2.25 - 2.10 (m, 4H), 2.10 - 1.95 (m, 2H), 1.95 - 1.65 (m, 6H), 1.65 - 1.49 (m, 1H), 1.12 - 0.95 (m, 2H).

LCMS m/z = 900.4 [M+1]+.

**Example 22: Preparation of compound 22** (Trans)

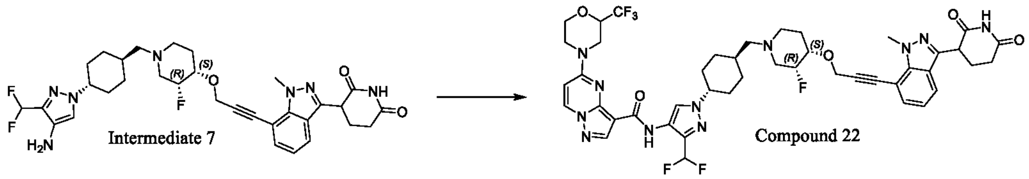


The above-mentioned crude intermediate 7 (0.12 g) was added to 5 mL of acetonitrile, and the above-mentioned crude intermediate 2 (0.08 g) and TCFH (0.084 g, 0.3 mmol) were added, and NMI (0.08 g, 0.97 mmol) was added at 0°C, and the mixture was reacted at room temperature for 16 h. The reaction system was concentrated under reduced pressure, and the residue was subjected to chiral preparative chromatography to obtain chiral isomer 1 (21.6 mg, yield: 8%) and chiral isomer 2 (22.7 mg, yield: 8%) of compound 22, respectively.

Chiral preparation method:

instrument and preparative column: Shimadzu LC-A HPLC preparative liquid chromatography, preparative column model Chiralpak IE, 250 × 30 mm, 10 μm; Mobile phase system: acetonitrile/methanol, isocratic elution: acetonitrile /methanol = 1:1; flow rate: 80 mL/min.

Analytical methods for target compounds:

instrument: Shimadzu LC-20AB with PDA detector; chromatographic column: Chiralpak IE-3;
Specification: 150 × 4.6 mm I.D., 3 μm ; mobile phase A: acetonitrile; mobile phase B: methanol;
column temperature: 35°C; flow rate: 1 mL/min; wavelength: 220 nm;
Elution program: Isocratic elution: mobile phase A: B = 3 : 7;
retention time of chiral isomer 1: 3.449 min.
$^1$H NMR (400 MHz, DMSO-$d_6$) δ 10.90 (s, 1H), 9.28 (s, 1H), 8.88 (d, 1H), 8.38 - 8.29 (m, 2H), 7.78 (d, 1H), 7.52 (d, 1H), 7.26 - 6.90 (m, 3H), 4.94 - 4.72 (m, 1H), 4.61 (s, 2H), 4.58 - 4.48 (m, 1H), 4.48 - 4.34 (m, 3H), 4.29 (s, 3H), 4.24 - 4.04 (m, 2H), 3.85 - 3.71 (m, 2H), 3.30 - 3.22 (m, 2H), 2.95 - 2.76 (m, 1H), 2.76 - 2.55 (m, 3H), 2.46 - 2.26 (m, 2H), 2.24 - 2.10 (m, 4H), 2.10 - 1.95 (m, 2H), 1.95 - 1.65 (m, 6H), 1.65 - 1.49 (m, 1H), 1.13 - 0.94 (m, 2H).
LCMS m/z = 924.4 [M+1]+.
retention time of chiral isomer 2: 4.165 min.
$^1$H NMR (400 MHz, DMSO-$d_6$) δ 10.90 (s, 1H), 9.28 (s, 1H), 8.88 (d, 1H), 8.38 - 8.29 (m, 2H), 7.79 (d, 1H), 7.52 (d, 1H), 7.26 - 6.90 (m, 3H), 4.94 - 4.72 (m, 1H), 4.61 (s, 2H), 4.58 - 4.48 (m, 1H), 4.48 - 4.34 (m, 3H), 4.29 (s, 3H), 4.24 - 4.04 (m, 2H), 3.85 - 3.71 (m, 2H), 3.30 - 3.22 (m, 2H), 2.95 - 2.76 (m, 1H), 2.76 - 2.55 (m, 3H), 2.46 - 2.26 (m, 2H), 2.24 - 2.10 (m, 4H), 2.10 - 1.95 (m, 2H), 1.95 - 1.65 (m, 6H), 1.65 - 1.49 (m, 1H), 1.13 - 0.94 (m, 2H).
LCMS m/z = 924.4 [M+1]+.

**Example 23: Preparation of compound 23** (Trans)

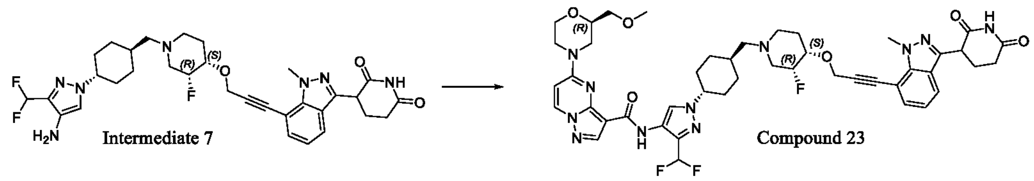

Intermediate 7

Compound 23

The above-mentioned crude intermediate 7 (0.12 g) was added to 5 mL of acetonitrile, and the above-mentioned crude intermediate 1 (0.073 g) and TCFH (0.084 g, 0.3 mmol) were added, and NMI (0.08 g, 0.97 mmol) was added at 0°C, and the mixture was reacted at room temperature for 16 h. The reaction system was concentrated under reduced pressure, and the residue was subjected to chiral preparative chromatography to obtain chiral isomer 1 (20.5 mg, two-step yield from compound 1-D: 8%) and chiral isomer 2 (17.8 mg, two-step yield from compound 1-D: 7%) of compound 23, respectively.

Chiral preparation method:

instrument and preparative column: Shimadzu LC-A HPLC preparative liquid chromatography, preparative column model Chiralpak IE, 250 × 30 mm, 10 μm; Mobile phase system: acetonitrile/methanol, isocratic elution: acetonitrile /methanol = 1:1; flow rate: 80 mL/min.

Analytical methods for target compounds:

instrument: Shimadzu LC-20AB with PDA detector; chromatographic column: Chiralpak IE-3;
Specification: 150 × 4.6 mm I.D., 3 μm; mobile phase A: acetonitrile; mobile phase B: methanol;
column temperature: 35°C; flow rate: 1 mL/min; wavelength: 220 nm; Elution program: Isocratic elution: mobile phase A: B = 3 : 7;
retention time of chiral isomer 1: 6.645 min.
$^1$H NMR (400 MHz, DMSO-$d_6$) δ 10.90 (s, 1H), 9.33 (s, 1H), 8.81 (d, 1H), 8.37 (s, 1H), 8.29 (s, 1H), 7.84 - 7.73 (m, 1H), 7.56 - 7.48 (m, 1H), 7.28 - 6.96 (m, 2H), 6.92 (d, 1H), 4.94 - 4.71 (m, 1H), 4.61 (s, 2H), 4.46 - 4.24 (m, 6H), 4.24 - 4.10 (m, 1H), 4.00 - 3.91 (m, 1H), 3.87 - 3.53 (m, 3H), 3.50 - 3.42 (m, 2H), 3.29 (s, 3H), 3.21 - 3.07 (m, 1H), 3.06 - 2.93 (m, 1H), 2.93 - 2.76 (m, 1H), 2.76 - 2.55 (m, 3H), 2.45 - 2.25 (m, 2H), 2.25 - 2.10 (m, 4H), 2.10 - 1.95 (m, 2H), 1.95 - 1.65 (m, 6H), 1.65 - 1.49 (m, 1H), 1.12 - 0.95 (m, 2H).
LCMS m/z = 900.5 $[M+1]^+$.
retention time of chiral isomer 2: 8.228 min.
$^1$H NMR (400 MHz, DMSO-$d_6$) δ 10.90 (s, 1H), 9.32 (s, 1H), 8.80 (d, 1H), 8.36 (s, 1H), 8.28 (s, 1H), 7.84 - 7.73 (m, 1H), 7.56 - 7.48 (m, 1H), 7.28 - 6.96 (m, 2H), 6.91 (d, 1H), 4.94 - 4.71 (m, 1H), 4.60 (s, 2H), 4.46 - 4.24 (m, 6H), 4.24 - 4.10 (m, 1H), 4.00 - 3.91 (m, 1H), 3.87 - 3.53 (m, 3H), 3.50 - 3.42 (m, 2H), 3.29 (s, 3H), 3.21 - 3.07 (m, 1H), 3.06 - 2.93 (m, 1H), 2.93 - 2.76 (m, 1H), 2.76 - 2.55 (m, 3H), 2.45 - 2.25 (m, 2H), 2.25 - 2.10 (m, 4H), 2.10 - 1.95 (m, 2H), 1.95 - 1.65 (m, 6H), 1.65 - 1.49 (m, 1H), 1.12 - 0.95 (m, 2H).
LCMS m/z = 900.4 $[M+1]^+$.

**Example 24: Preparation of compound 24** (Trans)

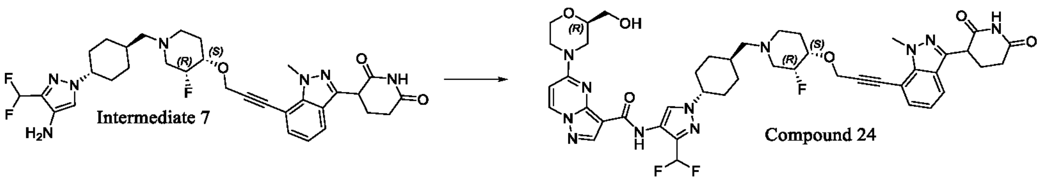

Intermediate 7

Compound 24

The above-mentioned crude intermediate 7 (0.12 g) was added to 5 mL of acetonitrile, and the above-mentioned crude intermediate 3 (0.07 g) and TCFH (0.084 g, 0.3 mmol) were added, and NMI (0.08 g, 0.97 mmol) was added at 0°C, and the mixture was reacted at room temperature for 16 h. The reaction system was concentrated under reduced pressure, and the residue was subjected to chiral preparative chromatography to obtain chiral isomer 1 (30.5 mg, two-step yield from compound 3-A: 8%) and chiral isomer 2 (28 mg, two-step yield from compound 3-A: 8%) of compound 24, respectively.

Chiral preparation method:

instrument and preparative column: Shimadzu LC-A HPLC preparative liquid chromatography, preparative column model Chiralpak IE, 250 × 30 mm, 10 μm; Mobile phase system: acetonitrile/methanol, isocratic elution: acetonitrile /methanol = 1 : 1; flow rate: 80 mL/min.

Analytical methods for target compounds:

instrument: Shimadzu LC-20AB with PDA detector; chromatographic column: Chiralpak IE-3;
Specification: 150 × 4.6 mm I.D., 3 μm; mobile phase A: acetonitrile; mobile phase B: methanol;
column temperature: 35°C; flow rate: 1 mL/min; wavelength: 220 nm;
Elution program: Isocratic elution: mobile phase A: B = 3 : 7;
retention time of chiral isomer 1: 5.434 min.
$^1$H NMR (400 MHz, DMSO-$d_6$) δ 10.89 (s, 1H), 9.34 (s, 1H), 8.81 (d, 1H), 8.36 (s, 1H), 8.29 (s, 1H), 7.79 (d, 1H), 7.52 (d, 1H), 7.27 - 6.94 (m, 2H), 6.89 (d, 1H), 4.93 - 4.72 (m, 2H), 4.61 (s, 2H), 4.52 - 4.10 (m, 7H), 4.03 - 3.92 (m, 1H), 3.87 - 3.70 (m, 1H), 3.67 - 3.46 (m, 4H), 3.20 - 3.06 (m, 1H), 3.05 - 2.93 (m, 1H), 2.93 - 2.76 (m, 1H), 2.75 - 2.56 (m, 3H), 2.44 - 2.30 (m, 2H), 2.25 - 2.10 (m, 4H), 2.09 - 1.95 (m, 2H), 1.95 - 1.66 (m, 6H), 1.65 - 1.50 (m, 1H), 1.12 - 0.95 (m, 2H). LCMS m/z = 886.4 [M+1]$^+$.
retention time of chiral isomer 2: 6.706 min.
$^1$H NMR (400 MHz, DMSO-$d_6$) δ 10.89 (s, 1H), 9.34 (s, 1H), 8.81 (d, 1H), 8.36 (s, 1H), 8.29 (s, 1H), 7.79 (d, 1H), 7.52 (d, 1H), 7.27 - 6.94 (m, 2H), 6.90 (d, 1H), 4.93 - 4.72 (m, 2H), 4.61 (s, 2H), 4.52 - 4.10 (m, 7H), 4.03 - 3.92 (m, 1H), 3.87 - 3.70 (m, 1H), 3.67 - 3.46 (m, 4H), 3.20 - 3.06 (m, 1H), 3.05 - 2.93 (m, 1H), 2.93 - 2.76 (m, 1H), 2.75 - 2.56 (m, 3H), 2.44 - 2.30 (m, 2H), 2.25 - 2.10 (m, 4H), 2.09 - 1.95 (m, 2H), 1.95 - 1.66 (m, 6H), 1.65 - 1.50 (m, 1H), 1.12 - 0.95 (m, 2H). LCMS m/z = 886.4 [M+1]$^+$.

**Example 25: Preparation of compound 25** (Trans)

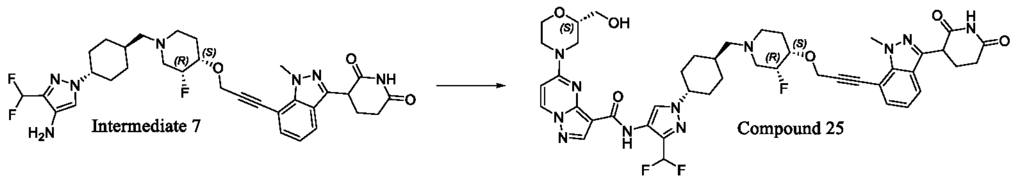


The above-mentioned crude intermediate 7 (0.12 g) was added to 5 mL of acetonitrile, and the above-mentioned crude intermediate 5 (0.07 g) and TCFH (0.084 g, 0.3 mmol) were added, and NMI (0.08 g, 0.97 mmol) was added at 0°C, and the mixture was reacted at room temperature for 16 h. The reaction system was concentrated under reduced pressure, and the residue was subjected to chiral preparative chromatography to obtain chiral isomer 1 (25 mg, two-step yield from compound 5-A: 19%) and chiral isomer 2 (26.4 mg, two-step yield from compound 5-A: 20%) of compound 25, respectively.

Chiral preparation method:

instrument and preparative column: Shimadzu LC-A HPLC preparative liquid chromatography, preparative column model Chiralpak IE, 250 × 30 mm, 10 μm; Mobile phase system: acetonitrile/methanol, isocratic elution: acetonitrile /methanol = 1:1; flow rate: 80 mL/min.

Analytical methods for target compounds:

instrument: Shimadzu LC-20AB with PDA detector; chromatographic column: Chiralpak IE-3;
Specification: 150 × 4.6 mm I.D., 3 μm; mobile phase A: acetonitrile; mobile phase B: methanol;
column temperature: 35°C; flow rate: 1 mL/min; wavelength: 220 nm;
Elution program: Isocratic elution: mobile phase A: B = 3 : 7;
retention time of chiral isomer 1: 5.364 min.
$^1$H NMR (400 MHz, DMSO-$d_6$) δ 10.89 (s, 1H), 9.34 (s, 1H), 8.81 (d, 1H), 8.36 (s, 1H), 8.28 (s, 1H), 7.79 (d, 1H), 7.52 (d, 1H), 7.27 - 6.94 (m, 2H), 6.90 (d, 1H), 4.93 - 4.72 (m, 2H), 4.61 (s, 2H), 4.52 - 4.10 (m, 7H), 4.03 - 3.92 (m, 1H), 3.87 - 3.70 (m, 1H), 3.67 - 3.46 (m, 4H), 3.20 - 3.06 (m, 1H), 3.05 - 2.93 (m, 1H), 2.93 - 2.76 (m, 1H), 2.75 - 2.56 (m, 3H), 2.44 - 2.30 (m, 2H), 2.25 - 2.10 (m, 4H), 2.09 - 1.95 (m, 2H), 1.95 - 1.66 (m, 6H), 1.65 - 1.50 (m, 1H), 1.12 - 0.94 (m, 2H).

LCMS m/z = 886.4 [M+1]+.
retention time of chiral isomer 2: 6.619 min.
$^1$H NMR (400 MHz, DMSO-$d_6$) δ 10.89 (s, 1H), 9.34 (s, 1H), 8.81 (d, 1H), 8.36 (s, 1H), 8.29 (s, 1H), 7.79 (d, 1H), 7.52 (d, 1H), 7.27 - 6.94 (m, 2H), 6.90 (d, 1H), 4.93 - 4.72 (m, 2H), 4.60 (s, 2H), 4.52 - 4.10 (m, 7H), 4.03 - 3.92 (m, 1H), 3.87 - 3.70 (m, 1H), 3.67 - 3.46 (m, 4H), 3.20 - 3.06 (m, 1H), 3.05 - 2.93 (m, 1H), 2.93 - 2.76 (m, 1H), 2.75 - 2.56 (m, 3H), 2.44 - 2.30 (m, 2H), 2.25 - 2.10 (m, 4H), 2.09 - 1.95 (m, 2H), 1.95 - 1.66 (m, 6H), 1.65 - 1.50 (m, 1H), 1.12 - 0.94 (m, 2H).
LCMS m/z = 886.4 [M+1]+.

**Example 26: Synthesis of compound 26** (Trans)

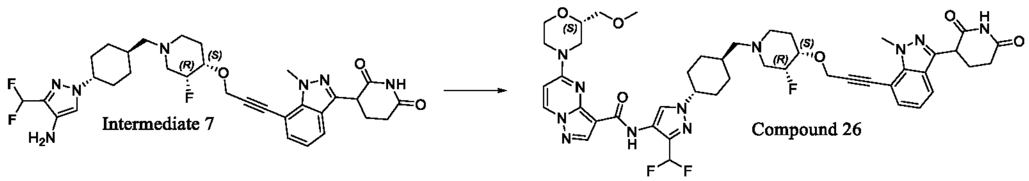


The above-mentioned crude intermediate 7 (0.12 g) was added to 5 mL of acetonitrile, and the above-mentioned crude intermediate 4 (0.073 g) and TCFH (0.084 g, 0.3 mmol) were added, and NMI (0.08 g, 0.97 mmol) was added at 0°C, and the mixture was reacted at room temperature for 16 h. The reaction system was concentrated under reduced pressure, and the residue was subjected to chiral preparative chromatography to obtain chiral isomer 1 (25.2 mg, two-step yield from compound 4-C: 12%) and chiral isomer 2 (18.9 mg, two-step yield from compound 4-C: 9%) of compound 26, respectively.

Chiral preparation method:

instrument and preparative column: Shimadzu LC-A HPLC preparative liquid chromatography, preparative column model Chiralpak IE, 250 × 30 mm, 10 μm; Mobile phase system: acetonitrile/methanol, isocratic elution: acetonitrile /methanol = 1 : 1; flow rate: 80 mL/min.
Analytical methods for target compounds:

instrument: Shimadzu LC-20AB with PDA detector; chromatographic column: Chiralpak IE-3;
Specification: 150 × 4.6 mm I.D., 3 μm; mobile phase A: acetonitrile; mobile phase B: methanol;
column temperature: 35°C; flow rate: 1 mL/min; wavelength: 220 nm; Elution program: isocratic elution: mobile phase A: B = 3 : 7;
retention time of chiral isomer 1: 6.733 min.
$^1$H NMR (400 MHz, DMSO-$d_6$) δ 10.90 (s, 1H), 9.33 (s, 1H), 8.81 (d, 1H), 8.37 (s, 1H), 8.29 (s, 1H), 7.84 - 7.73 (m, 1H), 7.56 - 7.48 (m, 1H), 7.28 - 6.96 (m, 2H), 6.92 (d, 1H), 4.94 - 4.71 (m, 1H), 4.61 (s, 2H), 4.46 - 4.24 (m, 6H), 4.24 - 4.10 (m, 1H), 4.00 - 3.91 (m, 1H), 3.87 - 3.53 (m, 3H), 3.50 - 3.42 (m, 2H), 3.29 (s, 3H), 3.21 - 3.07 (m, 1H), 3.06 - 2.93 (m, 1H), 2.93 - 2.76 (m, 1H), 2.76 - 2.55 (m, 3H), 2.45 - 2.25 (m, 2H), 2.25 - 2.10 (m, 4H), 2.10 - 1.95 (m, 2H), 1.95 - 1.65 (m, 6H), 1.65 - 1.49 (m, 1H), 1.12 - 0.95 (m, 2H).
LCMS m/z = 900.4 [M+1]+.
retention time of chiral isomer 2: 8.385 min.
$^1$H NMR (400 MHz, DMSO-$d_6$) δ 10.89 (s, 1H), 9.32 (s, 1H), 8.81 (d, 1H), 8.37 (s, 1H), 8.29 (s, 1H), 7.84 - 7.73 (m, 1H), 7.56 - 7.48 (m, 1H), 7.28 - 6.96 (m, 2H), 6.91 (d, 1H), 4.94 - 4.71 (m, 1H), 4.60 (s, 2H), 4.46 - 4.24 (m, 6H), 4.24 - 4.10 (m, 1H), 4.00 - 3.91 (m, 1H), 3.87 - 3.53 (m, 3H), 3.50 - 3.42 (m, 2H), 3.30 (s, 3H), 3.21 - 3.07 (m, 1H), 3.06 - 2.93 (m, 1H), 2.93 - 2.76 (m, 1H), 2.76 - 2.55 (m, 3H), 2.45 - 2.25 (m, 2H), 2.25 - 2.10 (m, 4H), 2.10 - 1.95 (m, 2H), 1.95 - 1.65 (m, 6H), 1.65 - 1.49 (m, 1H), 1.12 - 0.95 (m, 2H).
LCMS m/z = 900.5 [M+1]+.

**Example 27: Preparation of compound 27** (Trans)

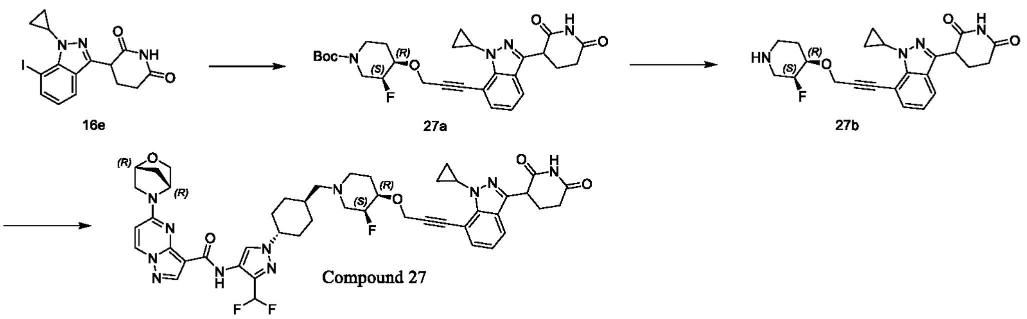

16e 27a 27b

Compound 27

Step 1: Preparation of 27a

16e (600 mg, 1.52 mmol), 10b (470 mg, 1.83 mmol), CuI (58 mg, 0.30 mmol), $PdCl_2(PPh_3)_2$ (110 mg, 0.157 mmol) and TEA (0.77 g, 7.61 mmol) were dissolved in 20 mL of DMF, and the mixture was subjected to nitrogen replacement three times, and reacted at 60°C for 5 h. The reaction system was cooled to room temperature, 80 mL of water was added, and the mixture was extracted with 100 mL of ethyl acetate. The organic phase was washed with 50 mL of water, dried over anhydrous sodium sulfate, and concentrated under reduced pressure. The crude was separated and purified by silica gel column chromatography (dichloromethane/ethyl acetate (v/v) = 1:1) to obtain 27a (550 mg, yield: 69%).

Step 2: Preparation of the trifluoroacetate salt of 27b

27a (500 mg, 0.95 mmol) was dissolved in 10 mL of dichloromethane, and 4 mL of trifluoroacetic acid was added and the mixture was reacted at room temperature for 2 h. The reaction system was concentrated under reduced pressure to obtain the trifluoroacetate salt of crude 27b (0.65 g).
LCMS m/z = 425.2 $[M+1]^+$.

Step 3: Synthesis of compound 27

The above-mentioned trifluoroacetate salt of the crude 27b (650 mg) was dissolved in 20 mL of DMA, and sodium bicarbonate (160 mg, 1.90 mmol) was added. After stirring at room temperature for 15 min, 6h (560 mg, 1.16 mmol), 0.15 mL of acetic acid and a 4 Å molecular sieve (2 g) were added. After stirring at room temperature for 2 h, sodium triacetoxyborohydride (410 mg, 1.93 mmol) was added and the mixture was reacted at room temperature for 16 h. 40 mL of aqueous saturated sodium bicarbonate solution and 20 mL of dichloromethane were added to the reaction system, liquid separation was carried out, and the organic phase was concentrated under reduced pressure. The crude was separated and purified by silica gel column chromatography (dichloromethane/methanol (v/v) = 100 : 1-20 : 1). The obtained crude was subjected to chiral preparative chromatography to obtain chiral isomer 1 (270 mg, yield: 26%) and chiral isomer 2 (200 mg, yield: 19%) of compound 27, respectively.

Chiral preparation method:

instrument and preparative column: Shimadzu LC-A HPLC preparative liquid chromatography, preparative column model Chiralpak IE, 250 × 30 mm, 10 μm; Mobile phase system: acetonitrile/methanol, isocratic elution: acetonitrile /methanol = 1 : 1; flow rate: 80 mL/min.
Analytical methods for target compounds:

instrument: Shimadzu LC-20AB with PDA detector; chromatographic column: Chiralpak IE-3;
Specification: 150 × 4.6 mm I.D., 3 μm; mobile phase A: acetonitrile; mobile phase B: methanol;
column temperature: 35°C; flow rate: 1 mL/min; wavelength: 220 nm;
Elution program: Isocratic elution: mobile phase A: B = 3 : 7;
retention time of chiral isomer 1: 5.139 min.
$^1H$ NMR (400 MHz, DMSO-$d_6$) δ 10.88 (s, 1H), 9.49 (d, 1H), 8.77 (d, 1H), 8.38 (d, 1H), 8.25 (d, 1H), 7.76 (d, 1H), 7.53 (d, 1H), 7.28 - 6.93 (m, 2H), 6.90 - 6.40 (m, 1H), 5.35 - 5.02 (m, 1H), 4.95 - 4.70 (m, 2H), 4.59 (s, 2H), 4.43 - 4.32 (m, 1H), 4.24 - 4.02 (m, 2H), 3.88 - 3.40 (m, 5H), 2.95 - 2.54 (m, 4H), 2.45 - 1.45 (m, 17H), 1.27 - 0.94 (m, 6H).
LCMS m/z = 894.5 $[M+1]^+$.
retention time of chiral isomer 2: 5.982 min.
$^1H$ NMR (400 MHz, DMSO-$d_6$) δ 10.88 (s, 1H), 9.49 (d, 1H), 8.77 (d, 1H), 8.38 (d, 1H), 8.25 (d, 1H), 7.76 (d, 1H), 7.53 (d, 1H), 7.28 - 6.93 (m, 2H), 6.90 - 6.40 (m, 1H), 5.35 - 5.02 (m, 1H), 4.95 - 4.70 (m, 2H), 4.59 (s, 2H), 4.43 - 4.32 (m, 1H),

4.24 - 4.02 (m, 2H), 3.88 - 3.40 (m, 5H), 2.95 - 2.54 (m, 4H), 2.45 - 1.45 (m, 17H), 1.27 - 0.94 (m, 6H).
LCMS m/z = 894.5 [M+1]$^+$.

**Example 28: Preparation of compound 28** (Trans)

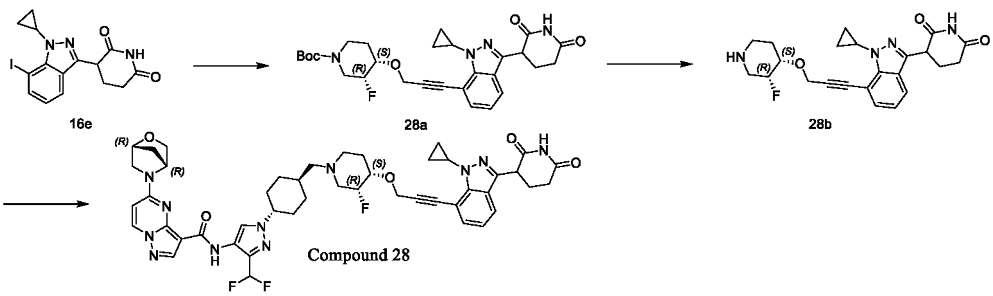

16e 28a 28b Compound 28

Using tert-butyl (3R,4S)-3-fluoro-4-(prop-2-yn-1-yloxy)piperidine-1-carboxylate+16e as the starting material and referring to the synthesis method of example 27, compound 28 was obtained.

Refining method: the crude was separated and purified by silica gel column chromatography (dichloromethane/methanol (v/v) = 100 : 1-20 : 1), and the obtained crude was subjected to chiral preparative chromatography to obtain chiral isomer 1 (290 mg, yield: 24%) and chiral isomer 2 (250 mg, yield: 21%) of compound 28, respectively.

Chiral preparation method:

instrument and preparative column: Shimadzu LC-A HPLC preparative liquid chromatography, preparative column model Chiralpak IE, 250 × 30 mm, 10 μm; Mobile phase system: acetonitrile/methanol, isocratic elution: acetonitrile /methanol = 1 : 1; flow rate: 80 mL/min.

Analytical methods for target compounds:

instrument: Shimadzu LC-20AB with PDA detector; chromatographic column: Chiralpak IE-3;
Specification: 150 × 4.6 mm I.D., 3 μm; mobile phase A: acetonitrile; mobile phase B: methanol;
column temperature: 35°C; flow rate: 1 mL/min; wavelength: 220 nm;
Elution program: Isocratic elution: mobile phase A: B = 3 : 7;
retention time of chiral isomer 1: 5.277 min.
$^1$H NMR (400 MHz, DMSO-$d_6$) δ 10.88 (s, 1H), 9.49 (d, 1H), 8.77 (d, 1H), 8.38 (d, 1H), 8.25 (d, 1H), 7.76 (d, 1H), 7.53 (d, 1H), 7.28 - 6.93 (m, 2H), 6.90 - 6.40 (m, 1H), 5.35 - 5.02 (m, 1H), 4.95 - 4.70 (m, 2H), 4.59 (s, 2H), 4.43 - 4.32 (m, 1H), 4.24 - 4.02 (m, 2H), 3.88 - 3.40 (m, 5H), 2.95 - 2.54 (m, 4H), 2.45 - 1.45 (m, 17H), 1.27 - 0.94 (m, 6H).
LCMS m/z = 447.9 [M/2 +1]$^+$.
retention time of chiral isomer 2: 6.017 min.
$^1$H NMR (400 MHz, DMSO-$d_6$) δ 10.89 (s, 1H), 9.50 (d, 1H), 8.78 (d, 1H), 8.38 (d, 1H), 8.26 (d, 1H), 7.76 (d, 1H), 7.53 (d, 1H), 7.28 - 6.93 (m, 2H), 6.90 - 6.40 (m, 1H), 5.35 - 5.02 (m, 1H), 4.95 - 4.70 (m, 2H), 4.59 (s, 2H), 4.43 - 4.32 (m, 1H), 4.24 - 4.02 (m, 2H), 3.88 - 3.40 (m, 5H), 2.95 - 2.54 (m, 4H), 2.45 - 1.45 (m, 17H), 1.27 - 0.94 (m, 6H).
LCMS m/z = 894.5 [M+1]$^+$.

**Biological test examples**

**Test example 1: Study on IRAK4 degradation activity in hPBMC cells (24 hours)**

hPBMC cells are human peripheral blood mononuclear cells. Peripheral venous blood was taken from healthy volunteers, and hPBMCs were isolated using Ficoll density gradient centrifugation (Ficoll-PaqueTM PLUS 1.077, GE, Cat. 17-1140-02). Culture conditions: RPMI-1640 + 10% FBS + 1% penicillin-streptomycin solution. The cells were cultured in an incubator at 37°C and 5% $CO_2$. The cells were plated in a 24-well plate at 1 × $10^6$ cells/well. After plating, compounds at different concentrations were added, and the cells were cultured in an incubator at 37°C and 5% $CO_2$ for 24 hours. After the culture was completed, the cells were collected, and RIPA lysis buffer (beyotime, Cat. P0013B) was added. The cells were lysed on ice for 20 minutes, and then centrifuged at 12000 rpm at 4°C for 10 minutes. The protein sample of the supernatant was collected, the protein was quantified using a BCA kit (Beyotime, Cat. P0009), and then the protein was diluted to 1 mg/mL. The expression of IRAK4 (CST, Cat. 4363S) and the internal reference cofilin (CST, Cat. 5175S) were detected

using a fully automated western blot quantitative analyzer (Proteinsimple). The expression level of IRAK4 relative to the internal reference was calculated using compass software. The remaining IRAK4 protein IRAK4% relative to the vehicle control group at different doses was calculated using formula (1), and the IRAK4 protein degradation relative to the vehicle control group at different doses was calculated using formula (2), where $IRAK4_{administration}$ was the expression level of IRAK4 in the administration groups at different doses, and $IRAK4_{vehicle}$ was the expression level of IRAK4 in the vehicle control group. An IRAK4 degradation-drug concentration curve was fit using Graphpad Prism 8 software, and the $DC_{50}$ was calculated.

$$IRAK4\% = IRAK4_{administration} / IRAK4_{vehicle} \times 100\% \qquad \text{formula 1}$$

$$IRAK4\ degradation\% = 100\% - IRAK4\% \qquad \text{formula 2}$$

Table 1 Degradation activity of test compounds against IRAK4 protein in hPBMC cells at 100 nM

| Compound No. | Remaining IRAK4 protein IRAK4% |
|---|---|
| Compound 1 | B |
| Compound 2 | A |
| Compound 6 | A |
| Compound 7 | A |
| Chiral isomer 1 of compound 8 | B |
| Compound 10 | A |
| Compound 11 | A |
| Compound 12 | B |
| Compound 13 | B |
| Trifluoroacetate salt of compound 15 | B |
| Compound 16 | A |
| Notes: in Table 1, A ≤ 10%, 10% < B ≤ 50%, and 50% < C | |

Table 2 $DC_{50}$ of test compounds for IRAK4 protein degradation in hPBMC cells

| Compound No. | $DC_{50}$ (nM) |
|---|---|
| Chiral isomer 1 of compound 19 | <20 |
| Chiral isomer 2 of compound 19 | <20 |
| Chiral isomer 1 of compound 20 | <20 |
| Chiral isomer 2 of compound 20 | <20 |
| Chiral isomer 2 of compound 23 | <20 |
| Chiral isomer 1 of compound 24 | <20 |
| Chiral isomer 1 of compound 25 | <20 |
| Chiral isomer 2 of compound 25 | <20 |
| Conclusion: The compounds of the present invention have certain degradation effect on IRAK4 protein in hPBMC cells at 24 h. | |

**Test example 2: Pharmacokinetic test in mice**

**Experimental animals:** Male ICR mice, about 25 g, 6 mice/compound, purchased from Chengdu Ddossy Experimental Animals Co., Ltd.

**Test design:** On the day of the experiment, 6 ICR mice were randomly grouped according to their body weights.

The animals were fasted with water available for 12 to 14 h one day before the administration, and were fed 4 h after the administration.

Table 2.1 Administration information

| Group | Number | Administration information | | | | | |
|---|---|---|---|---|---|---|---|
| | Male | Test compound | Administration dosage (mg/kg) | Administration concentration (mg/mL) | Administration volume (mL/kg) | Collected samples | Mode of administration |
| G1 | 3 | The compound of the present invention or control compound | 2.5 | 0.5 | 5 | Plasma | Intravenous administration |
| G2 | 3 | The compound of the present invention or control compound | 10 | 1 | 10 | Plasma | Intragastric administration |
| Vehicle for intravenous administration: 5% DMA + 5% Solutol + 90% Saline;<br>Vehicle for intragastric administration: 5% DMSO + 30% PEG400 + 65% (20% SBE-CD);<br>(DMSO: dimethyl sulfoxide; DMA: dimethylacetamide; Solutol: polyethylene glycol-15-hydroxystearate; PEG400: polyethylene glycol 400; SBE-β-CD: sulfobutyl-β-cyclodextrin; Saline: physiological saline; ) | | | | | | | |

Before and after the administration, 0.15 mL of blood was taken from the orbit of the animals under isoflurane anesthesia and placed in an EDTAK2 centrifuge tube. The blood was centrifuged at 5000 rpm and 4°C for 10 min to collect plasma. The blood collection time points for the intravenous administration group and intragastric administration group were 0 min, 5 min, 15 min, 30 min, 1 h, 2 h, 4 h, 7 h and 24 h. Before analysis and detection, all samples were stored at -60°C. The samples were analyzed quantitatively by LC-MS/MS.

Table 2.2 Pharmacokinetic parameters of compounds of the present invention in plasma of mice

| Test compound | Mode of administration* | $AUC_{0-t}$ (ng/mL·h) |
|---|---|---|
| Compound 10 | i.g. (10 mg/kg) | 17480±5264 |
| Compound 11 | i.g. (10 mg/kg) | 8748±4649 |
| Chiral isomer 1 of compound 19 | i.g. (10 mg/kg) | 13407±4034 |
| Trifluoroacetate salt of chiral isomer 2 of compound 19 | i.g. (10 mg/kg) | 8304±2852 |
| Chiral isomer 2 of compound 20 | i.g. (10 mg/kg) | 3938±1106 |
| Control compound 1 | i.g. (10 mg/kg) | 2322±146 |
| *Notes: i.g. (intragastrical) administration of compound;<br>Conclusion: The compounds of the present invention have good oral absorption in mice. | | |

**Test example 3. Test of effect on hERG potassium ion channel**

**Experimental platform:** electrophysiological manual patch-clamp system
Cell line: Chinese hamster ovary (CHO) cell line stably expressing hERG potassium ion channel
**Experimental method:** In CHO (Chinese Hamster Ovary) cells stably expressing hERG potassium channel, whole cell patch-clamp technique was used to record hERG potassium channel current at room temperature. The glass microelectrode was made of a glass electrode blank (BF150-86-10, Sutter) by a puller. The tip resistance after filling

the liquid in the electrode was about 2-5 MΩ. The glass microelectrode can be connected to the patch-clamp amplifier by inserting the glass microelectrode into an amplifier probe. The clamping voltage and data recording were controlled and recorded by the pClamp 10 software through a computer. The sampling frequency was 10 kHz, and the filtering frequency was 2 kHz. After the whole cell records were obtained, the cells were clamped at -80 mV, and the step voltage that induced the hERG potassium current ($I_{hERG}$) was depolarized from -80 mV to +20 mV for 2 s, then repolarized to -50 mV, and returned to -80 mV after 1 s. This voltage stimulation was given every 10 s, and the administration process was started after the hERG potassium current was confirmed to be stable (at least 1 minute). The compound was administered for at least 1 minute at each test concentration, and at least 2 cells (n ≥ 2) were tested at each concentration.

**Data processing:** Data analysis processing was carried out by using pClamp 10, GraphPad Prism 5 and Excel software. The inhibition degree of hERG potassium current (peak value of hERG tail current induced at -50 mV) at different compound concentrations was calculated by the following formula:

$$\text{Inhibition\%} = [1 - (I / Io)] \times 100\%$$

where Inhibition% represents the percentage of inhibition of hERG potassium current by the compound, and *I* and *I*o represent the amplitude of hERG potassium current after and before the administration, respectively.

Compound $IC_{50}$ was calculated using GraphPad Prism 5 software by fitting according to the following equation:

$$Y = Bottom + (Top\text{-}Bottom)/(1+10^{\wedge}((LogIC_{50}\text{-}X)*HillSlope))$$

where X represents the Log value of the tested concentration of the test sample, Y represents the inhibition percentage at the corresponding concentration, and Bottom and Top represent the minimum and maximum inhibition percentage, respectively.

Table 3. $IC_{50}$ values of test compounds for inhibitory effect on hERG potassium channel current

| Compound | $IC_{50}$ (μM) |
|---|---|
| Compound 10 | >30 |
| Compound 11 | >30 |
| Chiral isomer 1 of compound 19 | >20 |
| Trifluoroacetate salt of chiral isomer 2 of compound 19 | >40 |
| Compound 12 | >30 |
| Compound 2 | B |
| Chiral isomer 1 of compound 20 | >40 |
| Chiral isomer 2 of compound 20 | >40 |
| Chiral isomer 2 of compound 23 | >40 |
| Chiral isomer 1 of compound 24 | >40 |
| Chiral isomer 1 of compound 25 | >40 |
| Chiral isomer 2 of compound 25 | >40 |
| Control compound 1 | 0.6421 |
| Notes: in Table 3, 1 < B ≤ 30<br>Conclusion: The compounds of the present invention have no obvious inhibitory effect on hERG potassium channel. | |

The control compound 1 has the structure as follows, and is synthesized with reference to patent WO 2020113233 A1.

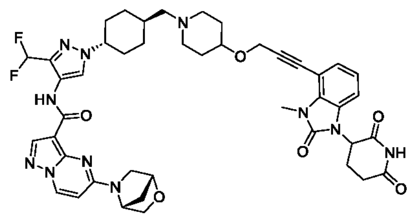

Control compound 1

## Test example 4: Pharmacokinetic test in rats

Experimental animals: Male SD rats, about 200 g, 6-8 weeks old, 6 rats/compound, purchased from Chengdu Ddossy Experimental Animals Co., Ltd.

Test design: On the day of the experiment, 6 SD rats were randomly grouped according to their body weights. The animals were fasted with water available for 12 to 14 h one day before the administration, and were fed 4 h after the administration.

Table 4.1 Administration information

| Group | Number | Administration information | | | | | |
|---|---|---|---|---|---|---|---|
| | Male | Test compound | Administration dosage (mg/kg) | Administration concentration (mg/mL) | Administration volume (mL/kg) | Collected samples | Mode of administration |
| G1 | 3 | The compound of the present invention or control compound | 2.5 | 0.5 | 5 | Plasma | Intravenous administration |
| G2 | 3 | The compound of the present invention or control compound | 10 | 1 | 10 | Plasma | Intragastric administration |
| Vehicle for intravenous administration: 5% DMA + 5% Solutol + 90% Saline;<br>Vehicle for intragastric administration: 5% DMSO + 30% PEG400 + 65% (20% SBE-CD);<br>(DMSO: dimethyl sulfoxide; DMA: dimethylacetamide; Solutol: polyethylene glycol-15-hydroxystearate; PEG400: polyethylene glycol 400; SBE-β-CD: sulfobutyl-β-cyclodextrin; Saline: physiological saline; ) | | | | | | | |

Before and after the administration, 0.15 mL of blood was taken from the orbit of the animals under isoflurane anesthesia and placed in an EDTAK2 centrifuge tube. The blood was centrifuged at 6000 rpm and 4°C for 5 min to collect plasma. The blood collection time points for the intravenous administration group and intragastric administration group were 0 min, 5 min, 15 min, 30 min, 1 h, 2 h, 4 h, 6 h, 8 h and 24 h. Before analysis and detection, all samples were stored at -60°C. The samples were analyzed quantitatively by LC-MS/MS. Conclusion: The compounds of the present invention have good oral absorption in rats.

## Test example 5: Pharmacokinetic test in beagle dogs

**Experimental animals:** Male beagle dogs, about 8-11 kg, 6 beagle dogs/compound, purchased from Beijing Marshall Biotechnology Co. Ltd.

**Experimental method:** On the day of the experiment, 6 beagle dogs were randomly grouped according to their body weights. The animals were fasted with water available for 14 to 18 h one day before the administration, and were fed 4 h after the administration.

Table 5.1 Administration information

| Group | Number | Administration information | | | | | |
|---|---|---|---|---|---|---|---|
| | Male | Test compound | Administration dosage (mg/kg) | Administration concentration (mg/mL) | Administration volume (mL/kg) | Collected samples | Mode of administration |
| G1 | 3 | The compound of the present invention or control compound | 1 | 1 | 1 | Plasma | Intravenous administration |
| G2 | 3 | The compound of the present invention or control compound | 5 | 1 | 5 | Plasma | Intragastric administration |
| Vehicle for intravenous administration: 10% DMA + 5% Solutol + 85% Saline;<br>Vehicle for intragastric administration: 5% DMSO + 30% PEG400 + 65% (20% SBE-CD); | | | | | | | |

Before and after the administration, 1 mL of blood was taken from the jugular veins or limb veins, and placed in an EDTAK2 centrifuge tube. The blood was centrifuged at 5000 rpm and 4°C for 10 min to collect plasma. The blood collection time points for the intravenous administration group and intragastric administration group were 0 min, 5 min, 15 min, 30 min, 1 h, 2 h, 4 h, 6 h, 8 h, 10 h, 12 h and 24 h. Before analysis and detection, all samples were stored at -60°C. The samples were analyzed quantitatively by LC-MS/MS.

Conclusion: The compounds of the present invention have good oral absorption in dogs.

**Test example 6: Pharmacokinetic test in monkeys**

**Experimental animals:** Male cynomolgus monkey, about 3-5 kg, 3-6 years old, 6 monkeys/compound, purchased from Suzhou Xishan Biotechnology Inc.

**Experimental method:** On the day of the experiment, 6 monkeys were randomly grouped according to their body weights. The animals were fasted with water available for 14 to 18 h one day before the administration, and were fed 4 h after the administration.

Table 6.1 Administration information

| Group | Number | Administration information | | | | | |
|---|---|---|---|---|---|---|---|
| | Male | Test compound | Administration dosage (mg/kg) | Administration concentration (mg/mL) | Administration volume (mL/kg) | Collected samples | Mode of administration |
| G1 | 3 | The compound of the present invention or control compound | 1 | 1 | 1 | Plasma | Intravenous administration |

(continued)

| Group | Number | Administration information | | | | | |
|---|---|---|---|---|---|---|---|
| | Male | Test compound | Administration dosage (mg/kg) | Administration concentration (mg/mL) | Administration volume (mL/kg) | Collected samples | Mode of administration |
| G2 | 3 | The compound of the present invention or control compound | 5 | 1 | 5 | Plasma | Intragastric administration |
| Vehicle for intravenous administration: 10% DMA + 5% Solutol + 85% Saline;<br>Vehicle for intragastric administration: 5% DMSO + 30% PEG400 + 65% (20% SBE-CD); | | | | | | | |

Before and after the administration, 1 mL of blood was taken from limb veins, and placed in an EDTAK2 centrifuge tube. The blood was centrifuged at 5000 rpm and 4°C for 10 min to collect plasma. The blood collection time points for the intravenous administration group and intragastric administration group were 0 min, 5 min, 15 min, 30 min, 1 h, 2 h, 4 h, 6 h, 8 h, 10 h, 12 h and 24 h. Before analysis and detection, all samples were stored at -60°C. The samples were analyzed quantitatively by LC-MS/MS.

Conclusion: The compounds of the present invention have good oral absorption in monkeys.

Test example 7: Test of stability in liver microsomes

In this experiment, liver microsomes of five species, including human, monkey, dog, rat and mouse, were used as *in vitro* models to evaluate the metabolic stability of the test compound.

At 37°C, 1 μM test compound was co-incubated with microsomal protein and coenzyme NADPH. At given time points of the reaction (5 min, 10 min, 20 min, 30 min and 60 min), the reaction was terminated by adding ice-cold acetonitrile containing an internal standard. The LC-MS/MS method was used to measure the concentration of the test compound in the sample. $T_{1/2}$ was calculated using the natural logarithm (In) of the residual rate of the drug in the incubation system and the incubation time. In addition, the intrinsic clearance in liver microsomes $CL_{int(mic)}$ and the intrinsic clearance in liver $CL_{int(Liver)}$ were further calculated. Conclusion: The compounds of the present invention have good stability in liver microsomes.

**Test example 8: CYP450 enzyme inhibition test**

The purpose of this study was to evaluate the effect of the test compound on the activity of five isoenzymes (CYP1A2, CYP2C9, CYP2C19, CYP2D6 and CYP3A4) of human liver microsomal cytochrome P450 (CYP) by using an *in vitro* testing system. The specific probe substrates of CYP450 isoenzymes were incubated with human liver microsomes and test compounds of different concentrations, and reduced nicotinamide adenine dinucleotide phosphate (NADPH) was added to initiate the reaction. After the completion of the reaction, the sample was treated and liquid chromatography-tandem mass spectrometry (LC-MS/MS) was used to quantitatively detect metabolites produced by specific substrates, changes in CYP enzyme activity were determined, and $IC_{50}$ value was calculated to evaluate the inhibitory potential of the test compound on each CYP enzyme subtype.

Conclusion: The compounds of the present invention have no obvious inhibitory effect on CYP450 enzyme subtypes.

**Test example 9: Caco2 permeability test**

The experiment used a monolayer of Caco-2 cells incubated in triplicate in a 96-well Transwell plate. A transport buffer solution (HBSS, 10 mM HEPES, pH 7.4±0.05) containing the compound of the present invention (2 μM) or the control compounds of digoxin (10 μM), nadolol (2 μM) and metoprolol (2 μM) was added to the administration end hole on the apical side or the basal side. DMSO-containing transport buffer solution was added to the corresponding receiving end hole. After incubation for 2 hours at 37 ± 1°C, the cell plate was removed and an appropriate amount of samples were taken from the apical side and basal side and transferred to a new 96-well plate. Acetonitrile containing internal standard was then added to precipitate the protein. Samples were analyzed using LC MS/MS and the concentrations of the compounds of the present invention and the control compounds were determined. Concentration data were used to calculate apparent

permeability coefficients for transport from the apical side to the basal side, and from the basal side to the apical side of the cell monolayer, and thus to calculate the efflux ratio. The integrity of the cell monolayer after 2 hours of incubation was assessed by leakage of Lucifer Yellow.

Conclusion: The compounds of the present invention have good $CaCO_2$ permeability.

**Test example 10: Study on Ikaros degradation activity in hPBMC cells (24 hours)**

hPBMC cells are human peripheral blood mononuclear cells. Peripheral venous blood was taken from healthy volunteers and hPBMCs were isolated using Ficoll density gradient centrifugation (Ficoll-PaqueTM PLUS 1.077, GE, Cat. 17-1140-02). Culture conditions: RPMI-1640 + 10% FBS + 1% penicillin-streptomycin solution. The cells were cultured in an incubator at 37°C and 5% $CO_2$. The cells were plated in a 24-well plate at $1 \times 10^6$ cells/well. After plating, compounds at different concentrations were added, and the cells were cultured in an incubator at 37°C and 5% $CO_2$ for 24 hours. After the culture was completed, the cells were collected, and RIPA lysis buffer (beyotime, Cat. P0013B) was added. The cells were lysed on ice for 20 minutes, and then centrifuged at 12000 rpm at 4°C for 10 minutes. The protein sample of the supernatant was collected, and the protein was quantified using a BCA kit (Beyotime, Cat. P0009), and then the protein was diluted to 1 mg/mL. The expression of Ikaros (CST, Cat. # 14859S) and the internal reference β-actin (CST, Cat. # 4970S) were detected using a fully automated western blot quantitative analyzer (Proteinsimple). The expression level of Ikaros relative to the internal reference was calculated using compass software. The remaining Ikaros protein relative to the vehicle control group at different doses was calculated using formula (3), and the Ikaros protein degradation relative to the vehicle control group at different doses was calculated using formula (4) to obtain the maximum degradation rate (Dmax) of Ikaros protein by the drug.

$$\text{Remaining Ikaros\%} = \text{Ikaros}_{\text{compound}} / \text{Ikaros}_{\text{vehicle}} \times 100\% \quad \text{formula (3)}$$

$$\text{Ikaros degradation\%} = 100\% - \text{formula (3)} \quad \text{formula (4)}$$

Conclusion: The compounds of the present invention have no obvious degradation effect on Ikaros protein in hPBMC cells.

**Test example 11: Study on Aiolos degradation activity in hPBMC Cells (24 hours)**

hPBMC cells are human peripheral blood mononuclear cells. Peripheral venous blood was taken from healthy volunteers and hPBMCs were isolated using Ficoll density gradient centrifugation (Ficoll-PaqueTM PLUS 1.077, GE, Cat. 17-1140-02). Culture conditions: RPMI-1640 + 10% FBS + 1% penicillin-streptomycin solution. The cells were cultured in an incubator at 37°C and 5% $CO_2$. The cells were plated in a 24-well plate at $1 \times 10^6$ cells/well. After plating, compounds at different concentrations were added, and the cells were cultured in an incubator at 37°C and 5% $CO_2$ for 24 hours. After the culture was completed, the cells were collected, and RIPA lysis buffer (beyotime, Cat. P0013B) was added. The cells were lysed on ice for 20 minutes, and then centrifuged at 12000 rpm at 4°C for 10 minutes. The protein sample of the supernatant was collected, and the protein was quantified using a BCA kit (Beyotime, Cat. P0009), and then the protein was diluted to 1 mg/mL. The expression of Aiolos (CST, Cat. # 15103) and the internal reference β-actin (CST, Cat. 5175S) were detected using a fully automated western blot quantitative analyzer (Proteinsimple). The expression level of Aiolos relative to the internal reference was calculated using compass software. The remaining Aiolos protein relative to the vehicle control group at different doses was calculated using formula (5), and the Aiolos protein degradation relative to the vehicle control group at different doses was calculated using formula (6) to obtain the maximum degradation rate (Dmax) of Aiolos protein by the drug.

$$\text{Remaining Aiolos\%} = \text{Aiolos}_{\text{compound}} / \text{Aiolos}_{\text{vehicle}} \times 100\% \quad \text{formula (5)}$$

$$\text{Aiolos degradation\%} = 100\% - \text{formula (5)} \quad \text{formula (6)}$$

Conclusion: The compounds of the present invention have no obvious degradation effect on Aiolos protein in hPBMC cells.

**Test example 12: Study on IRAK4 degradation activity in hPBMC cells (4 hours)**

hPBMC cells are human peripheral blood mononuclear cells. Peripheral venous blood was taken from healthy

volunteers and hPBMCs were isolated using Ficoll density gradient centrifugation (Ficoll-PaqueTM PLUS 1.077, GE, Cat. 17-1140-02). Culture conditions: RPMI-1640 + 10% FBS + 1% penicillin-streptomycin solution. The cells were cultured in an incubator at 37°C and 5% $CO_2$. The cells were plated in a 24-well plate at $1 \times 10^6$ cells/well. After plating, compounds at different concentrations were added, and the cells were cultured in an incubator at 37°C and 5% $CO_2$ for 4 hours. After the culture was completed, the cells were collected, and RIPA lysis buffer (beyotime, Cat. P0013B) was added. The cells were lysed on ice for 20 minutes, and then centrifuged at 12000 rpm at 4°C for 10 minutes. The protein sample of the supernatant was collected, and the protein was quantified using a BCA kit (Beyotime, Cat. P0009), and then the protein was diluted to 1 mg/mL. The expression of IRAK4 (CST, Cat. 4363S) and the internal reference cofilin (CST, Cat. 5175S) were detected using a fully automated western blot quantitative analyzer (Proteinsimple). The expression level of IRAK4 relative to the internal reference was calculated using compass software. The remaining IRAK4 protein relative to the vehicle control group at different doses was calculated using formula (7), and the IRAK4 protein degradation relative to the vehicle control group at different doses was calculated using formula (8), where $IRAK4_{compound}$ was the expression level of IRAK4 in the administration groups at different doses, and $IRAK4_{vehicle}$ was the expression level of IRAK4 in the vehicle control group. An IRAK4 degradation-drug concentration curve was fit using Graphpad Prism 8 software, and the $DC_{50}$ was calculated.

$$\text{Remaining IRAK4\%} = IRAK4_{compound} / IRAK4_{vehicle} \times 100\% \quad \text{formula (7)}$$

$$\text{IRAK4 degradation\%} = 100\% - \text{formula (7)} \quad \text{formula (8)}$$

Conclusion: The compounds of the present invention have a given degradation effect on IRAK4 protein in hPBMC cells at 4 h.

## Claims

1. A compound or a stereoisomer, deuterated compound, solvate, prodrug, metabolite, pharmaceutically acceptable salt or eutectic crystal thereof, wherein the compound is a compound represented by general formula (I),

B-L-K (I);

L is selected from -Ak1-Cy1-Ak2-Cy2-Ak3-Cy3-Ak4-Cy4-Ak5-;
Ak1, Ak2, Ak3, Ak4 and Ak5 are each independently selected from $-(CH_2)_q-$, O, $-(CH_2)_qNR^L-$, NR'C=O, $C{=}ONR^L$, C=O, $-R^LC{=}CR^L-$, C≡C or a bond;
$R^L$ is selected from H or $C_{1-6}$ alkyl;
Cy1, Cy2, Cy3 and Cy4 are each independently selected from a bond, 4- to 7-membered mono-heterocyclic ring, 4- to 10-membered fused-heterocyclic ring, 5- to 12-membered spiro-heterocyclic ring, 7- to 10-membered bridged-heterocyclic ring, 3- to 7-membered monocycloalkyl, 4- to 10-membered fused cycloalkyl, 5- to 12-membered spiro cycloalkyl, 7- to 10-membered bridged cycloalkyl, 5- to 10-membered heteroaryl or 6- to 10-membered aryl, wherein the aryl, heteroaryl, cycloalkyl, mono-heterocyclic ring, fused-heterocyclic ring, spiro-heterocyclic ring or bridged-heterocyclic ring is optionally further substituted with 0 to 4 substituents selected from H, F, Cl, Br, I, OH, C(=O)OH, CN, $NH_2$, =O, $C_{1-4}$ alkyl, halogen-substituted $C_{1-4}$ alkyl, hydroxyl-substituted $C_{1-4}$ alkyl or $C_{1-4}$ alkoxy, and the heteroaryl, mono-heterocyclic ring, fused-heterocyclic ring, spiro-heterocyclic ring or bridged-heterocyclic ring contains 1 to 4 heteroatoms selected from O, S, or N;
B is selected from

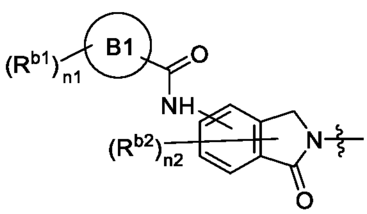 or 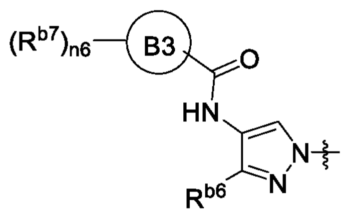 ;

B1 and B3 are each independently selected from $C_{6-10}$ aryl, 5- to 10-membered heteroaryl or 4-to 10-membered heterocyclyl, wherein the heteroaryl or heterocyclyl contains 1 to 4 heteroatoms selected from O, S or N;

$R^{b1}$ and $R^{b7}$ are each independently selected from H, F, Cl, Br, I, =O, OH, $NH_2$, CN, $CF_3$, C(=O)OH, $CHF_2$, $C_{1-4}$ alkyl, $C_{1-4}$ alkoxy, $C_{3-6}$ cycloalkyl, $-(CH_2)_n-R^{b21}$, $-OR^{b21}$, $-N(R^{b21})_2$, $C_{6-10}$ aryl, 5- to 10-membered heteroaryl or 4- to 10-membered heterocyclyl, wherein the alkyl, alkoxy, cycloalkyl, heterocyclyl, aryl or heteroaryl is optionally further substituted with 0 to 4 substituents selected from H, F, Cl, Br, I, OH, =O, $-N(R^{b21})_2$, CN, $CF_3$, C(=O)OH, $C_{1-4}$ alkyl, $C_{1-4}$ alkoxy, $C_{3-6}$ cycloalkyl, 5- to 10-membered heteroaryl, 4- to 10-membered heterocyclyl or $R^{b7a}$, and the heteroaryl or heterocyclyl contains 1 to 4 heteroatoms selected from O, S, or N;

$R^{b7a}$ is selected from $C_{1-4}$ alkyl, $-C_{3-6}$ cycloalkyl, 4- to 10-membered heterocyclyl, $-C_{1-4}$ alkylene $-C_{3-6}$ cycloalkyl, $-C_{1-4}$ alkylene 4- to 10-membered heterocyclyl, $-O-C_{3-6}$ cycloalkyl, -O-4- to 10-membered heterocyclyl, $-NH-C_{3-6}$ cycloalkyl, -NH-4- to 10-membered heterocyclyl, $-N(C_{1-4}$ alkyl)$-C_{3-6}$ cycloalkyl or $-N(C_{1-4}$ alkyl)-4- to 10-membered heterocyclyl, wherein the $R^{b7a}$ is optionally substituted with 1 to 4 substituents selected from H, F, Cl, Br, I, OH, =O, $-N(R^{b21})_2$, CN, $CF_3$, C(=O)OH, $C_{1-4}$ alkyl, $C_{1-4}$ alkoxy, $C_{3-6}$ cycloalkyl or 4- to 10-membered heterocyclyl, and the heterocyclyl contains 1 to 4 heteroatoms selected from O, S, or N;

$R^{b2}$ and $R^{b6}$ are each independently selected from H, F, Cl, Br, I, =O, OH, $-C(=O)N(R^{b21})_2$, $-N(R^{b21})_2$, CN, $CF_3$, C(=O)OH, $CHF_2$, $C_{1-4}$ alkyl, $C_{1-4}$ alkoxy, $C_{3-6}$ cycloalkyl, $-(CH_2)_n-R^{b21}$, $-OR^{b21}$, $C_{6-10}$ aryl, 5- to 10-membered heteroaryl or 4- to 10-membered heterocyclyl, wherein the alkyl, alkoxy, cycloalkyl, aryl, heteroaryl or heterocyclyl is optionally further substituted with 0 to 4 substituents selected from H, F, Cl, Br, I, OH, =O, $NH_2$, CN, $CF_3$, C(=O) OH, $C_{1-4}$ alkyl, $C_{1-4}$ alkoxy, $C_{3-6}$ cycloalkyl, 5- to 10-membered heteroaryl or 4- to 10-membered heterocyclyl, and the heteroaryl or heterocyclyl contains 1 to 4 heteroatoms selected from O, S, or N;

each $R^{b21}$ is independently selected from H, $C_{1-6}$ alkyl, $C_{1-4}$ alkoxy, $C_{3-6}$ cycloalkyl, $C_{6-10}$ aryl, 5- to 10-membered heteroaryl or 4- to 10-membered heterocyclyl, wherein the alkyl, alkoxy, cycloalkyl, aryl, heteroaryl or heterocyclyl is optionally further substituted with 0 to 4 substituents selected from H, F, Cl, Br, I, OH, =O, $NH_2$, CN, $CF_3$, C(=O) OH, $C_{1-4}$ alkyl, $C_{3-6}$ cycloalkyl or $C_{1-4}$ alkoxy, and the heteroaryl or heterocyclyl contains 1 to 4 heteroatoms selected from O, S, or N;

n is selected from 0, 1, 2, 3 or 4;

K is selected from

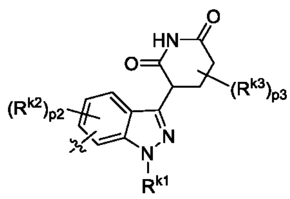,
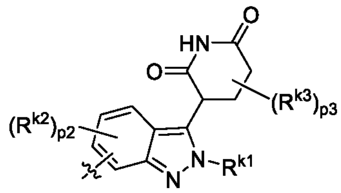,

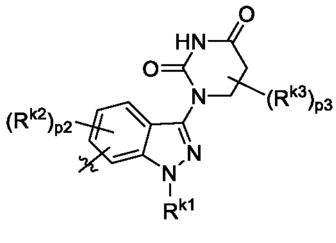
or
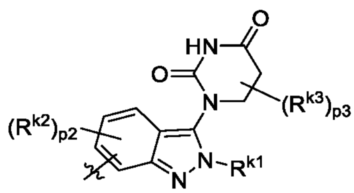;

each $R^{k1}$ is independently selected from H, $C_{1-4}$ alkyl, $C_{2-6}$ alkenyl, $C_{2-6}$ alkynyl, $C_{3-6}$ cycloalkyl or 3-to 6-membered heterocycloalkyl, wherein the alkyl, cycloalkyl or heterocycloalkyl is optionally further substituted with 0 to 4 substituents selected from H, F, Cl, Br, I, OH, $NH_2$, CN, $CF_3$, $C_{1-6}$ alkyl, $C_{1-6}$ alkoxy, $C_{2-6}$ alkenyl, $C_{2-6}$ alkynyl or $C_{3-6}$cycloalkyl;

$R^{k2}$ and $R^{k3}$ are each independently selected from H, F, Cl, Br, I, OH, =O, $NH_2$, $CF_3$, CN, C(=O)OH, $C(=O)NH_2$, $C_{1-4}$ alkyl or $C_{1-4}$ alkoxy, wherein the alkyl or alkoxy is optionally further substituted with 0 to 4 substituents selected from H, F, Cl, Br, I, OH, or $NH_2$;

or two $R^{k3}$ together with the carbon atoms or ring backbones to which they are directly attached form 3- to 6-membered carbocycle or 3- to 7-membered heterocycle, wherein the carbocycle or heterocycle is optionally further substituted with 0 to 4 substituents selected from H, F, Cl, Br, I, OH, =O, $NH_2$, CN, C(=O)OH, $C(=O)NH_2$, $C_{1-4}$ alkyl or $C_{1-4}$ alkoxy, and the heterocycle contains 1 to 4 heteroatoms selected from O, S or N;

q is selected from 0, 1, 2, 3 or 4;

n1, n2 and n6 are each independently selected from 0, 1, 2 or 3;

p2 and p3 are each independently selected from 0, 1, 2, 3 or 4;

optionally, 0 to 50 H of the compound represented by general formula (I) are replaced by 0 to 50 D.

2. The compound or the stereoisomer, deuterated compound, solvate, prodrug, metabolite, pharmaceutically acceptable salt or eutectic crystal thereof according to claim 1, wherein Cy1, Cy2, Cy3 and Cy4 are each independently selected from a bond, 4- to 7-membered nitrogen-containing mono-heterocyclic ring, 4- to 10-membered nitrogen-containing fused-heterocyclic ring, 5- to 12-membered nitrogen-containing spiro-heterocyclic ring, 7- to 10-membered nitrogen-containing bridged-heterocyclic ring, 3- to 7-membered monocycloalkyl, 4-to 10-membered fused cycloalkyl, 5- to 12-membered spiro cycloalkyl, 7- to 10-membered bridged cycloalkyl, 5- to 10-membered heteroaryl or 6- to 10-membered aryl, wherein the mono-heterocyclic ring, fused-heterocyclic ring, bridged-heterocyclic ring, spiro-heterocyclic ring, cycloalkyl, aryl or heteroaryl is optionally further substituted with 0 to 4 substituents selected from H, F, Cl, Br, I, OH, C(=O)OH, CN, $NH_2$, =O, $C_{1-4}$ alkyl, halogen-substituted $C_{1-4}$ alkyl, hydroxyl-substituted $C_{1-4}$ alkyl or $C_{1-4}$ alkoxy, and the mono-heterocyclic ring, fused-heterocyclic ring, bridged-heterocyclic ring, spiro-heterocyclic ring or heteroaryl contains 1 to 4 heteroatoms selected from O, S, or N.

3. The compound or the stereoisomer, deuterated compound, solvate, prodrug, metabolite, pharmaceutically acceptable salt or eutectic crystal thereof according to claim 2, wherein

$R^L$ is selected from H, methyl or ethyl;

Cy1, Cy2, Cy3 and Cy4 are each independently selected from a bond or one of the following substituted or unsubstituted groups: cyclopropyl, cyclobutyl, cyclopentyl, cyclohexyl, azetidinyl, azacyclopentyl, piperidyl, morpholinyl, piperazinyl, phenyl, cyclopropyl-fused-cyclopropyl, cyclopropyl-fused-cyclobutyl, cyclopropyl-fused-cyclopentyl, cyclopropyl-fused-cyclohexyl, cyclobutyl-fused-cyclobutyl, cyclobutyl-fused-cyclopentyl, cyclobutyl-fused-cyclohexyl, cyclopentyl-fused-cyclopentyl, cyclopentyl-fused-cyclohexyl, cyclohexyl-fused-cyclohexyl, cyclopropyl-spiro-cyclopropyl, cyclopropyl-spiro-cyclobutyl, cyclopropyl-spiro-cyclopentyl, cyclopropyl-spiro-cyclohexyl, cyclobutyl-spiro-cyclobutyl, cyclobutyl-spiro-cyclopentyl, cyclobutyl-spiro-cyclohexyl, cyclopentyl-spiro-cyclopentyl, cyclopentyl-spiro-cyclohexyl, cyclohexyl-spiro-cyclohexyl, cyclopropyl-fused-azetidinyl, cyclopropyl-fused-azacyclopentyl, cyclopropyl-fused-azacyclohexyl, cyclobutyl-fused-azetidinyl, cyclobutyl-fused-azacyclopentyl, cyclobutyl-fused-azacyclohexyl, cyclopentyl-fused-azetidinyl, cyclopentyl-fused-azacyclopentyl, cyclopentyl-fused-azacyclohexyl, cyclohexyl-fused-azetidinyl, cyclohexyl-fused-azacyclopentyl, cyclohexyl-fused-azacyclohexyl, azetidinyl-fused-azetidinyl, azetidinyl-fused-azacyclopentyl, azetidinyl-fused-azacyclohexyl, azacyclopentyl-fused-azacyclopentyl, azacyclopentyl-fused-azacyclohexyl, azacyclohexyl-fused-azacyclohexyl, cyclobutyl-spiro-azetidinyl, cyclobutyl-spiro-azacyclopentyl, cyclobutyl-spiro-azacyclohexyl, cyclopentyl-spiro-azetidinyl, cyclopentyl-spiro-azacyclopentyl, cyclopentyl-spiro-azacyclohexyl, cyclohexyl-spiro-azetidinyl, cyclohexyl-spiro-azacyclopentyl, cyclohexyl-spiro-azacyclohexyl, azetidinyl-spiro-azetidinyl, azetidinyl-spiro-azacyclopentyl, azetidinyl-spiro-azacyclohexyl, azacyclopentyl-spiro-azacyclopentyl, azacyclopentyl-spiro-azacyclohexyl, azacyclohexyl-spiro-azacyclohexyl,

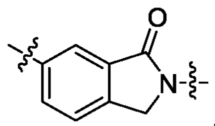 ,

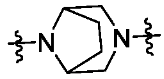 , 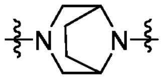 , 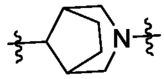 , 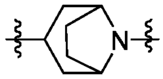 , 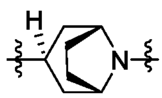 , 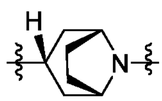 ,

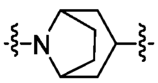 , 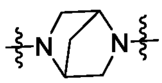 , 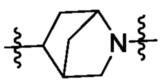 , 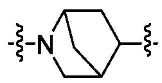 , 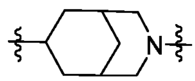 , 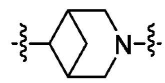 ,

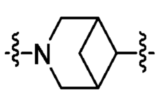 , 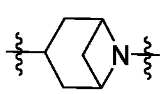 , 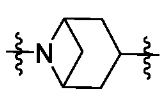 , 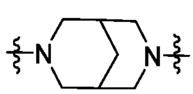 , 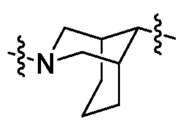 , 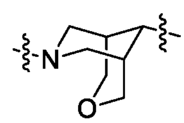

or

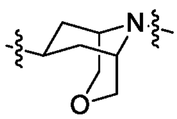, 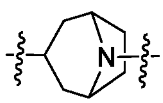,

which, when substituted, is optionally further substituted with 0 to 4 substituents selected from H, F, Cl, Br, I, OH, $NH_2$, C(=O)OH, CN, =O, $C_{1-4}$ alkyl, halogen-substituted $C_{1-4}$ alkyl, hydroxyl-substituted $C_{1-4}$ alkyl or $C_{1-4}$ alkoxy; B1 and B3 are each independently selected from pyrazolyl, oxazolyl, dioxazolyl, oxadiazolyl, triazolyl, imidazolyl, tetrazolyl, pyrrolyl, thienyl, thiazolyl, thiadiazolyl, pyridyl, phenyl, pyrazinyl, pyrimidyl, pyridazinyl, thienopyrazinyl, benzimidazolyl, pyridotriazolyl, pyrimidopyrazolyl, imidazopyridazinyl, pyridopyrazolyl, pyrrolopyridazinyl or

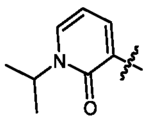;

$R^{b1}$ and $R^{b7}$ are each independently selected from H, F, Cl, Br, I, =O, OH, $NH_2$, CN, $CF_3$, $CHF_2$, $CH_2F$, methyl, ethyl, methoxy, ethoxy, phenyl, pyrrolyl, pyridyl, morpholinyl,

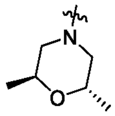, 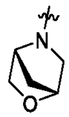, 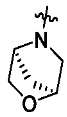,

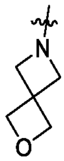, 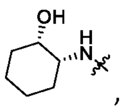, 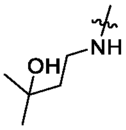, 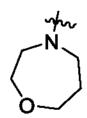, 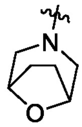, 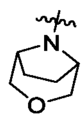, 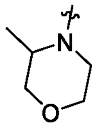, 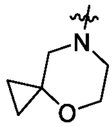, 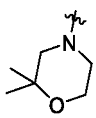, 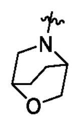, 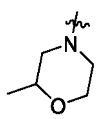,

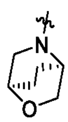, 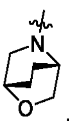, 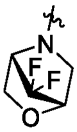, 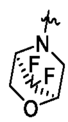 or 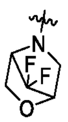,

wherein the methyl, ethyl, methoxy, ethoxy, phenyl, pyrrolyl, pyridyl or morpholinyl is optionally further substituted with 0 to 4 substituents selected from H, F, Cl, Br, I, OH, CN, $CF_3$, $NH_2$, $NHC_{1-4}$ alkyl, $N(C_{1-4}$ alkyl$)_2$, $NHCH_2C_{3-6}$ cycloalkyl, $C_{1-4}$ alkyl, $C_{1-4}$ alkoxy, $C_{3-6}$ cycloalkyl or $R^{b7a}$;

or $R^{b1}$ and $R^{b7}$ are each independently selected from azetidinyl, azacyclopentyl, piperidyl, piperazinyl, morpholinyl or 2-oxa-5-azabicyclo[2.2.1]heptanyl, wherein the $R^{b1}$ and $R^{b7}$ are optionally substituted with 1 to 4 substituents selected from F, Cl, Br, I, OH, =O, CN, $CF_3$, $NH_2$, $NHC_{1-4}$ alkyl, $N(C_{1-4}$ alkyl$)_2$, $NHCH_2C_{3-6}$ cycloalkyl, halogen-substituted $C_{1-4}$ alkyl, cyano-substituted $C_{1-4}$ alkyl, $-C_{1-4}$ alkylene -OH, $C_{1-4}$ alkyl, $C_{1-4}$ alkoxy, $-CH_2$-O-$C_{1-4}$ alkyl, $-CH_2$-$C_{3-6}$ cycloalkyl, -O-$C_{3-6}$ cycloalkyl, -NH-$C_{3-6}$ cycloalkyl, $C_{3-6}$ cycloalkyl, $-CH_2$-4- to 7-membered heterocycloalkyl, -O-4- to 7-membered heterocycloalkyl, -NH-4- to 7-membered heterocycloalkyl, or 4- to 7-membered heterocycloalkyl, and the heterocyclyl contains 1 to 4 heteroatoms selected from O, S, or N; $R^{b2}$ and $R^{b6}$ are each independently selected from H, F, Cl, Br, I, =O, $CF_3$, $CHF_2$, OH, $NH_2$, NH(methyl), NH(ethyl), NH(propyl), NH(isopropyl), N(methyl$)_2$, N(ethyl$)_2$, CN, methyl, ethyl, methoxy, ethoxy, propoxy, isopropyloxy, morpholinyl, piperazinyl, pyrrolidyl, piperidyl or oxazolidinyl, wherein the methyl, ethyl, methoxy, ethoxy, propoxy, isopropyloxy, morpholinyl, piperazinyl, pyrrolidyl, piperidyl or oxazolidinyl is optionally further substituted with 0 to 4 substituents selected from H, F, Cl, Br, I, OH, CN, $CF_3$, $C_{1-4}$ alkyl, $C_{1-4}$ alkoxy or $C_{3-6}$ cycloalkyl; each $R^{k1}$ is independently selected from H, methyl, ethyl, propyl, isopropyl, ethenyl, propenyl, allyl, ethynyl, propynyl, propargyl, cyclopropyl, cyclobutyl, cyclopentyl, cyclohexyl, azetidinyl, azacyclopentyl, piperidyl, oxacyclobutyl, oxacyclopentyl or oxacyclohexyl, wherein the methyl, ethyl, propyl, isopropyl, cyclopropyl, cyclobutyl, cyclopentyl, cyclohexyl, azetidinyl, azacyclopentyl, piperidyl, oxacyclobutyl, oxacyclopentyl or oxacyclohexyl is optionally further substituted with 0 to 4 substituents selected from H, F, Cl, Br, I, OH, CN, $CF_3$, $C_{1-4}$ alkyl, $C_{1-4}$ alkoxy, ethenyl, propenyl, allyl, ethynyl, propynyl, propargyl or $C_{3-6}$ cycloalkyl;

$R^{k2}$ and $R^{k3}$ are each independently selected from H, F, Cl, Br, I, OH, =O, $NH_2$, $CF_3$, CN, C(=O)OH, C(=O)$NH_2$, methyl, ethyl, methoxy or ethoxy, wherein the methyl, ethyl, methoxy or ethoxy is optionally further substituted with 0 to 4 substituents selected from H, F, Cl, Br, I, OH or $NH_2$; each p2 or p3 is independently selected from 0, 1 or 2.

4. The compound or the stereoisomer, deuterated compound, solvate, prodrug, metabolite, pharmaceutically acceptable salt or eutectic crystal thereof according to claim 3, wherein Cy1, Cy2, Cy3 and Cy4 are each independently selected from a bond or one of the following substituted or unsubstituted groups:

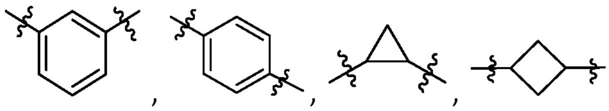
, , , ,

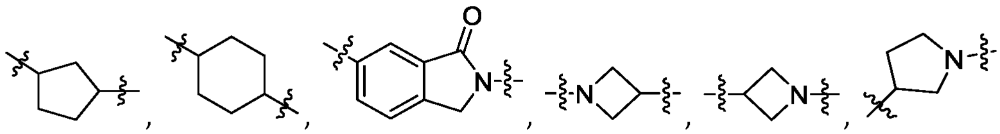
, , , , , ,

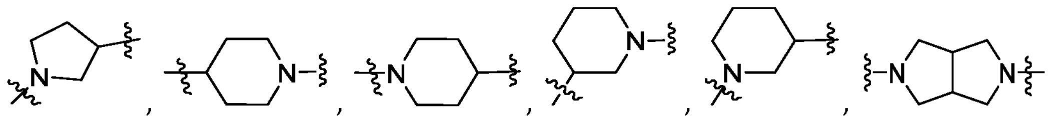
, , , , , ,

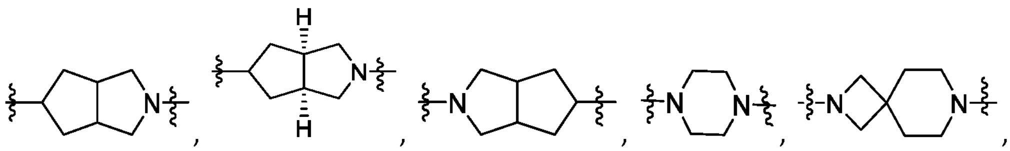
, , , , ,

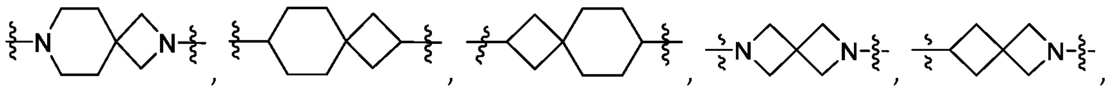
, , , , ,

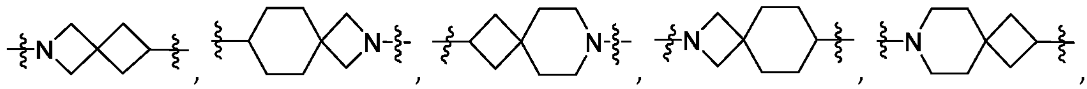
, , , , ,

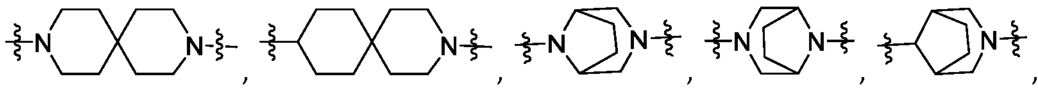
, , , , ,

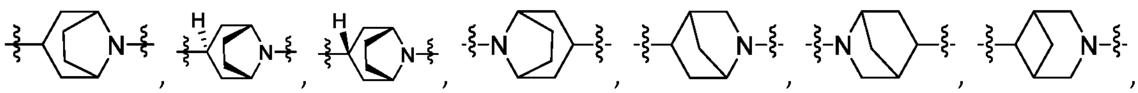
, , , , , , ,

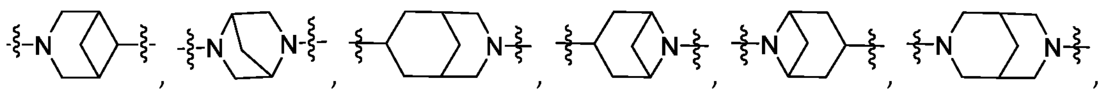
, , , , , ,

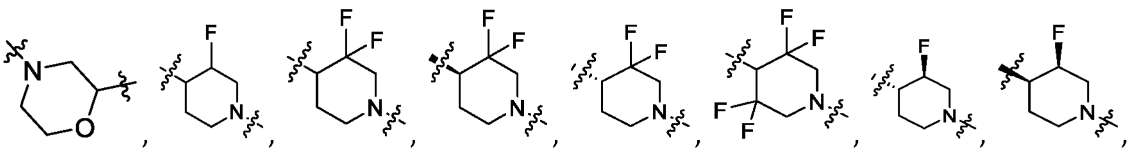
, , , , , , , ,

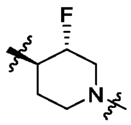, 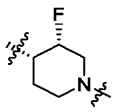, 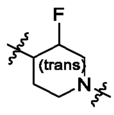 or 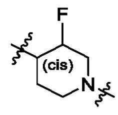,

which, when substituted, is optionally further substituted with 0 to 4 substituents selected from H, F, $CF_3$, methyl, =O, hydroxymethyl, C(=O)OH, CN or $NH_2$;

B is selected from

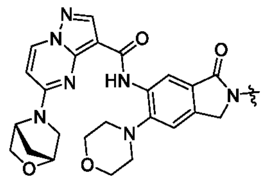, 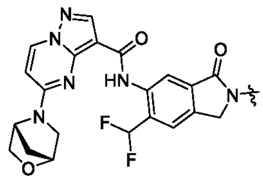, 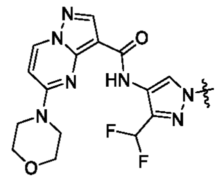,

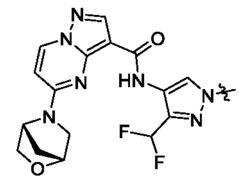, 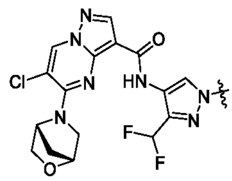, 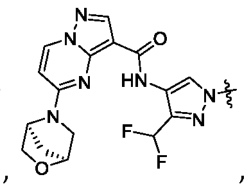, 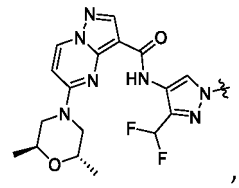,

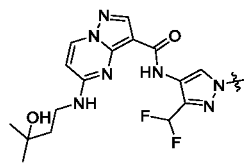, 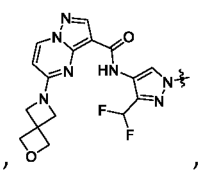, 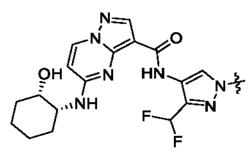, 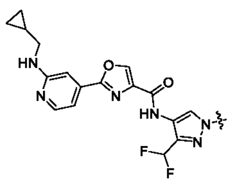,

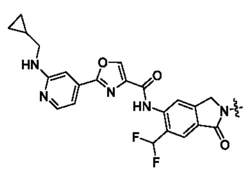, 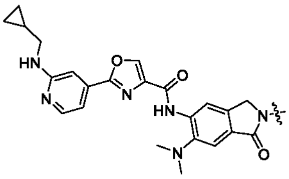, 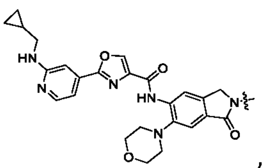, 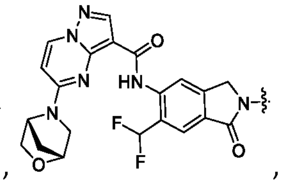,

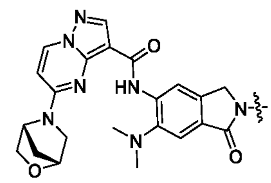, 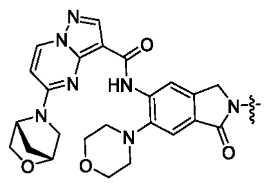, 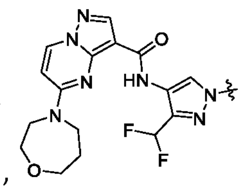, 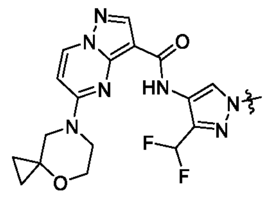,

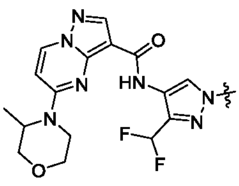, 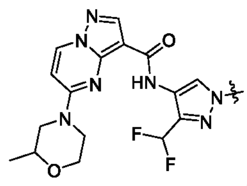, 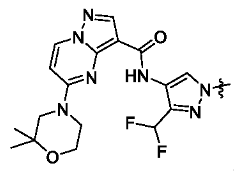, 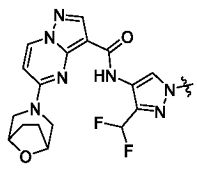, 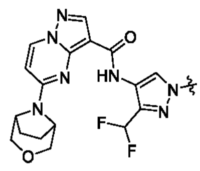,

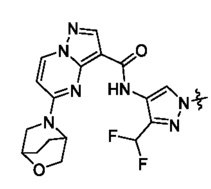, 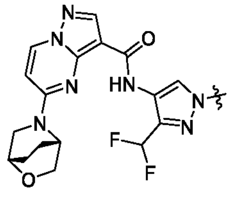, 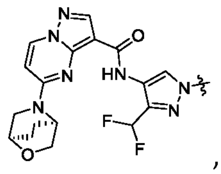, 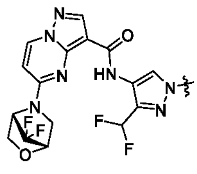, 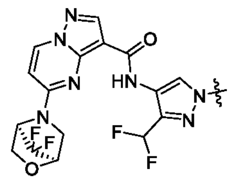,

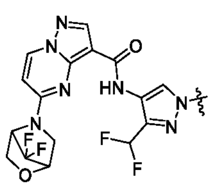, 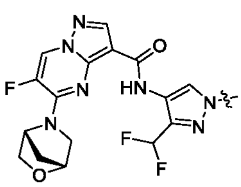, 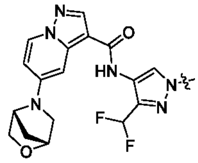, 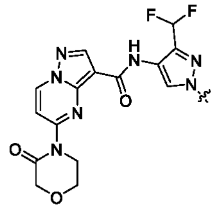, 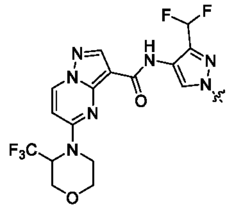,

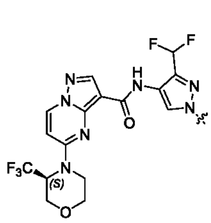, 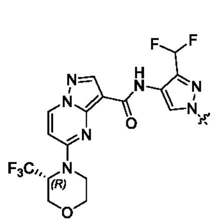, 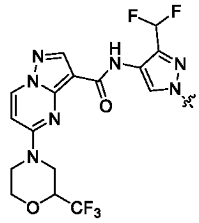, 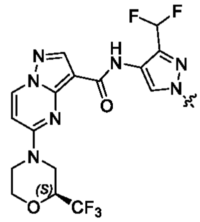, 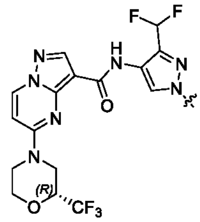,

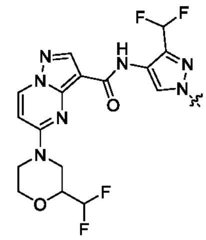, 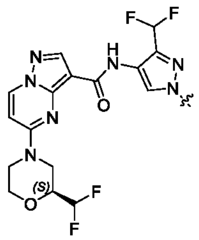, 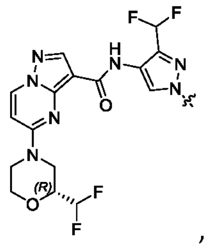, 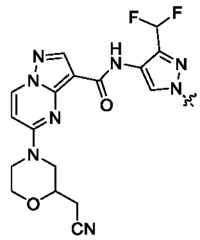, 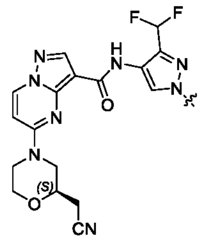,

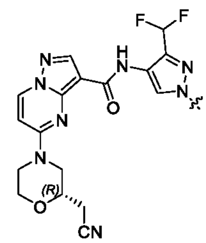, 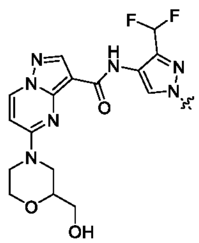, 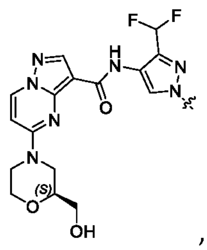, 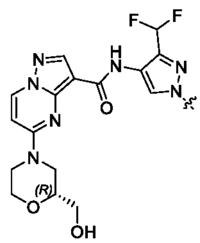, 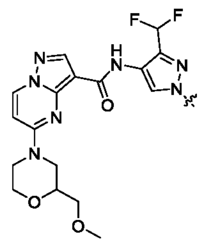,

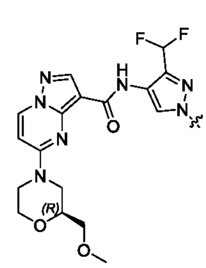, 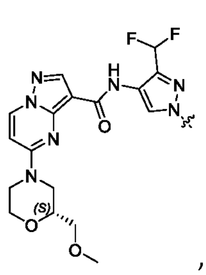, 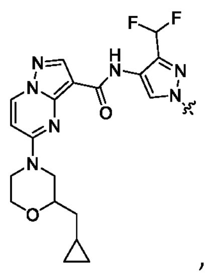, 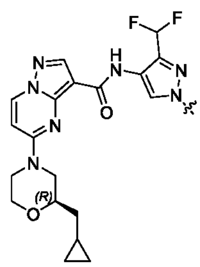, 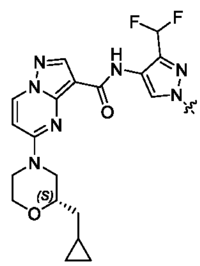,

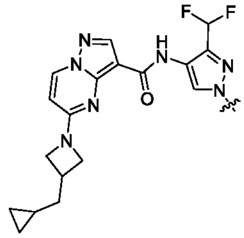, 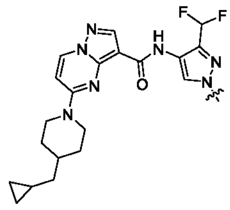, 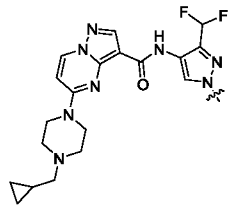, 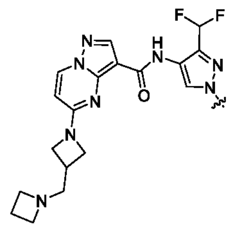,

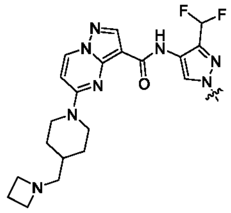, 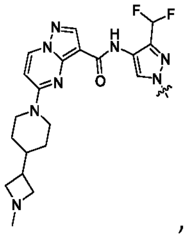, 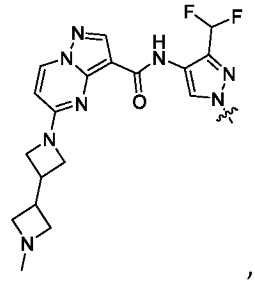, 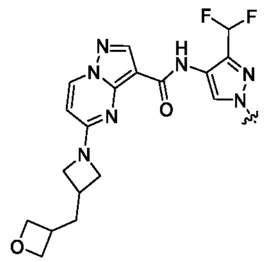,

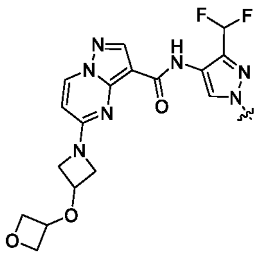, 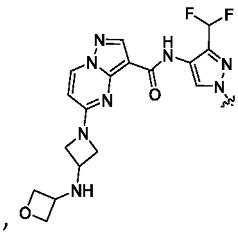, 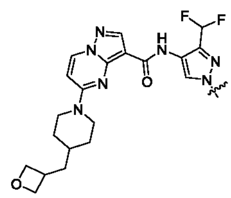, 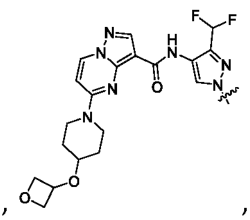,

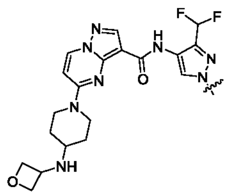, 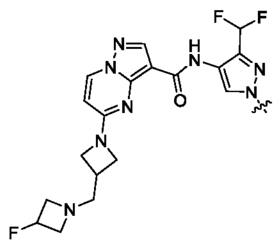 or 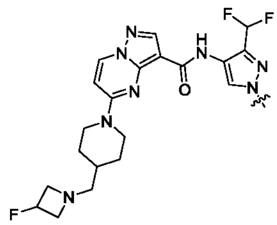

**5.** The compound or the stereoisomer, deuterated compound, solvate, prodrug, metabolite, pharmaceutically acceptable salt or eutectic crystal thereof according to claim 4, wherein L is selected from a bond, -Cy1-, -Cy1-Ak2-, -Cy1-Ak2-Ak3-, -Cy1-Ak2-Ak3-Ak4-Ak5-, -Cy1-Cy2-, - Cy1-Ak2-Cy2-, -Cy1-Cy2-Ak3-, -Cy1-Cy2-Ak3-Cy4-, -Cy1-Ak2-Cy2-Ak3-, -Cy1-Ak2-Cy2-Ak3-Ak4-, - Cy1-Ak2-Cy2-Cy3-Ak4-, -Cy1-Cy2-Ak3-Ak4-, -Cy1-Cy2-Ak3-Ak4-Ak5-, -Cy1-Ak2-Cy2-Ak3-Ak4-Ak5-, -Cy1-Ak2-Ak3-Cy3-Ak4-, -Cy1-Ak2-Ak3-Cy3-Ak4-Ak5-, -Cy1-Ak2-Ak3-Ak4-Cy4-Ak5-, -Cy1-Cy2-Cy3-, -Cy1-Ak2-Cy2-Cy3-, -Cy1-Cy2-Cy3-Ak4-, -Cy1-Ak2-Cy2-Cy3-Ak4-, -Cy1-Ak2-Cy2-Ak3-Cy3-, -Cy1-Ak2-Cy2-Cy3-Ak4-Ak5, -Cy1-Ak2-Cy2-Ak3-Cy3-Ak4-Ak5-, -Cy1-Cy2-Cy3-Cy4-, -Cy1-Cy2-Ak3-Cy3-Cy4-, -Cy1-Cy2-Cy3-Ak4-Cy4-, -Ak1-Cy1-Ak2-Cy2-, -Ak1-Cy1-Ak2-Cy2-Ak3-, -Ak1-Ak2-Cy2-Ak3-, - Ak1-Ak2-Cy2-, -Ak1-Ak2-Cy2-Cy3-Ak4-, -Ak1-Ak2-Ak3-Cy3-Ak4-, -Ak1-Cy1-Ak2-, -Ak1-Cy1-Cy2-Ak3-Ak4-, -Ak1-Cy1-Cy2-Ak3-, -Ak1-Cy1-Ak2-Ak3-Ak4-, -Ak1-Cy1-, -Ak1-Cy1-Ak2-Ak3-, -Ak1-Ak2-Cy2-Ak3-Ak4-, -Ak1-Cy1-Ak2-Cy2-Ak3-Ak4-, -Cy1-Ak2-Ak3-Cy3-Cy4-Ak5-, -Ak1-Cy1-Ak2-Ak3-Ak4-Ak5-, -Cy1-Ak1-Ak2-Ak3-, -Ak1-Cy1-Cy2-, -Ak1-Ak2-Ak3-Ak4- or -Cy1-Ak2-Cy2-Ak3-Cy3-Ak4-; Ak1, Ak2, Ak3, Ak4 and Ak5 are each independently selected from O, C=C, $CH_2$, $CH_2CH_2$, $CH_2CH_2CH_2$, $CH_2CH_2CH_2CH_2$, $CH_2N(CH_3)$, $CH_2CH_2N(CH_3)$, $N(CH_3)$, NH, C(=O), $C(=O)N(CH_3)$, $N(CH_3)C(=O)$, C(=O)NH or NHC(=O).

**6.** The compound or the stereoisomer, deuterated compound, solvate, prodrug, metabolite, pharmaceutically acceptable salt or eutectic crystal thereof according to claim 4, wherein

L is selected from

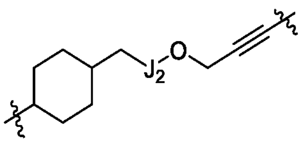, 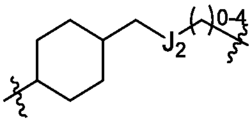, 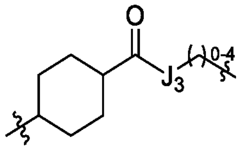,

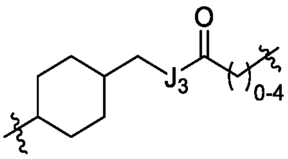, 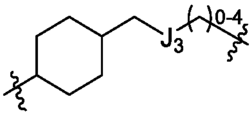, 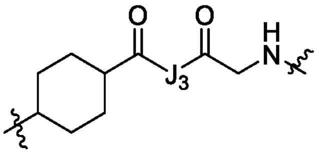,

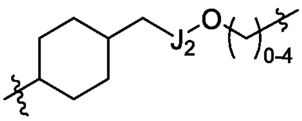, 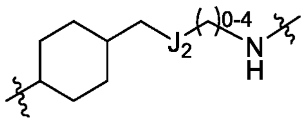, 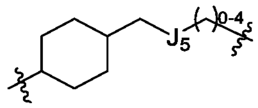,

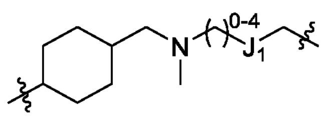, 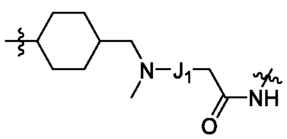, 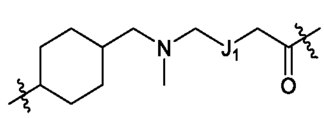,

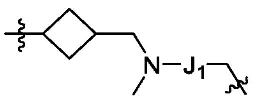, 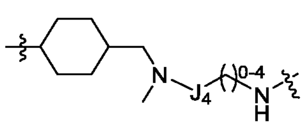, 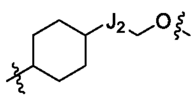, 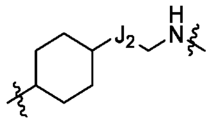,

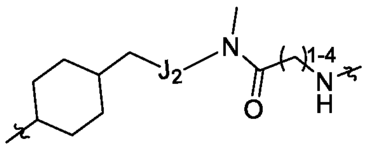, 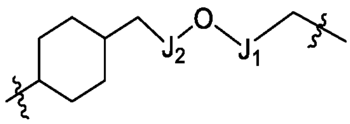, 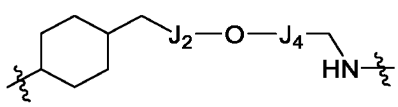,

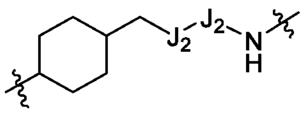, 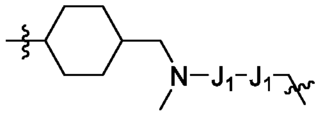, 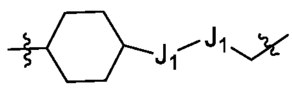,

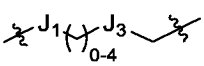, 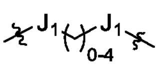, 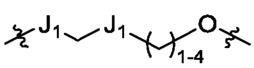, 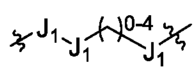,

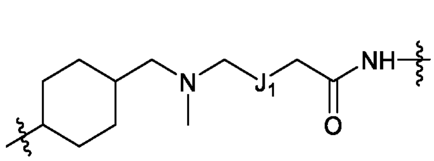, 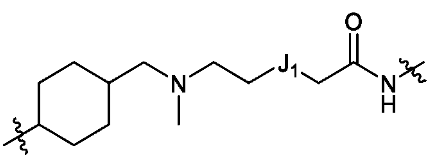,

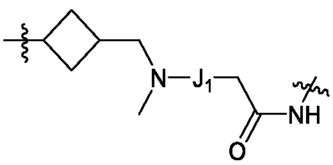, 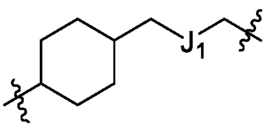, 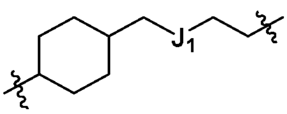,

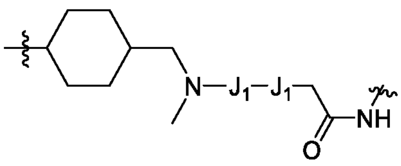, 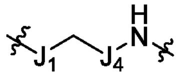, 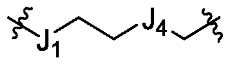, 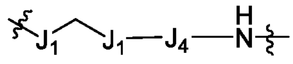,

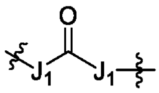, 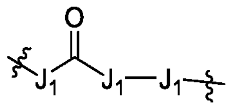, 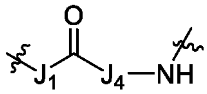, 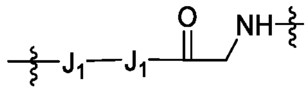,

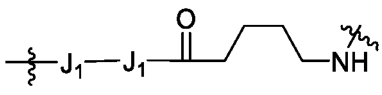, 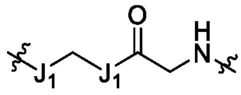, 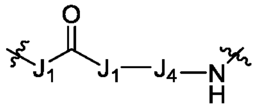,

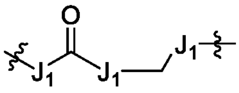, 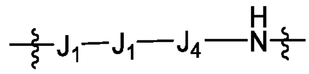, 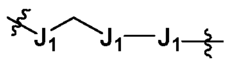, 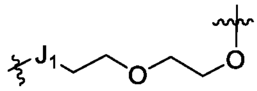,

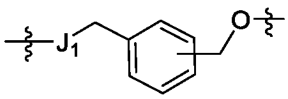, 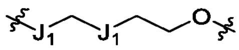, 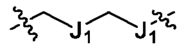, 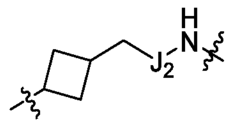,

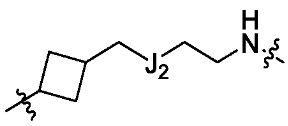, 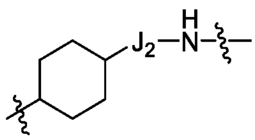, 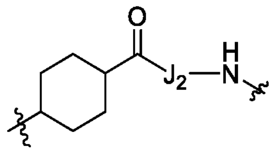,

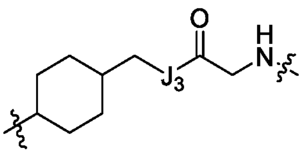, 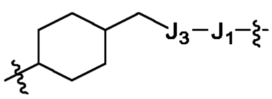, 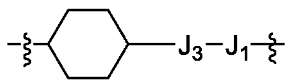, 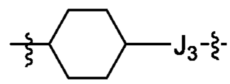,

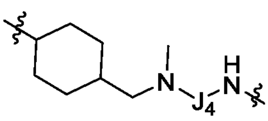, 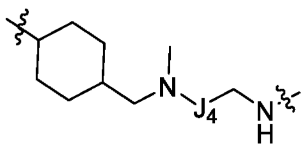, 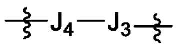, 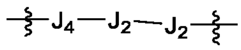,

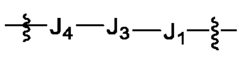 or 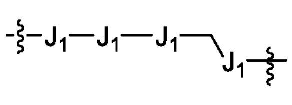;

each $J_1$ is independently selected from

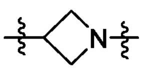, 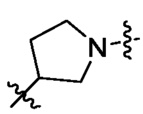 or 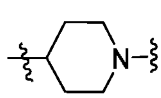;

each $J_2$ is independently selected from

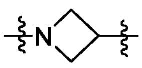, 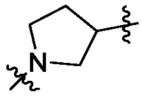, 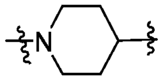, 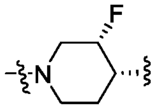,

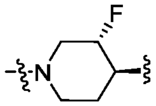, 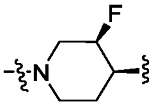, 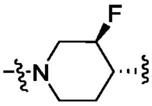, 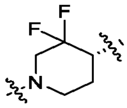, 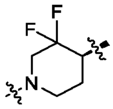, 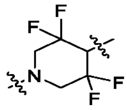, 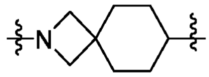

or

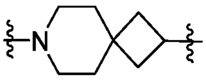;

each $J_3$ is independently selected from

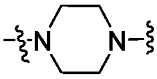, 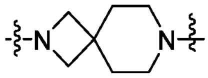, 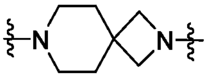,

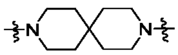, 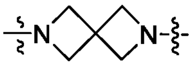 or 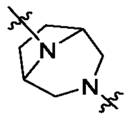;

each $J_4$ is independently selected from

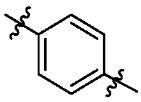, 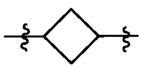, 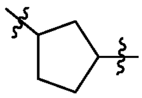, 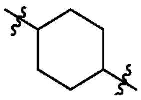,

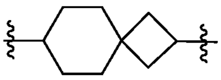 or 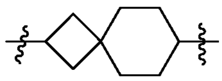;

each $J_5$ is independently selected from

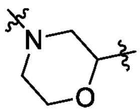;

or, L is selected from

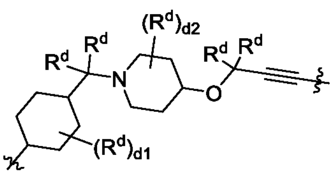;

$R^d$ is selected from H or D, and at least one of $R^d$ is selected from D;
d1 is selected from 0, 1, 2, 3, 4, 5, 6, 7, 8, 9 or 10;
d2 is selected from 0, 1, 2, 3, 4, 5, 6, 7, 8 or 9.

**7.** The compound or the stereoisomer, deuterated compound, solvate, prodrug, metabolite, pharmaceutically acceptable salt or eutectic crystal thereof according to claim 4, wherein L is selected from a group shown in Table L-1, wherein the left side of the group is linked to B.

**8.** The compound or the stereoisomer, deuterated compound, solvate, prodrug, metabolite, pharmaceutically acceptable salt or eutectic crystal thereof according to claims 5-7, wherein
K is selected from

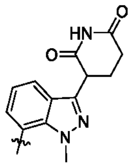 , 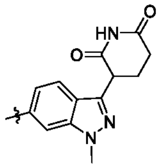 , 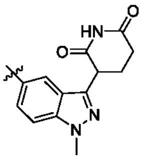 , 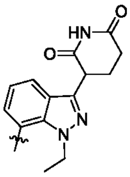 , 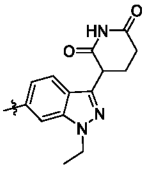 , 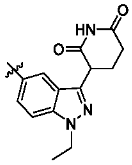 ,

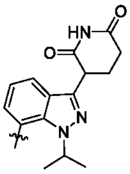 , 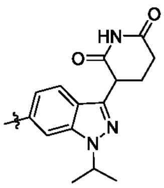 , 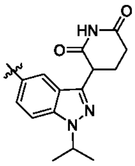 , 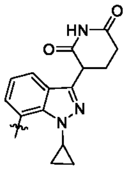 , 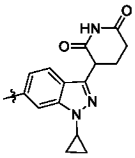 , 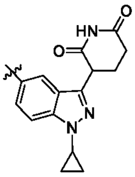 , 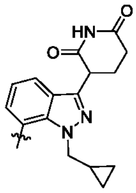 ,

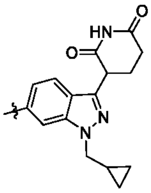 , 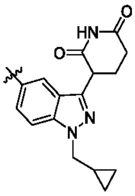 , 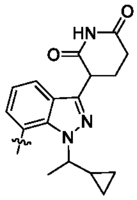 , 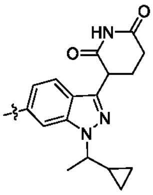 , 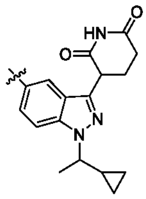 , 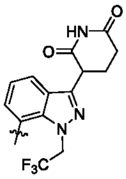 , 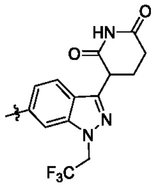 ,

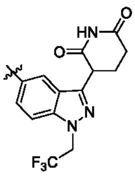 , 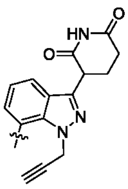 , 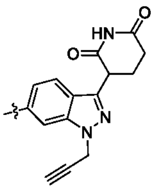 , 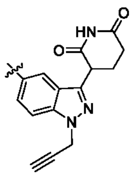 , 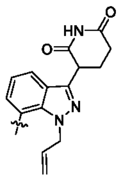 , 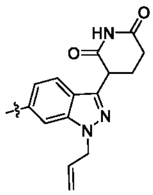 , 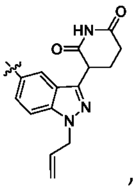 ,

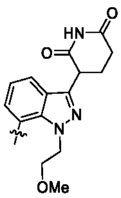 , 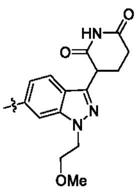 , 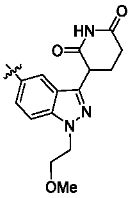 , 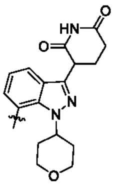 , 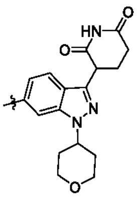 , 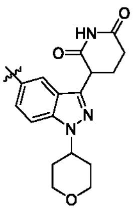 , 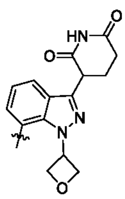 , 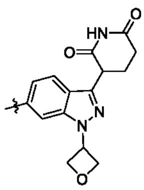 ,

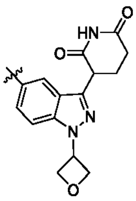 , 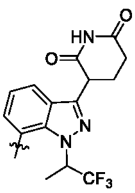 , 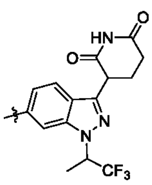 , 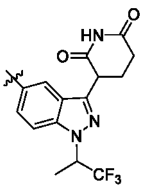 , 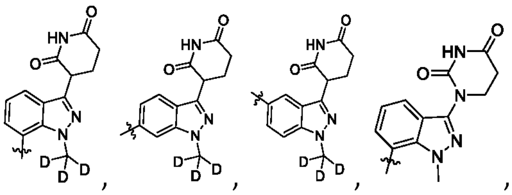 , , , ,

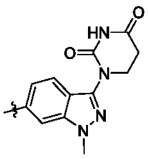 , 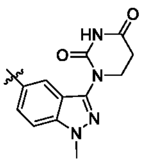 , 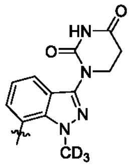 , 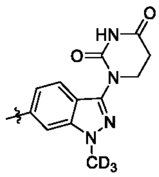 , 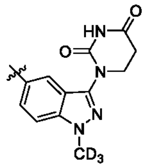 , 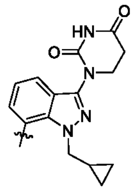 , 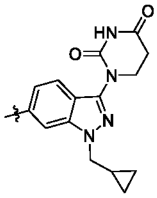 ,

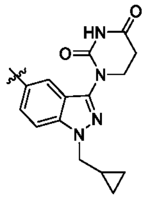 , 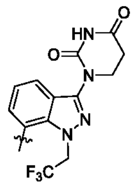 , 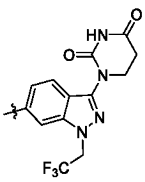 , 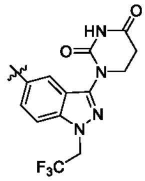 , 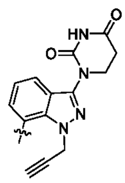 , 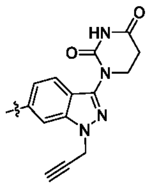 , 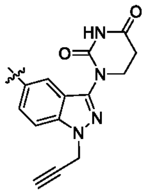 ,

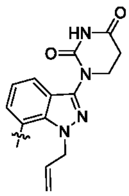 , 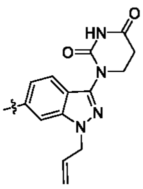 , 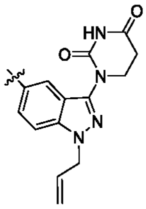 , 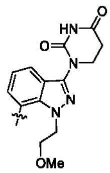 , 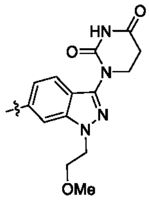 , 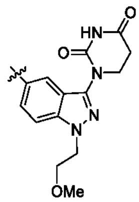 , 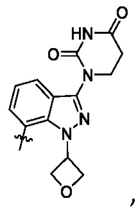 ,

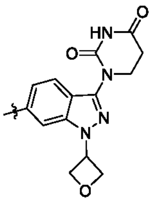 , 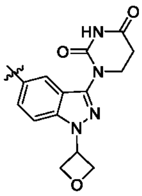 , 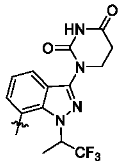 , 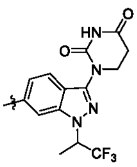 , 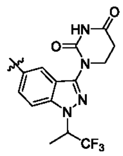 , 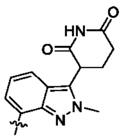 , 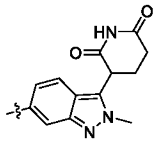 ,

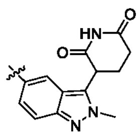 , 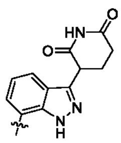 , 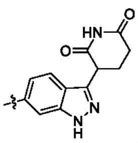 , 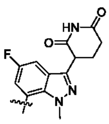 or 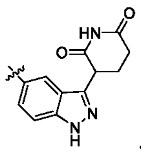 .

**9.** The compound or the stereoisomer, deuterated compound, solvate, prodrug, metabolite, pharmaceutically acceptable salt or eutectic crystal thereof according to claim 1, wherein the compound is selected from one of the structures shown in Table P-1.

**10.** A pharmaceutical composition, comprising the compound or the stereoisomer, deuterated compound, solvate, prodrug, metabolite, pharmaceutically acceptable salt or eutectic crystal thereof according to any one of claims 1-9, and a pharmaceutically acceptable carrier, wherein preferably, the pharmaceutical composition comprises 1-1500 mg of the compound or the stereoisomer, deuterated compound, solvate, prodrug, metabolite, pharmaceutically acceptable salt, or eutectic crystal thereof according to any one of claims 1-9.

**11.** Use of the compound or the stereoisomer, deuterated compound, solvate, prodrug, metabolite, pharmaceutically acceptable salt or eutectic crystal thereof according to any one of claims 1 to 9, or the pharmaceutical composition according to claim 10 in the preparation of a medicament for the treatment of a disease related to the activity or expression quantity of IRAK4.

**12.** Use of the compound or the stereoisomer, deuterated compound, solvate, prodrug, metabolite, pharmaceutically acceptable salt or eutectic crystal thereof according to any one of claims 1 to 9, or the pharmaceutical composition according to claim 10 in the preparation of a medicament for the treatment of a disease related to the inhibition or degradation of IRAK4.

**13.** The use according to claim 12, wherein the disease is selected from an autoimmune disease, an inflammatory disease or cancer.

**14.** A method for treating a disease in a mammal, the method comprising administering to a subject a therapeutically effective amount of the compound or the stereoisomer, deuterated compound, solvate, prodrug, metabolite, pharmaceutically acceptable salt, or eutectic crystal thereof according to any one of claims 1-9, or the pharmaceutical composition according to claim 10, wherein the therapeutically effective amount is preferably 1-1500 mg, and the disease is preferably an autoimmune disease, an inflammatory disease or cancer.

## INTERNATIONAL SEARCH REPORT

International application No.
**PCT/CN2023/070367**

**A. CLASSIFICATION OF SUBJECT MATTER**

C07D519/00(2006.01)i;C07D487/04(2006.01)i;A61K31/519(2006.01)i;A61K47/55(2017.01)i;A61P29/00(2006.01)i;A61P35/00(2006.01)i;A61P37/00(2006.01)i

According to International Patent Classification (IPC) or to both national classification and IPC

**B. FIELDS SEARCHED**

Minimum documentation searched (classification system followed by classification symbols)

IPC: C07D 519/-; C07D487/-; A61L31/-; A61K47/-; A61P29/-; A61P35/-; A61P37/-

Documentation searched other than minimum documentation to the extent that such documents are included in the fields searched

Electronic data base consulted during the international search (name of data base and, where practicable, search terms used)

VEN, DWPI, CNTXT, CNKI, CAplus, REG: IRAK4, E3, 泛素, ubiqutin, 根据权利要求通式, search for structural formula in STN based on general formulas of claims

**C. DOCUMENTS CONSIDERED TO BE RELEVANT**

| Category* | Citation of document, with indication, where appropriate, of the relevant passages | Relevant to claim No. |
|---|---|---|
| PX | WO 2022161414 A1 (INCRELAND) 04 August 2022 (2022-08-04)<br>claims 1-8, 19-21 | 1-13 |
| E | CN 115772180 A (MEDSHINE DISCOVERY INC.) 10 March 2023 (2023-03-10)<br>claims 1-19, description, paragraphs [0162], [0167], [0173] | 1-5, 7, 9-13 |
| PY | CN 114437035 A (HEIXIDAE MEDICINE GROUP STOCK LIMITED COMPANY) 06 May 2022 (2022-05-06)<br>claims 1-10 | 1-13 |
| PY | WO 2022147465 A1 (KYMERA THERAPEUTICS INC.) 07 July 2022 (2022-07-07)<br>claims, description embodiments | 1-13 |
| PY | WO 2022266258 A1 (ARVINAS OPERATIONS, INC.) 22 December 2022 (2022-12-22)<br>claims, description embodiments | 1-13 |
| Y | WO 2020264499 A1 (KYMERA THERAPEUTICS INC.) 30 December 2020 (2020-12-30)<br>description, page 39, formula I-F-3, page 52, line 4, structure fragment, page 67, last line, structure fragment, page 68, lines 1-2, structure fragment, pages 243-289, table B, table 1, compounds I-331, I-472-I-475 etc. and claims 2 and 17-26 | 1-13 |

☑ Further documents are listed in the continuation of Box C. ☑ See patent family annex.

* Special categories of cited documents:
"A" document defining the general state of the art which is not considered to be of particular relevance
"D" document cited by the applicant in the international application
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **12 April 2023** | **19 April 2023** |
| Name and mailing address of the ISA/CN<br>**China National Intellectual Property Administration (ISA/CN)**<br>**China No. 6, Xitucheng Road, Jimenqiao, Haidian District, Beijing 100088**<br>Facsimile No. **(86-10)62019451** | Authorized officer<br><br>Telephone No. |

Form PCT/ISA/210 (second sheet) (July 2022)

**INTERNATIONAL SEARCH REPORT**

International application No. **PCT/CN2023/070367**

**C. DOCUMENTS CONSIDERED TO BE RELEVANT**

| Category* | Citation of document, with indication, where appropriate, of the relevant passages | Relevant to claim No. |
|---|---|---|
| Y | WO 2021158634 A1 (KYMERA THERAPEUTICS, INC.) 12 August 2021 (2021-08-12) claim 2, formulas I-a-16, I-a-19, I-a-22, I-a-25 etc., claims 6-17, description, paragraph [00224], table 1, compound | 1-13 |
| Y | WO 2021127586 A1 (CALICO LIFE SCIENCES LLC et al.) 24 June 2021 (2021-06-24) description pages 17-19, page 56 table 1 compounds 8, 9, 28 etc. | 1-13 |
| Y | WO 2021127561 A1 (C4 THERAPEUTICS INC.) 24 June 2021 (2021-06-24) claim 2, description pages 186-192 | 1-13 |
| Y | WO 2021170109 A1 (CULLGEN SHANGHAI INC.) 02 September 2021 (2021-09-02) description formula I, page 42 formulas 5j, 5k et al. | 1-13 |
| Y | CN 113646002 A (CULLGEN SHANGHAI INC.) 12 November 2021 (2021-11-12) claims 1, 42, 46, 47, formulas 5j, 5k, CRBN-4 | 1-13 |
| Y | WO 2020113233 A1 (KYMERA THERAPEUTICS INC.) 04 June 2020 (2020-06-04) description, paragraph [00661], table 1, and claims | 1-13 |
| Y | WO 2021247899 A1 (KYMERA THERAPEUTICS, INC.) 09 December 2021 (2021-12-09) claims | 1-13 |
| Y | WO 2021247897 A1 (KYMERA THERAPEUTICS, INC.) 09 December 2021 (2021-12-09) claims | 1-13 |
| Y | WO 2021127278 A1 (KYMERA THERAPEUTICS, INC.) 24 June 2021 (2021-06-24) claims | 1-13 |
| Y | CN 112105385 A (KYMERA THERAPEUTICS, INC.) 18 December 2020 (2020-12-18) claims | 1-13 |

Form PCT/ISA/210 (second sheet) (July 2022)

**INTERNATIONAL SEARCH REPORT**

International application No.
**PCT/CN2023/070367**

**Box No. II Observations where certain claims were found unsearchable (Continuation of item 2 of first sheet)**

This international search report has not been established in respect of certain claims under Article 17(2)(a) for the following reasons:

1. ☑ Claims Nos.: **14**
because they relate to subject matter not required to be searched by this Authority, namely:

Claim 14 relates to a method for treatment of the human or animal body by therapy. (see PCT Rule 39.1(iv))

2. ☐ Claims Nos.:
because they relate to parts of the international application that do not comply with the prescribed requirements to such an extent that no meaningful international search can be carried out, specifically:

3. ☐ Claims Nos.:
because they are dependent claims and are not drafted in accordance with the second and third sentences of Rule 6.4(a).

Form PCT/ISA/210 (continuation of first sheet) (July 2022)

**INTERNATIONAL SEARCH REPORT**
**Information on patent family members**

International application No.
**PCT/CN2023/070367**

| Patent document cited in search report | Publication date (day/month/year) | Patent family member(s) | Publication date (day/month/year) |
|---|---|---|---|
| WO 2022161414 A1 | 04 August 2022 | None | |
| CN 115772180 A | 10 March 2023 | WO 2023036175 A1 | 16 March 2023 |
| CN 114437035 A | 06 May 2022 | None | |
| WO 2022147465 A1 | 07 July 2022 | None | |
| WO 2022266258 A1 | 22 December 2022 | None | |
| WO 2020264499 A1 | 30 December 2020 | None | |
| WO 2021158634 A1 | 12 August 2021 | EP 4100004 A1 | 14 December 2022 |
| WO 2021127586 A1 | 24 June 2021 | JP 2023508914 A | 06 March 2023 |
| | | CA 3162364 A1 | 24 June 2021 |
| | | AU 2020407244 A1 | 28 July 2022 |
| | | UY 38999 A | 30 July 2021 |
| | | EP 4077319 A1 | 26 October 2022 |
| | | TW 202136250 A | 01 October 2021 |
| WO 2021127561 A1 | 24 June 2021 | None | |
| WO 2021170109 A1 | 02 September 2021 | None | |
| CN 113646002 A | 12 November 2021 | EP 3930759 A1 | 05 January 2022 |
| | | EP 3930759 A4 | 22 March 2023 |
| | | WO 2020173440 A1 | 03 September 2020 |
| | | US 2023073777 A1 | 09 March 2023 |
| WO 2020113233 A1 | 04 June 2020 | None | |
| WO 2021247899 A1 | 09 December 2021 | None | |
| WO 2021247897 A1 | 09 December 2021 | None | |
| WO 2021127278 A1 | 24 June 2021 | EP 4076536 A1 | 26 October 2022 |
| CN 112105385 A | 18 December 2020 | AU 2018396142 A1 | 16 July 2020 |
| | | SG 11202005912 PA | 29 July 2020 |
| | | US 2019192668 A1 | 27 June 2019 |
| | | US 10874743 B2 | 29 December 2020 |
| | | MX 2020006812 A | 06 November 2020 |
| | | BR 112020012997 A2 | 01 December 2020 |
| | | CA 3086763 A1 | 04 July 2019 |
| | | JP 2021508703 A | 11 March 2021 |
| | | EP 3731869 A1 | 04 November 2020 |
| | | EP 3731869 A4 | 22 September 2021 |
| | | IL 275649 A | 31 August 2020 |
| | | WO 2019133531 A1 | 04 July 2019 |
| | | US 11318205 B1 | 03 May 2022 |

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 2021158634 A
- CN 113512025
- WO 2020113233 A
- WO 2020264499 A
- WO 2021247899 A
- WO 2021247897 A
- WO 2020113233 A1

### Non-patent literature cited in the description

- *CHEMICAL ABSTRACTS*, 87413-09-0
- *CHEMICAL ABSTRACTS*, 98327-87-8
- *CHEMICAL ABSTRACTS*, 51364-51-3
- *CHEMICAL ABSTRACTS*, 95464-05-4